(19) Europäisches Patentamt
European Patent Office
Office européen des brevets

(11) EP 0 339 978 B1

(12) **EUROPEAN PATENT SPECIFICATION**

(45) Date of publication and mention of the grant of the patent: **21.08.1996 Bulletin 1996/34**

(21) Application number: **89304168.1**

(22) Date of filing: **26.04.1989**

(51) Int. Cl.$^6$: **C07D 401/04**, A61K 31/415, C07D 401/14, C07D 403/04, C07D 409/04, C07D 409/14, C07D 413/04, C07D 413/14, C07D 417/04, C07D 417/14, C07D 498/04

(54) **Fused polycyclic compounds, compositions, methods of manufacture, and their use as PAF antagonists, antihistamines and/or antiinflammatory agents**

Kondensierte polyzyklische Verbindungen, Zusammenstellungen, Verfahren zur Herstellung und deren Anwendung als PAF-antagonistische, antihistaminische und/oder anti-inflammatorische Agenzien

Composés polycycliques condensés, compositions, procédés de préparation et leur utilisation comme agents PAF antagonistiques, antihistaminiques et/ou anti-inflammatoires

(84) Designated Contracting States:
**AT BE CH DE ES FR GB GR IT LI LU NL SE**

(30) Priority: **28.04.1988 US 187105**

(43) Date of publication of application:
**02.11.1989 Bulletin 1989/44**

(73) Proprietor: **SCHERING CORPORATION**
**Kenilworth New Jersey 07033 (US)**

(72) Inventors:
- **Friary, Richard J.**
  **West Orange New Jersey 07052 (US)**
- **Green, Michael J.**
  **Skillman New Jersey 08558 (US)**
- **Piwinski, John J.**
  **Parsippany New Jersey 07054 (US)**

(74) Representative: **Ritter, Stephen David et al**
**Mathys & Squire**
**100 Grays Inn Road**
**London WC1X 8AL (GB)**

(56) References cited:

| | |
|---|---|
| EP-A- 0 015 156 | EP-A- 0 075 140 |
| EP-A- 0 260 642 | EP-A- 0 270 818 |
| EP-A- 0 286 346 | EP-A- 0 293 777 |
| FR-A- 1 371 443 | FR-E- 91 084 |
| US-A- 3 442 903 | US-A- 3 458 522 |
| US-A- 3 485 846 | US-A- 3 491 103 |
| US-A- 3 994 915 | US-A- 4 174 397 |

Remarks:
- Consolidated with 89905353.2/0412988 (European application No./publication No.) by decision dated 09.09.91.
- The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 339 978 B1

## Description

BACKGROUND OF THE INVENTION

The present invention relates to compounds useful as PAF antagonists, antihistamines and/or antiinflammatory agents, and to methods of treating mammals in need of such compounds.

Galantay et al. in the Journal of Medicinal Chemistry, 1974, Vol. 17, No. 12, pp 1316-1327, disclose various tricyclic heterocyclic compounds, e.g., the compounds of formulas 4, 6 and 7 and the compounds in Table I of formula I on page 1319 and in Table IV on pages 1320 and 1321. The compounds of Table IV are disclosed as having CNS depressant activities, see page 1324. Some of the compounds of formula I in Table I are disclosed as having antiinflammatory activity.

Ketotifen is a known antihistamine having the formula

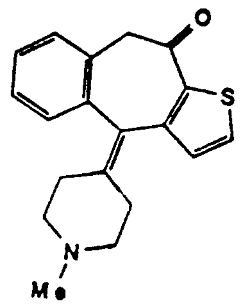

No PAF-antagonist activity for ketotifen has been disclosed as far as we are aware.

Numerous other patents disclose antihistaminic activity in certain benzo[5,6]cyclohepta[1,2-b]pyridine derivatives. See, for example, U.S. patent Nos.3,326,924, 3,409,621, 3,419,565, 3,357,986, 4,282,233 and 4,335,036.

U.S. Patent No. 3458522 discloses compounds of the formulas:

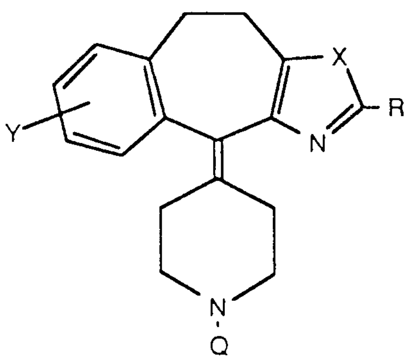
,
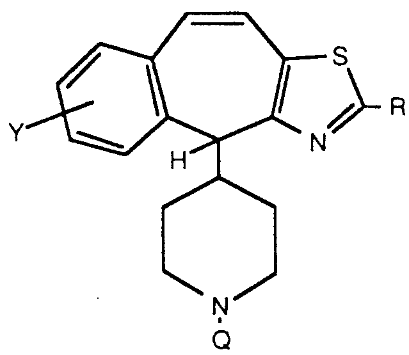
and

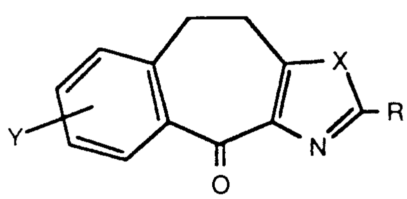

where Y is H or halo, R is lower alkyl, X is O or S, and Q is H, lower alkyl, CN, 2-hydroxyethyl or acetoxyethyl. The first two types of compounds are said to be useful as antihistamines, anti-Parkinsonian agents, tranquilizers and antidepressants.

U.S. Patent No. 3485846 discloses compounds of the formulas

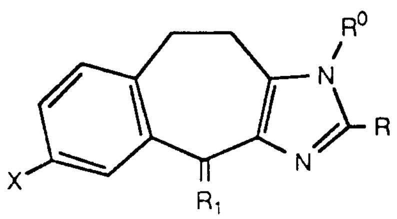

and

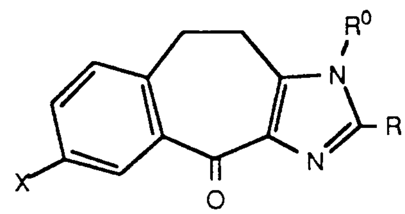

where N is lower alkyl, $R^0$ is H or lower alkyl, $R_1$ is $=CHCH_2CH_2NR'R''$ or 1-loweralkyl-4-piperidylidene, R' and R" are lower alkyl, and X is H or halo.

The former compounds are said to be antihistamines, anti-cholinergic agents and tranquilizers.

Our earlier-filed European patent application no. 87115890.3, published on 15 June 1988 as EP-A1-0270818, discloses anti-allergic and anti-inflammatory compounds of formula:

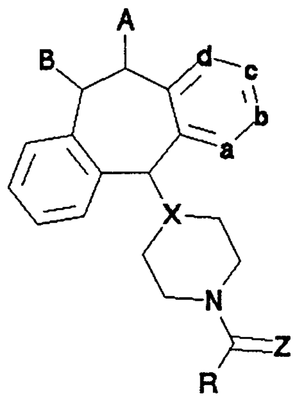

wherein one of a,b,c and d can be N and R can represent H, alkyl, cycloalkyl, aryl, heteroaryl in which the heteroatom is O, S or N, "$N(R^{10})_2$", thioalkyl, alkoxy or, in combination with one of "$R^{5-8}$", a fused ring. These compounds disclosed in 87115890.3 are excluded from the present invention by specific wording in Claim 1.

US Patent No. 3,442,903 discloses compounds of the formulas:

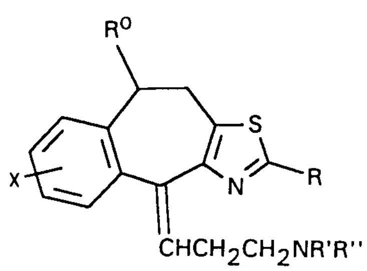

and

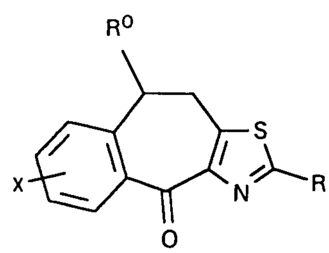

where R is lower alkyl, $R^0$ is H or phenyl, R' is H or lower alkyl , R" is lower alkyl, and X is H or halo. The former compounds are disclosed as tranquilizers.

It has now surprisingly been found that compounds of formula 1.0 below may have utility for one or more of the following applications: inhibiting platelet aggregating factor (PAF) as antihistamine agents and/or anti-inflammatory agents. These compounds have the structural formula

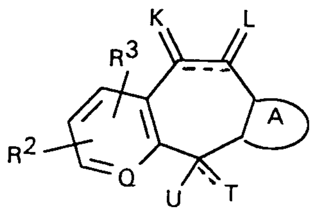

1.0

or a pharmaceutically acceptable salt or solvate thereof, wherein U and T represent =O, or U represents H and T represents

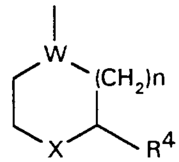

or U and T together represent

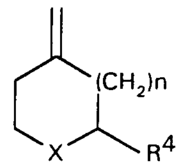

Q is CH, N or N→O;

$R^2$ and $R^3$ may be the same or different and each independently represents H, alkyl, $CF_3$, $NO_2$, halo, $OR^5$, $NR^5R^6$, or $-S(O)_m$-alkyl, in which:

$R^5$ and $R^6$ may be the same or different and each is independently selected from H, alkyl, acyl or aroyl, and m is 0, 1 or 2;

K represents -H, -H or -H, -alkyl, or alkyl, alkyl, or -H, -OH or =O;

L represents -H, -H or -H, -alkyl, or alkyl, alkyl, or -H, -OH or =O, with the proviso that when L or K is -H, -OH or =O, the other of K or L, respectively, is -H, -H or -H, -alkyl or alkyl, alkyl;

ring

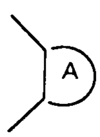

represents

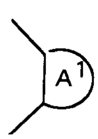

when T represents

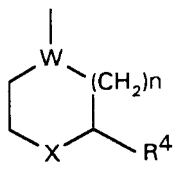

or

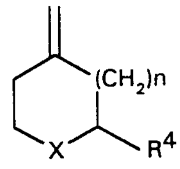

and ring

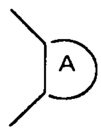

represents

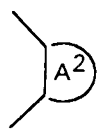

when T represents =O;

ring

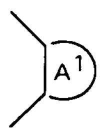

represents a fused five-membered heterocyclic aromatic ring having at least one heteroatom selected from O, S or N in the ring, said ring optionally being substituted by 1 to 3 R groups selected from alkyl, aryl, and arylmethyl or by two R groups on adjacent ring atoms which R groups together with such adjacent ring atoms represent a fused benzene or fused pyridine ring, or ring

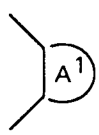

represents a fused six-membered heterocyclic aromatic ring having 1, 2 or 3 ring nitrogen atoms, provided that when there is 1 ring nitrogen atom, X is defined in the claims to distinguish over EP-A-0270818, a citation under Article 54(3);

ring

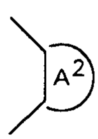

represents

(i) when Q is N or N→O

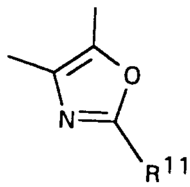

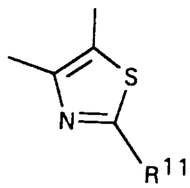

or

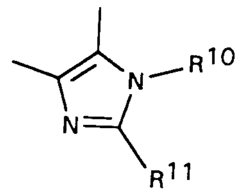

or (ii) when Q is CH, N or N→O

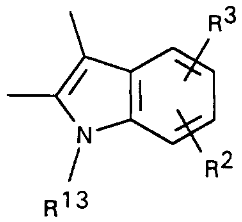

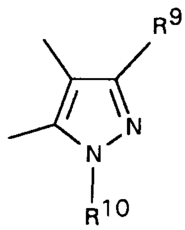

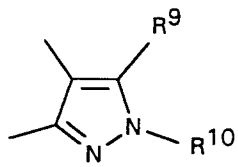

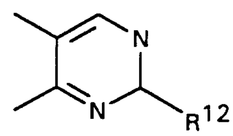

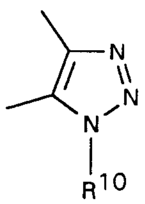

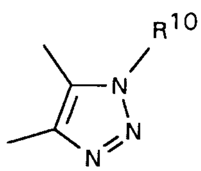

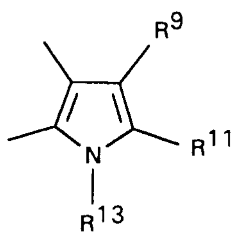

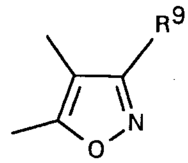

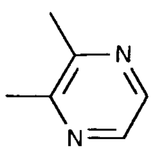

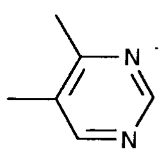

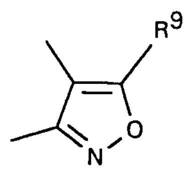

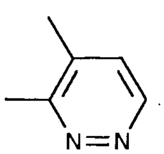

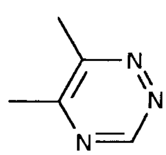

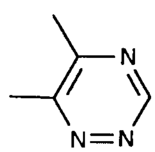

U is -H when the bond between W and the cyclohepta ring is a single bond;
W is N or N→O;
the broken line in the seven membered ring represents an optional double bond.
n is O, 1, 2 or 3;
X represents

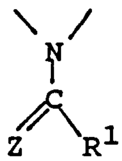,
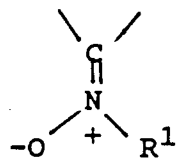
or
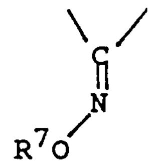

in which:

$R^7$ represents H, alkyl, acyl or aroyl,

Z is O or S, and

$R^1$ is H, alkyl, cycloalkyl, $CF_3$, aryl, heteroaryl, $NR^5R^6$ wherein $R^5$ and $R^6$ are as defined above, alkylthio or alkoxy, or $R^1$ and $R^4$ together represent $-(CH_2)_k-$ where k is 1, 2, or 3 so as to form a fused ring;

$R^x$ represents alkyl, aralkyl or aryl; and

$R^4$ represents H, alkyl or aryl.

$R^9$ is H, alkyl or aryl,

$R^{10}$ is H, alkyl, aryl, arylalkyl, acyl, aroyl and heteroaroyl wherein the aryl moiety is optionally substituted by one or more substituents selected from H, halogen, $NO_2$, $CF_3$, -SH, -S-(alkyl), $-S(O)_m$-alkyl, alkyl, $CO_2H$, $CH_2OH$, C(OH)-(lower alkyl), $NH_2$, NH-(lower alkyl), N-(lower alkyl)$_2$, OH, O-(lower alkyl), O-(aryl), O-(aroyl), O-(heteroaroyl), NH(acyl), N(acyl)$_2$, NH-aroyl, N-(aroyl)$_2$, NH-(heteroaroyl), NH-(heteroaroyl)$_2$, NH(alkyl) N-(alkyl)$_2$,

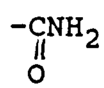, 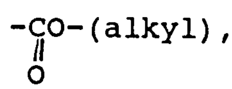,

-CHO,

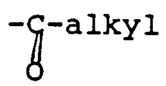, 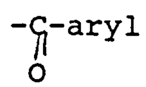,

and -C≡N

$R^{11}$ represents H, alkyl, aryl or aralkyl;

$R^{12}$ represents H, lower alkyl, OH, O-(loweralkyl), SH,S-(lower alkyl), $NH_2$, NH-(lower alkyl), N-(lower alkyl)$_2$, and NH(C=O)-(lower alkyl);

$R^{13}$ is H, alkyl, aryl or arylmethyl; and

The invention does not include those compounds where Q is CH, ring $A^1$ is a fused, 6 membered heterocyclic ring having one ring nitrogen and X represents

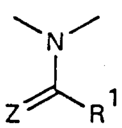

wherein

(a) Z = S or Z = O and $R^1$ is part of a fused ring or is selected from H, alkyl, cycloalkyl, aryl, alkylthio, $-NH_2$, N(alkyl)$_2$, and heteroaryl in which the heteroatom is O, S or N; or

(b) Z = O and $R^1$ is $CF_3$; or

(c) Z = S and $R^1$ is alkoxy.

These compounds are excluded from the claims and are retained in general terms when describing the invention only for their relevance to analagous compounds of the invention.

The present invention also comprises a method for treating PAF disorders, allergy and/or inflammation in a mammal comprising administering an effective amount of a compound of formula 1.0 to said mammal.

The present invention also relates to the use of a compound of formula 1.0 and the pharmaceutically acceptable salts thereof for the preparation of a medicament for the treatment of PAF disorders, allergy and/or inflammation.

Preferred structures are those of formula 1.1:

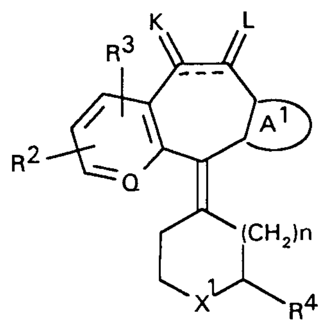

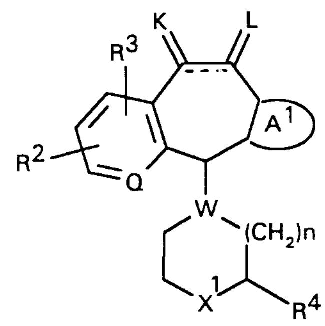

1.1

where $X^1$ represents

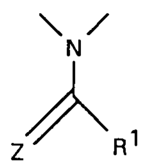

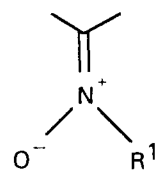

or

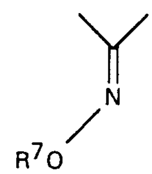

Preferably, ring

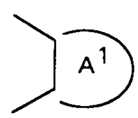

in formula 1.0 represents

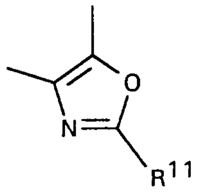 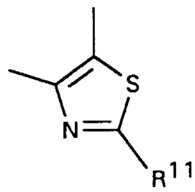 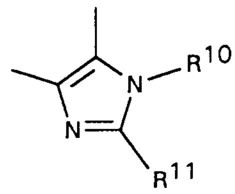 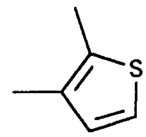

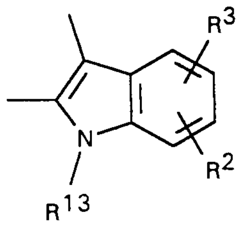 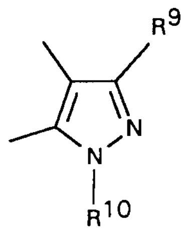 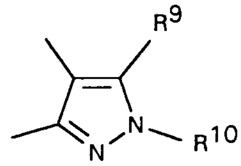 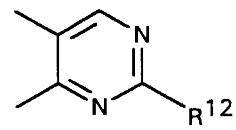

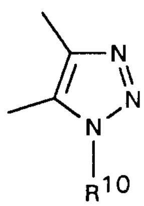 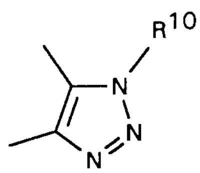 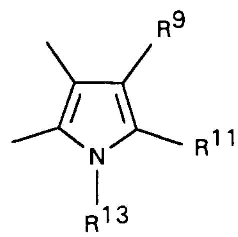 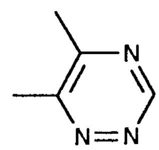

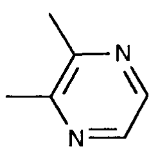 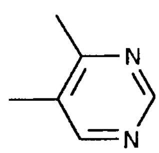 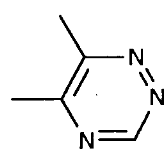 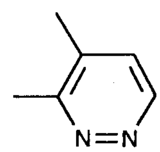

A preferred ring

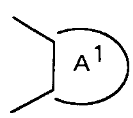

is

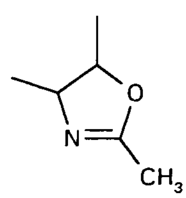

,

Q is preferably N or N→O and $R^2$ and $R^3$ are preferably both H. When Q is CH, $R^2$ is preferably H and $R^3$ is Cl and the chloro group is in the position shown, ie

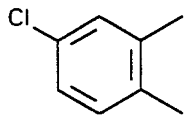

When present, W preferably represents N.

$X^1$ preferably represents

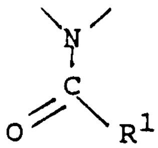

$R^1$ is preferably methyl or 2-, 3-, or 4-pyridyl.

$R^4$ is preferably H and n is preferably 1.

K and L preferably both represent -H,-H.

In another preferred embodiment, ring

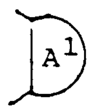

represents

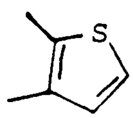

,

K represents H,H; and L represents -OH, -H or =O.

The following compounds are preferred species of formula 1.1:

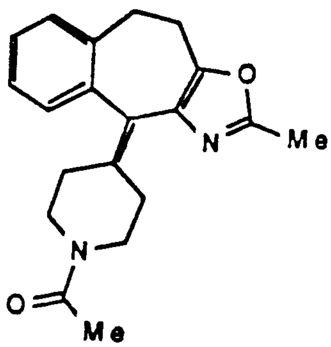

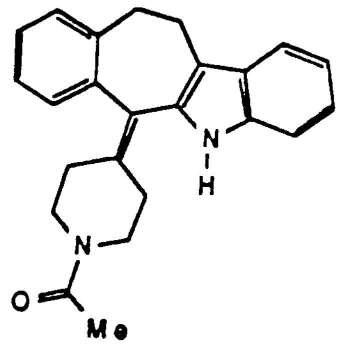

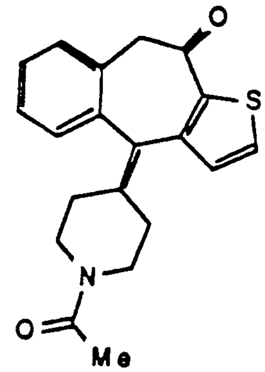

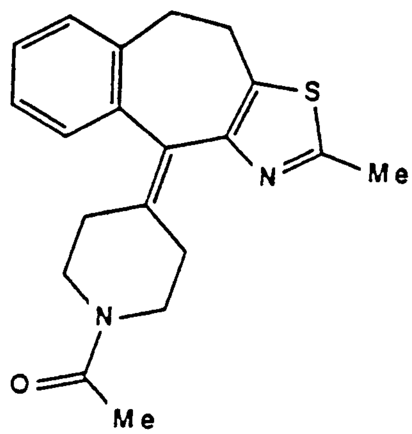

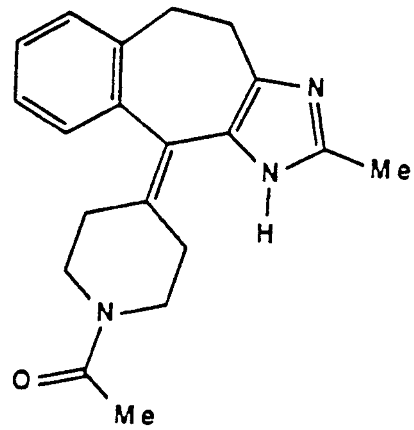

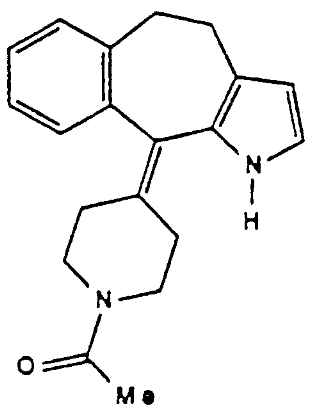

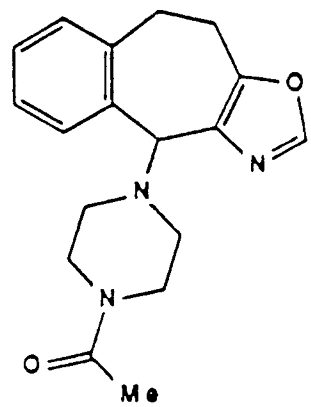

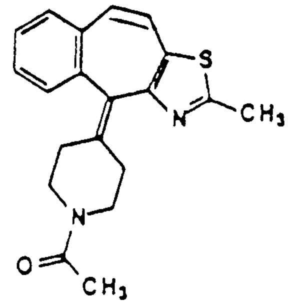

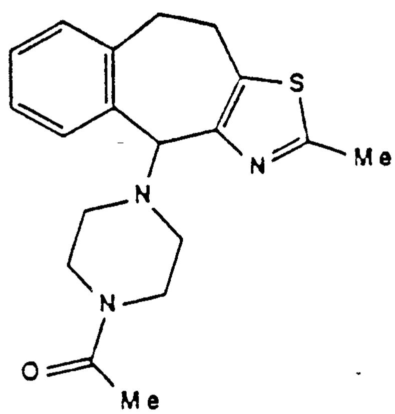

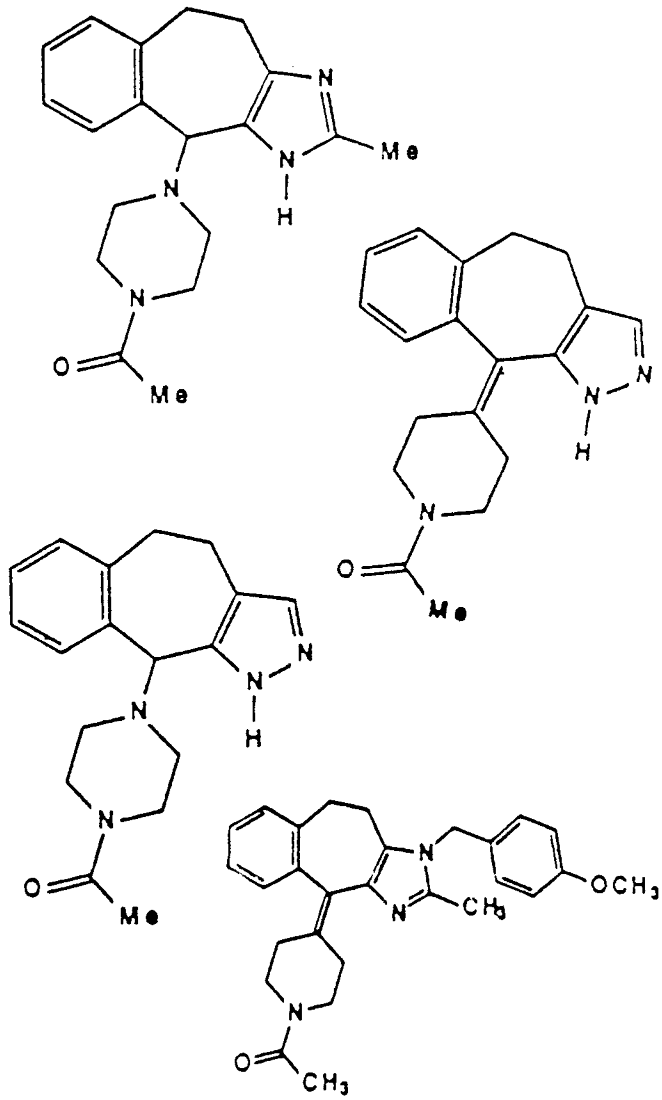

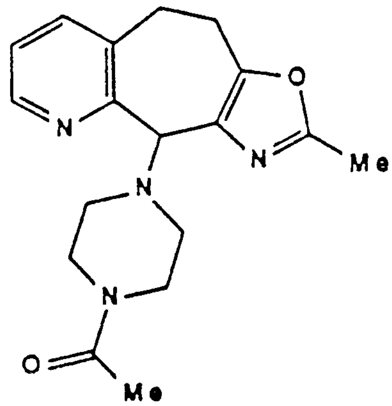

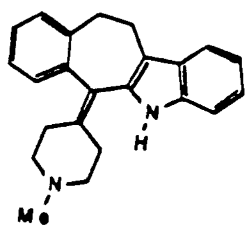

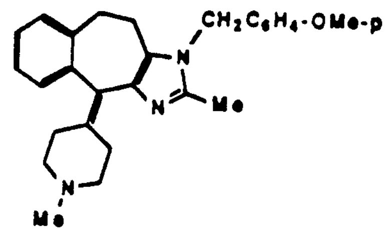

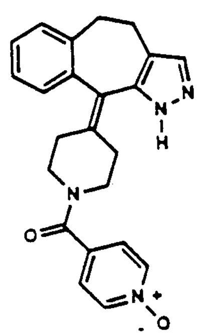

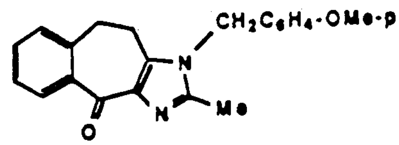

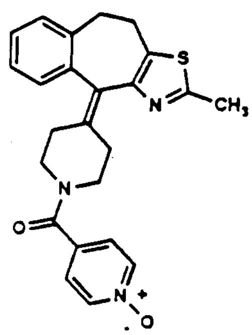

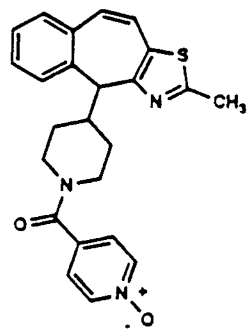

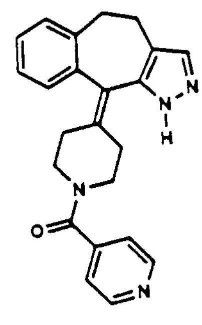

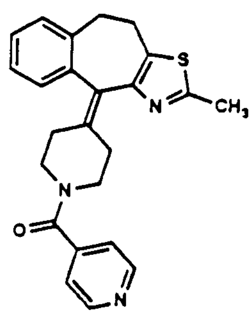

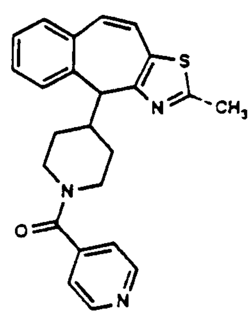

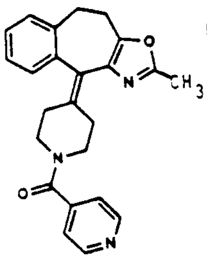
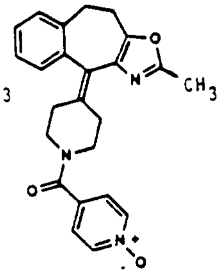
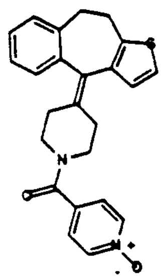
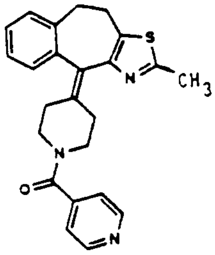
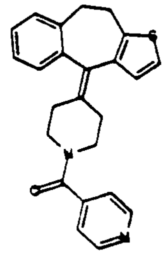
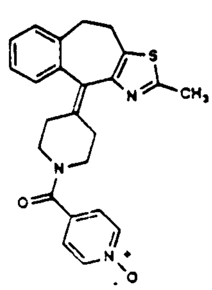
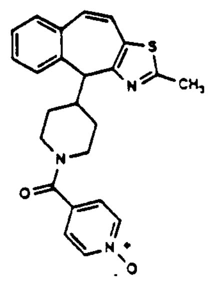
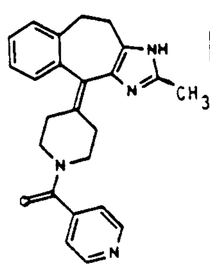
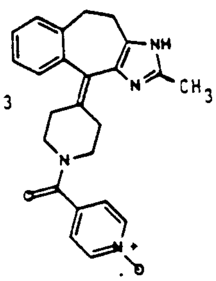
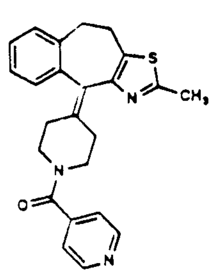
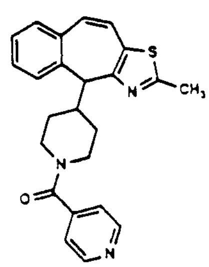

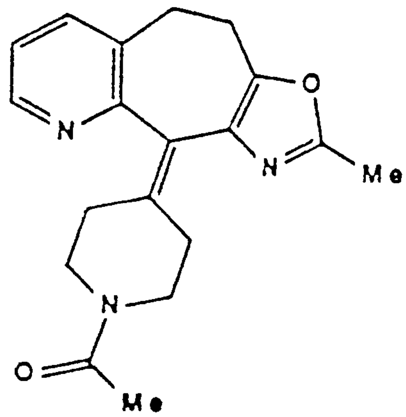

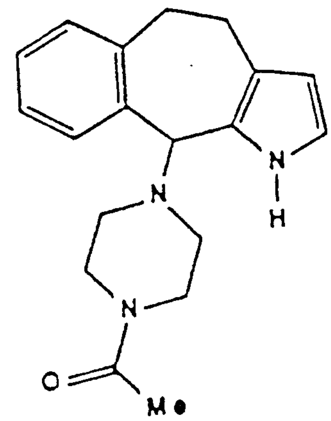

wherein Me = methyl.

Due to their PAF-antagonist activity the compounds of formula 1.1 are useful in treating allergic reactions in mammals, e.g., man. In general, the compounds of formula 1.1 may be used to treat any condition in which mediation of PAF is involved.

Preferred compounds of formula 1.0 which are particularly useful as antiinflammatory agents and also as intermediates for preparing compounds of formulae 1.1 and 2.0 are compounds of formula 3.0

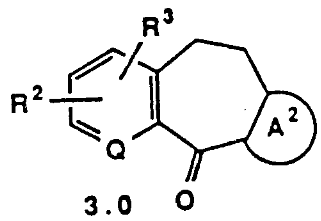

3.0

or a pharmaceutically acceptable salt or solvate thereof, wherein m, Q, $R^2$, $R^3$, $R^5$, $R^6$ $R^9$, $R^{10}$, $R^{11}$, $R^{12}$, $R^{13}$, and $R^{14}$ are as previously defined.

m is 0, 1 or 2;

ring

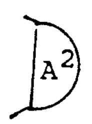

represents

(i) when Q is N or N→O:

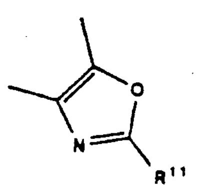 , 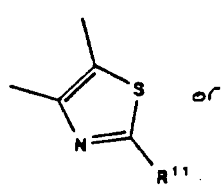 or 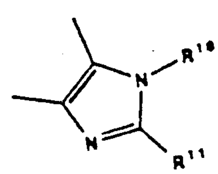 ,

or (ii) when Q is CH, N or N→O:

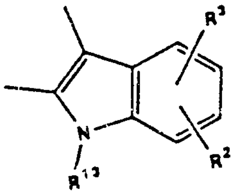
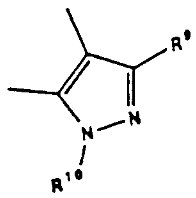
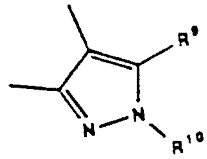
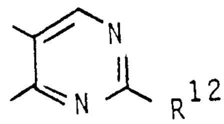

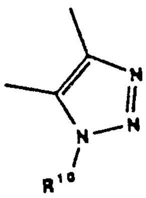
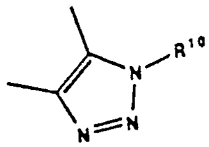
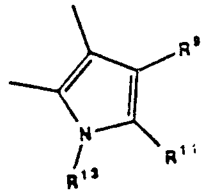

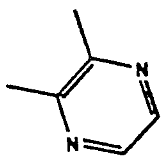
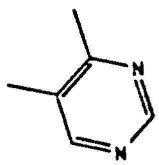
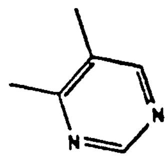

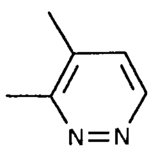
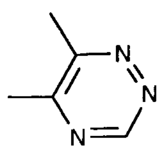
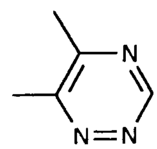

In formula 3.0, the preferences stated above for Q, $R^2$ and $R^3$ also apply. Ring

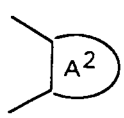

is preferably

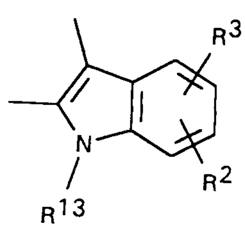

The present invention also is directed at pharmaceutical compositions comprising a composition of formula 1.0, and a pharmaceutically acceptable carrier.

The present invention further comprises a method of making a pharmaceutical composition comprising admixing a compound of formula 1.0, with a pharmaceutically acceptable carrier.

The invention further comprises a method for making a compound of formula 1.0, comprising

a) where $X^1$ represents

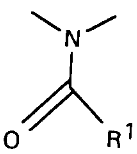

reacting a compound of formula 9.1 with compound 8.1 in the presence of base

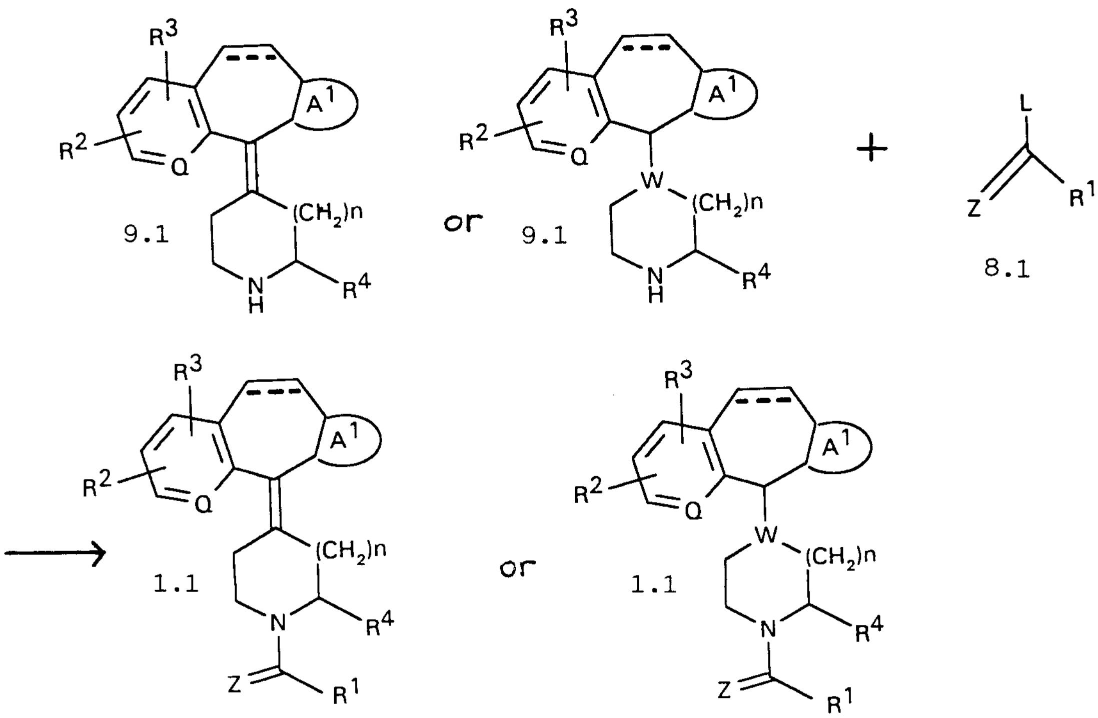

9.1 or 9.1 + 8.1

1.1 or 1.1

b) where $X^1$ represents

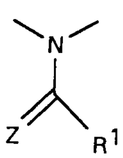

where Z is =O or =S, reacting a compound of formula 11.1 with a compound of formula 8.1

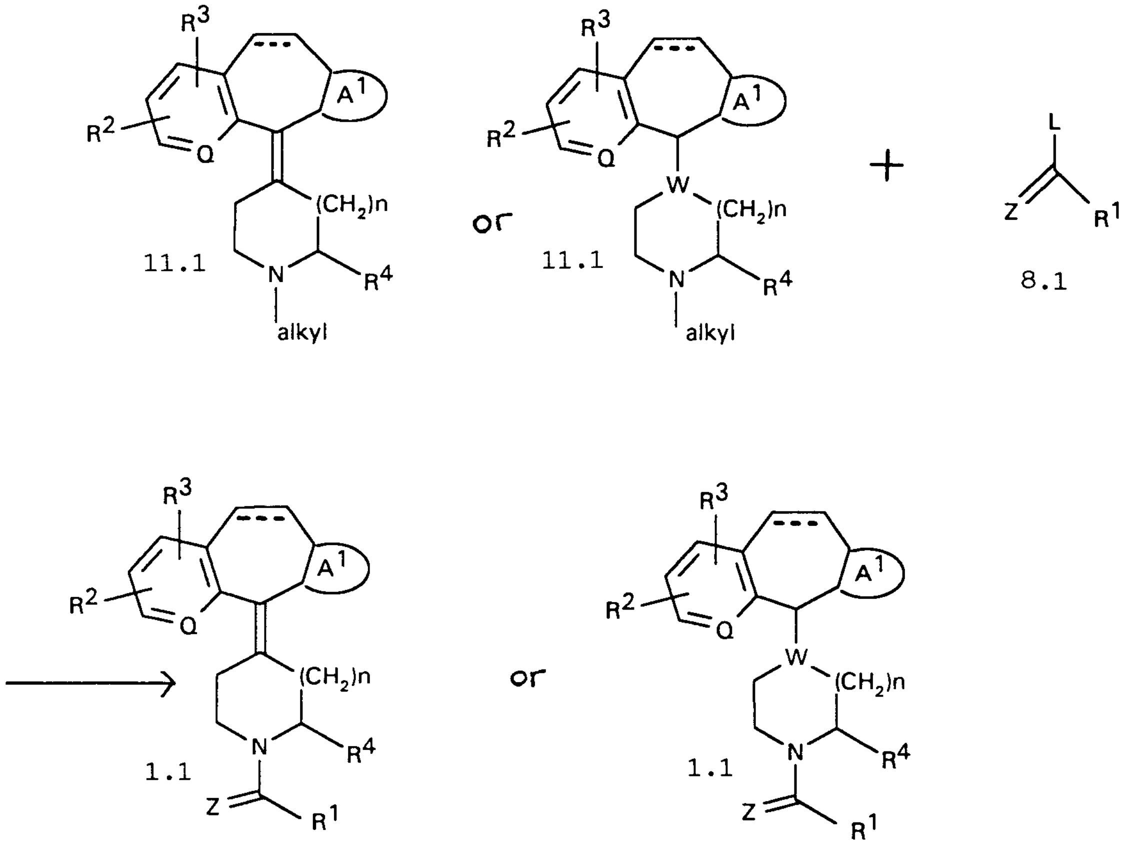

11.1 or 11.1 8.1

1.1 or 1.1

c) where $X^1$ represents

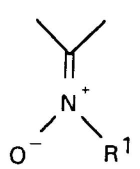

or

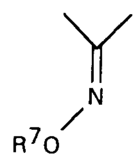

reacting a compound of formula 13 in the presence of hydroxylamine or O-substituted hydroxylamine or N-substituted hydroxylamine:

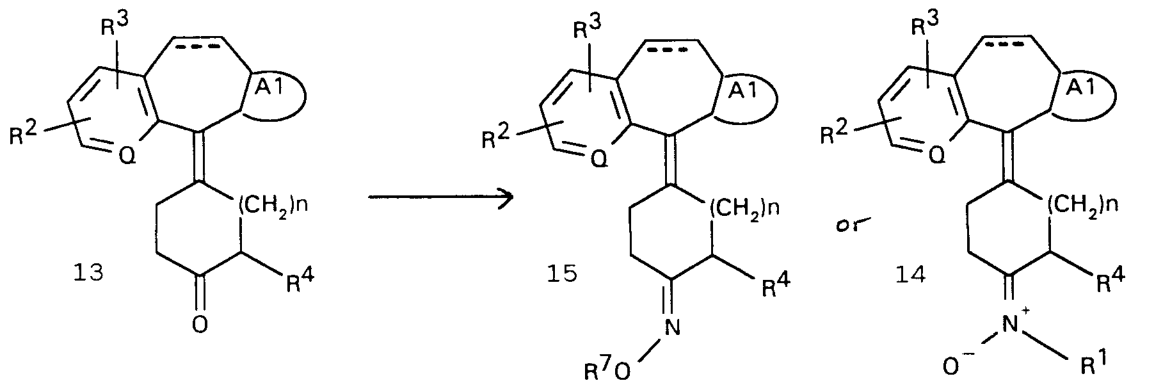

13 15 or 14

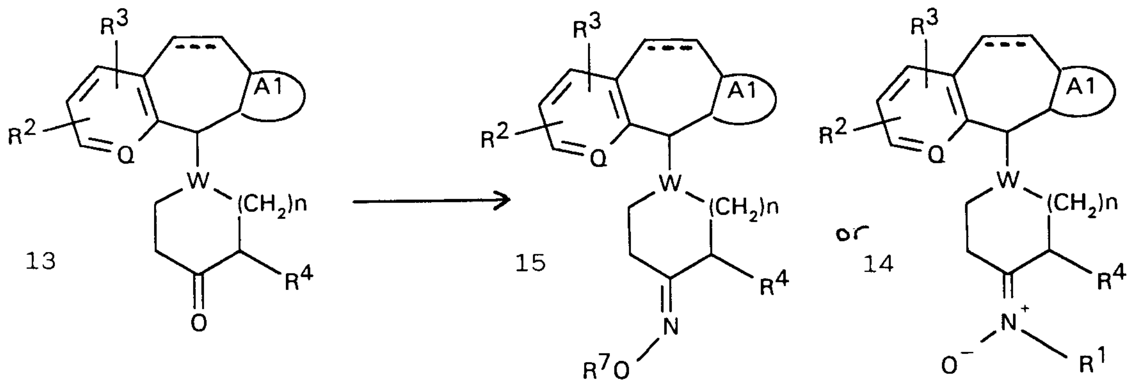

13 15 or 14

d) Reacting a compound of formula 9.2 with an acid

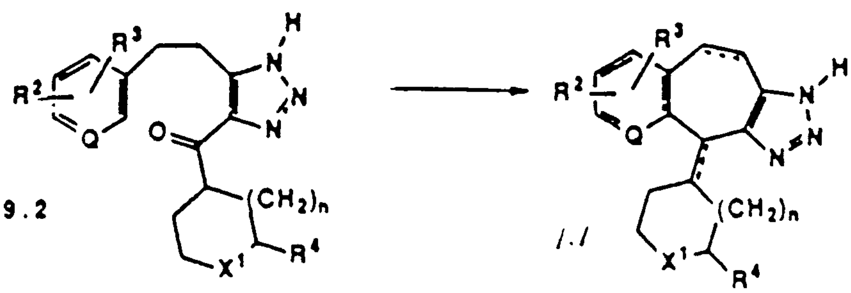

9.2 1.1

e) Reacting a compound of formula 200 with an alkali or alkaline metal hydroxide

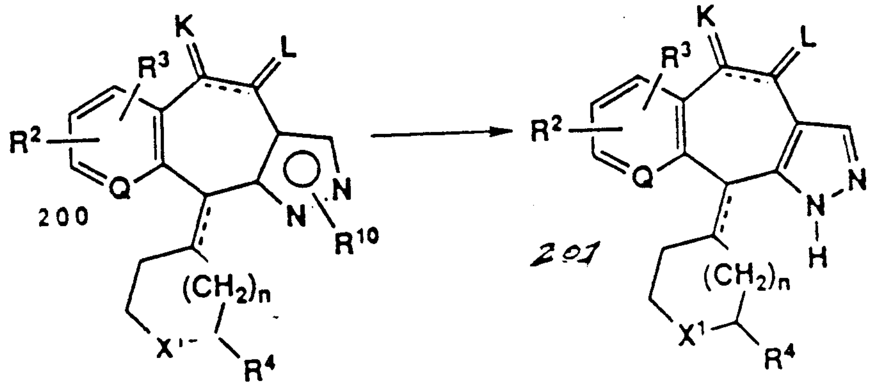

200 201

f) Reacting a compound or formula 17 with a suitable acid

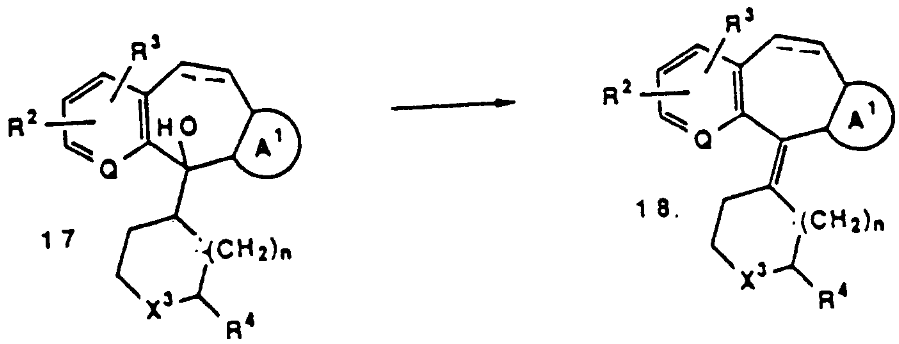

17 18.

wherein $X^3$ represents

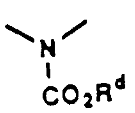 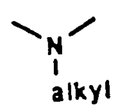 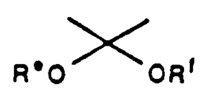 or 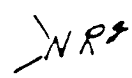

$R^d$ represents an optionally substitutted alkyl, alkenyl, aryl or aralkyl group;

$R^e$ and $R^f$ each independently represent lower alkyl or together represent an alkanediyl chain of 2 or 3 methylene groups;

$R^g$ is H or $C(Z)R^1$.

g) Reacting a compound of formula 19 with a compound of formula 20 where L is a leaving group and $X^3$ represents $NR^g$ or

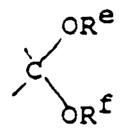

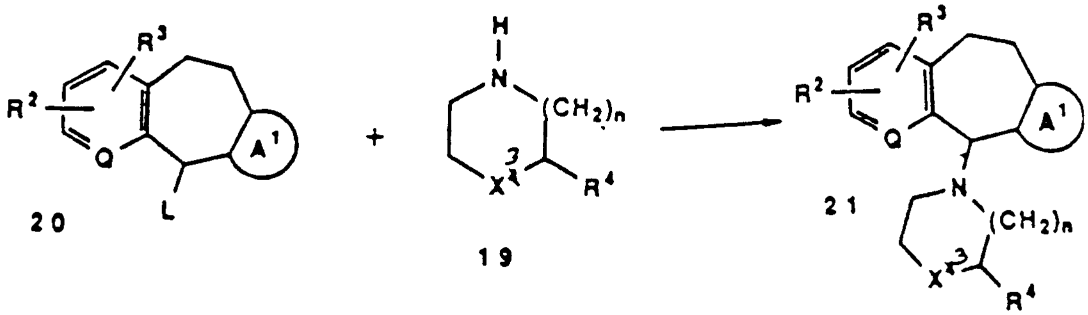

h) Reacting a compound of formula 4.0 with a compound of formula 19

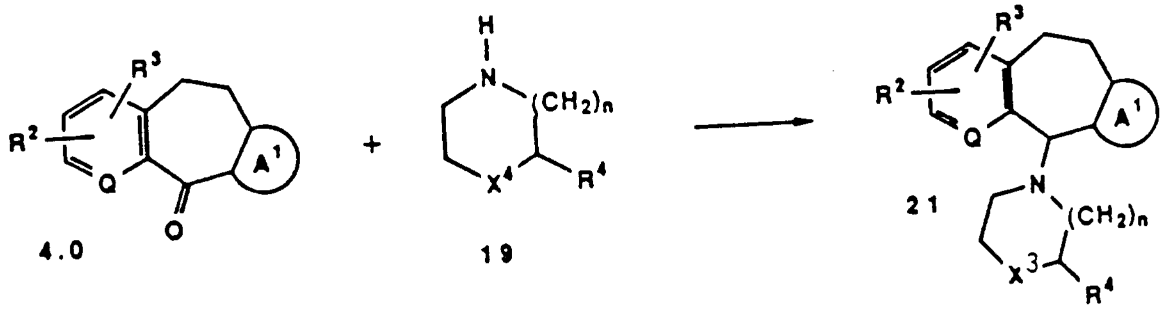

i) Dehydrating a compound of formula 26 to produce compound 27

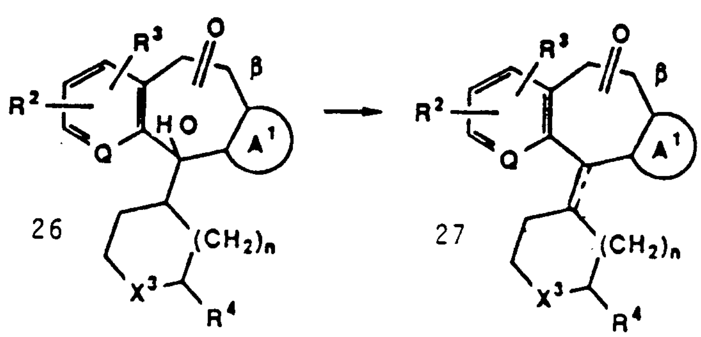

j) Hydrolyzing compound 30 with a strong aqueous acid to produce compound 31

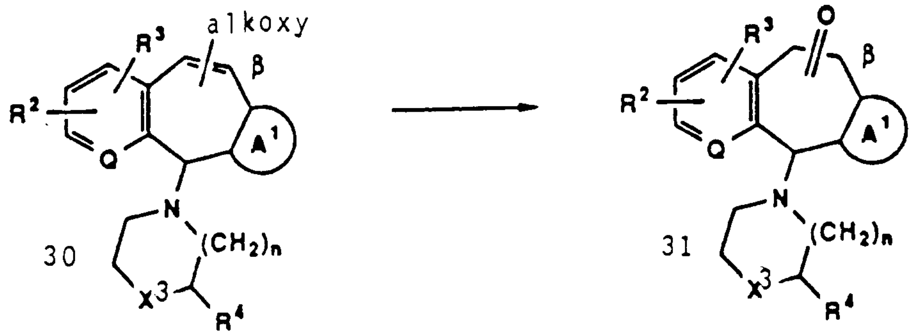

30

31

k) Reacting a compound of formula 5.1 with a compound of formula $R^{11}CO_2H$, and $(R^{11}CO)_2O$ or $(R^{11}CO_2)_2O$ alone in the presence of HCl

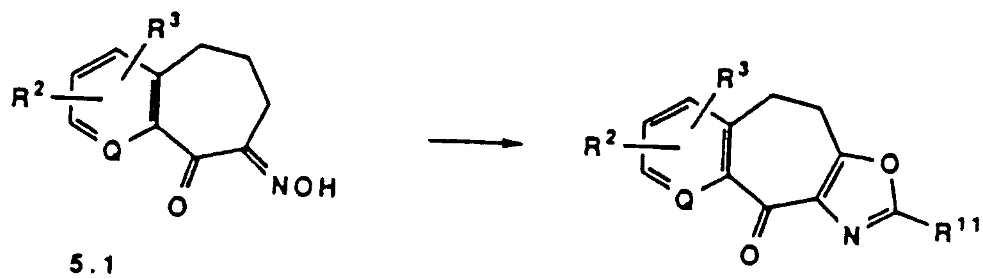

5.1

4.1

l) Reacting a compound of formula 5.2 with an acidic reagent wherein Ar represents an appropriate aryl group

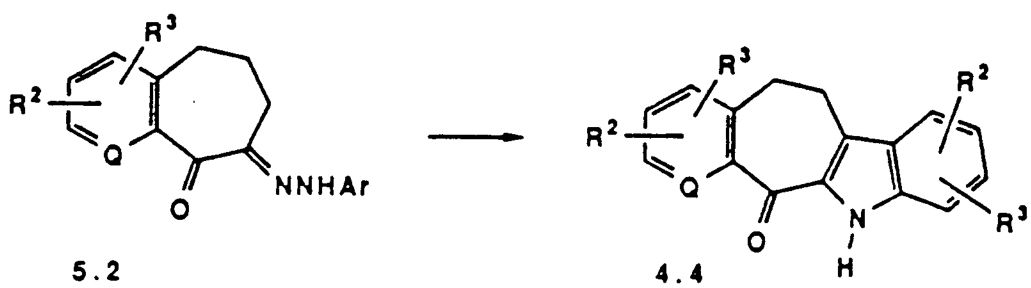

5.2

4.4

m) Reacting a compound of formula 5.3 with a hydrazine reagent $R^{10}NHNH_2$ and an acid catalyst

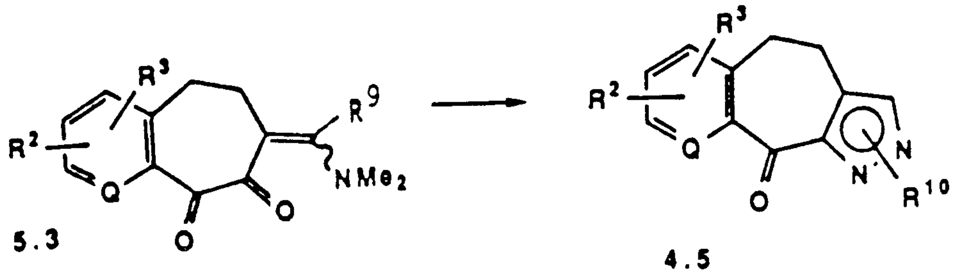

5.3 4.5

n) Reacting a compound of formula 5.4 with an acid or peracid, to produce a compound of formula 4.6

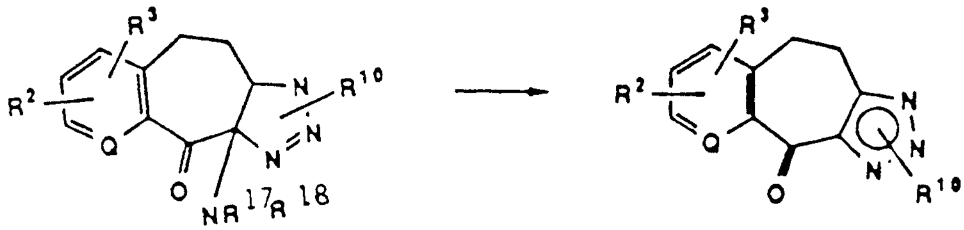

5.4 4.6

o) Reacting a compound of formula 5.5 with an appropriate alkali metal dithionite in a hot solvent

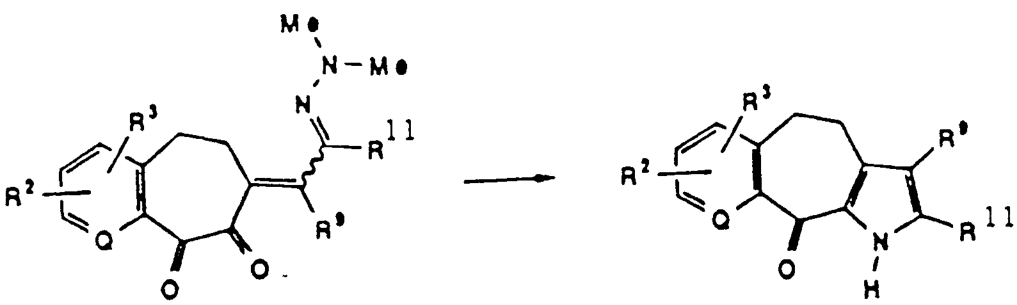

5.5 4.7

p) Reacting a compound of formula 5.3 with a hydroxylamine hydrochloride

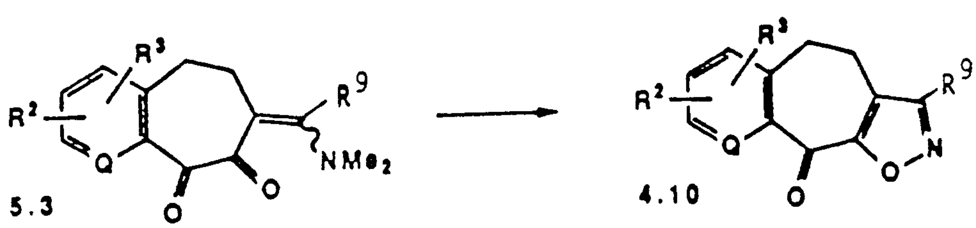

5.3 4.10

q) Reacting a compound of formula 5.3 with a compound of formula $H_2N\text{-}C(=NH)R^y$ where $R^y$ represents one of H, lower alkyl, OH, O-(lower alkyl), SH, S-(lower alkyl), $NH_2$, NH-(lower alkyl), N-(lower alkyl)$_2$, and NH(C=O)-(lower alkyl), of formula

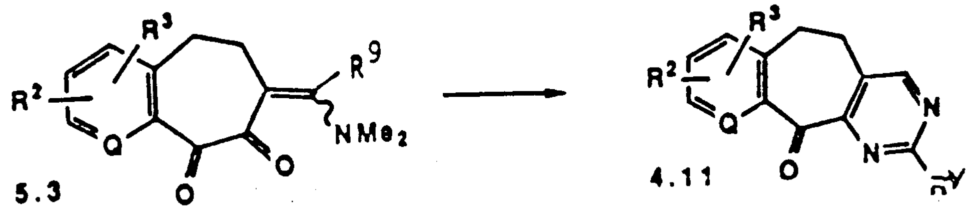

5.3 4.11

r) Reacting a compound of formula 5.3 with a compound of formula $H_2NC(=O)CH_2E$, in which E is taken from among CHO, C(=O)-(lower alkyl), $CO_2$-(lower alkyl), $CONH_2$, $NO_2$, CN, $SO_2CH_3$, and $CF_3$:

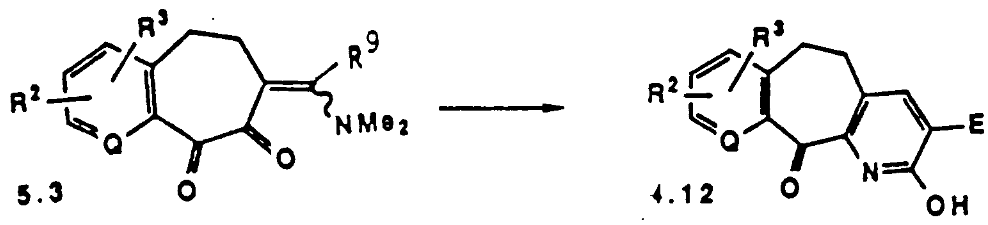

5.3 4.12

s) Reacting a compound of formula 6.0 with formamide, p-toluenesulfonic acid, and N,N',N"-methylidynetrisformamide ($HC(NHCHO)_3$)

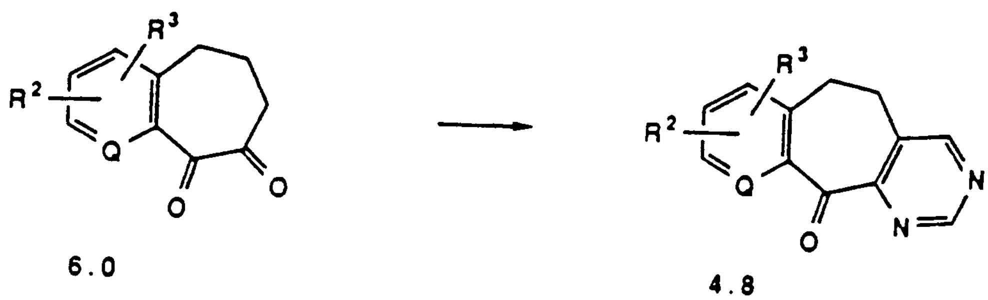

6.0 4.8

t) Reacting a compound of 5.6 with hydrazine followed by oxidation

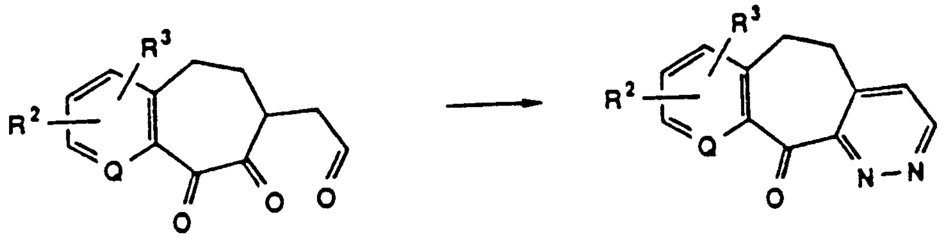

5.6

4.9

DETAILED DESCRIPTION OF THE INVENTION

Certain compounds of formula 1.0, may exist in different isomeric as well as conformational forms. The invention contemplates all such isomers both in pure form and in admixture, including racemic mixtures.

The compounds of formula 1.0, can exist in unsolvated as well as solvated forms, including hydrated forms, e.g., hemihydrate. In general, the solvated forms, with pharmaceutically acceptable solvents such as water, ethanol and the like are equivalent to the unsolvated forms for purposes of the invention.

As noted above, the structures of formula 1.0, may contain one or more substituents, e.g., $R^2$ and $R^3$. In compounds where there is more than one such substituent, they may be the same or different. Thus, compounds having combinations of different substituents are within the scope of the invention. Also, the lines drawn into the rings from the $R^2$ and $R^3$ groups indicate that such groups may be attached at any of the appropriate available positions. For example, the $R^2$ and $R^3$ groups may be attached at any of the four positions in the left-hand ring of formula 1.0, provided that Q is CH, and at the three positions when Q is N.

The definitions of K and L are such that both may not represent -H,-OH or =O or -H,-alkyl or alkyl,alkyl at the same time. However, one of K or L may represent -H,alkyl or alkyl,alkyl when the other is -H,OH or =O or -H,-H. Also, one of K or L may represent -H,-H when the other is -H,-OH or =O.

Certain compounds of formula 1.0, will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic compounds of formula 1.0, also form pharmaceutically acceptable salts, e.g., acid addition salts and quaternary ammonium salts. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The quaternary ammonium salts are prepared by conventional methods, e.g., by reaction of a tertiary amino group in a compound of formula 1.0, with a quaternizing compound such as an alkyl iodide, etc. The free base forms differ from their respective salt forms somewhat in certain physical properties, such as solubility in polar solvents, but the salts are otherwise equivalent to their respective free base forms for purposes of the invention.

All such acid, base and quaternary salts are intended to be pharmaceutically acceptable salts within the scope of the invention and all acid and base salts are considered equivalent to the free forms of the corresponding compounds for purposes of the invention.

Unless otherwise indicated herein, the terms listed below have the following meanings:

alkyl (including the alkyl portions of $-(SO_2)_m$-alkyl, alkylthio, alkoxy, alkylamino and dialkylamino) - represents a straight or branched, saturated hydrocarbon chain having from 1 to 8 carbon atoms, e.g., methyl, n-propyl, isopropyl, tertiary butyl, secondary butyl, etc.;

halo - represents fluoro, chloro, bromo or iodo;

acyl - represents a group

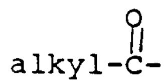

wherein alkyl is as described above;

cycloalkyl - represents a saturated carbocyclic ring having from 3 to 8 carbon atoms, preferably 5 to 7 carbon atoms, e.g., cyclohexyl;

aryl (including the aryl portion of aroyl and arylalkyl) - represents a carbocyclic group having from 6 to 15 carbon atoms and at least one benzene ring, with all of the available substitutable carbon atoms of the carbocyclic group being intended as possible points of attachment, wherein the carbocyclic group may be optionally substituted with 1 to 3 substituents independently selected from halo, alkyl, hydroxy, alkoxy, alkyl-$S(O)_m$-phenoxy, $CF_3$, amino, alkylamino, dialkylamino, $NO_2$, carboxyl or -COOalkyl, and wherein aryl preferably represents phenyl or phenyl substituted with 1 to 3 substituents as described above;

alkanediyl - represents a divalent, straight or branched hydrocarbon chain having from 1 to 20 carbon atoms, preferably 1 to 6 carbon atoms, the two available bonds being from the same or different carbon atoms thereof, e.g., methylene, ethylene, ethylidene, $-CH_2CH_2CH_2-$,

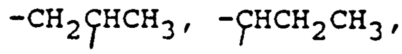

etc.

alkenyl - represents straight and branched carbon chains having at least one carbon to carbon double bond and containing from 2 to 12 carbon atoms, preferably from 3 to 6 carbon atoms;

aroyl - represents

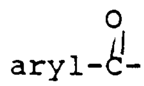

where aryl is as described above; and

heteroaryl - represents a cyclic group having at least one heteroatom selected from O, S, N→O and/or N interupting a carbocyclic ring structure and having a sufficient number of delocalized pi electrons to provide aromatic character, wherein heteroaryl groups preferably have from 2 to 14 carbon atoms and are optionally substituted with 1 to 3 substituents independently selected from halo, alkyl, hydroxy, alkoxy, alkyl-$S(O)_m$-, phenoxy, $CF_3$, amino, alkylamino, dialkylamino, $NO_2$, carboxyl or -COOalkyl, and wherein any available carbon atoms thereof may function as the point of attachment of the heteroaryl group, suitable heteroaryl groups including 2- or 3-furanyl, 2- or 3-thienyl, 2-, 3- or 4-pyridinyl, 2-, 4- or 5-thiazolyl, 2-, 4- or 5-imidazolyl, 2-, 3-, 5- or 6-pyrimidinyl, 2- or 3-pyrazinyl, 3- or 4-pyridazinyl, 3-, 5- or 6-[1,2,4-triazinyl], 2-, 3-, 4-, 5-, 6- or 7-benzofuranyl, 2-, 3-, 4-, 5-, 6- or 7-indolyl, 3-, 4- or 5-pyrazolyl, 2-, 4- or 5-oxazolyl, etc.

heteroaroyl - represents

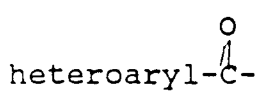

where the heteroaryl is as defined above.

The ring

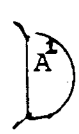

represents a fused, 5-membered heterocyclic aromatic ring having at least one heteroatom selected from O, S or N in the ring or a 6-membered heterocyclic aromatic ring having 2 or 3 ring nitrogen atoms in addition to carbon atoms. The 5-membered rings have two double bonds and the 6-membered rings have three double bonds. One skilled in the art will recognize the possible combinations of O, S and N heteroatoms in such fused aromatic rings, and all are part of the

invention. For example, in the 5-membered fused ring system containing one O or S atom as the only ring heteroatom, the O or S may be at any of three positions. Similarly, when two N atoms are in the 6-membered heterocyclic aromatic ring, there are six possible isomeric variations for the two nitrogen atoms. All such possibilities are considered to be within the scope of the present invention.

The compounds of formula 4.0

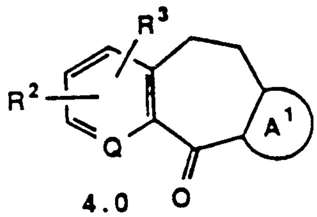

4.0

may be prepared according to general procedures known in the art. For example, the compounds of formula 4.0 may be prepared by conventional heterocycle-forming reactions, e.g., intramolecular or intermolecular ring closures. One particularly useful starting material for such ring closures is a compound of formula 6.0

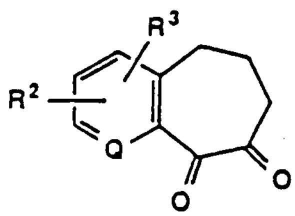

6.0

or an appropriate derivative thereof. Examples of appropriate derivatives and heterocycle-forming reactions for forming compounds of formula 4.0 are exemplified below and others are known to those skilled in the art. Also, certain rings

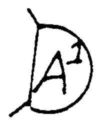

may be converted to other rings

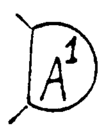

by known techniques. The hydroxy and carbonyl groups on the bridge carbon atoms (i.e., as in K and L) may be added subsequent to heterocycle-forming reactions as also described below.

The compounds of formula 4.0 may be prepared by the ring closure or other reactions exemplified in sections A - I below:

A. To prepare a compound of formula 4.1 wherein ring

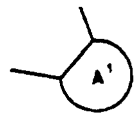

represents

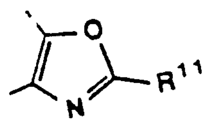

,

a compound of formula 5.1 is reacted with, for example, a compound of the formula $R^{11}CO_2H$ and $(R^{11}CO)_2O$, or $(R^{11}CO)_2O$ alone or the like, and anhydrous, gaseous HCl:

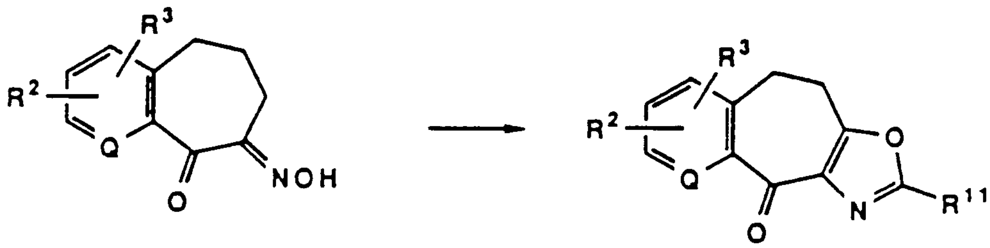

5.1

4.1

This reaction is a standard, general method disclosed by J. Med. Chem. (1974, 17, 1316), Chem. Commun. (1970, 754), and by Chem. Commun. (1970, 274). Hydrogen bromide, but not hydrogen iodide or fluoride, may be substituted for the chloride. Aqueous rather than gaseous hydrogen bromide may be used, provided enough $(R^{11}CO)_2O$ is used to react with the water introduced. Similarly, aqueous hydrogen chloride may be used instead of the gaseous acid. Another, and preferred, substitute for anhydrous, gaseous hydrogen chloride is a 1:1 mixture of anhydrous ethanol (or methanol) and $R^{11}COCl$. Alcohol and acid chloride react to produce the needed, anhydrous hydrogen chloride as well as an ester.

The compound of formula 5.1 may be prepared as disclosed in J. Med. Chem. (1974, 17, 1316) and J. Med. Chem. (1984, 27, 20). Use of cerain commercially available starting materials according to standard methods disclosed by these references furnishes the α-oximinoketones 5.1. 1-Benzosuberone and cycloheptenylpyridine exemplify these starting materials, and they are commercially available from the Aldrich Chemical Co. Milwaukee, Wisconsin, U.S.A.

B. The compounds of formula 4.1 may be converted to compounds of formula 4.0 wherein ring

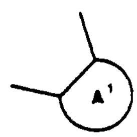

represents

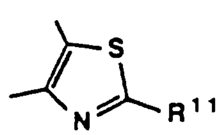

by reacting a compound of formula 4.1 with, for example, $H_2S$ and potassium tertiary butoxide in N,N-dimethylfor-

mamide:

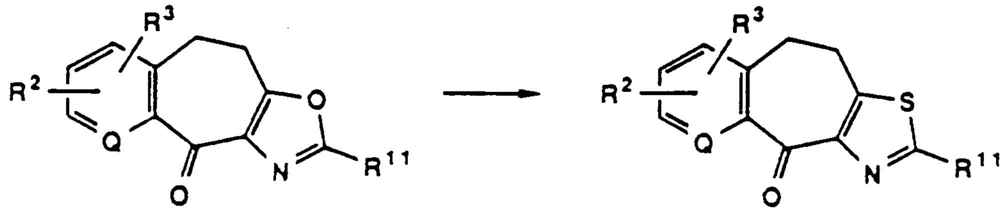

4.1 4.2

J. Med. Chem. (1974, 17, 1316) discloses the general method above. Potassium hydrogen sulfide should also work, but the combination of hydrogen sulfide and potassim tert.-butoxide is preferred. Also, any alkali metal cation may replace the potassium ion, and any liquid tertiary amide may replace N,N-dimethylformamide. Any suitable temperature may be employed, e.g., 0-100°C, preferably about 25°C.

C. The compounds of formula 4.1 may also be converted to compounds of formula 4.0 wherein ring

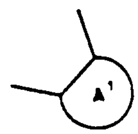

represents

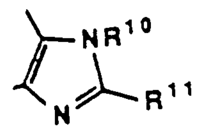

by reacting a compound of formula 4.1 with a compound of formula $R^{10}NH_2$:

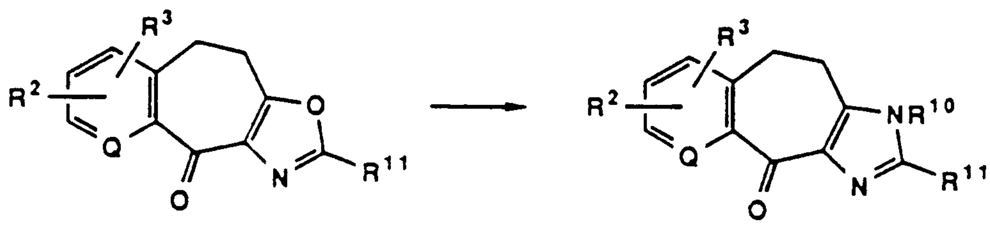

4.1 4.3

This reaction represents a general method for making imidazoloketones (4.3) from oxazoloketones (4.1). J. Med. Chem. (1974, 17, 1316) discloses several examples of this reaction. Preferably, the reaction may be carried out in a sealed tube, i.e., at a pressure higher than atmospheric. It is preferable to use no solvent whatsoever, but inert, high boiling solvents may be employed. Any suitable temperature may be employed, but the temperature is preferably elevated (e.g., >100°C); more preferably, the temperature should be about 130-140°C.

D. A compound of formula 4.0 wherein ring

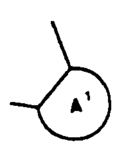

represents

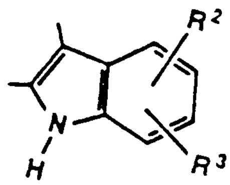

may be prepared by reacting a compound of the formula 5.2 with an acidic reagent:

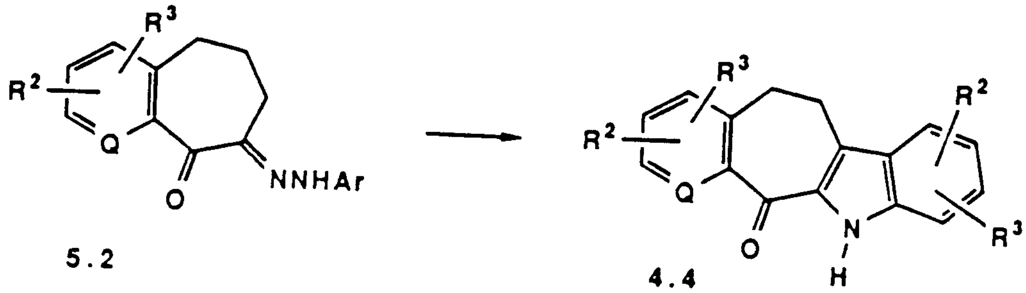

5.2 4.4

wherein Ar represents an appropriate aryl group to result in the desired $R^2$ and $R^3$ substituents. This type of reaction is well-known, as taught by Robinson's monograph "The Fischer Indole Synthesis" (Wiley, New York, 1983). The Fischer indole synthesis is also the subject of numerous reviews cited by March ("Advanced Organic Chemistry: Reactions, Mechanisms, and Structure", Wiley, New York, 1985, p. 1033). An acidic reagent is the only one needed, and the reagent as well as solvents and temperatures, may be varied widely. The aryl hydrazones of formula 5.2 are easily prepared from compounds of formula 6.0 above by procedures well known in the art.

E. A compound of formula 4.0 wherein ring

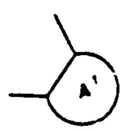

represents

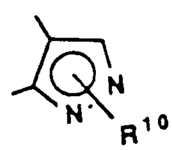

may be prepared by reacting a compound of formula 5.3 with a hydrazine reagent $R^{10}NHNH_2$ and an acid catalyst:

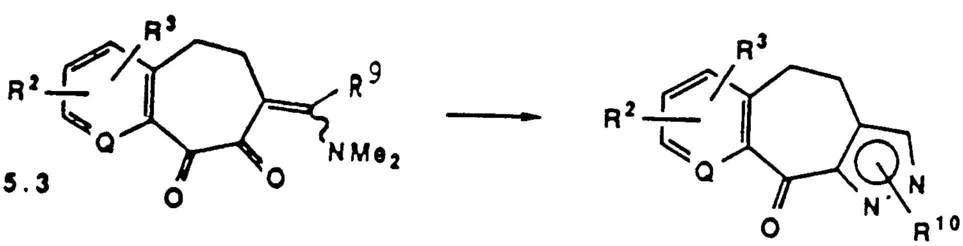

5.3 4.5

This reaction is illustrated in J. Het. Chem. (1982, 19, 1355). Suitable catalysts include, for example, acetic and toluenesulfonic acids. The reaction may be carried out in a variety of solvents, among which n-butanol is preferred. Toluene may also be used as a solvent. The time and temperature of the reaction are varied according to the particular enaminone of formula 5.3 used as substrate.

The compound of formula 5.3 can be prepared by reacting a compound of formula 6.0 with an appropriate amide acetal such as N,N-dimethylformamide dimethylacetal:

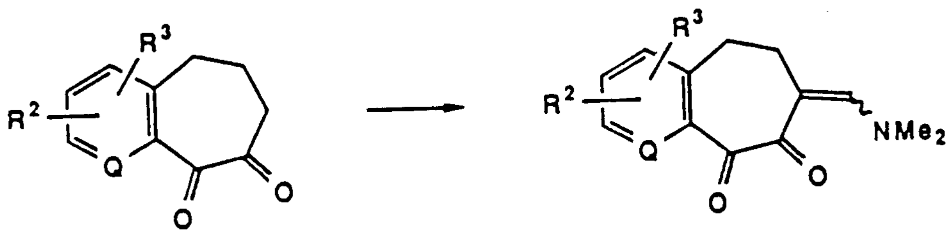

6.0 5.3

This reaction is also illustrated in J. Het. Chem. (1982, 19, 1355). Making enaminone 5.3 requires N,N-dimethylformamide dimethylacetal as the only reagent, and the structure of this preferred amide-acetal may be varied. Thus, $R^a(R^b)NC(O)R^c$ may be used in place of it. $R^a$ and $R^b$, which may be the same or different, may be chosen from among lower alkyl, or together may represent a chain of C-atoms varying in length from 4 to 6. The resulting azacycle is then 5- to 7-membered. $R^c$ may be H or lower alkyl. The reaction may be carried out at temperatures ranging from 0 to > 100°C, and a preferred temperature is that of the boiling point of the amide-acetal.

F. A compound of formula 4.0 wherein ring

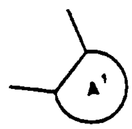

represents

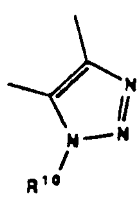

or

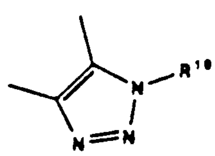

may be also prepared by reacting an appropriate compound of formula 5.4 with an acid or peracid:

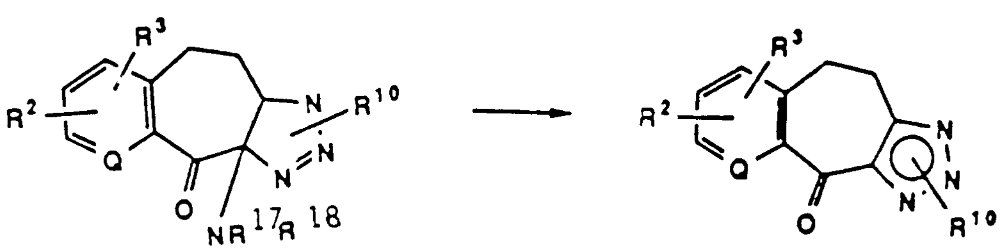

5.4 4.6

$R^{17}$ and $R^{18}$ may be the same or different and may be $C_1$-$C_6$ linear, branched or cyclic alkyls or $R^{17}$ and $R^{18}$ may be combined with each other to produce a heterocycle which optionally may have an additional heteroatom which interrupts the methylene groups (e.g. morpholine).

Suitable acids and peracids include p-toluenesulfonic, trifluoroacetic, and trifluoromethanesulfonic acids; and perbenzoic, m-chloroperbenzoic, peracetic and per trifluoroacetic acids. Suitable solvents include benzene, toluene, xylenes, and tert.-butylbenzene, dichloromethane, carbontetrachloride, chloroform, and 1,2-dichloroethane. When an acid is used to effect the reaction, the solution is preferably heated, more preferably to the boiling point of the solvent. When a peracid is used, heating is typically unnecessary but may be desirable. If the solution containing peracid is heated, a stabilizer should be added, and a preferred stabilizer is 4,4′-thiobis-6-tert.-butyl-m-cresol.

The compound of formula 5.4 can be made from a compound of formula 7.0.

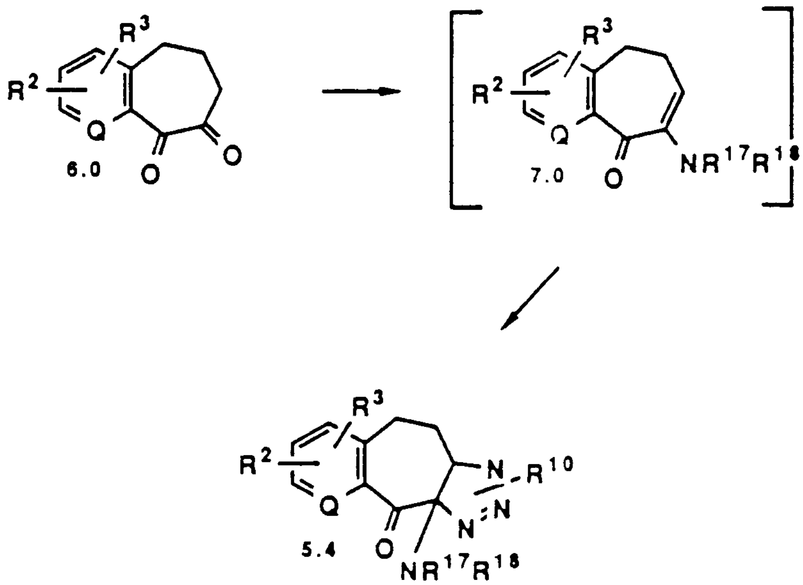

6.0 7.0 5.4

The reactions above are well known to those skilled in the art, see, e.g., Finley's monograph, "Triazoles: 1,2,3" (in "The Chemistry of Heterocyclic Compounds", Vol. 39, Wiley, New York, 1980). In the present case, the reaction that produces the intermediate triazoline 5.4 may be carried out by heating a mixture of the azide ($R^{10}N_3$) and enamine 7.0 in an inert, high boiling solvent. The temperature of reaction is elevated (> 100°C), and the preferred temperature is that of the boiling point of the solvent. A preferred solvent is toluene.

Methods for making the needed enamines 7.0 from α-diketone 6.0 are standard; they may be found in "Enamines" (Chapters 2 and 8, Marcel Dekker, New York, 1969).

Triazoline (5.4) formation and enamine (7.0) formation may advantageously be combined in a single process beginning with α-diketone 6.0. The advantage of the combination is that the intermediate enamine (7.0) need not be isolated or purified, but is formed and used in situ. In this process, compound 6.0 is heated in a solvent containing both an amine ($R^{17}R^{18}NH$) and an azide ($R^{10}N_3$). An inert, high boiling solvent is used, and the temperature of reaction is elevated (> 100°C). The preferred temperature is that of the boiling point of the solvent, and a preferred solvent is toluene. Other solvents like xylenes and tert-butylbenzene may also be used.

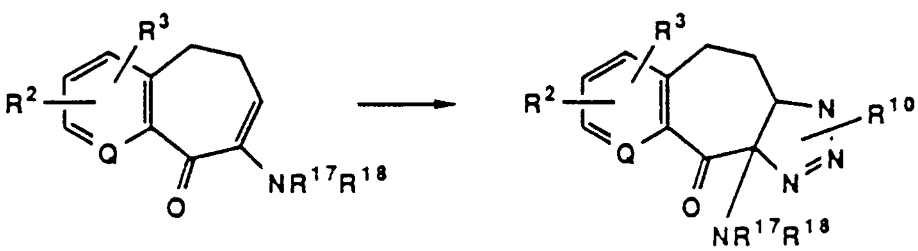

7.0 5.4

G. A compound of formula 4.0 wherein ring

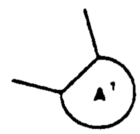

represents

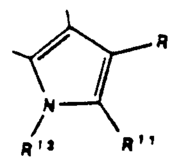

can be prepared from a compound of formula 5.5 where $R^9$, $R^{11}$ and $R^{13}$ are as previously defined:

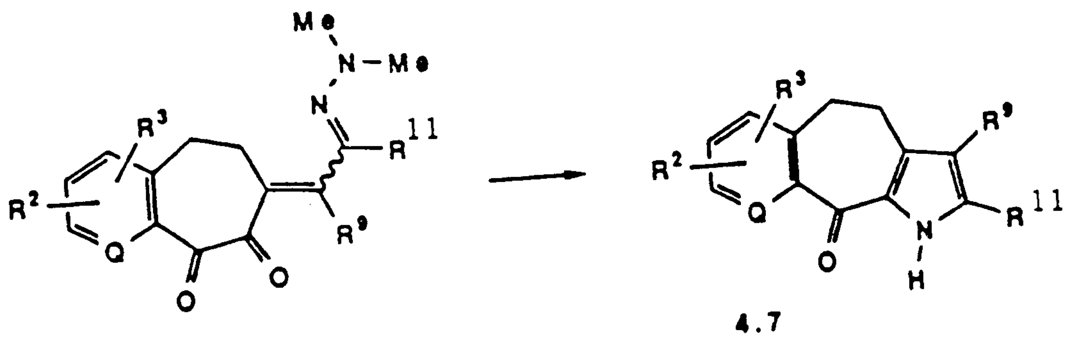

5.5 4.7

This reaction may be carried out by reducing intermediate 5.5 with an appropriate alkali metal dithionite ($M_2S_2O_4$) in a hot solvent, whereby the unisolated reduction product spontaneously cyclizes to pyrroloketone 4.7. Sodium is the preferred metal cation, and the preferred temperature is that of the boiling point of the solvent. The solvent is preferably aqueous, and contains a water-soluble alcohol, preferably a water-miscible alcohol like methanol or ethanol.

The compound of formula 5.5 can be made from a compound of formula 6.0:

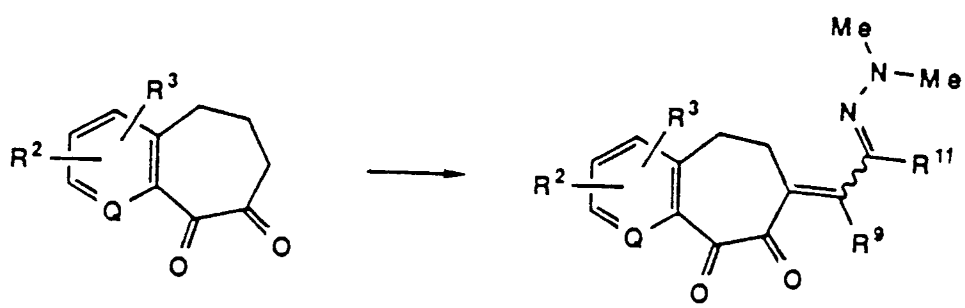

6.0 5.5

In this reaction, the intermediate α-diketone 6.0 may be treated with the monohydrazone ($O=C(R^9)-C(R^{10})=N-NMe_2$ of an α-diketone and dimethylamine. The hydrazone and 6.0 are dissolved in an anhydrous alcohol containing an alkali metal alkoxide, and allowed to react, e.g., for 15 minutes to 4 days at temperatures ranging from 0 to 60°C.

Secondary amines other than the preferred dimethylamine may also be used; other such amines are represented by $HNR^{17}R^{18}$, wherein $R^{17}$ and $R^{18}$ are as previously defined. The alcohol and alkoxide respectively used as solvent and base are preferably ethanol and ethoxide. The preferred alkali metal cation is potassium ion.

The synthesis of pyrroloketones from α-methylene ketones is well known to those skilled in the art and is, in part, the subject of review (Synthesis, 1976, 281-304). A method more recent than those of the review is taught by J. Chem. Soc. (1987, Perkin Trans. I, 2829) and by Chem. Commun. (1986, 303). Chem. Ber. (1977, 110, 491) teaches the specific method used here.

H. To prepare a compound of formula 4.0 wherein ring

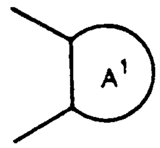

represents

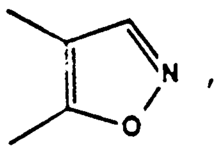

a compound of formula 5.3 is allowed to react with hydroxylamine hydrochloride:

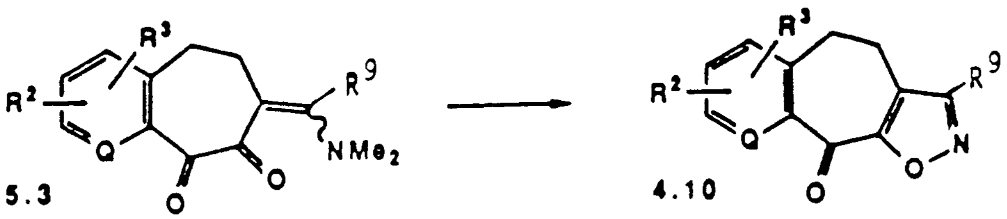

This reaction is a standard general method disclosed by the Journal of Heterocyclic Chemistry (1977, 14, 345) and by Izvest. Akad. Nauk S.S.S.R., Otdel., Khim. Nauk (1954, 47) (see also Chem. Abstr. (1955), 49, 6090i)). The reaction is carried out in a solvent which may be water, a lower alcohol, a combination of water and a water-miscible lower alcohol, or a combination of water and water-miscible ethereal solvent. Examples of water-miscible alcoholic and ethereal solvents are methanol and ethanol, and dioxane and 1,2-dimethoxyethane. Preferable solvents are, e.g., methanol and aqueous dioxane.

The reaction is carried out at temperatures ranging from 0°C to the boiling point of the solvent employed, preferably at the boiling point of the solvent.

I. To prepare a compound of formula 4.11 wherein ring

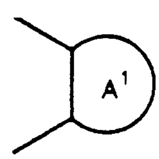

represents

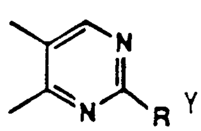

and $R^y$ represents one of H, lower alkyl, OH, O-(lower alkyl), SH, S-(lower alkyl), $NH_2$, NH-(lower alkyl), N-(lower alkyl)$_2$, and NH(C=O)-(lower alkyl), a compound of formula 5.3 is allowed to react with a compound of formula $H_2N$-C(=NH) $R^y$, in which $R^y$ is as defined above:

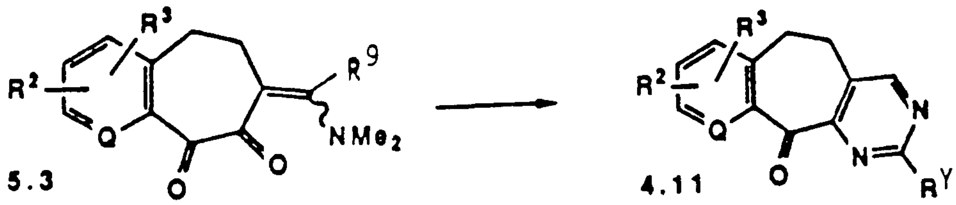

5.3 4.11

Compounds of formula $H_2N$-C (=NH)$R^y$ are commercially available or may be prepared by methods known to those skilled in the art. For example, the Aldrich Chemical Co. sells compounds in which $R^y$ is $NH_2$ (guanidine), OH (urea), H (formamidine), and $SCH_3$ (S-methylthiopseudourea).

The reaction of a compound of formula 5.3 that yields a compound of formula 4.11 is a standard method disclosed by the Journal of Heterocyclic Chemistry (1983, 20, 649) and by Chemische Berichte (1964, 97, 3397).

Compounds of formula 4.11 in which $R^y$ is S-(lower alkyl) may be transformed into compounds of formula 4.11 in which $R^y$ is H, OH, O-(lower alkyl), SH, $NH_2$, NH-(lower alkyl), N-(lower alkyl), N-(lower alkyl)$_2$, and NH(C=O)-(lower alkyl). Methods for carrying out these transformation are standard and known to one skilled in the art.

J. To prepare a compound of formula 4.12 in which

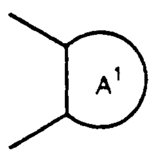

represents

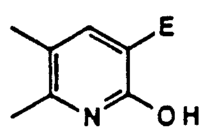

,

a compound of formula 5.3 is allowed to react with a compound of formula $H_2NC(=O)CH_2E$, in which E is taken from among CHO, C(=O)-(lower alkyl), $CO_2$-(lower alkyl), $CONH_2$, $NO_2$, CN, $SO_2CH_3$, and $CF_3$:

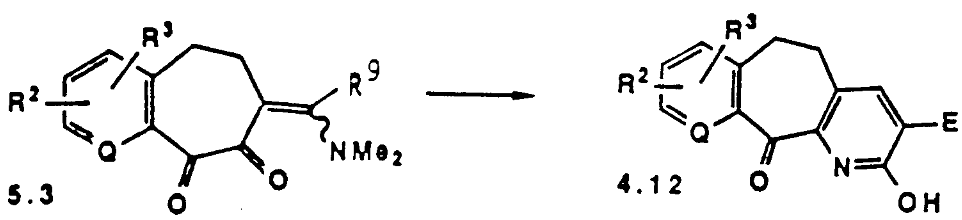

5.3 4.12

Compounds of formula $H_2NC$ (=O) $CH_2E$ are commercially available or may be prepared by methods known to those skilled in the art.

The reaction of a compound of formula 5.3 that yields a compound of formula 4.12 is a standard method disclosed by Izvest. Akad. Nauk S.S.S.R., Otdel., Khim. Nauk (1954, 47) (see also Chem. Abstr. (1955, 49, 6090i).

K. A compound of formula 4.0 wherein ring

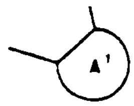

represents

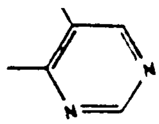

may also be prepared directly from a compound of formula 6.0:

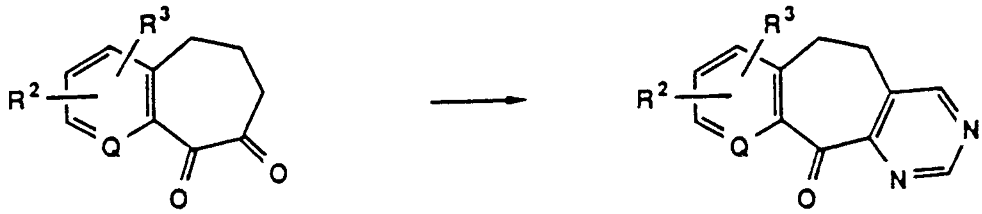

6.0

4.8

J. Chem. Soc. (1969, C, 1635) teaches how to make such pyrimidine ketones from α-methylene ketones like 6.0. The reaction requires that the latter be treated with formamide, p-toluenesulfonic acid, and N,N′,N″-methylidyne-trisformamide ($HC(NHCHO)_3$) at an elevated temperature for a time sufficient to produce the desired reaction. The preferred temperature is about 160°C.

L. A compound of formula 4.0 wherein ring

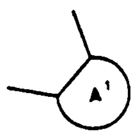

represents

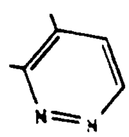

may be prepared from a compound of formula 5.6:

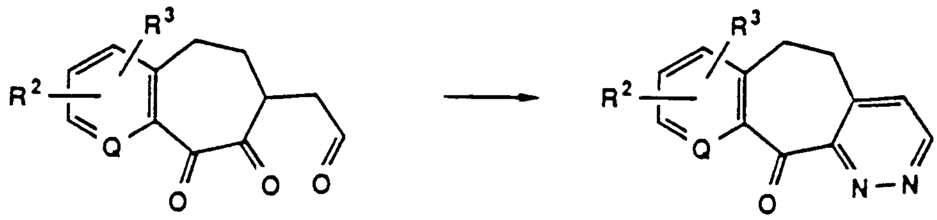

5.6 4.9

This reaction may be carried out in two steps as discussed in J. Am. Chem. Soc. (1979, 101, 766). In the first step, intermediate 5.6 is treated with hydrazine in an aqueous or in an anhydrous solvent. The preferred solvent is aqueous tetrahydrofuran. Other inert, water-soluble solvents like dioxane, 1,2-dimethoxyethane, and ethanol may also be used in combination with water. The first step may be carried out at temperatures ranging from -5 to 50°C, and the preferred temperature is 25°C. The reaction time may also be varied from a few minutes to 24 hours, depending on the particular substrate used.

When the first step is complete, as determined by monitoring, the second step is begun. An oxidizing agent is added to the foregoing solution which is then stirred in the presence of air. The preferred oxidizing agent is $PtO_2$, and other oxidizing agents may also be used. Platinum exemplifies them. The second step is carried out at temperatures ranging from about 25 to about 100°C, the maximum temperature being determined by the boiling point of the solvent used.

The compound of formula 5.6 may be prepared from a compound of formula 7.0:

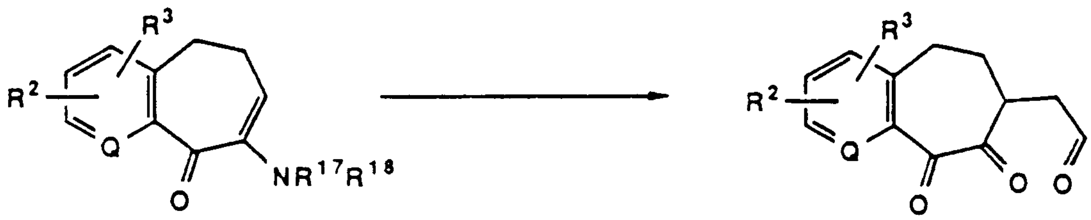

7.0 5.6

This enamine alkylation is well known to those versed in the art, and may be brought about by a standard method, e.g., "Enamines" (Chapters 4 and 8, Marcel Dekker, New York, 1969). The reaction requires α-haloacetaldehyde as a reactant, and any halogen substituent except for fluorine is useful. The preferred reactant is α-bromoacetaldehyde.

The common intermediate of formula 6.0 may be prepared by various procedures, e.g., those disclosed in J. Org. Chem (1966, 31, 3446) and J. Med. Chem. (1984, 27, 20):

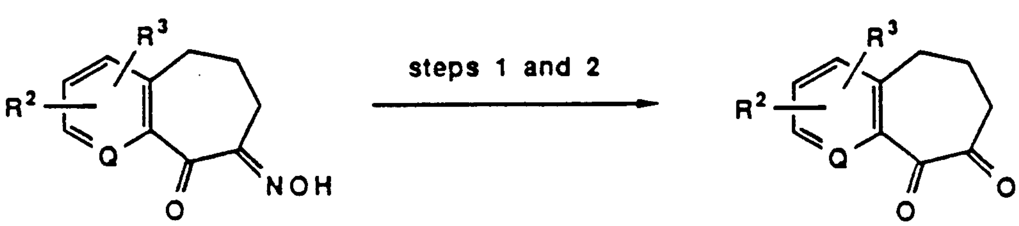


5.1 6.0

The reagent required for the first step is sodium bisulfite in an appropriate solvent such as ethanol. Another alkali metal cation may be substituted for sodium ion. Other alcohols may be used in place of ethanol. This step is preferably carried out at reflux temperature, and may be terminated if the resulting bisulfite adduct precipitates. Otherwise, the reaction, which is insensitive to long times, may be carried out for varying times.

In the second step, the precipitated bisulfite adduct is hydrolyzed to the desired α-diketone 6.0. The hydrolysis is

carried out by stirring at suitable temperature, e.g., about 25°C, a two-phase mixture of the adduct, aqueous dilute aqueous hydrochloric acid, and dichloromethane. The function of the last of these is to extract the desired compound of formula 6.0 out of water as fast as it forms; doing so may be unnecessary, however. Other dilute aqueous acids, e.g., sulfuric or acetic, may be substituted for hydrochloric acid. Any inert, water-imiscible solvent may be used in place of dichloromethane for the extraction; examples are carbon tetrachloride, chloroform, toluene, and 1,2-dichloroethane. The hydrolysis may also be carried out at temperatures ranging from 0° to 100°C, and a 25°C temperature is preferred.

A compound of formula 1.1 wherein $X^1$ represents

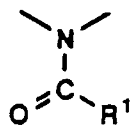

may be prepared by processes (a) or (b) below, and certain other compounds of formula 1.0, wherein Q represents CH or N→O, $X^1$ represents

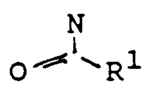

and

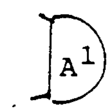

represents

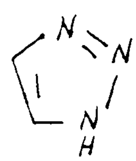

may be prepared by process (c) below.

(a) A compound of general formula 9.1 may be reacted with compound 8.1 in the presence of base to produce compounds of general structural formula 1.0 wherein $X^1$ is

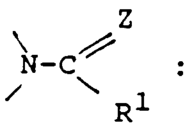

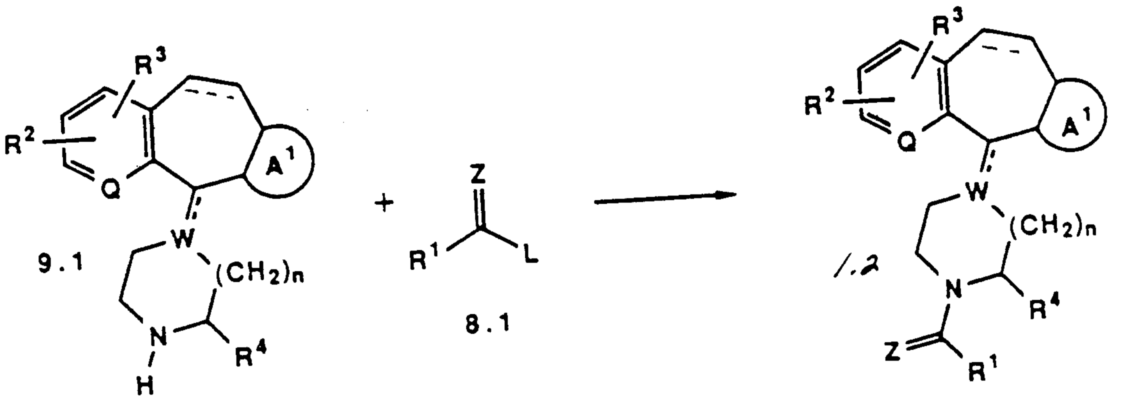

9.1

8.1

1.2

Representative examples of appropriate bases are pyridine and triethylamine. L designates a suitable leaving group. For example, if Z is O or S, a compound of formula 8.1 may be an acyl halide (e.g., L = halo) or acyl anhydride, (e.g., L is

$$-O-\overset{O}{\overset{\|}{C}}-R^1) .$$

Alternatively, if the leaving group is hydroxy a coupling reagent may be employed to form compound 1.1. Examples of coupling agents include N,N′-dicyclohexylcarbodiimide (DCC) and N,N′-carbonyldiimidazole (CDI). The leaving group may also be alkoxy, in which case the compounds of formula 1.1 may be produced by refluxing a compound of formula 9.1 with an excess of a compound of formula 8.1.

Compounds of general formula 9.1 may be prepared by cleaving the group $COOR^d$ from the corresponding carbamates 10.1, for example, via acid hydrolysis (e.g., HCl) or base hydrolysis (e.g., KOH):

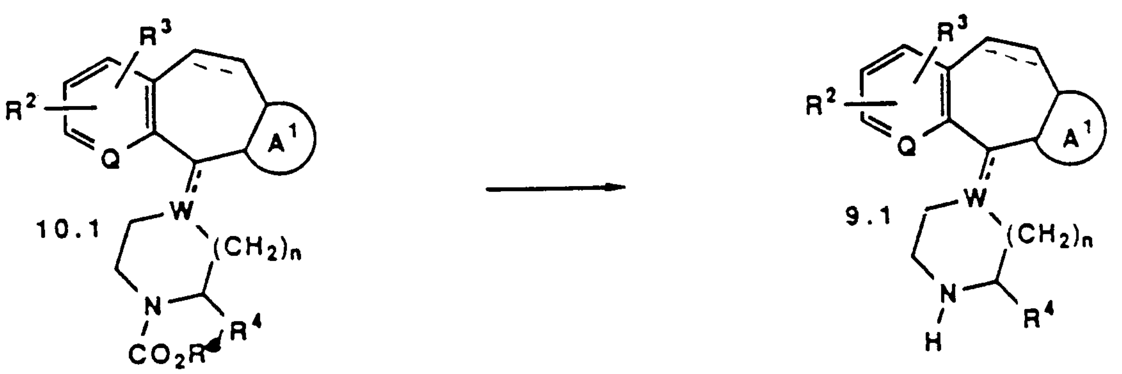

10.1

9.1

wherein $R^d$ is a group which does not prevent the cleavage reaction, e.g., $R^d$ is an optionally substituted alkyl, alkenyl, aryl or aralkyl group, such as ethyl, $Cl_3CCH_2$ or vinyl. Alternatively, depending upon the nature of $R^d$, as determined by one skilled in the art, compound 10.1 may be treated with an organometallic reagent (e.g., $CH_3Li$), a reductant or reducing agent (e.g., Zn in acid), etc., to form compounds of formula 9.1.

A compound of formula 10.1 wherein $R^d$ is $-CH_2CCl_3$ may also be converted directly into a compound of formula 1.1 without isolation of a compound of formula 9.1 by reacting such compound of formula 10.1 with Zn, $R^1CO_2H$ and heat and then with $(R^1CO)_2)$ and heat.

Compound 9.1 also may be prepared from the N-alkyl compound shown as formula 11.1 below, in the manner disclosed in U.S. patents 4,282,233 and 4,335,036.

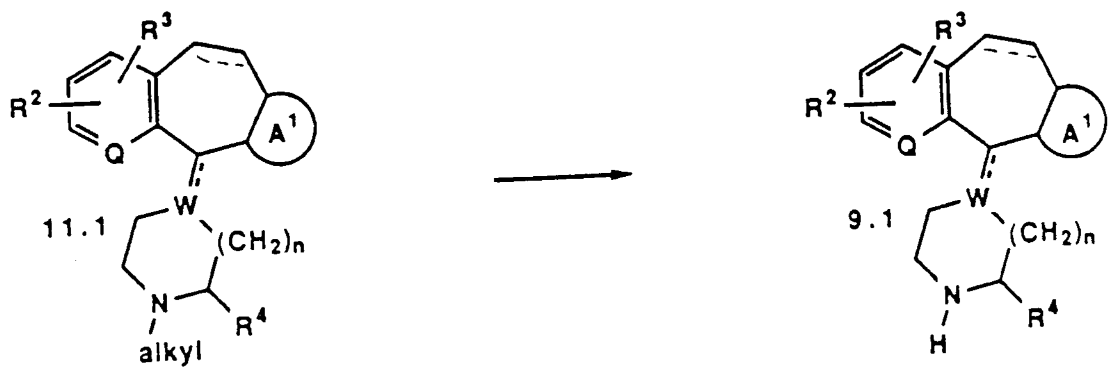

11.1 9.1

It also will be apparent to one skilled in the art that there are other methods for converting compound 11.1 to compound 9.1. For example, treatment of compound 11.1 with BrCN via von Braun reaction conditions would provide nitrile 12. Subsequent hydrolysis of the nitrile under either aqueous basic or acidic conditions or reduction of it with e.g. lithium aluminum hydride would produce compound 9.1.

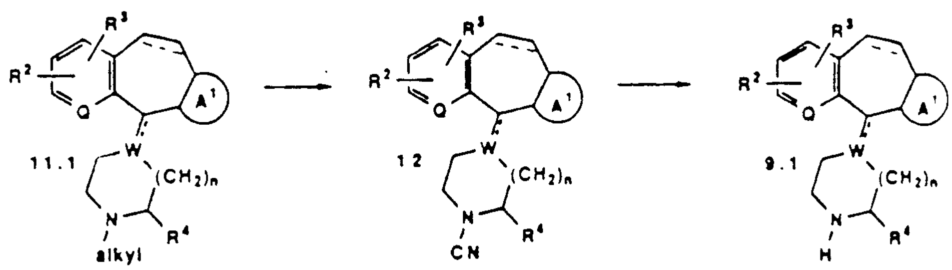

11.1 12 9.1

(b) The compounds of formula 1.2 where Z is O or S may be made by an alternative process using direct conversion of the N-alkyl compound 11.1 with an appropriate compound of formula 8.1 such as an acyl halide or acyl anhydride. Preferably, the reaction is run in the presence of an appropriate nucleophile (e.g. LiI, etc.) and solvent (e.g., toluene, dioxane or xylenes). An appropriate base, may be added, and heating may be required. Typically, a temperature ranging from 50-150°C (preferably 100-120°C) is utilized.

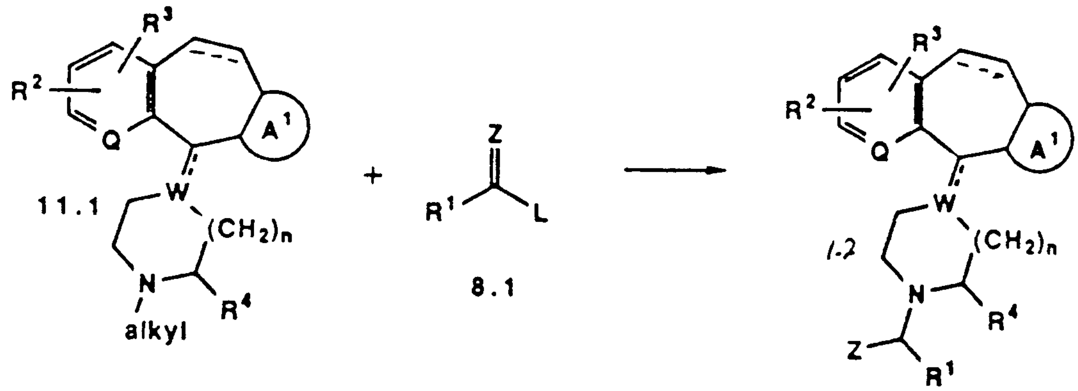

11.1

8.1

1.2

Compounds of formula 1.1 wherein $X^1$ represents

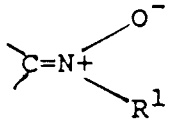

or $C=N-OR^7$ may be prepared from a compound of formula 13:

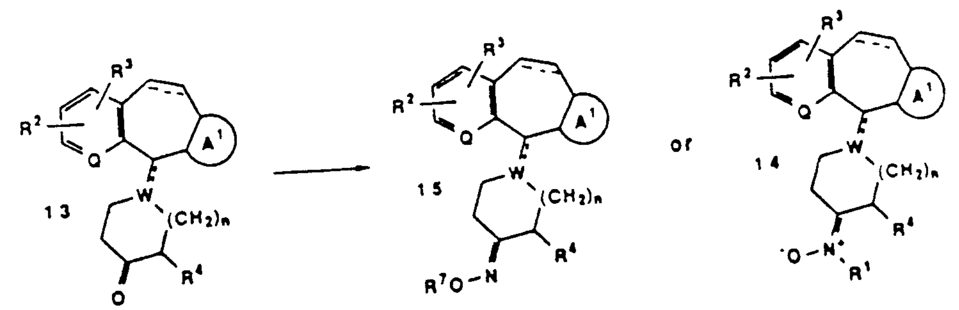

13

15

or

14

The illustrated reaction takes place between the ketone of formula 13 and 1-2 equivalents of hydroxylamine or an O-substituted hydroxylamine (i.e., $H_2NOR^7$), or an N-substituted hydroxylamine (i.e., $R^1NHOH$). Formation of an oxime (15) from hydroxylamine and an oxime ether (14) from an O-substituted hydroxylamine are reactions well known to those skilled in the art. So is the reaction of an N-substituted hydroxylamine to form the nitrone of formula 14 in which the illustrated N-atom bears a positive charge.

Each of these reactions is carried out in a solvent, e.g., an alcohol solvent, like methanol, ethanol, or butanol. Mixtures of an alcohol solvent with water are also suitable. A preferred solvent is ethanol.

The reactions may be carried out at various temperatures ranging from 0°C to that of the reflux temperature of the solvent chosen. A preferred temperature range for oxime and oxime O-ether formation is 0° to 25°C. The preferred temperature for reaction of a ketone with an N-substituted hydroxyamine is that of the reflux temperature of the solvent used.

It may be necessary to liberate the chosen reagent from an acid addition salt, if the reagent is supplied in this form. A base is needed to do so, and suitable bases are tertiary amine bases, $M_2^+CO_2^-$, and $M^+OH^-$. Pyridine, triethylamine, and diisopropylethylamine exemplify suitable tertiary amine bases, whereas the cation $M^+$ may be $Na^+$, $K^+$, or $Li^+$. These bases may be combined with the acid addition salt, the ketone, and the solvent, so as to liberate the reagent in situ and to carry out the desired condensation. Liberating the reagent in situ is the preferred method.

(c) Certain compounds of the invention having formula 1.0 in which Q represents CH or N→O, $X^1$ represents

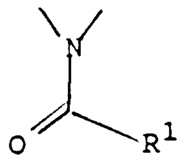

and

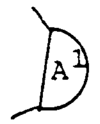

represents

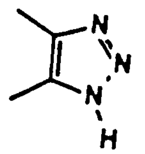

,

are prepared by allowing a compound of formula 9.2 to react with an acid:

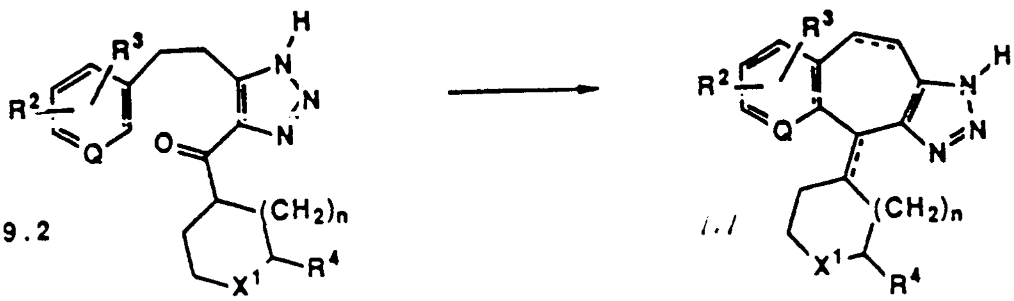

9.2

1.1

Suitable acids are mineral or strong organic acids. Concentrated sulfuric, hydrochloric, hydrobromic, hydrofluoric, and polyphosphoric acids exemplify suitable mineral acids. Trifluoromethanesulfonic acid, methanesulfonic acid, and Eaton's reagent, a mixture of methanesulfonic acid and phosphorous pentoxide, exemplify strong organic acids. A preferable acid is trifluoromethanesulfonic acid.

The reaction can be carried out at temperatures from about -10°C to +150°C, and is preferably carried out at about +25°C to +100°C.

To prepare a compound of formula 9.2, a compound of formula 100 is allowed to react with trimethylsilyl azide, which has the formula $(CH_3)_3SiN_3$ and is commercially available from the Aldrich Chemical Co.:

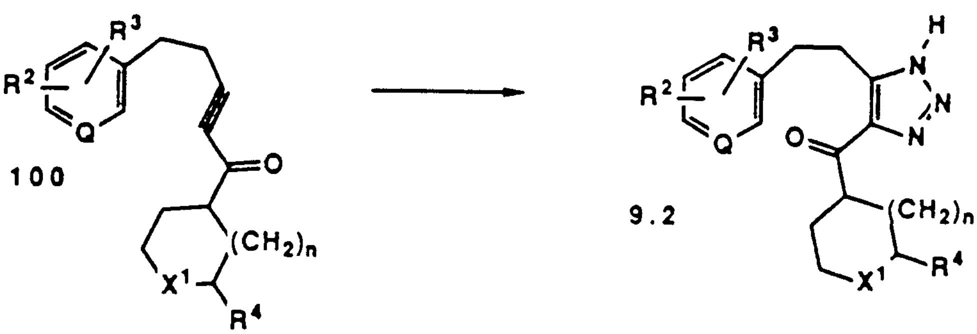

100

9.2

The reaction yielding a compound of formula 9.2 is a standard method disclosed by "1,3-Dipolar Cycloaddition Chemistry" (ed. A. Padwa, Wiley, New York, 1984, p. 628).

To prepare a compound of formula 100, compounds of formulas 101 and 102 are allowed to react with one another

in the presence of a base:

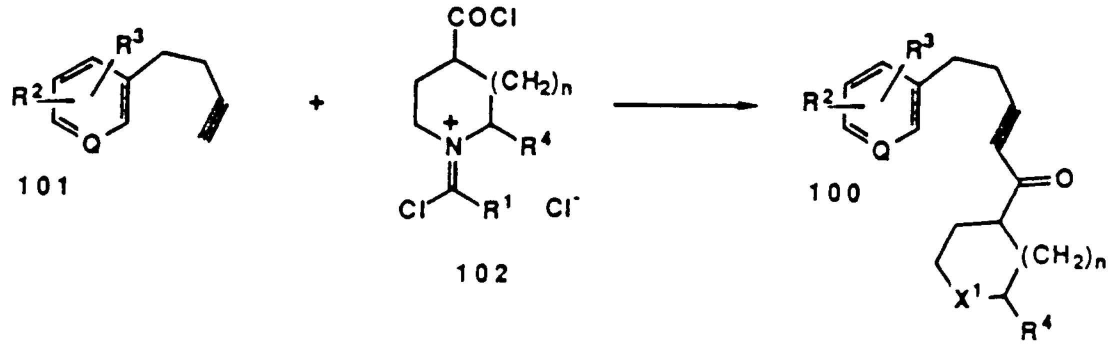

The reaction of compounds of formulas 101 and 102 represents a standard general method disclosed by Synthesis (1978,307), Bull. Soc. Chim. France (1975, 779), and Tetrahedron Letters (1970, 2659).

Compounds of formula 101 are commercially available or are made by methods known to one skilled in the art. For example, Farchan Laboratories sells a compound (4-phenyl-1-butyne) of formula 101 in which Q is CH and both $R^2$ and $R^3$ are H.

To prepare a compound of formula 102, a compound of formula 103 is allowed to react with a reagent taken from among thionyl chloride, oxalyl chloride, phosgene, phosphorous oxychloride, or phosphorous pentachloride:

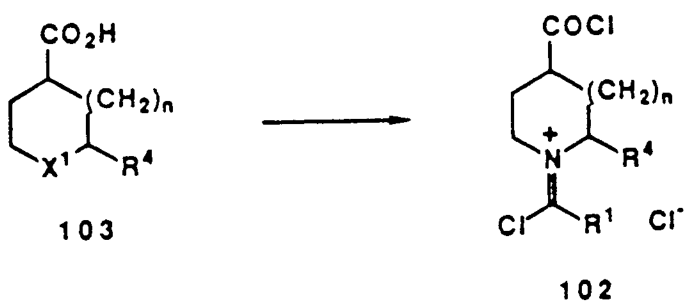

The reaction of compound 103 with the aforementioned reagent is a standard one, well known to one skilled in the art.

Compounds of formula 103 are commercially available or are made by methods known to one skilled in the art. For example, Lancaster Synthesis, Ltd., sells a compound (1-acetyl-4-piperidinecarboxylic acid) of formula 103 in which n is 1, $R^4$ is H, and $X^1$ is acetamido.

Compounds of formula 1.1 wherein

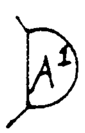

represents

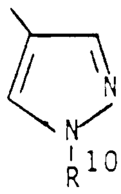 or 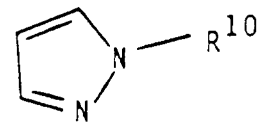

where $R^{10}$ represents acyl, aroyl and heteroaroyl may be prepared by allowing a compound of formula 200 to react with an alkali or alkaline metal hydroxide with a dilute organic or inorganic acid or with water.

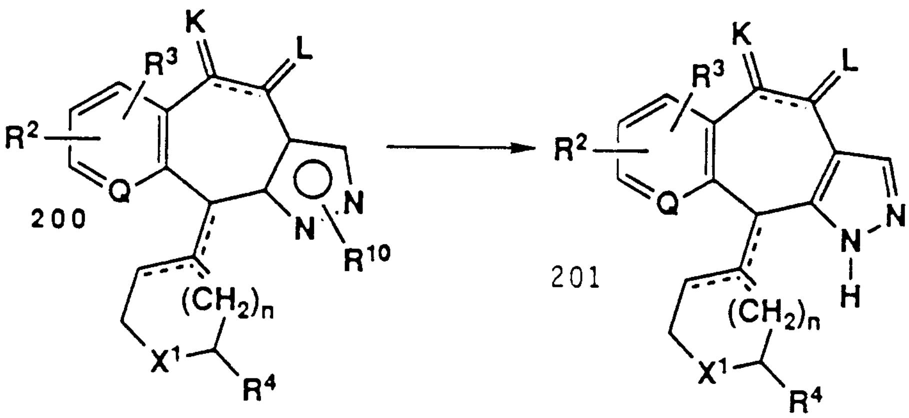

200 201

The reaction described above is described in more detail in "Heterocyclic Compounds" Chapter 5, p. 139, John Wiley and Son (1967).

The compounds of formula 18 wherein W represents C and the dotted line represents a double bond can be prepared from compounds of formula 4.0 as described below:

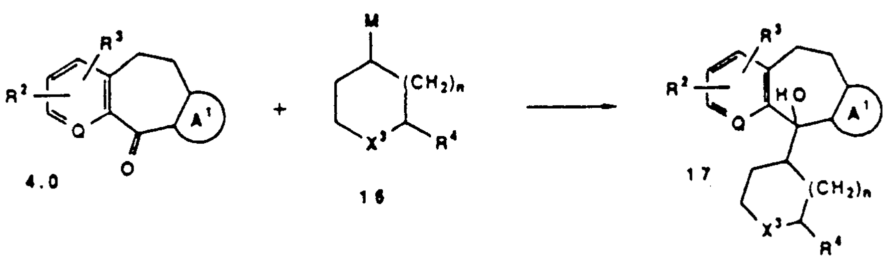

4.0 + 16 17

wherein $X^3$ represents

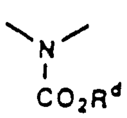 - 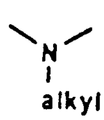 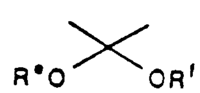 or 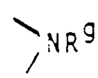

and $R^d$ is as defined above, and $R^e$ and $R^f$ each independently represent lower alkyl or together represent an alkanediyl chain of 2 or 3 methylene groups, and M is a metal, e.g., MgX (X = Cl, Br or I), Li, Na, K, etc. $R^g$ is H, or $C(Z)R^1$. These reactions generally are conducted in an inert solvent such as ether, toluene, or THF at a temperature range of about -78 to about +50°C.

A compound of formula 18 below may be prepared by reacting a compound of formula 17 with any suitable acid.

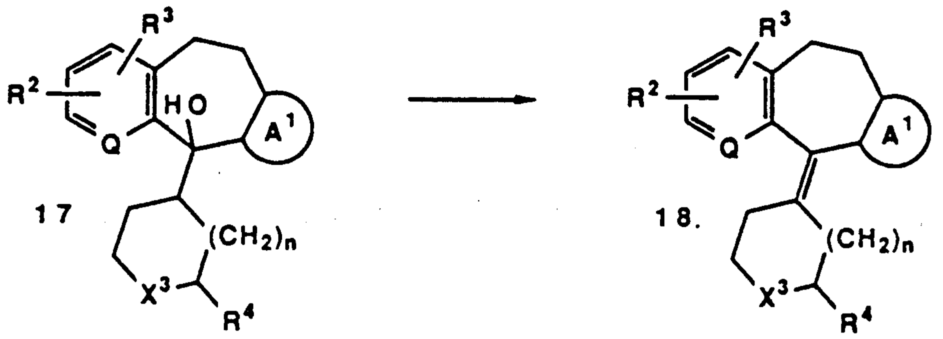

17

18.

Suitable acids include hydrochloric acid dissolved in a mixture of acetic acid and acetic anhydride. Preferably, acetyl chloride reacting with acetic acid is the source of hydrochloric acid. A preferred solvent is a mixture of excess acetic acid and acetic anhydride. A suitable temperature for dehydration is about 100°C; and the preferred temperature should be determined by monitoring. Lower temperatures avoid any side reactions. For further details of this process, see also J. Med. Chem. (1974, 17, 1316).

Compounds of formula 10.1 and 11.1 wherein W represents N and the dotted line is absent may be prepared via alkylation of the appropriately substituted compound 19 with compound 20 containing as the leaving group L the appropriately substituted halide (such as Cl, Br, I) or other similar leaving group (tosyloxy or mesyloxy). The reaction usually is conducted in an inert solvent such as THF or toluene, optionally with a base such as triethylamine or potassium carbonate, typically at a temperature range of ambient to reflux to produce compound 21:

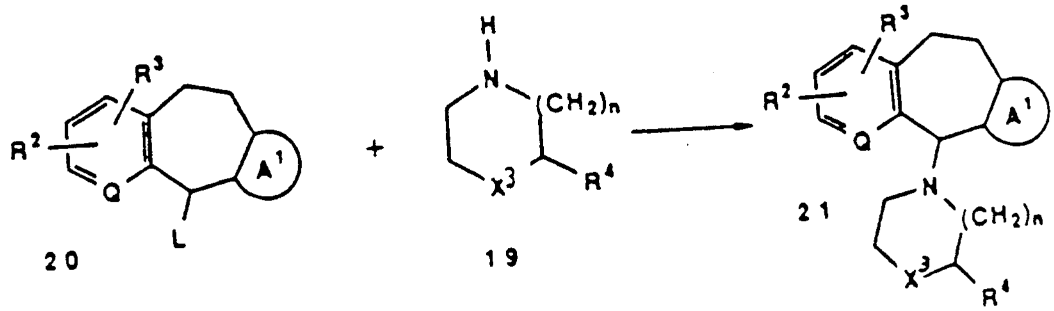

20

19

21

wherein $X^3$ represents N-$R^g$ or

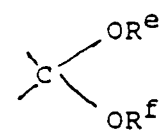

,

The preparation of compound 20 where L is Cl is analogous to the procedure described in U.S. Patent No. 3,409,621. When $R^g$ is C(Z)$R^1$ compounds of formula 1.0 are prepared. When $R^g$ is H, alkyl or $CO_2R^d$, the compounds are converted to compounds of formula 1.0 by processes previously described herein.

An alternative route for generating compound 21 is by reductive amination of the ketone 4.0, with the appropriately substituted compound of formula 19 where if $X^3$ is C(Z)$R^1$, then Z is not =S.

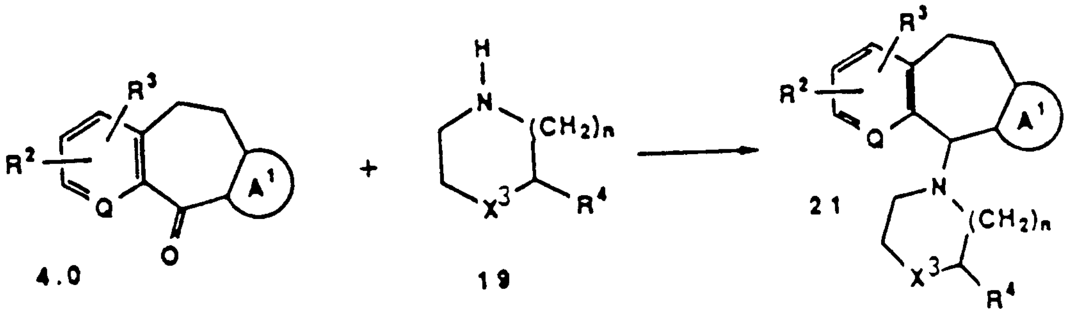

4.0 19 21

The reaction typically is carried out in a polar solvent, such as methanol or ethanol optionally in the presence of a dehydrating agent such as 3Å molecular sieves. In the first step of the reaction, an iminium salt forms and, in a second step, is reduced to compound 21 by employing a variety of reducing agents such as $Na(CN)BH_3$ or catalytic hydrogenation, for example, hydrogen over Pd/C.

To prepare compounds of formula 13, the compounds of formula 22, are treated with diluted aqueous acid:

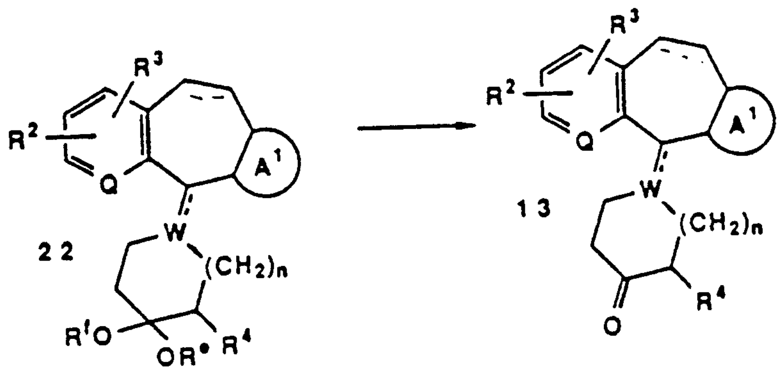

22 13

Suitable acids include toluenesulfonic acid, trifluoroacetic acid, hydrochloric acid, sulfuric acid, etc. Any suitable temperature may be employed, e.g., from 0°C to the boiling point of the reaction mixture. A water miscible cosolvent may be employed such as tetrahydrofuran, acetone, methyl ethyl ketone, etc.

SUBSTITUTION ON THE TWO-CARBON BRIDGE

The following process may be employed to produce compounds of structural formulas 1.0 and 2.0 substituted at one of the bridge carbon atoms. In the compounds drawn below, the substitution group has a bond drawn into the cycloheptane ring between the bridging atoms, rather than to a specific bridge carbon atom. This is used to indicate that attachment of the substitution group may be to either bridge carbon atom. For example, the methoxy group of compound 24 below may be attached to either of the bridge carbons and the corresponding carbonyl group on the bridge of compound 27 will be positioned at the same carbon.

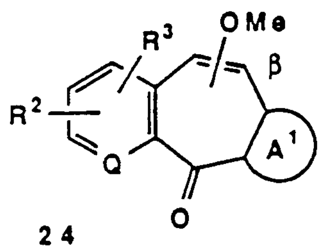

24

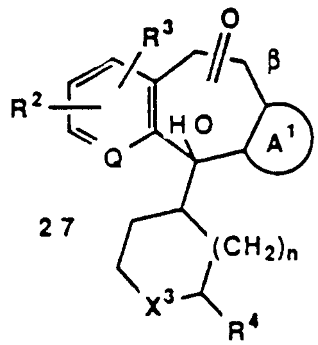

27

One bridge carbon (α or β) of compound 4,0., 4.0, which is analogous for present purpose to the compounds disclosed in U.S. Patent 3,326,924, is first brominated with an appropriate brominating agent, such as N-bromosuccinimide (NBS) in the presence of an initiator, such as azobisisobutyryl nitrile (ABIN), benzoyl peroxide or the like in an inert solvent, such as $CCl_4$, benzene or a similar solvent. Heat or light may be required to initiate the reaction. The bromine on the bridge carbon may then be eliminated with base to form the olefinic compound 23. Examples of suitable bases for elimination include diazabicycloundecane (DBU), diazabicyclononane (DBN) and diazabicyclooctane (DABCO). Elimination is typically performed in an inert solvent at reflux temperature. Examples of suitable inert solvents include $CH_2Cl_2$, $CCl_4$, toluene, tetrahydrofuran (THF), dioxane, and $CHCl_3$, with $CHCl_3$ being preferred.

Alternatively, compound 4.0 may be refluxed in the presence of an oxidizing agent to yield compound 23. Representative examples of oxidizing agents suitable for oxidizing compound 4.0 include 2,3-dichloro-5,6-dicyano-1,4-quinone (DDQ) and $SeO_2$.

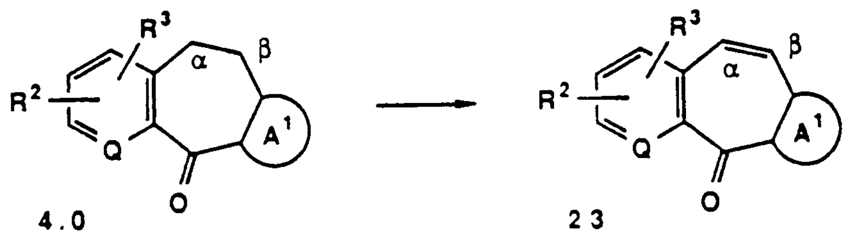

4.0 23

Compound 23 may be converted to compound 24 by adding excess powdered $AgNO_3$ in methanol, followed by the addition of excess $Br_2$, which bromoetherificates the unsubstituted bridge carbon atoms. The bridge-substituted bromine is then eliminated with excess base, such as DBU to provide a compound of formula 24. The reaction may be run in an inert solvent such as $CHCl_3$ at reflux temperature. The resultant isomeric mixture may be separated by column chromatography or any other appropriate method.

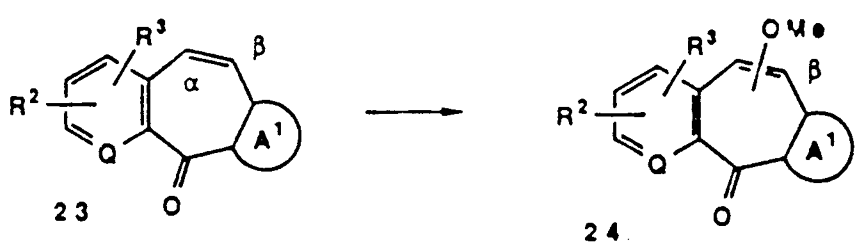

23 24.

A compound of formula 26 is prepared by treating a compound of formula 24 with a Grignard reagent 25 or similar metalated reagent in an inert solvent, such as ether, benzene, or tetrahydrofuran (THF). Compound 25 is prepared in a known manner from magnesium and the appropriate chloro derivative of compound 16. The reaction mixture may be refluxed, if necessary, after which it may be quenched with $NH_4Cl$ to form compound 26.

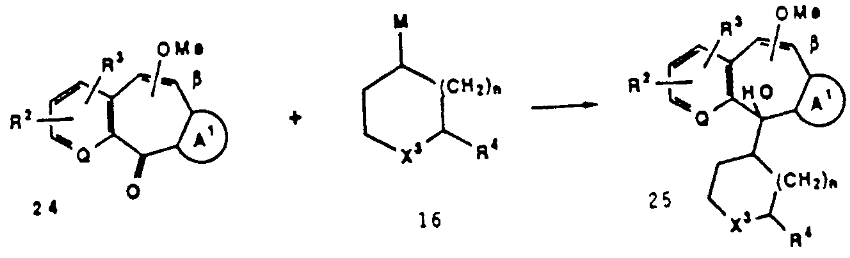

24 16 25

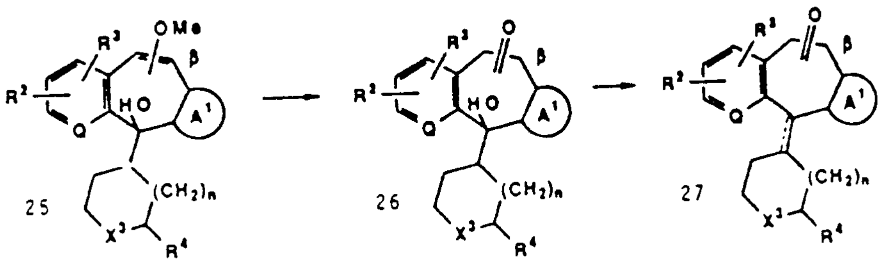

25 26 27

A compound of formula 25 may be hydrolyzed with any strong, aqueous acid, for example, 80-95% $H_2SO_4$ or HCl, having a pH less than 1, at a temperature not higher than room temperature for not generally longer than one hour to produce an intermediate compound of formula 26.

After complete hydrolysis, compound 26 may be dehydrated with $CF_3SO_3H$ (triflic acid) or a similar acid to yield compound 27. Examples of other acids for dehydrating compound 26 include, for example, $HF/BF_3$, $CH_3SO_3H/BF_3$, and a mixture of acetyl chloride, acetic acid and acetic anhydride, etc. The reaction can be performed in the absence of or with an inert co-solvent such as $CH_2Cl_2$. The temperature and time of the reaction vary with the acid employed. When triflic acid is used, the temperature may be controlled to minimize side reactions.

Compound 27 can, if necessary, then be converted to compounds of the invention as previously described.

Ketone 24 can be reduced to the corresponding alcohol 28 using a variety of reducing agents (e.g. $NaBH_4$ in MeOH, $LiAlH_4$ in ether). The alcohol can then be converted to an appropriate leaving group (L) such as halide (e.g. Cl, Br, I) or other derivative (e.g. tosyloxy) thereby providing compound 29. For example, the chloride of 29 (L=Cl) can be obtained from the alcohol 28 using $SOCl_2$ in an inert solvent such as toluene.

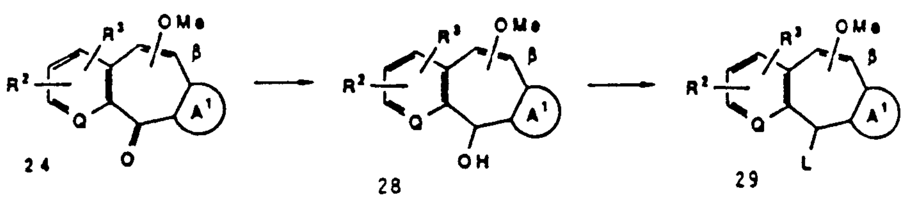

24 28 29

Alkylation of the appropriately substituted compound 19 with 29 then provides 30 below. The reaction is usually conducted in an inert solvent such as THF or toluene, optionally with base, such as triethylamine or potassium carbonate, typically at ambient to reflux temperature.

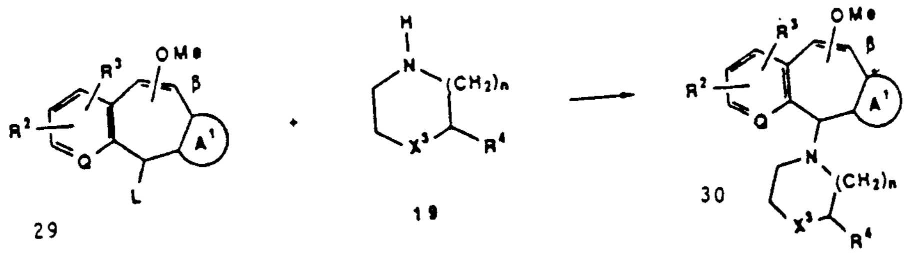

29 19 30

Compound 30 can then be hydrolyzed with any strong aqueous acid, for example 80-95% $H_2SO_4$ or HCl, to provide the desired keto compound 31.

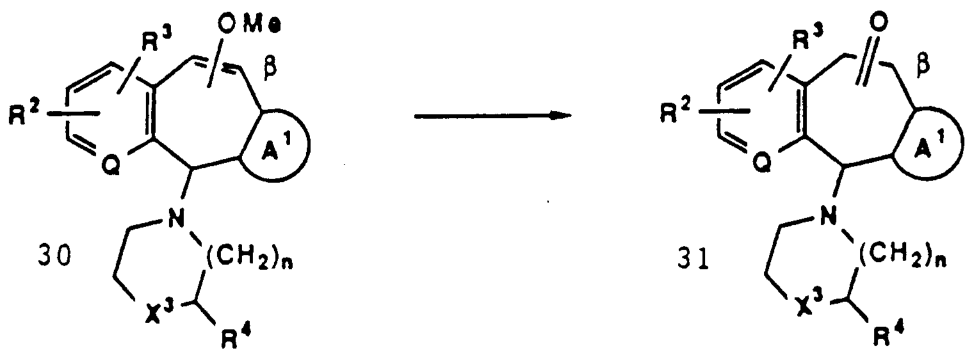

30 31

The bridge carbonyl of compound 27 may be reduced to a hydroxy group by treating either compound with an appropriate reducing agent, such as $NaBH_4$ in $CH_3OH$ or $LiAlH_4$ in ether to produce a compound of formula 32.

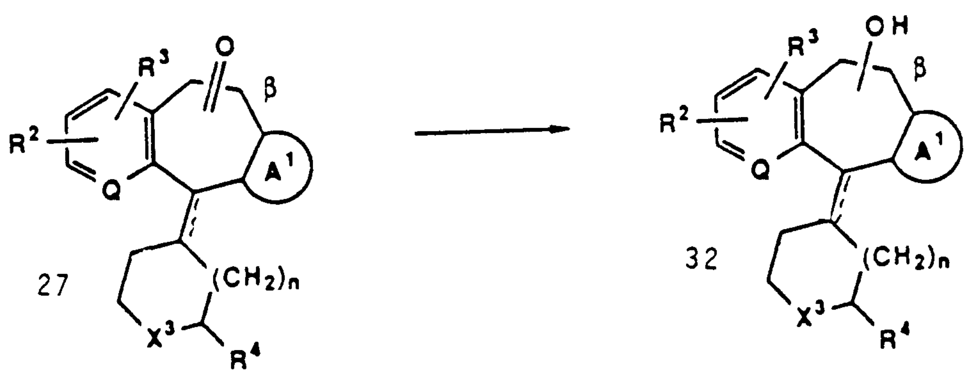

27 32

The compounds of formulas 27 and 32 may be converted, if necessary, e.g., where $X^3$ represents N-alkyl or N-$COOR^d$, to compounds of formula 1.1 by the procedures described above.

Compounds of formula 1.1 wherein L and/or K represents -H, -alkyl or alkyl, alkyl (e.g., two methyl groups on one of the bridging carbons $\alpha$ or $\beta$) may be prepared as described below:

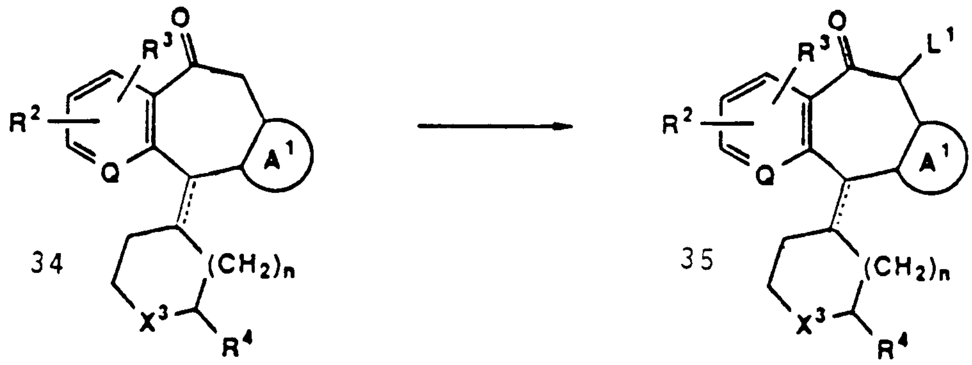

34 35

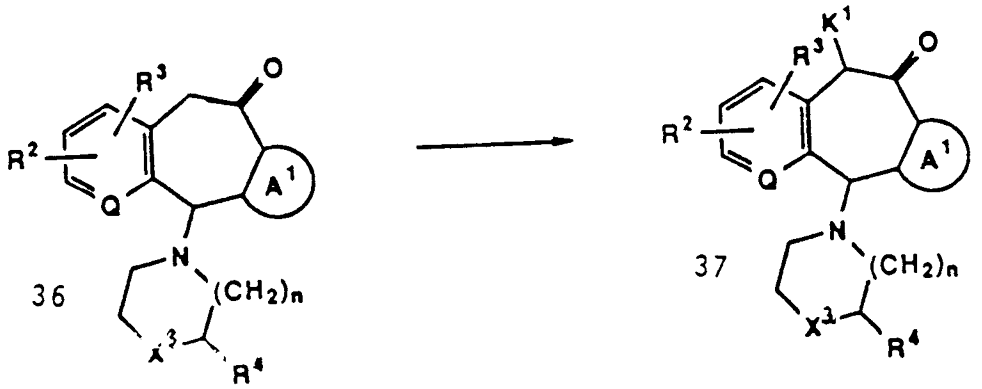

36 37

wherein $L^1$ and $K^1$ each represent -H, -alkyl or alkyl, alkyl.

To prepare compounds 35 and 37, compounds of formulas 34 and 36, respectively, are allowed to react in a suitable solvent with an alkyl halide and a base. Suitable solvents include 1,2-dimethoxyethane, tetrahydrofuran (THF), N,N-dimethylformamide (DMF), dimethylsulfoxide (DMSO), tertiary-butanol and the like. Bases may be chosen from among sodium hydride, $M^+$ t-butoxide (in which $M^+$ is $K^+$, $Na^+$, or $Li^+$), lithium diisopropylamine, lithium bistrimethylsilylamide, among others. Suitable alkyl halides include alkyl chlorides, bromides, and iodides; and alkyl iodides are preferred. The reaction may be carried out at temperatures ranging from -40°C to the reflux temperature of the solvent employed. A preferred temperature is about 50°C.

The carbonyl groups of compounds of formulas 35 and 37 may be reduced to the corresponding alcohols, for example, by any suitable reductant, such as $NaBH_4$. These carbonyl groups in compounds 35 and 37 may also be reduced to the corresponding methylene groups by other suitable reductive methods. For example, a suitable method is to treat the carbonyl groups of compounds 35 and 37 with ethanedithiol in toluene or benzene containing a catalytic amount of toluenesulfonic acid; then the resulting thioketals are reduced to the methylene compounds with Raney nickel in an alcohol solvent, like ethanol.

The compounds of the invention where Q represents N→O and/or W represents N→O may be prepared by reacting an appropriate compound of formula 1.0, wherein Q is N and/or W is N with an appropriate oxidizing agent, e.g. a peracid. Suitable oxidizing agents include $CF_3CO_3H$, aryl-$CO_3H$, $CH_3CO_3H$, etc. The reaction may be performed in a suitable solvent such as $CH_2Cl_2$, $CHCl_3$, $CCl_4$, 1,2-dichloroethane, etc. and at any suitable temperature, e.g., 0°C to reflux, preferably about 25°C. If the temperature exceeds about 25°C a stabilizer may be employed such as 4,4′-thiobis-6-(tertiary butyl)-m-cresol.

To make a compound of formula 1.1 in which Z=S, a compound of formula 1.1 where Z is oxygen is reacted with $P_2S_5$, Lawesson's reagent, or another reagent capable of introducing sulfur in place of oxygen.

The reaction may take place at elevated temperature in pyridine, toluene or other suitable solvents. Lawesson's reagent has the formula

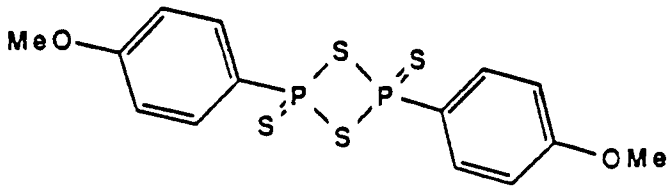

In this and other reactions, numerous conversions of a compound of formula I (Z = 0) to another compound of formula I (Z = S) are possible.

As is apparent to one skilled in the art, depending on substituents already present in the molecule e.g., different $R^2$, $R^3$, $R^4$, K, L etc.), one or more such substituents may have to be protected during some of the reactions above. For example, the groups listed in column 1 of the following table may be protected as indicated in column 2 of the table. It is also readily apparent that, subsequent to the above reactions, certain final compounds can be transformed into other final compounds by trivial reactions well known in the art.

| 1. Group to be Protected | 2. Protected Group |
|---|---|
| -COOH | -COOalkyl, -COObenzyl, -COOphenyl |
| >NH | >N-$CO_2$alkyl, >N-$CO_2$benzyl, >N-$CO_2CH_2CCl_3$ |
| >CO | 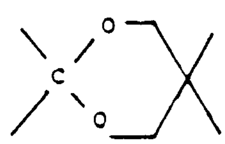 , 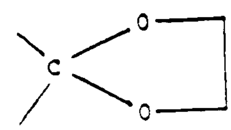 |
| -OH | 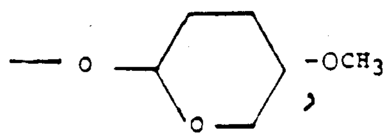 , |
| -$NH_2$ | 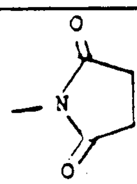 |

The compounds of formulas 1.1 and 2.0 possess platelet-activating factor ("PAF") antagonistic properties. The compounds are, therefore, useful when PAF is a factor in the disease or disorder. This includes allergic diseases such as asthma, adult respiratory distress syndrome, urticaria and inflammatory diseases such as rheumatoid arthritis and osteoarthritis. For example, PAF is an important mediator of such processes as platelet aggregation, smooth muscle contraction (especially in lung tissue), vascular permeability and neutrophil activation. Recent evidence implicates PAF as an underlying factor involved in airway hyperreactivity, shock, edema, hypersensitivity, disseminated loss of platelets by pregnant women, and in diseases associated with implantation of embyro <u>in utero</u>, in particular.

The PAF antagonistic properties of the compounds of formulas 1.1 and 2.0 may be demonstrated by use of standard pharmacological testing procedures as described below. These test procedures are standard tests used to determine PAF antagonistic activity and to evaluate the usefulness of said compounds for counteracting the biological effects

of PAF. The in vitro assay is a simple screening test, while the in vivo test mimics clinical use of PAF antagonists to provide data which simulate clinical use of the compounds of formulas 1.1 and 2.0 described herein.

A. PAF Antagonism Assay

In vitro Assay:

Preparation of platelet-rich plasma (PRP): Human blood (50 ml) is collected from healthy male donors. The blood is mixed with an anticoagulant solution (5 ml) containing sodium citrate (3.8%) and dextrose (2%). Blood is centrifuged at 110 X g for 15 min. and the supernatant PRP is carefully transferred into a polypropylene tube. Platelet-poor-plasma (PPP) is prepared by centrifuging PRP at 12,000 X g for 2 min. in a Beckman Microfuge B. PRP is used within 3 hours of drawing the blood.

Platelet Aggregation Assay: When an aggregating agent such as PAF is added to PRP, platelets aggregate. An aggregometer quantifies this aggregation by measuring light (infra-red) transmission through PRP and comparing it to transmission through PPP. The aggregation assays are performed using a dual-channel aggregometer (Model 440, Chrono-Log Corp., Havertown, PA). PRP (0.45 ml) in aggregometer curettes is continually stirred (37°C). Solutions of test compounds or vehicle are added to the PRP, and, after incubation for 2 min., 10-15 µl aliquots of pAF solution are added so as to achieve a final concentration of 1-5 X $10^{-8}$M. Incubations are continued until the increase in light transmission reaches a maximum (usually about 2 min). Values for inhibition are calculated by comparing maximal aggregation obtained in the absence and the presence of the compound. For each experiment, a standard PAF antagonist, such as alprazolam, is used as a positive internal control. The inhibitory concentration is the concentration of compound in micromoles at which the indicated % inhibition of the aggregation is found, as measured by the light transmission through each sample of PRP as compared to that through each sample of PPP. Table I below presents data for PAF aggregate inhibitory concentrations.

TABLE 1

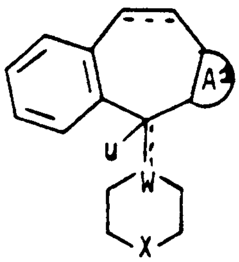

| | $A^1$ | 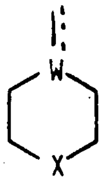 | Bridge Double Bond | U | Dose (μm) | Inhibition (%) |
|---|---|---|---|---|---|---|
| 1 | 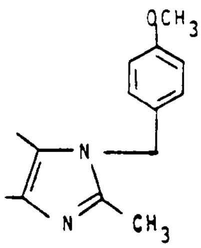 | 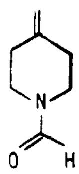 | No | - | 50 | 49 |
| 2. | 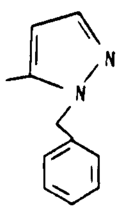 * | 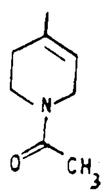 | Yes | H | 50 | 100 |
| | | | | | 5 | 97 |
| | | | | | 2.5 | 63 |
| | | | | | 1.2 | 51 |
| 3. | 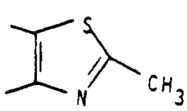 | 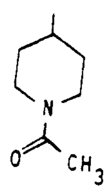 | No | -- | 0.5 | 80 |
| | | | | | 0.25 | 63 |
| | | | | | 0.1 | 36 |

*The product group is attached to one of the nitrogens. The regioisomeric structure has not been determined.

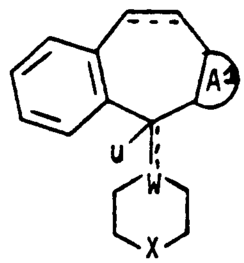

| | $A^1$ | 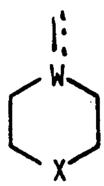 | Bridge Double Bond | U | Dose (μm) | Inhibition (%) |
|---|---|---|---|---|---|---|
| 4. | 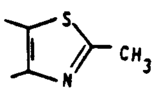 | 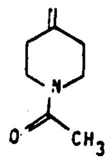 | No | — | 50<br>25<br>12 | 100<br>71<br>29 |
| 5. | 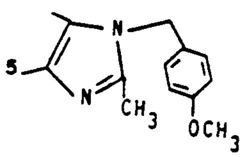 | 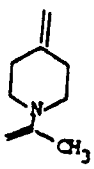 | No | — | 50 | 67 |
| 6. | 6. 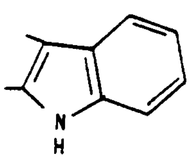 | 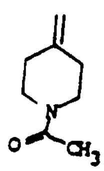 | No | OH | | |
| 7. | 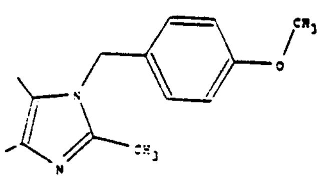 | 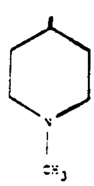 | No | H | 50<br>25 | 84<br>42 |

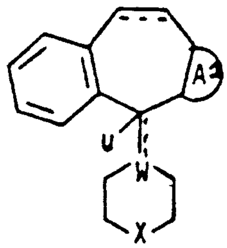

| $A^1$ | 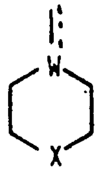 | Bridge Double Bond | U | Dose (µm) | Inhibition (%) |
|---|---|---|---|---|---|
| 8. 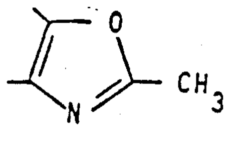 | 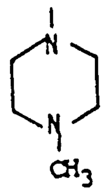 | No | — | 50 | 100 |
| | | | | 5 | 78 |
| | | | | 2.5 | 51 |
| 9. 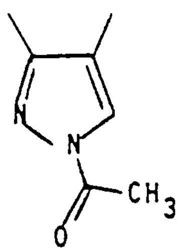 | 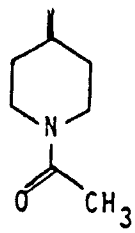 | No | — | 12.5 | 89 |
| | | | | 5 | 54 |
| | | | | 2.5 | 43 |
| | | | | 0.5 | 8 |

PAF is also a known bronchoconstrictive agent in mammals. Hence, PAF antagonism can be evaluated by measuring inhibition by the compounds of the invention in PAF-induced bronchoconstriction in guinea pigs.

B. PAF-Induced Bronchospasm in Guinea Pigs

In Vivo Assay

Non-sensitized guinea pigs are fasted overnight, and the following morning are anesthetized with 0.9 ml/kg i.p. of dialurethane (0.1 g/ml of diallybarbituric acid, 0.4 g/ml of ethylurea and 0.4 g/ml of urethane). The trachea is cannulated and the animals are ventilated by a Harvard rodent respirator at 55 strokes/min. with a stroke volume of 4 ml. A side arm to the tracheal cannula is connected to a Harvard pressure transducer to obtain a continuous measure of intratracheal pressure, which is recorded on a Harvard polygraph. The jugular vein is cannulated for the administration of compounds. The animals are challenged i.v. with PAF (0.4 ug/kg in isotonic saline containing 0.25% bovine serum albumin (BSA)) and the peak increase in inflation pressure that occurred within 5 min. after challenge is recorded. Test compounds are administered either orally (2 hrs. prior to PAF as a suspension in 0.4% methylcellulose vehicle) or intravenously (10 min. prior to PAF as a solution in dimethylsulfoxide).

Data for PAF-induced bronchospasm in guinea pigs is presented in Table II below:

TABLE II

| 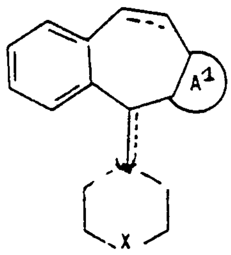 A¹ | 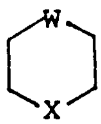 | Bridge Double Bond | U | Dose mg/kg | Inhibition (%) |
|---|---|---|---|---|---|
| 1. 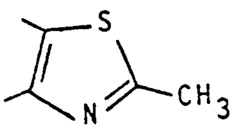 | 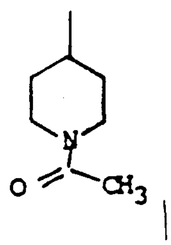 | Yes | H | 10 | 9 |
| 2. 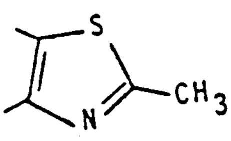 | 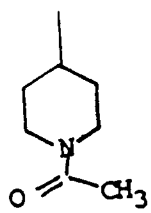 | No | H | 10 | 38 |
| 3. 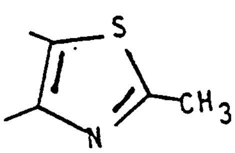 | 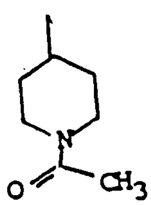 | No | — | 10 | 16 |

C. Antihistamine Activity Assay

Prevention of Histamine-Induced Lethality in Guinea Pigs. The compounds of formula 1.1 also possess antihistaminic properties which may be assessed by test procedure C below. Test procedure C, "Prevention of histamine-induced lethality", demonstrates basic antihistaminic activity of representative compounds of formula 1.0. Protection against histamine lethality is indicative of strong antihistaminic properties. Compounds may be evaluated for antihistamine activity by their ability to protect female albino guinea pigs (250-350 g) against death induced by the intravenous injection of histamine dihydrochloride at 1.1 mg/kg, which is approximately twice the $LD_{99}$. Doses of the antagonists are administered orally to separate groups of fasted animals 1 hour prior to the challenge with histamine and protection from death is recorded for 30 minutes after histamine. $ED_{50}$ values are determined for each drug by probit analysis.

The PAF-antagonist and antihistamine dosage of the compounds of formulas 1.1 and 2.0 will vary depending upon the severity of the condition being treated, the compound employed, etc. A typical recommended dosage is from about .01 to 1000 mg/kg, preferably from about 10 mg/kg to about 100 mg/kg, preferably orally.

Compounds 3.0 of this invention are useful for the treatment of inflammation; thus, they are useful for the treatment of arthritus, bursitis, tendonitis, gout, and other inflammatory conditions. The antiinflammatory use of these compounds may be demonstrated by test procedure (D), "Inhibition of Cellular and Fluid Influx to Rat Pleural Cavity", as set forth below. Protection against cellular and fluid influx strongly indicates antiinflammatory properties.

D. Antiinflammatory Activity Assay

Inhibition of Cellular and Fluid Influx to Rat Pleural Cavity. Groups of 4 male rats are injected in the penile vein with antigen (1 mg BSA in 0.2 mL of saline per rat) and 0.5 hour later injected in the pleural cavity with antibody (1.0 mg antibody protein in the IgG fraction of rabbit anti-BSA in 0.2 mL). Sham control animals are treated as RPAR animals but do not receive BSA antigen. After 4 hours the animals are killed with $CO_2$, and the pleural cavities are opened and the exudate is drained into a graduated conical glass centrifuge tube containing indomethacin (1.8 ug) and nordihydroguaiaretic acid (NDGA) (15 ug) to block ex vivo metabolite synthesis. The volume of the exudate is measured. The cavity is then washed out with saline-EDTA to achieve a final volume of 5.0 mL. The number of cells is determined in a Coulter Counter. The cells are spun-down (1000 x g) and the exudate supernatant is added to 4 volumes of 95% ethanol and samples are kept on ice for 30 minutes. After removal of the protein precipitate (2,500 x g) the ethanol extract of the exudate is dried under $N_2$ and then is stored at -20°C. For radioimmunoassay analysis the samples are redissolved in water to a volume of 1 mL per rat. The recoveries of $^3$H-$TXB_2$ (tritiated thromboxane $B_2$) and $LTE_4$ (leukotriene $E_4$) from cell free exudate are 84 ± 2 (SEM) and 89 ± 2% (N=4), respectively. These samples are directly assayed in duplicate with a commercial $^3$H-$TBX_2$ radioimmunoassay kit from New England Nuclear and with a $^3$H-$LTC_4$/$D_4$/$E_4$ kit from Amersham. 11,12-Dihydrobenzo[5,6]cyclohepta[1,2-b]indol-6(5H)-one (Compound E) at 25 mg 1 kg p.o. reduced cellular and fluid influx to the pleural cavity in such procedure by 65 and 25%, respectively.

The compounds of formula 3.0 can be administered by any therapeutically useful method, such as orally, topically or parenterally, in single or divided daily doses. When used orally or parenterally for the treatment of inflammation, the compounds of formula 3.0 can be administered in an amount ranging from about 0.01 mg/kg to about 100 mg/kg, preferably from 0.1 mg/kg to about 10 mg/kg per day.

Determination of the proper dosage of a compound of formula 1.0, 2.0 or 3.0 for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the compounds of formulas 1.0, 2.0 and 3.0 and the pharmaceutically acceptable salts thereof will be requlated according to the judgement of the attending clinician considering such factors as age, condition and size of the patient as well as severity of the symptom being treated. A typical recommended dosage regimen is oral administration of from 10 mg to 1500 mg/day preferably 10 to 750 mg/day, in two to four divided doses to achieve relief of the symptoms.

Compounds of formula 1.0 may be administered by any suitable mode, e.g., orally, parenterally, intravenously, topically, etc., as explained further below, depending upon the allergic or inflammatory condition being treated.

For preparing pharmaceutical compositions from the compounds of formula 1.0, the compounds may be mixed with inert, pharmaceutically acceptable carriers which can be either solid or liquid. Solid form preparations include but are not limited to powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The active ingredient contained in the powders or tablets preferably ranges from about 5 to about 70 percent of the tablet or powder weight. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parental injection. Liquid preparations can also be formulated in solution in aqueous polyethylene glycol solution.

Liquid form preparations may also include solutions for intranasal administration.

Aerosol preparations suitable for inhalation may also include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners, solubilizing agents and the like.

The compounds of formula 1.0 may be applied topically, e.g., to the skin, eyes, nose, or lungs. Dermatitis, urticaria, acne, and psoriasis exemplify skin conditions in which the compounds are useful. Conjunctivitis, rhinitis, and asthma are examples of diseases or conditions in which the compounds may be advantageously applied to eyes, nose, and lungs, respectively.

Formulations for topical application, e.g., for use in treating psoriasis, may include the above liquid forms, creams, aerosols, sprays, dusts, powders, lotions, drops and ointments which are prepared by combining an active ingredient according to this invention with conventional pharmaceutical diluents and carriers commonly used in topical dry, liquid, cream and aerosol formulations.

The topical pharmaceutical compositions according to the invention may also contain other active ingredients such as antimicrobial agents, particularly antibiotics, anesthetics, analgesics and antipruritic agents.

The compounds of formula 1.0 may also be deliverable transdermally. The transdermal compositions can take the form of creams, lotions, aerosols and/or emulsions and can be included in a transdermal patch of the matrix or reservoir type as is conventional in the art for this purpose.

Preferably, the compounds of formula 1.0 are administered orally.

The pharmaceutical preparation is preferably in unit dosage form. In such form, the preparation is subdivided into unit doses containing appropriate quantities of the active component, e.g., from 0.1 to 1000 mg, preferably from 1 mg to 100 mg, according to the particular application. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The following examples are intended to illustrate, but not to limit, the present invention.

## EXAMPLE 1

### 1-Acetyl-4-(9,10-dihydro-4H-benzo[5,6]cyclohept[1,2-d]oxazol-4-ylidene)-piperidine

Add zinc dust (0.338g) to a hot (70°C) solution of trichloroethyl 4-(9,10-dihydro-2-methyl-4H-benzo[5,6]cyclohepta[1,2-d]oxazol-4-ylidene)-1-piperidinecarboxylate (1.18 g) in acetic acid (6 ml). After 15 minutes, add a second portion (0.338g) of zinc dust. Stir the heated mixture for 1.5 hours, and then add acetic anhydride (6 ml). Reflux the resulting mixture overnight, cool it, and add it slowly to 50% (wt./wt.) aqueous sodium hydroxide solution (20 g) diluted with ice to a volume of 75 ml. Extract with dichloromethane, and wash the extracts with water. Dry, filter, and concentrate the dichloromethane solution to give 1-acetyl-4-(9,10-dihydro-2-methyl-4H-benzo[5,6]cyclohept[1,2-d]oxazol-4-ylidene)-piperidine, $\nu_{max}$ ($CH_2Cl_2$) 1640 $cm^{-1}$, as a glass.

By using essentially the same procedure, the following compounds may be prepared:

1-acetyl-4-(6,5,11,12-tetrahydrobenzo[5,6]cyclohept[1,2-b]indol-6-ylidene)piperidine, as a glass eluted from silica gel by $CH_3OH$-$CH_2Cl_2$(95-5), HRFAB-MS: found, m/e 357.1956 (M + 1]$^+$);

2-methyl-4-(1-acetyl-4-piperidinyl)benzo[5,6]cyclohept[1,2-d]thiazole m/e 338 (7%, $M^+$);

1-acetyl-1,2,3,6-tetrahydro-4-(1,4,5,10-tetrahydro-1-(phenylmethyl)benzo[5,6]cyclohepta[1,2-c]pyrazol-10-yl]pyridine, m/e 397 (73%, $M^+$); and

2-acetyl-10-(1-acetyl-4-piperidinylidene)-2,4,5,10-tetrahydrobenzo[5,6]cyclohepta[1,2-c]pyrazole, m.p. 197-200.5°C, EtOAc.

## EXAMPLE 2

### 4,9-Dihydro-4-(1-acetyl-4-piperidinylidene)-10H-benzo[4,5]cyclohepta[1,2-b]thiophen-10-one

Add pyridine (47 µl), and acetic anhydride (55 µl) to a solution of 4,9-dihydro-4-(4-piperidinylidene)-10H-benzo[4,5]cyclohepta[1,2-b]thiophen-10-one (127 mg) in dry dichloromethane (5 ml) at -10°C under a nitrogen atmosphere. After 1 hour, pour the mixture into 10% aqueous sodium hydroxide solution, and extract twice with dichloromethane. Wash the combined organic portions with 5% aqueous HCl, and with water. Dry, filter, and concentrate the combined solutions to yield a crude product, and chromatograph it over silica gel. Elute the column with ethyl acetate and crystallize the eluate to yield 4,9-dihydro-4-(1-acetyl-4-piperidinylidene)-10H-benzo[4,5]cyclohepta[1,2-b]thiophen-10-one, m/e 337 ($M^+$, 100%), from dichloromethane-ether-hexanes.

By using essentially the same procedure, the following compound may be prepared:

1-acetyl-4-(1,4,9,10-tetrahydro-1-(4-methoxyphenylmethyl)-2-methylbenzo[4,5]cyclohept[1,2-d]imidazol-4-ylidene)piperidine, m/e 441 (51%, $M^+$).

EXAMPLE 3

11,12-Dihydrobenzo[5,6]cyclohept[1,2-b]indol-6(5H)-one

Add concentrated sulfuric acid (12 ml) to a stirred suspension of the (5-substituted) phenyl hydrazone of 6,7,8,9-tetrahydro-5H-benzocycloheptene-5,6-dione (2.39 g) and ethanol (72 ml); reflux the resulting mixture for 2.5 hours. Add the mixture to ice (150 ml), and collect the resulting precipitate on a filter. Wash the collected precipitate with water and dry it. Crystallize the solid to give 11,12-dihydrobenzo[5,6]cyclohept[1,2-b]indol-6(5H)-one, m.p. 186.0-187.5°C from $CH_2Cl_2$-95% ethanol.

EXAMPLE 4

4,5-dihydro-1-(phenylmethyl)benzo[5,6]cyclohepta[1,2-c] pyrazol-10(1H)-one and 4,5-dihydro-2-(phenylmethyl)benzo [5,6]cyclohepta[1,2-c]pyrazol-10(2H)-one

Add a solution of 7-[(dimethylamino)methylene]-8,9-dihydro-5H-benzocycloheptene-5,6(7H)-dione (7.10 g) in n-butanol (100 ml) to a stirred suspension of benzylhydrazine dihydrochloride (5.85 g) and n-butanol (25 ml). Add acetic acid (4.3 ml), and reflux the mixture for 2 hours. Concentrate the solution, and dissolve the residue in dichloromethane. Wash the dichloromethane solution with water, 1M sodium bicarbonate solution, and again with water. Dry the organic solution over sodium sulfate, filter and concentrate it to give an oil. Chromatograph the oil over silica gel, and elute the column with dichloromethane to give isomers 4,5-dihydro-1-(phenylmethyl)benzo[5,6]cyclohepta[1,2-c]pyrazol-10(1H)-one and 4,5-dihydro-2-(phenylmethyl)benzo[5,6]cyclohepta[1,2-c]pyrazol-10(2H)-one as an oil, m.p. 96-99°C from isopropyl acetate, CI-MS: m/e 289 ($[M + 1]^+$), 91($[C_7H_7]^+$).

By using essentially the same procedure, the following compound may be prepared:

4,5-dihydro-benzo[5,6]cyclohepta[1,2-c]pyrazol-10(1H)-one, m.p. 142-145.5°C, MeCN.

PREPARATIVE EXAMPLE 1

Trichloroethyl 4-(9,10-dihydro-2-methyl-4H-benzo[5,6]-cyclohepta[1,2-d]oxazol-4-ylidene)-1-piperidinecarboxylate

Add trichloroethyl chloroformate (4.3 ml) to a hot (100°C) solution of 1-methyl-4-(9,10-dihydro-2-methyl-4H-benzo[5,6]cyclohept[1,2-d]oxazol-4-ylidene)piperidine (J. Med. Chem., 1974, 17, 1316) (1.84g) and triethylamine (4.4 ml) in toluene (53 ml) under nitrogen. Heat the mixture for 1.5 hours, cool it to room temperature, and wash it with 5% (wt./wt.) aqueous sodium hydroxide solution, with water, and with saturated saline solution. Dry the organic solution, filter and concentrate it. Dilute the residue with methanol, and cool the resulting solution to give trichloroethyl 4-(9,10-dihydro-2-methyl-4H-benzo[5,6]cyclohept[1,2-d]oxazol-4-ylidene)-1-piperidinecarboxylate, m.p. 162.0-164.5°C from methanol.

Using essentially the foregoing procedure, the following compounds may also be prepared:

2,2,2-trichloroethyl 4-(5,6,11,12-tetrahydrobenzo[5,6]cyclohept[1,2-b]indol-6-ylidene)-1-piperidinecarboxylic acid, oil; EI-MS: m/e 488 (88%, $M^+$ for $^{35}Cl$);

2,2,2-trichloroethyl 4-(2-methyl-4H-benzo[5,6]cyclohepta[1,2-d]thiazol-4-yl)-1-piperidinecarboxylate, δ6.95 (d, J = 12 Hz, 1 H, vinyl), 6.75 (d, J = 12 Hz, 1 H, vinyl), 4.14 (d, $J_{CH-CH}$ = 10 Hz, CH)ppm;

2,2,2-trichloroethyl-1,2,3,6-tetrahydro-4-[1,4,5,10-tetrahydro-1-(phenylmethylbenzo[5,6]cyclohepta[1,2-c]pyrazol-10-yl]-1-pyridinecarboxylate, δ 6.32 (d, $J_{eq}$ = 7 Hz, 1 H, vinyl), 5.32 (d, $J_{AB}$ = 15 Hz, 1H, $CH_AH_BPh$), 5.12 (d, $J_{AB}$ = 15 Hz, 1 H, $CH_AH_BPh$) ppm; and

2,2,2-trichloroethyl-4-(1-acetyl-1,4,5,10-tetrahydrobenzo[5,6]cyclohepta[1,2-c]pyrazol-10-ylidene)-1-piperidinecarboxylate, δ 4.8 ($CO_2CH_2CCl_3$) ppm, or

2,2,2-trichloroethyl-4-(2-acetyl-2,4,5,10-tetrahydrobenzo[5,6]cyclohepta[1,2-c]pyrazol-10-ylidene)-1-piperidinecarboxylate.

PREPARATIVE EXAMPLE 2

4,9-dihydro-4-(4-piperidinylidene)-10H-benzo[4,5]cyclohepta[1,2-b]thiophen-10-one

Add 2,2,2-trichloroethyl chloroformate (600 µl) over 20 minutes to a mixture of 4,9-dihydro-4-(1-methyl-4-piperidinylidene)-10H-benzo[4,5]cyclohepta[1,2-b]thiophen-10-one (361 mg) and triethylamine (220 µl) in dry toluene (10 ml) at 90°C and under nitrogen. After 60 minutes' heating, add another portion of 2,2,2-trichloroethyl chloroformate (100 µl). After another 20 minute period, pour the mixture into 10% aqueous sodium hydroxide solution and extract with ether. Wash the combined organic extracts with 5% aqueous HCl solution and with brine. Dry the organic solution, filter and concentrate it to give a crude product.

Dissolve the crude product in glacial acetic acid (6 ml) and heat the solution to 80°C under nitrogen. Add zinc dust (709 mg) to the solution, and heat it for 1 hour. Pour the reaction mixture into 10% aqueous sodium hydroxide solution and extract with dichloromethane. Dry the combined organic extracts, filter and concentrate them; chromatograph the residue over silica gel. Elute the column with a mixture of dichloromethane and methanol saturated with dry ammonia (3:97) to yield 4,9-dihydro-4-(4-piperidinylidene-10H-benzo[4,5]cyclohepta[1,2-b]thiophen-10-one as a glass, m/e 295 ($M^+$, 100%).

Helv. Chim. Acta (1976, 59, 866) teaches how to obtain the starting material (4,9-dihydro-4-(1-methyl-4-piperidinylidene)-10H-benzo[4,5]cyclohepta[1,2-b]thiophen-10-one) required for this Preparative Example.

EXAMPLE 5

9,10-dihydro-2-methyl-4H-oxazolo[4′,5′:5,6]cyclohepta [1,2-b]pyridin-4-one

Add acetyl chloride (7.3 ml) to a suspension of 6,7-dihydro-5H-cyclohepta[b]pyridine-8,9-dione 8-oxime 4.50 g) (prepared according to J. Med. Chem. 1984, 27, 20), acetic anhydride (47 ml), and acetic acid (5.0 ml) in an 85°C oil bath. Stir the resulting mixture at 85°C for 1.25 hours. Add the resulting, cooled solution to a mixture of 50% aqueous sodium hydroxide (83 g) and ice (ca. 140 g). Add more ice as needed to keep the temperature at ≦20°C. When the addition is complete, allow the mixture to stir for 1 hour at 25°C. Extract it with dichloromethane, combine the extracts, and wash them With saturated aqueous sodium chloride solution. Dry the organic solution over sodium sulfate, and concentrate it to dryness. Triturate the residue with ethyl acetate, collect the solid on a filter, and crystallize it to give 9,10-dihydro-2-methyl-4H-oxazolo[4′,5′:5,6]cyclohepta[1,2-b]pyridin-4-one, m.p. 210-211°C from ethanol.

EXAMPLE 6

5,6,11,12-Tetrahydro-6-(1-methyl-4-piperidinylidene)-benzo[5,6]cyclohept[1,2-b]indole

Add 5,6,11,12-tetrahydro-6-(1-methyl-4-piperidinyl)-benzo[5,6]cyclohepta[1,2-b]indol-6-ol (4.18 g) to glacial acetic acid (8.5 ml) containing acetic anhydride (2.5 ml) and acetyl chloride (2.5 ml). Stir the resulting solution at 25°C for 3 hours, cool and dilute it with dichloromethane. Add the cooled solution to a stirred mixture of 50% aqueous sodium hydroxide solution (19 g) and ice (ca. 100 g). If necessary, adjust the pH to 9 with more sodium hydroxide. Separate the layers and extract the aqueous layer with dichloromethane; wash the combined dichloromethane solutions with water. Dry the solution over sodium sulfate, filter and concentrate it. Chromatograph the residue over silica gel, and elute the column with dichloromethane - (methanol-ammonium hydroxide) (95-(9-1) by vol.) to give 5,6,11,12-tetrahydro-6-(1-methyl-4-piperidinylidene)-benzo[5,6]cyclohept[1,2-b]indole as a solid, EI-MS: m/e 328 (100%, $M^+$).

By using essentially the same procedure, the following compounds may be prepared:

3,4,9,10-tetrahydro-1-(4-methoxyphenylmethyl)-2-methyl-4-(1-methyl-4-piperidinylidene)benzo[4,5]cyclohepta[1,2-d]imidazole, eluted from silica gel by $CH_3OH$-$NH_4OH$-$CH_2Cl_2$ (4.5-0.5-95), m.p. 191-194°C from $CH_3CN$;

3,4,9,10-tetrahydro-2-methyl-4-(1-methyl-4-piperidinylidene)-benzo[5,6]cyclohepta[1,2-d]thiazole, disclosed by J. Med. Chem. 1974, 17, 1316;

2-methyl-4-(1-methyl-4-piperidinyl)-4H-benzo[5,6]cyclohepta[1,2-d]thiazole δ 6.92 (d, J ($H_A$-$H_B$) = 10 Hz, 1 H), 6.73 (d, J ($H_B$-$H_A$) = 10 Hz, 1 H)ppm;

1-acetyl-1,4,5,10-tetrahydrobenzo[5,6] cyclohepta[1,2-c]pyrazole-10-(1-methyl-4-piperidinylidene), δ 2.7 ($CH_3C(C{=}O)$ ppm, or

2-acetyl-2,4,5,10-tetrahydrobenzo[5,6] cyclohepta[1,2-c]pyrazole-10-(1-methyl-4-piperidinylidene).

PREPARATIVE EXAMPLE 3

5,6,11,12-Tetrahydro-6-(1-methyl-4-piperidinyl)-benzo[5,6]cyclohept[1,2-b]indol-6-ol

Add 11,12-dihydrobenzo[5,6]cyclohept[1,2-b]indol-6(5H)-one (1.00 g) to a solution of 1-methylpiperidine magnesium chloride (12.5 ml of a 1M solution) in cold (ice bath) tetrahydrofuran in an atmosphere of nitrogen. Stir the resulting mixture 2 hours in the ice bath and 12 hours at 25°C. Cool the resulting solution in an ice bath, and add saturated ammonium chloride solution (13 ml). Evaporate the tetrahydrofuran and extract the residue with ethyl acetate. Combine extracts, and wash them with water and with brine. Dry the combined extracts over sodium sulfate, filter and concentrate them to give 5,6,11,12-tetrahydro-6-(1-methyl-4-piperidinyl)-benzo[5,6]cyclohept[1,2-b]indol-6-ol, m.p. 205-209°C (d) from ether-dichloromethane.

Using essentially the foregoing procedure, the following compounds may be prepared:

1-methyl-4-hydroxy-4-(2-methyl-3-(4-methoxyphenylmethyl)-9,10-dihydro-4H-benzo[5,6]cyclohepta[1,2-d]imidazol)-1-piperidine, m.p. 186-189°C from ethyl acetate;

9,10-dihydro-2-methyl-4-(1-methyl-4-piperidinyl)-4H-benzo[4,5]cyclohepta[1,2-d]thiazol-4-ol, m.p. 141.5-145°C, disclosed by J. Med. Chem. 1974, 17, 1316;

5,7,8,9-tetrahydro-5-hydroxy-5-(1-methyl-4-piperidinyl)-6H-benzocyclohepten-6-one, m.p. 159-163°C from ethyl acetate;

1,4,5,10-tetrahydro-10-(1-methyl-4-piperidinyl)benzo[5,6]cyclohepta[1,2-c]pyrazol-10-ol, m.p. 209-211°C, MeCN; and

1,4,5,10-tetrahydro-10-(1-methyl-4-piperidinyl)-1-(phenylmethyl)benzo[5,6] cyclohepta[1,2-c]pyrazol-10-ol, δ 2.1 ($CH_3$) ppm.

PREPARATIVE EXAMPLE 4

6,7,8,9-tetrahydro-5H-benzocycloheptene-5,6-dione, phenyl hydrazone

Add phenylhydrazine hydrochloride (4.15 g) to a solution of 6,7,8,9-tetrahydro-5H-benzo-cycloheptene-5,6-dione (5.00 g) in ethanol (50 ml), and reflux the resulting solution for 2.5 hours. Filter the cooled solution and wash the collected solid with cold ethanol. Dry the solid on the filter to give as dark yellow needles; use this solid directly in the next step.

PREPARATIVE EXAMPLE 5

7-[(dimethylamino)methylene]-8,9-dihydro-5H-benzocycloheptene-5,6(7H)-dione

Add N,N-dimethylformamide dimethylacetal (3.2 ml) to 6,7,8,9-tetrahydro-5H-benzocycloheptene-5,6-dione (2.00 g) in an atmosphere of nitrogen. Stir the resulting solution at reflux for 1.5 hours, cool the solution, and concentrate it to dryness. Chromatograph the resulting oil over silica gel, and elute the column with 99.5-0.5 (vol./vol.) dichloromethane - methanol to give 7-[(dimethylamino)methylene]-8,9-dihydro-5H-benzocycloheptene-5,6(7H)-dione as an oil, CI-MS: m/e 230 ($[M + 1]^+$), 239 ($M^+$), 201 ($[M - CO]^+$); m.p. 124-126°C EtOAc.

PREPARATIVE EXAMPLE 6

2-Methyl-3-(4-methoxybenzyl)-9,10-dihydro-4H-benzo[5,6]cyclohepta[1,2-d]imidazol-4-one

Heat a mixture of 2-methyl-9,10-dihydro-4H-benzo[5,6]cyclohepta[1,2-d]oxazol-4-one (2.00 g) (prepared according to J. Med. Chem. 1974, 17, 1316) and 4-methoxybenzylamine (2.60 g) in an atmosphere of nitrogen in a sealed tube for 2.5 hours at 130°C and 3 hours at 140°C. Cool the mixture, open the tube, and dissolve the contents in dichloromethane - (methanolammonium hydroxide) (99-(9-1), vol./vol.). Add the solution to a silica gel column, and elute the column with the same solvent to give 2-methyl-3-(4-methoxybenzyl)-9,10-dihydro-4H-benzo[5,6]cyclohepta[1,2-d]imidazol-4-one as a semi-solid, $^1$H-NMR: δ($CDCl_3$ 5.01 (s, 2H, $CH_2$-$C_6H_4$OMe-4) ppm.

EXAMPLE 7

3a,4,5,10a-tetrahydro-1-(4-methoxyphenyl)-10a-(1-pyrrolidinyl)benzo[4,5]cyclohepta[1,2-d]triazol-10(1H)-one

Add pyrrolidine (0.29 ml) to a solution of 6,7,8,9-tetrahydro-5H-benzocycloheptene-5,6-dione (500 mg), p-methoxybenzyl azide (0.937 g), and toluene (15 ml). Reflux the resulting solution for 17.5 hours in an atmosphere of nitrogen

and under a Dean-Stark trap. Cool the solution, evaporate the solvent, and chromatograph the residue over silica gel. Elute the column with dichloromethane-methanol (99.5-0.5, vol./vol.) to give 3a,4,5,10a-tetrahydro-1-(4-methoxyphenyl)-10a-(1-pyrrolidinyl)-benzo[4,5]cyclohepta[1,2-d]triazol-10(1H)- one as a glass, EI-MS: m/e 390 (2,$M^+$), 121 (100, $[C_8H_9O]^+$).

PREPARATIVE EXAMPLE 7

8,9-Dihydro-6-(1-pyrrolidinyl-5H-benzocycloheptene-5-one

Reflux a solution of 6,7,8,9-tetrahydro-5H-benzocycloheptene-5,6-dione (2.00 g), pyrrolidine (0.96 ml), p-toluenesulfonic acid monohydrate (23 mg), and benzene (25 ml) in an atmosphere of nitrogen and under a Dean-Stark trap for 1.5 hours. Cool and concentrate the solution to give 8,9-dihydro-6-(1-pyrrolidinyl-5H-benzocyclohepten-5-one as an oil, $v_{max}$ 1680, 1620 $cm^{-1}$.

PREPARATIVE EXAMPLE 8

6,7,8,9-tetrahydro-5H-benzocycloheptene-5,6-dione

Mix 6,7,8,9-tetrahydro-5H-benzocycloheptene-5,6-dione oxime (10.1 g) (prepared according to J. Med. Chem. 1974, 17, 1316) with a solution of sodium bisulfite (19.4 g) in 50% aqueous ethanol (100 ml), and reflux the resulting mixture for 2.75 hours. Cool the mixture and collect the precipitated bisulfite adduct on a filter; wash the solid with cold 50% aqueous ethanol, and dry it on the filter under a rubber dam. Stir the dried adduct at 25°C for 1.75 hours with 2N hydrochloric acid (185 ml) and dichloromethane (139 ml.) Separate the layers, and extract the aqueous layer with more solvent. Combine the organic extracts, wash them with water, dry them over sodium sulfate, and concentrate the (filtered) solution to give 6,7,8,9-tetrahydro-5H-benzocycloheptene-5,6-dione, as yellow crystals, m.p. 54.0-56.5°C from dichloromethane (J. Chem. Soc. (1952, 603) gives m.p. 45-49°C after distillation).

EXAMPLE 8

1-FORMYL-4-(1,4,9,10-TETRAHYDRO-1-(4-METHOXYPHENYLMETHYL)-2-METHYLBENZO[4,5]CYCLOHEPT[1,2-d]IMIDAZOL-4-YLIDENE)PIPERIDINE AND 1,4,9-TETRAHYDRO-1-[(4-METHOXYPHENYL)METHYL]-2-METHYL-4-(4-PIPERIDINYLIDENE)BENZO[4,5]CYCLOHEPT[1,2-d]IMIDAZOLE

Add a solution of 4-[1,4,9,10-tetrahydro-1-[(4-methoxyphenyl)methyl]-2-methylbenzo[4,5]cyclohept[1,2-d]imidazo-4-ylidene-1-piperidinecarbonitrile (100 mg) in dry tetrahydrofuran (5 ml) to a stirred suspension of lithium aluminum hydride (49 mg) and dry tetrahydrofuran (5 ml) under a nitrogen atmosphere. Reflux the reaction mixture for 3 hrs., and let it stand at room temperature overnight. Cautiously add ethyl acetate (0.65 ml), followed by saturated aqueous sodium potassium tartrate solution. Decant the supernatant solution from the coagulated solid salts, and concentrate the solution. Chromatograph the residue on preparative chromatographic plates bearing silica gel as the adsorbent. View the developed plates in ultraviolet light, and separately collect the bands of silica gel by scraping the plates. Extract the collected silica gel with a suitable solvent, e.g., dichloromethane-methanol (9:1). Separately concentrate the extracts to give 1-formyl-4-(1,4,9,10-tetrahydro-1-(4-methoxyphenylmethyl)-2-methylbenzo[4,5]cyclohept[1,2-d]imidazol-4-ylidene)piperidine, m.p. 191-194°C, crystallized from acetonitrile, and 1,4,9-tetrahydro-1-[(4-methoxyphenyl)methyl]-2-methyl-4-(4-piperidinylidene)benzo[4,5]cyclohept[1,2-d]imidazole, m/e 400 (24%, $[M + 1]^+$), as a semi-solid.

EXAMPLE 9

4-[1,4,9,10-TETRAHYDRO-1-[(4-METHOXYPHENYL)METHYL-2-METHYLBENZO[4,5]CYCLOHEPT[1,2-d]IMIDAZOL-4-YLIDENE]-1-PIPERIDINECARBONITRILE

Add 3,4,9,10-tetrahydro-1-(4-methoxyphenylmethyl)-2-methyl-4-(1-methyl-4-piperidinylidene)-benzo[4,5]cyclohepta[1,2-d]imidazole (387 mg) to cyanogen bromide (173 mg) dissolved in benzene (4 ml), and allow the reaction mixture to stir at 25°C for one week. Dilute the mixture with chloroform so as to dissolve solids, and wash the solution sequentially with 1 M aqueous sodium bicarbonate solution, water, and saturated sodium chloride solution. Separate the organic layer, dry it over sodium sulfate, filter and concentrate it. Chromatograph the residue over a column of silica gel, and elute the column with dichloromethane-methanolconcentrated aqueous ammonia (97:2.7:0.3). Analyze the fractions by thin-layer chromatography and combine and concentrate the appropriate ones to give 4-[1,4,9,10-tetrahydro-1-[(4-methoxyphenyl)methyl]-2-methylbenzo[4,5]cyclohept[1,2-d]imidazol-4-ylidene]-1-piperidinecarbonitrile, as a semi-solid, m/e 424 (38%, $M^+$).

EXAMPLE 10

1H-10-(1-ACETYL-4-PIPERIDINYLIDENE)-2,4,5,10-TETRAHYDRO[5,6]CYCLOHEPTA[1,2-c]PYRAZOLE

Dissolve 2-acetyl-10-(1-acetyl-4-piperidinylidene)-2,4,5,10-tetrahydro[5,6]cyclohepta[1,2-c]pyrazole (10 Me) in 95% ethanol (1.5 ml) and stir the solution at 25°C. Add an excess of sodium hydroxide solution (50% by weight), and monitor the ensuing reaction by thin-layer chromatography. When the reaction is complete, concentrate the solution, dilute it with water, and extract it with dichloromethane. Sequentially wash the combined extracts with dilute hydrochloric acid, water, 1N sodium bicarbonate solution, and with water. Dry the organic solution with sodium sulfate, filter and concentrate the solution to give 1H-10-(1-acetyl-4-piperidinylidene)-2,4,5,10-tetrahydro[5,6]cyclohepta[1,2-c]pyrazole as an oil, δ 2.11, 2.13 (2 $CH_3CON$) ppm, m/e 308 (100%, $[M+1]^+$).

PREPARATIVE EXAMPLE 9

5,7,8,9-TETRAHYDRO-5-HYDROXY-5-(1-METHYL-4-PIPERIDINYL)-6H-BENZOCYCLOHEPTEN-6-ONE (8)

Add sodium bisulfite (1.50 g) to a solution of 5,7,8,9-tetrahydro-5-hydroxy-5-(1-methyl-4-piperidinyl)-6H-benzocyclohepten-6-one, oxime (1.16 g) in 50% aqueous ethanol (1.2 ml), and reflux the solution for 2 hrs. Cool the solution in an ice bath, and acidify it with concentrated hydrochloric acid; stir the acidic solution at 25°C for 2 hrs. Wash the solution with ether, and discard the washings. Basify the aqueous solution with 50% sodium hydroxide solution, and extract the basic aqueous solution with several portions of ether. Combine the extracts and wash them sequentially with saturated sodium chloride solution. Dry the ether solution over sodium sulfate, filter and concentrate it. Chromatograph the residue over a column of silica gel, and elute the column with dichloromethane-methanol-concentrated aqueous ammonia (97.5:2.25:0.25). Monitor the progress of the chromatography by thin-layer chromatography, combine appropriate fractions and concentrate them to give 5,7,8,9-tetrahydro-5-hydroxy-5-(1-methyl-4-piperidinyl)-6H-benzocyclohepten-5-one, (FAB-ms) m/e 274 (100%, $[M + 1]^+$, as an oil.

The following examples illustrate pharmaceutical compositions which may employ as an active ingredient a compound of formula 1.0.

Pharmaceutical Dosage Form Examples

Example A

| Tablets | | | |
|---|---|---|---|
| No. | Ingredient | mg/tablet | mg/tablet |
| 1. | Active Compound | 100 | 500 |
| 2. | Lactose USP | 122 | 113 |
| 3. | Corn Starch, Food Grade, as a 10% paste in Purified Water | 30 | 40 |
| . | Corn Starch, Food Grade | 45 | 40 |
| 5. | Magnesium Stearate | 3 | 7 |
| | Total | 300 | 700 |

Method of Manufacture

Mix item nos. 1 and 2 in a suitable mixer for 10-15 minutes. Granulate the mixture with item no. 3. Mill the damp granules through a coarse screen (e.g., 1/4") if needed. Dry the damp granules. Screen the dried granules if needed and mix with item no. 4 and mix for 10-15 minutes. Add item no. 5 and mix for 1-3 minutes. Compress the mixture to appropriate size and weight on a suitable tablet machine.

Example B

| Capsules | | | |
|---|---|---|---|
| No. | Ingredient | mg/capsule | mg/capsule |
| 1. | Active Compound | 100 | 500 |
| 2. | Lactose USP | 106 | 123 |
| 3. | Corn Starch, Food Grade, | 40 | 70 |
| 4. | Magnesium Stearate NF | 4 | 7 |
| | Total | 250 | 700 |

Method of Manufacture

Mix item nos. 1, 2 and 3 in a suitable blender for 10-15 minutes. Add item no. 4 and mix for 1-3 minutes. Fill the mixture into suitable two-piece hard gelatin capsules on a suitable encapsulating machine.

While the present invention has been described in connection with certain specific embodiments thereof, it will be evident to one of ordinary skill in the art that many alternatives, modifications and variations may be made. All such alternatives, modifications and variations are intended to be included within the spirit and scope of the invention.

**Claims**

**Claims for the following Contracting States : AT, BE, CH, DE, FR, GB, IT, LI, LU, NL, SE**

**1.** A compound of formula 1.0

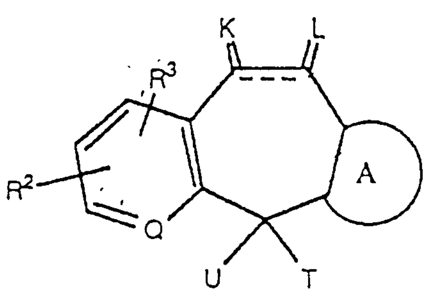

or a pharmaceutically acceptable salt or solvate thereof, wherein

U and T together represent =O, or U represents H and T represents

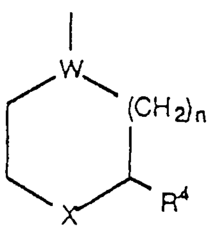

,

or

U and T together represent

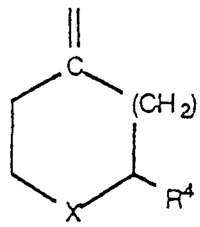 ;

Q is CH, N or N→O;

$R^2$ and $R^3$ may be the same or different and each independently represents H, $C_1$-$C_8$ alkyl, $CF_3$, $NO_2$, halo, $OR^5$, $NR^5R^6$, or $C_1$-$C_8$ $S(O)_m$-alkyl, in which:

$R^5$ and $R^6$ may be the same or different and each is independently selected from H, $C_1$-$C_8$ alkyl or $C_1$-$C_8$ acyl, $C_7$-$C_{16}$ aroyl, and m is O, 1 or 2;

$R^4$ represents H , $C_1$-$C_8$ alkyl or $C_6$-$C_{15}$ aryl

K represents -H, -H or -H, $C_1$-$C_8$ alkyl or $C_1$-$C_8$ alkyl, $C_1$-$C_8$ alkyl, or -H, -OH or=O;

L represents -H, -H or -H, $C_1$-$C_8$ alkyl or $C_1$-$C_8$ alkyl, $C_1$-$C_8$ alkyl or -H, -OH or =O, with the proviso that when L or K is -H, -OH or =O, then the other of K or L, respectively, is -H, -H or -H, $C_1$-$C_8$ alkyl or $C_1$-$C_8$ alkyl, $C_1$-$C_8$ alkyl;

ring

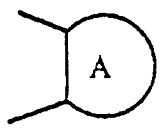

represents

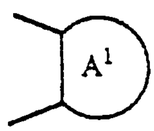

when U represents H and T represents

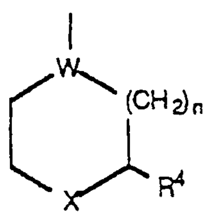

or when T and U together represent

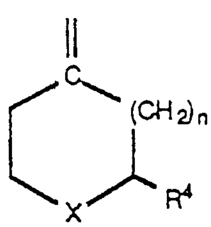 ;

and ring

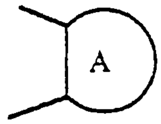

represents

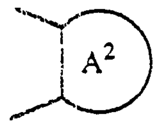

when T and U together represent =O;

ring

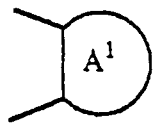

represents a fused 5-membered heterocyclic aromatic ring having at least one heteroatom selected from O, S or N in the ring, said ring optionally being substituted by 1 to 3 R groups selected from $C_1$-$C_8$ alkyl, $C_6$-$C_{15}$ aryl and $C_7$-$C_{16}$ arylmethyl or by two R groups on adjacent ring atoms which R groups together with such adjacent ring atoms represent a fused benzene or fused pyridine ring, or ring

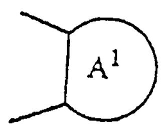

represents a fused 6-membered heterocyclic aromatic ring having 1,2 or 3 ring nitrogen atoms;

ring

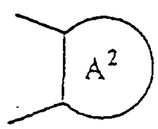

represents

(i) when Q is N or N→O:

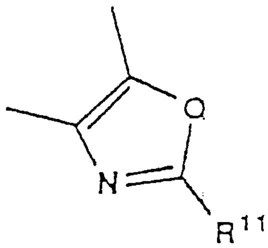 , 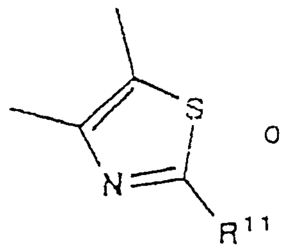 or 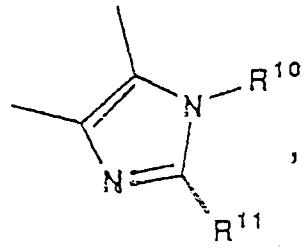 ,

or (ii) when Q is CH, N or N→O:

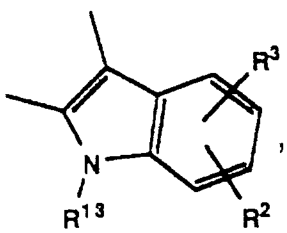 , 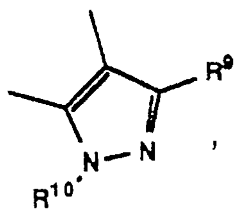 , 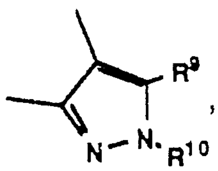 , 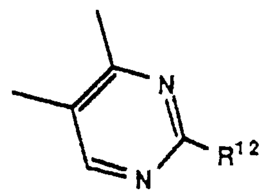 ,

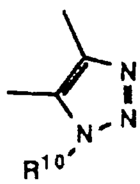 , 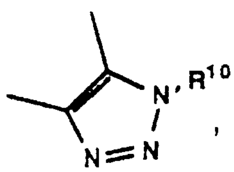 , 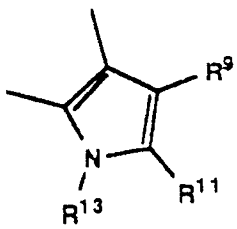 , 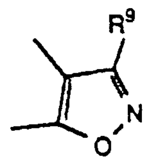 ,

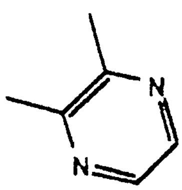 , 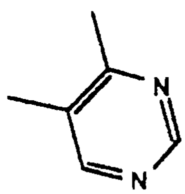 , 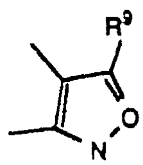 , 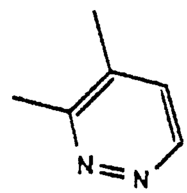 ,

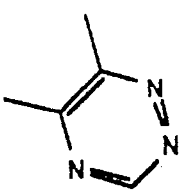 or 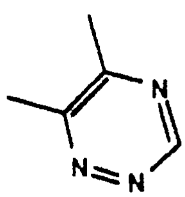 ;

in which:

$R^9$ is H, $C_1$-$C_8$ alkyl or $C_6$-$C_{15}$ aryl;

$R^{10}$ is H, $C_1$-$C_8$ alkyl, $C_6$-$C_{15}$ aryl, $C_7$-$C_{23}$ arylalkyl, $C_1$-$C_8$ acyl, $C_7$-$C_{16}$ aroyl and $C_2$-$C_{14}$ heteroaroyl wherein the aryl moiety is optionally substituted by one or more substituents selected from H, halogen, $NO_2$, $CF_3$, -SH, $C_1$-$C_8$ S-alkyl, $C_1$-$C_8$ $S(O)_m$-alkyl, $C_1$-$C_8$ alkyl, $CO_2H$, $CH_2OH$, C(OH)-($C_1$-$C_8$ lower alkyl), $NH_2$, NH-($C_1$-$C_8$ lower alkyl), N-($C_1$-$C_8$ lower alkyl)$_2$, OH, O-($C_1$-$C_8$ lower alkyl), O-($C_6$-$C_{15}$ aryl), O-($C_7$-$C_{16}$ aroyl), O-($C_2$-$C_{14}$ heteroaroyl), NH($C_1$-$C_8$ acyl), N($C_1$-$C_8$ acyl)$_2$, NH ($C_7$-$C_{16}$ aroyl), N($C_7$-$C_{16}$ aroyl)$_2$, NH($C_2$-$C_{14}$ heteroaroyl), N($C_2$-$C_{14}$ heteroaroyl)$_2$,

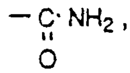, 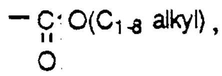,

—CHO,

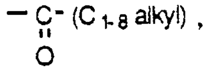, 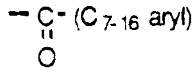

and —C≡N;

$R^{11}$ represents H, $C_1$-$C_8$ alkyl, $C_6$-$C_{15}$ aryl or $C_7$-$C_{23}$ aralkyl;

$R^{12}$ represents H, $C_1$-$C_8$ alkyl, OH, O-($C_1$-$C_8$ alkyl), SH S($C_1$-$C_8$ alkyl), $NH_2$, NH-($C_1$-$C_8$ alkyl), N-($C_1$-$C_8$ alkyl)$_2$, and NH(C=O)-($C_1$-$C_8$ alkyl);

$R^{13}$ is H, $C_1$-$C_8$ alkyl, $C_6$-$C_{15}$ aryl or $C_7$-$C_{16}$ arylmethyl; and

W is N or N→O;

the broken line in the seven-membered ring represents an optional double bond;

n is 0,1,2 or 3;

X represents

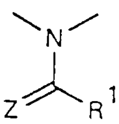, 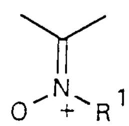 or 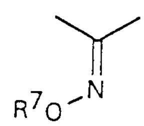

in which:

$R^7$ represents H, $C_1$-$C_8$ alkyl, $C_1$-$C_8$ acyl or $C_7$-$C_{16}$ aroyl,

Z is O or S, and $R^1$ is H, $C_1$-$C_8$ alkyl, $C_5$-$C_8$ cycloalkyl, $CF_3$, $C_6$-$C_{15}$ aryl, $C_2$-$C_{14}$ heteroaryl, $NR^5R^6$ wherein $R^5$ and $R^6$ are as defined above, $C_1$-$C_8$ alkylthio or $C_1$-$C_8$ alkoxy, or $R^1$ and $R^4$ together represent-$(CH_2)_k$- where k is 1,2, or 3 so as to form a fused ring,

excluding those compounds where Q is CH, ring $A^1$ is a fused, 6 membered heterocyclic ring having one ring nitrogen and X represents

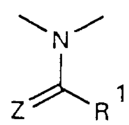

wherein

(a) Z = S or Z = O and $R^1$ is part of a fused ring or is selected from H, alkyl, cycloalkyl, aryl, alkylthio, $-NH_2$, N(alkyl)$_2$, and heteroaryl in which the heteroatom is O, S or N; or

(b) Z = O and $R^1$ is $CF_3$; or

(c) Z = S and $R^1$ is alkoxy.

**2.** The compound of claim 1 further characterised by having the structure:

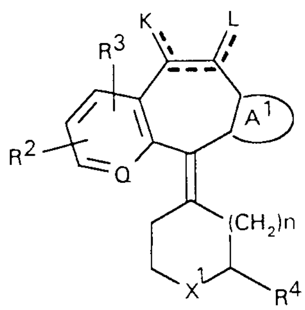

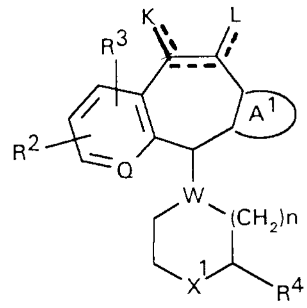

wherein $X^1$ represents

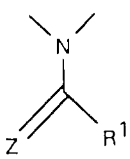

,

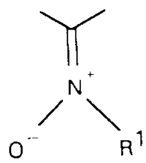

or

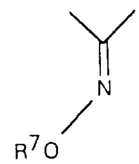

and where ring

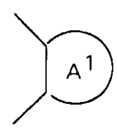

represents

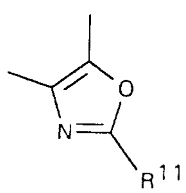

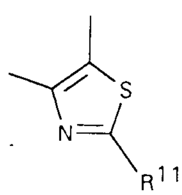

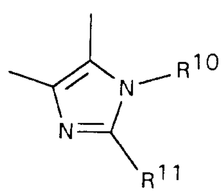

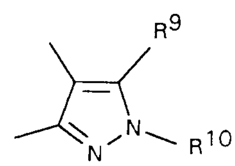

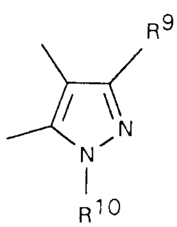

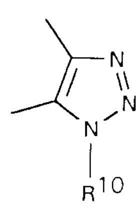

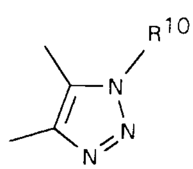

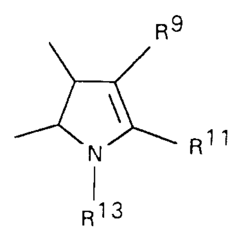

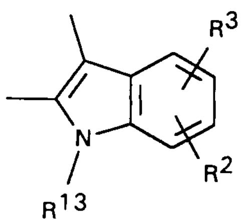
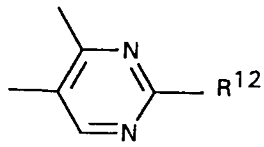
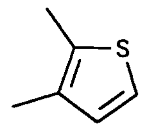

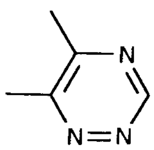
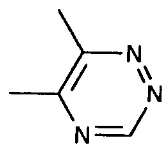
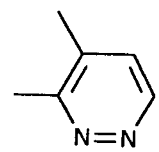

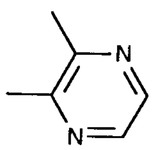
or
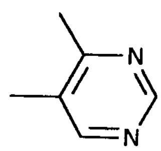

wherein

$R^9$ is selected from H, $C_1$-$C_8$ alkyl or $C_6$-$C_{15}$ aryl;
$R^{10}$ is selected from H, $C_1$-$C_8$ alkyl, $C_6$-$C_{15}$ aryl or $C_6$-$C_{15}$ arylmethyl;
$R^{11}$ is selected from H, $C_1$-$C_8$ alkyl, $C_6$-$C_{15}$ aryl or $C_6$-$C_{15}$ arylmethyl; and
$R^{13}$ is selected from H, $C_1$-$C_8$ alkyl, $C_6$-$C_{15}$ aryl or $C_6$-$C_{15}$ arylmethyl.

**3.** A compound of claim 1 further characterised by having the structural formula:

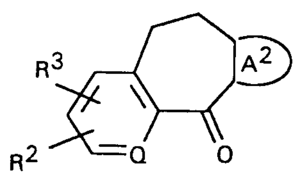

**4.** A compound of any of claims 1 or 2 further characterised by K and L both representing H, H and wherein $R^2$, $R^3$ and $R^4$ all represent H.

**5.** A compound of any of claims 1 - 4 having the structural formula:

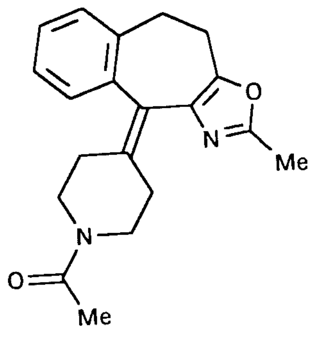

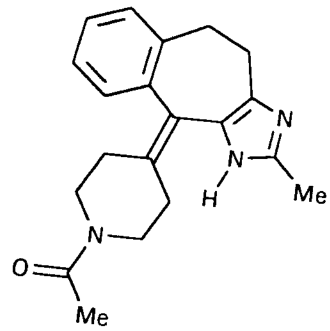

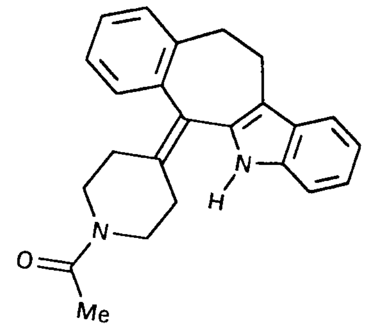

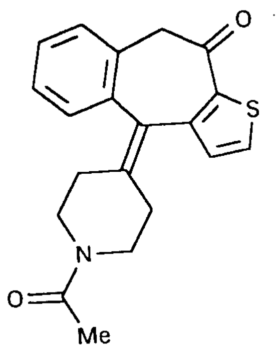

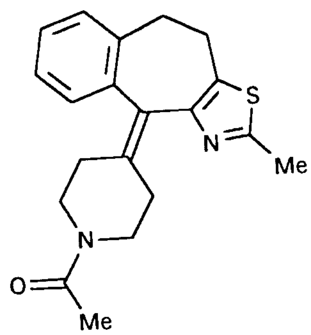

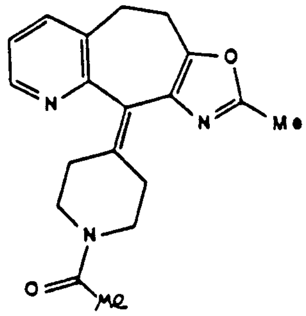

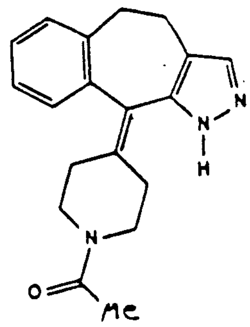

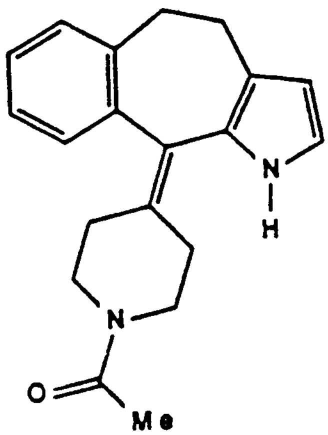

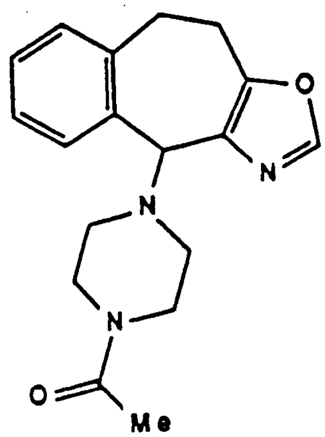

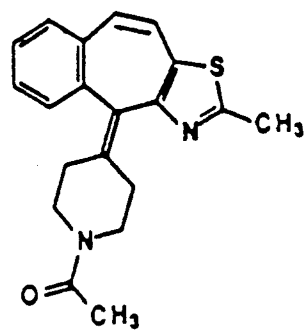

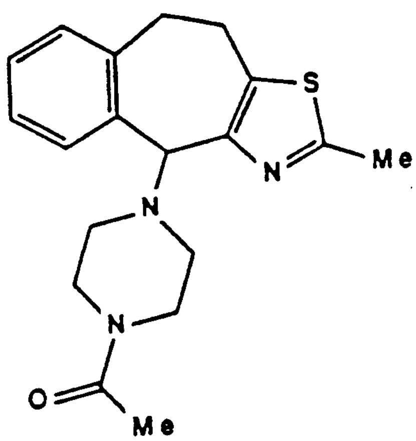

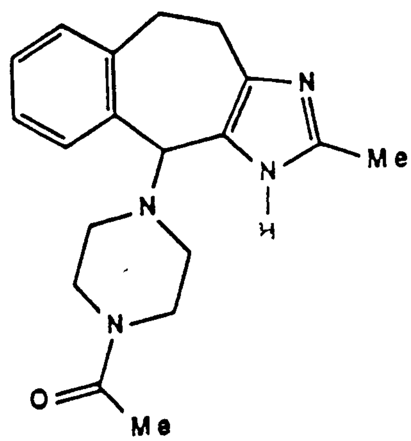

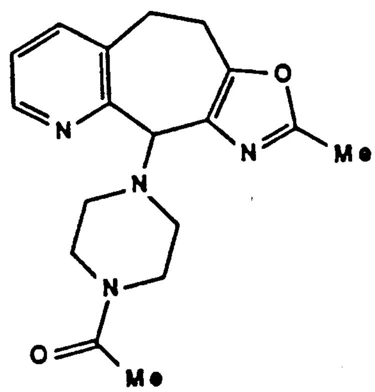

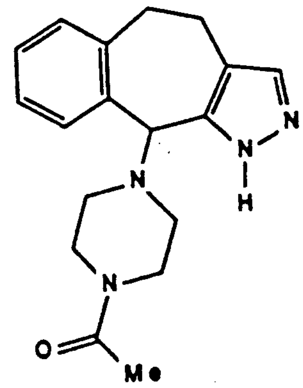

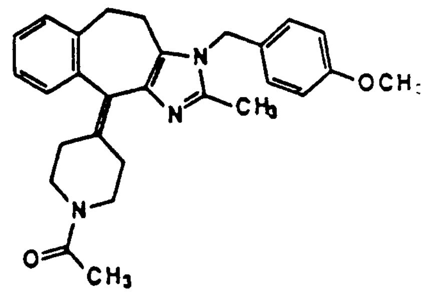

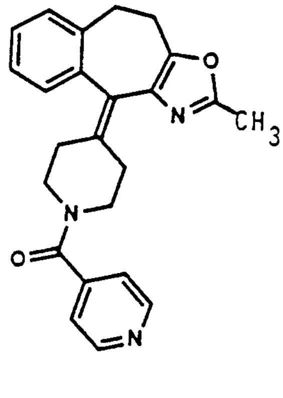

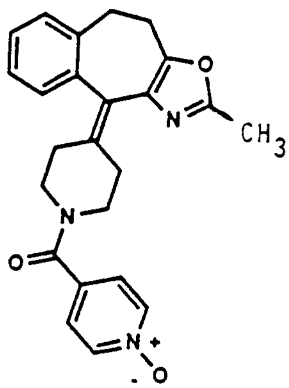

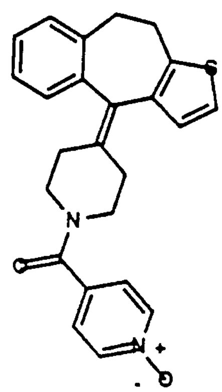

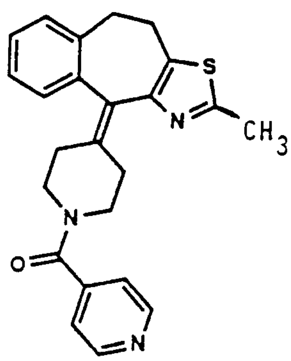

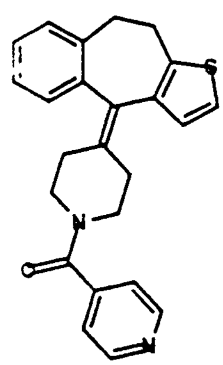

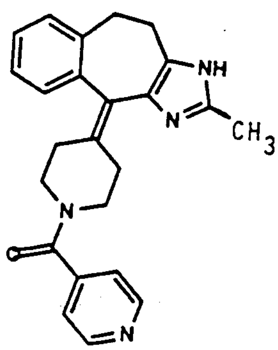

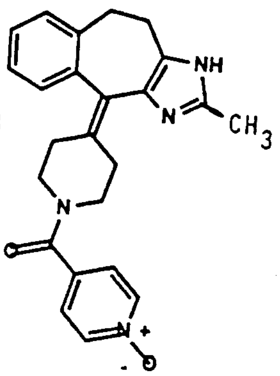

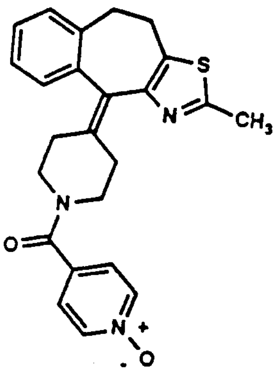

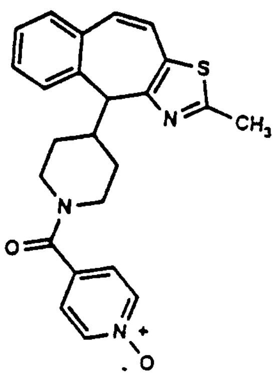

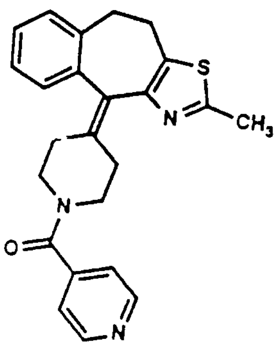

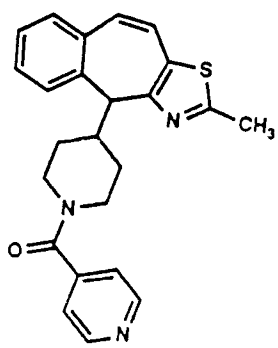

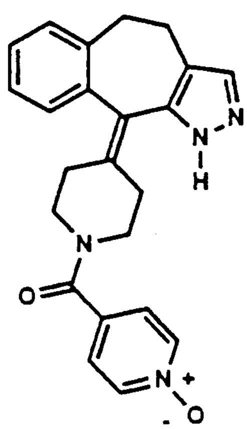

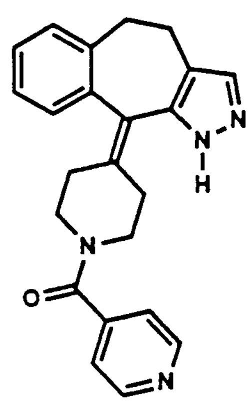

6. A pharmaceutical composition comprising a compound of formula 1.0 of claim 1 in combination with a pharmaceutically acceptable carrier.

7. A method of preparing a pharmaceutical composition comprising admixing a composition of formula 1.0 of claim 1 with a pharmaceutically acceptable carrier.

8. The use of a compound of formula 1.0 of claim 1 and the pharmaceutically acceptable salts thereof for the preparation of a medicament for the treatment of PAF disorders, allergy and/or inflammation.

9. A method for making a compound of formula 1.0 of claim 1 comprising:

a) where X represents

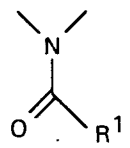

reacting a compound of formula 9.1 with compound 8.11 in the presence of base

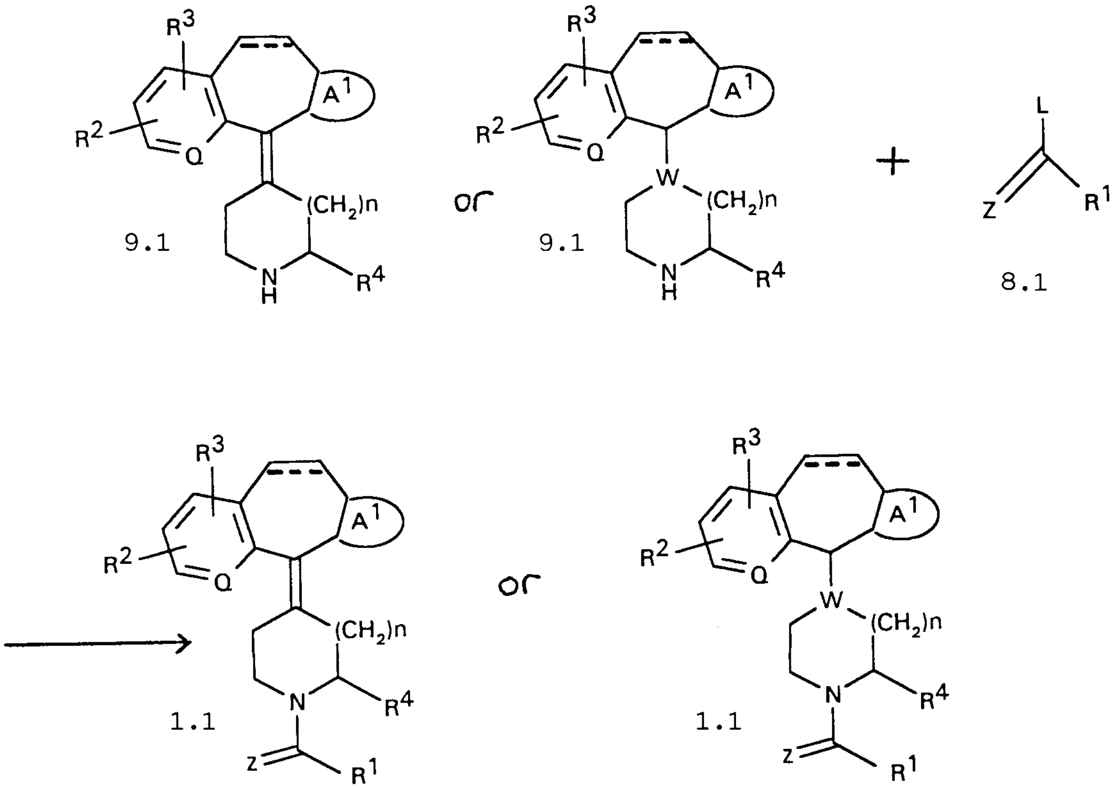

9.1 or 9.1 + 8.1

1.1 or 1.1

b) where X represents

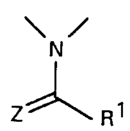

where Z is =O or =S, reacting a compound of formula 11.1 with a compound of formula 8.1

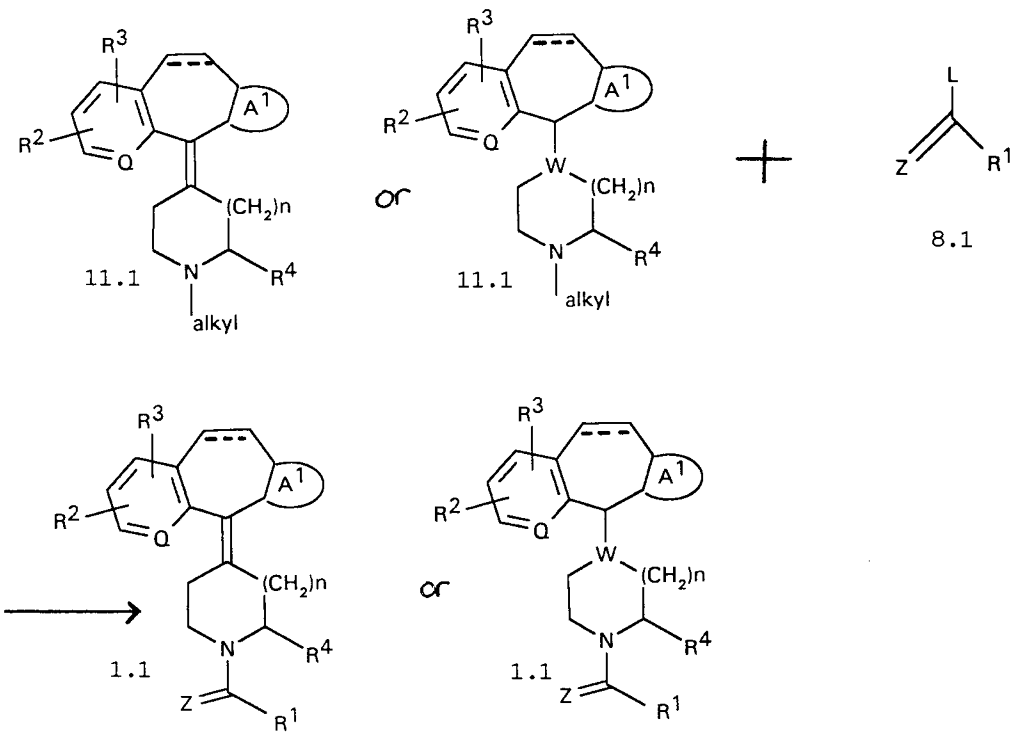

11.1 or 11.1 + 8.1

1.1 or 1.1

c) where X represents

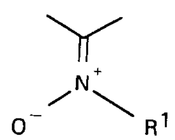

or

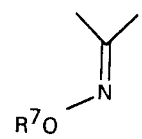

reacting a compound of formula 13 in the presence of hydroxylamine or O-substituted hydroxylamine or N-substituted hydroxylamine;

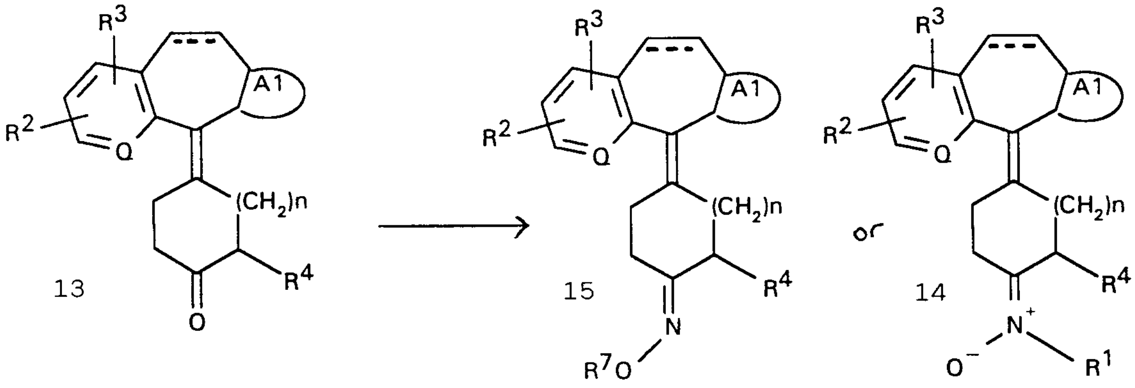

13 → 15 or 14

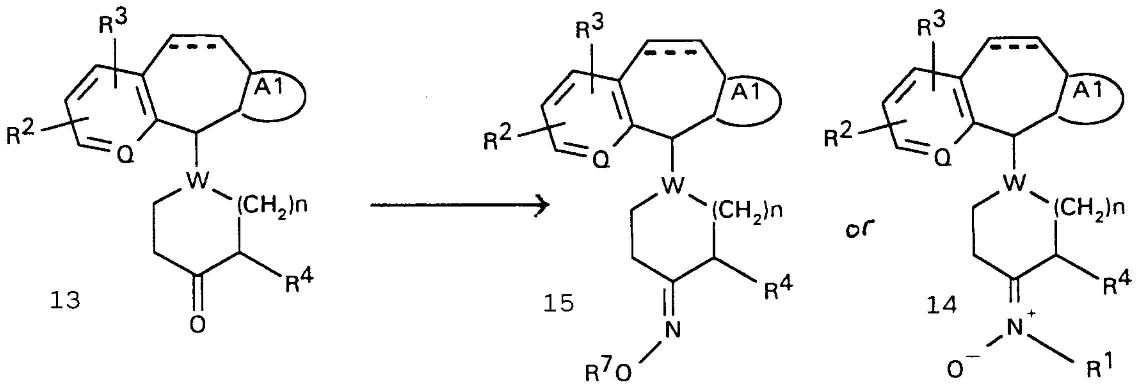

13 → 15 or 14

d) reacting a compound of formula 9.2 with an acid

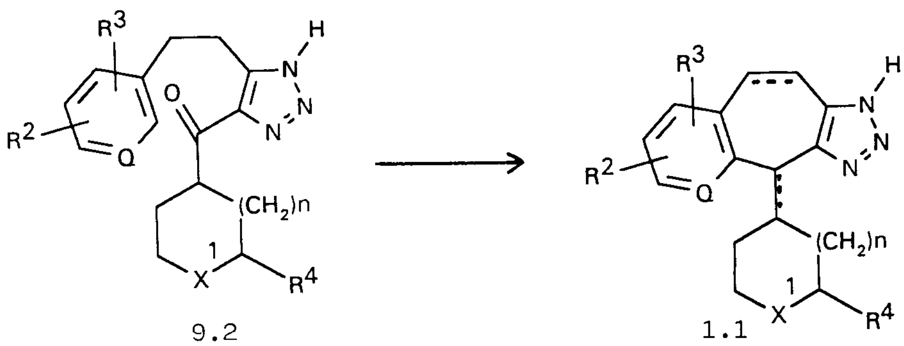

9.2 → 1.1

e) reacting a compound of formula 200 with an alkali or alkaline metal hydroxide

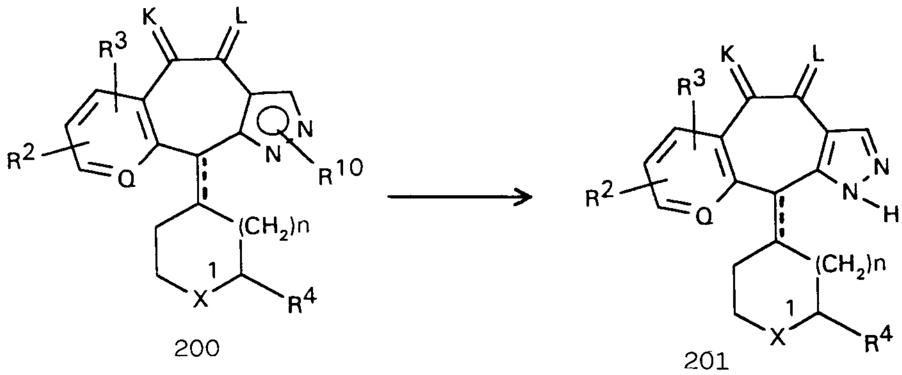

200

201

f) Reacting a compound of formula 17 with a suitable acid

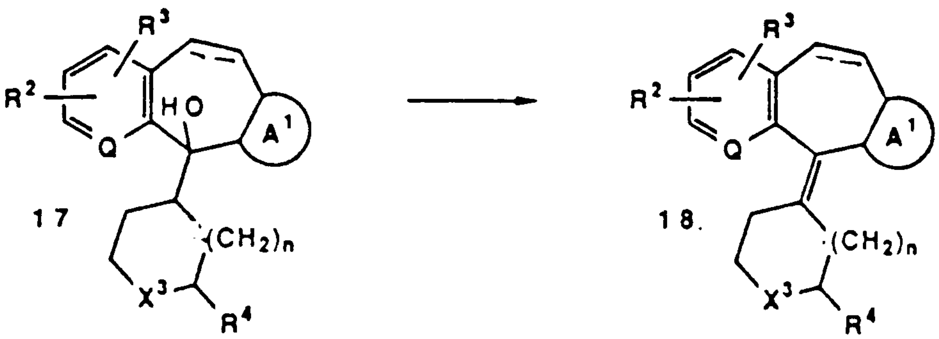

17

18.

wherein $X^3$ represents

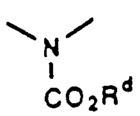

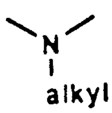

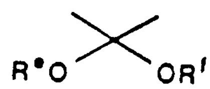

or

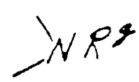

$R^d$ represents an optionally substituted alkyl, alkenyl, aryl or aralkyl group;

$R^e$ and $R^f$ each independently represent lower alkyl or together represent an alkanediyl chain of 2 or 3 methylene groups;

$R^g$ is H or $C(Z)R^1$;

g) Reacting a compound of formula 19 with a compound of formula 20 where L is a leaving group and $X^3$ represents $NR^g$ or

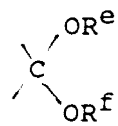

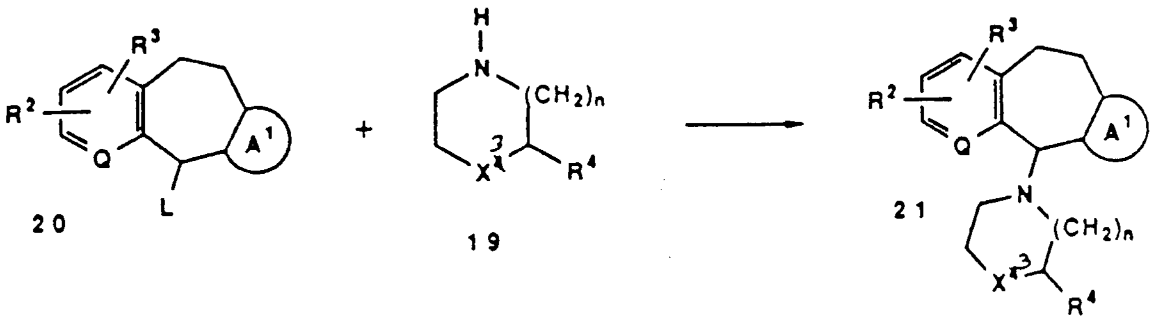

20 19 21

h) Reacting a compound of formula 4.0 with a compound of formula 19

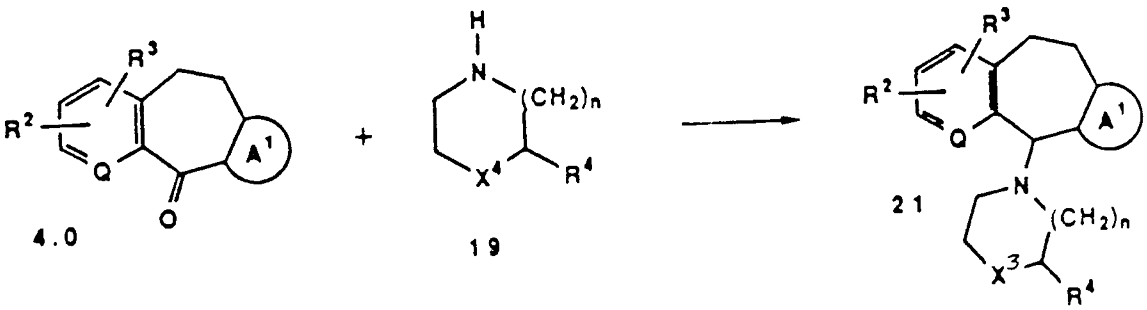

4.0 19 21

i) Dehydrating a compound of formula 26 to produce compound 27

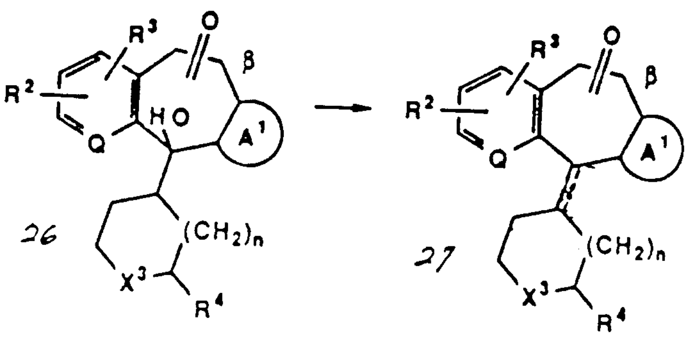

26 27

j) Hydrolyzing compound 30 with a strong aqueous acid to produce compound 31

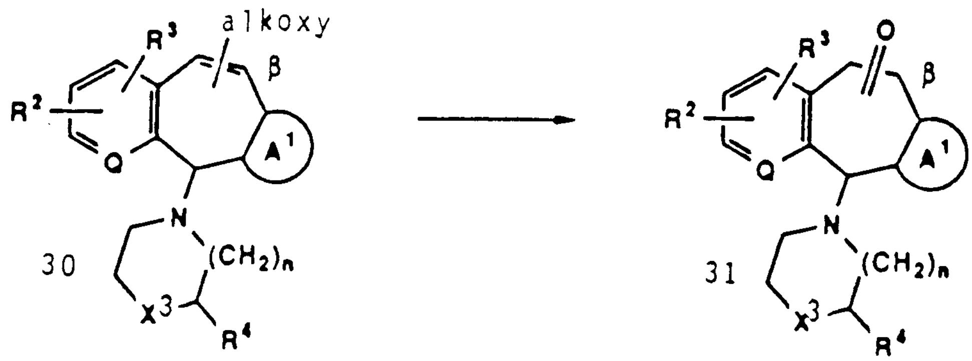

30

31

k) Reacting a compound of formula 5.1 with a compound jof formula $R^{11}CO_2H$, and $(R^{11}CO)_2O$ or $(R^{11}CO_2)_2O$ alone in the presence of HCl

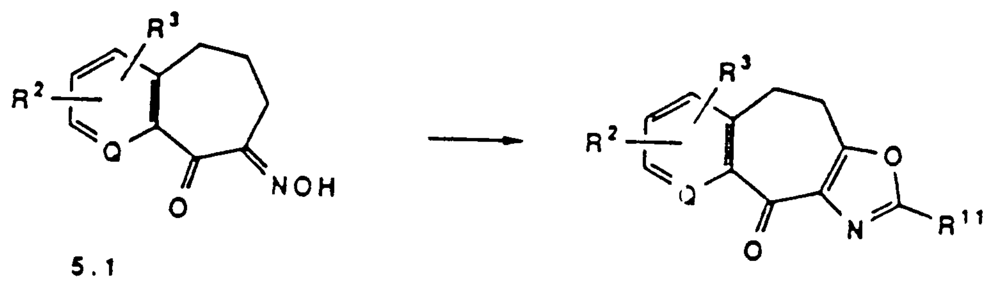

5.1

4.1

l) Reacting a compound of formula 5.2 with an acidic reagent and wherein Ar represents an appropriate aryl group

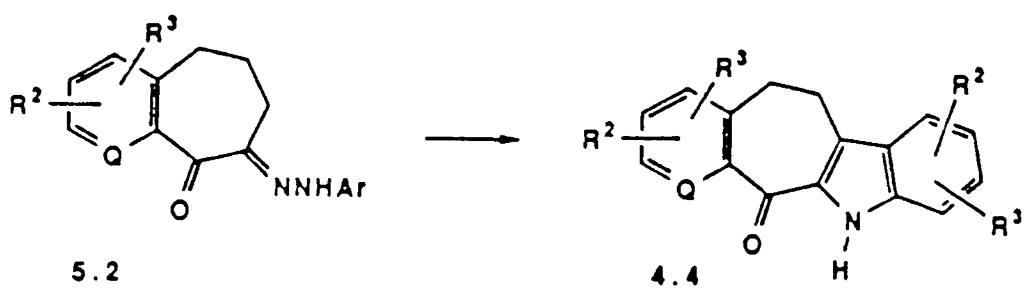

5.2

4.4

m) Reacting a compound of formula 5.3 with a hydrazine reagent $R^{10}NHNH_2$ and an acid catalyst

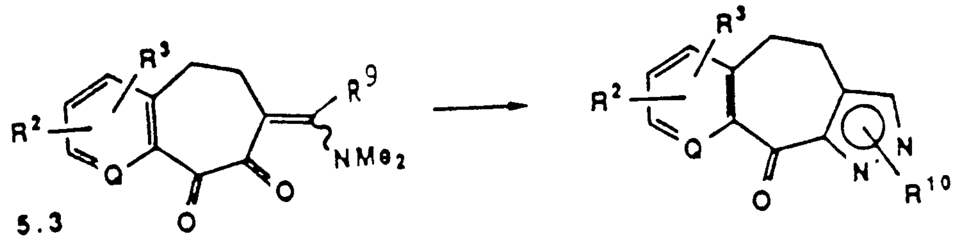

5.3

4.5

n) Reacting a compound of formula 5.4 with an acid or peracid, to produce a compound of formula 4.6

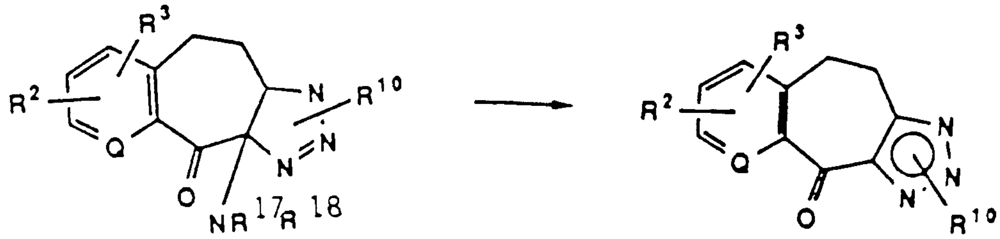

5.4

4.6

o) Reacting a compound of formula 5.5 with an appropriate alkali metal dithionite in a hot solvent

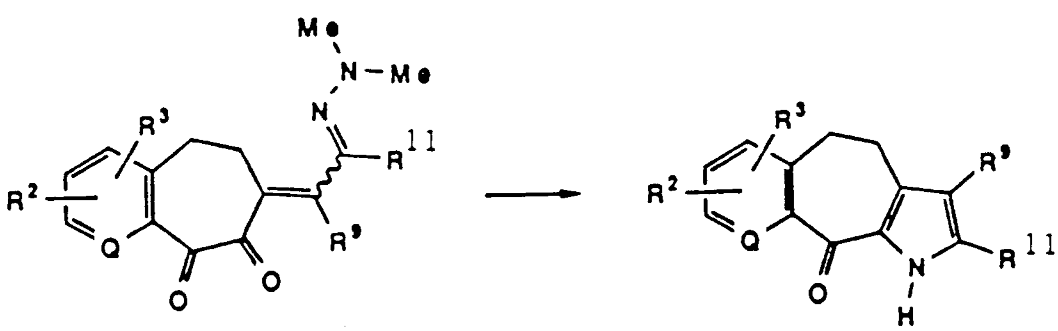

5.5

4.7

p) Reacting a compound of formula 5.3 with a hydroxylamine hydrochloride

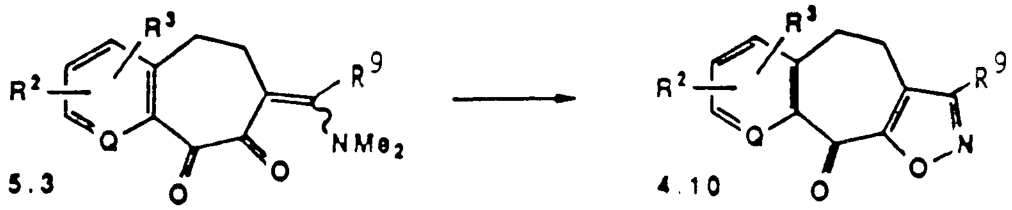

5.3 4.10

q) Reacting a compound of formula 5.3 with a compound of formula $H_2N-C(=NH)R^y$ where $R^y$ represents one of H, lower alkyl, OH, O-(lower alkyl), SH, S-(lower alkyl), $NH_2$, NH-(lower alkyl), N-(lower $alkyl)_2$, and NH(C=O)-(lower alkyl)

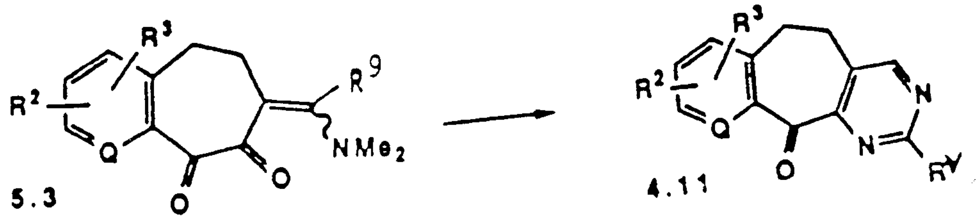

5.3 4.11

r) Reacting a compound of formula 5.3 with a compound of formula $H_2NC(=O)CH_2E$, in which E is taken from among CHO, C(=O)-(lower alkyl), $CO_2$-(lower alkyl), $CONH_2$, $NO_2$, CN, $SO_2CH_3$, and $CF_3$:

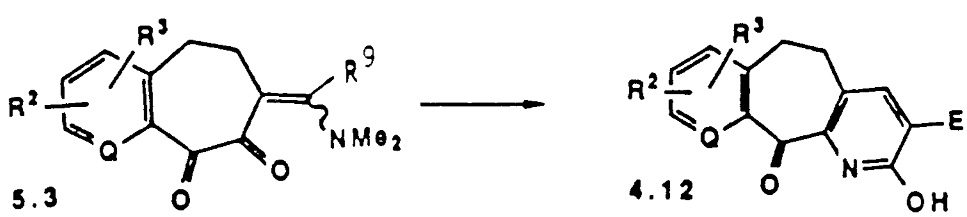

5.3 4.12

s) Reacting a compound of formula 6.0 with formamide, p-toluenesulfonic acid, and N,N′,N″-methylidynetris-formamide $(HC(NHCHO)_3)$

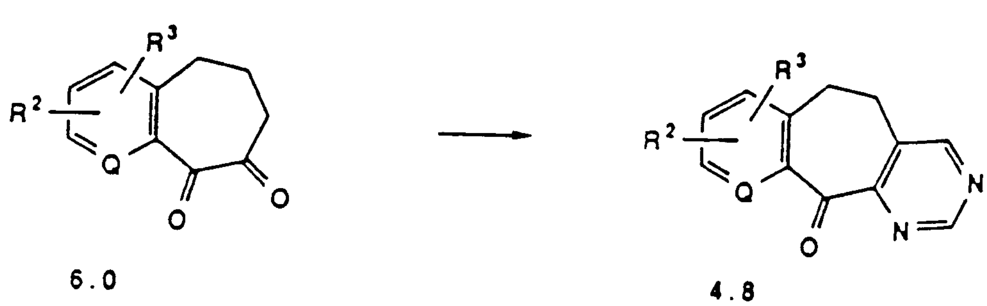

6.0 4.8

t) Reacting a compound of 5.6 with hydrazine followed by oxidation

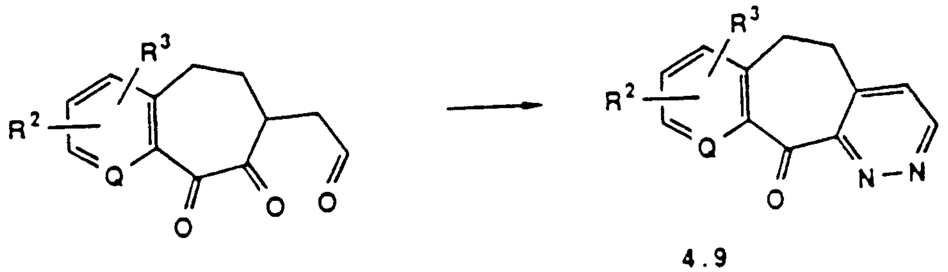

5.6

4.9

**Claims for the following Contracting States : GR, ES**

1. A method of making a compound of formula 1.0

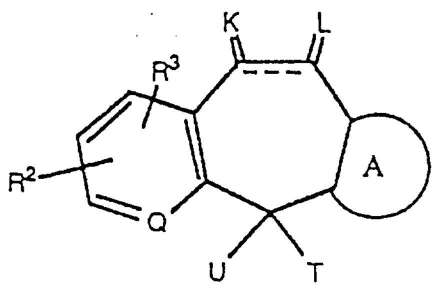

or a pharmaceutically acceptable salt or solvate thereof, wherein

U and T together represent =O, or U represents H and T represents

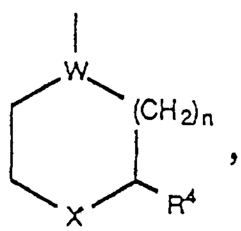

or

U and T together represent

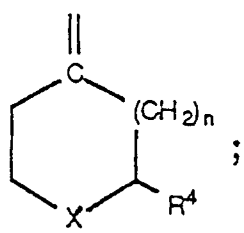

Q is CH, N or N→O;

$R^2$ and $R^3$ may be the same or different and each independently represents H, $C_1$-$C_8$ alkyl, $CF_3$, $NO_2$, halo, $OR^5$, $NR^5R^6$, or $C_1$-$C_8$ $S(O)_m$-alkyl, in which:

$R^5$ and $R^6$ may be the same or different and each is independently selected from H, $C_1$-$C_8$ alkyl or $C_1$-$C_8$

acyl, $C_7$-$C_{16}$ aroyl, and m is O, 1 or 2;

$R^4$ represents $H_1$ $C_1$-$C_8$ alkyl or $C_6$-$C_{15}$ aryl

K represents -H, -H or -H, $C_1$-$C_8$ alkyl or $C_1$-$C_8$ alkyl, $C_1$-$C_8$ alkyl, or -H, -OH or =O;

L represents -H, -H or -H, $C_1$-$C_8$ alkyl or $C_1$-$C_8$ alkyl, $C_1$-$C_8$ alkyl or -H, -OH or =O, with the proviso that when L or K is -H, -OH or =O, then the other of K or L, respectively, is -H, -H or -H, $C_1$-$C_8$ alkyl or $C_1$-$C_8$ alkyl, $C_1$-$C_8$ alkyl;

ring

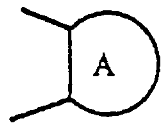

represents

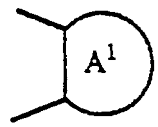

when U represents H and T represents

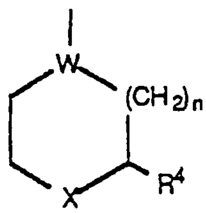

or when T and U together represent

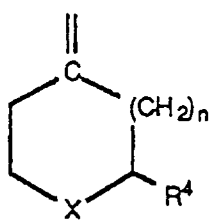

;

and ring

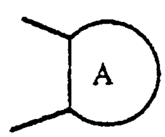

represents

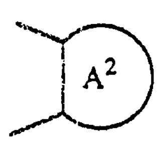

when T and U together represent =O;

ring

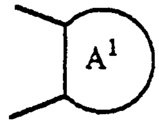

represents a fused 5-membered heterocyclic aromatic ring having at least one heteroatom selected from O, S or N in the ring, said ring optionally being substituted by 1 to 3 R groups selected from $C_1$-$C_8$ alkyl, $C_6$-$C_{15}$ aryl and $C_7$-$C_{16}$ arylmethyl or by two R groups on adjacent ring atoms which R groups together with such adjacent ring atoms represent a fused benzene or fused pyridine ring, or ring

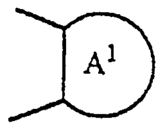

represents a fused 6-membered heterocyclic aromatic ring having 2 or 3 ring nitrogen atoms such as

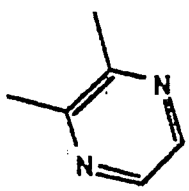 , 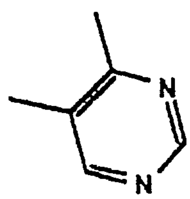 , 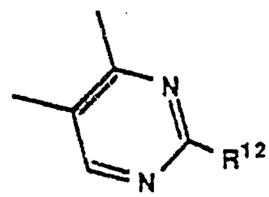 ,

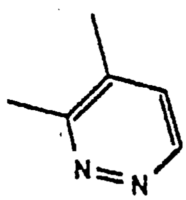 , 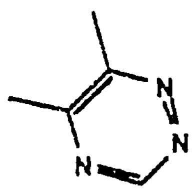 or 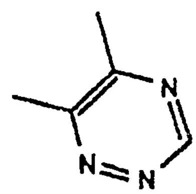 ;

or ring

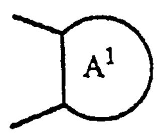

represents a fused 6-membered heterocyclic aromatic ring having 1 ring nitrogen atom and in which Q is CH and X represents

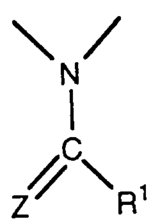

and $R^1$ represents $C_{2-14}$ heteroaryl wherein the hetero atom is N→O;

ring

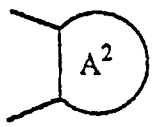

represents

(i) when Q is N or N→O:

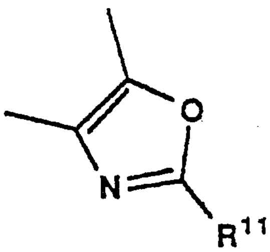 , 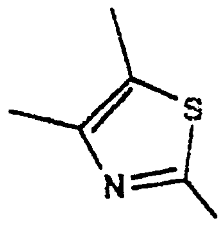 or 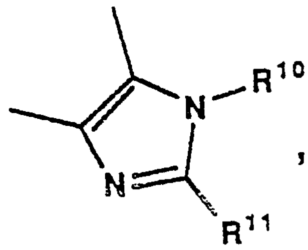 ,

or (ii) when Q is CH, N or N→O:

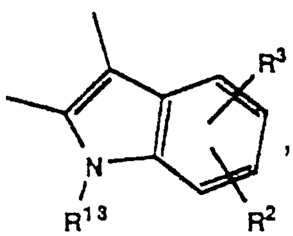 , 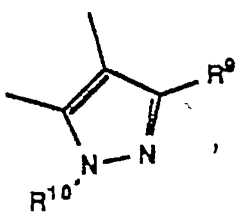 , 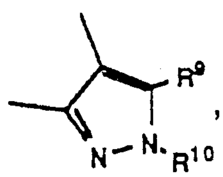 , 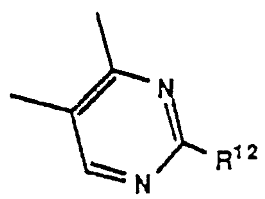 ,

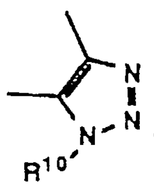 , 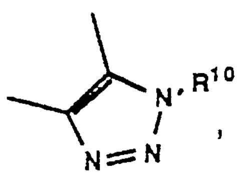 , 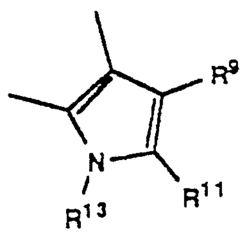 , 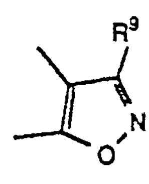 ,

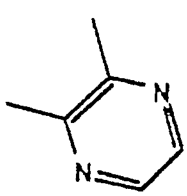 , 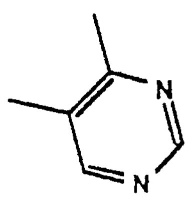 , 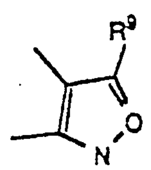 , 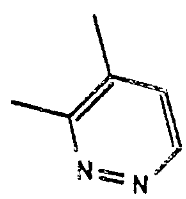 ,

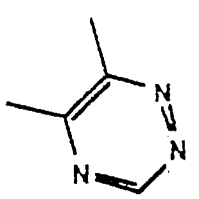 or 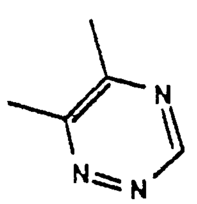 ;

in which:

$R^9$ is H, $C_1$-$C_8$ alkyl or $C_6$-$C_{15}$ aryl;

$R^{10}$ is H, $C_1$-$C_8$ alkyl, $C_6$-$C_{15}$ aryl, $C_7$-$C_{23}$ arylalkyl, $C_1$-$C_8$ acyl, $C_7$-$C_{16}$ aroyl and $C_2$-$C_{14}$ heteroaroyl wherein the aryl moiety is optionally substituted by one or more substituents selected from H, halogen, $NO_2$, $CF_3$, -SH, $C_1$-$C_8$ S-alkyl, $C_1$-$C_8$ $S(O)_m$-alkyl, $C_1$-$C_8$ alkyl, $CO_2H$, $CH_2OH$, C(OH)-($C_1$-$C_8$ lower alkyl), $NH_2$, NH-($C_1$-$C_8$ lower alkyl), N-($C_1$-$C_8$ lower alkyl)$_2$, OH, O-($C_1$-$C_8$ lower alkyl), O-($C_6$-$C_{15}$ aryl), O-($C_7$-$C_{16}$ aroyl), O-($C_2$-$C_{14}$ heteroaroyl), NH($C_1$-$C_8$ acyl), N($C_1$-$C_8$ acyl)$_2$, NH($C_7$-$C_{16}$ aroyl), N($C_7$-$C_{16}$ aroyl)$_2$, NH($C_2$-$C_{14}$ heteroaroyl, N($C_2$-$C_{14}$ heteroaroyl)$_2$,

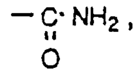 , 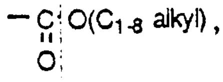 ,

-CHO,

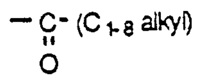 , 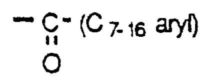

and -C≡N;

$R^{11}$ represents H, $C_1$-$C_8$ alkyl, $C_6$-$C_{15}$ aryl or $C_7$-$C_{23}$ aralkyl;

$R^{12}$ represents H, $C_1$-$C_8$ alkyl, OH, O-($C_1$-$C_8$ alkyl), SH, S($C_1$-$C_8$ alkyl), $NH_2$, NH-($C_1$-$C_8$ alkyl), N-($C_1$-$C_8$ alkyl)$_2$, and NH(C=O)-($C_1$-$C_8$ alkyl);

$R^{13}$ is H, $C_1$-$C_8$ alkyl, $C_6$-$C_{15}$ aryl or $C_7$-$C_{16}$ arylmethyl; and

$R^{14}$ represents H, $CO_2H$, $CH_2OH$, C(OH)-($C_1$-$C_8$ alkyl) $NH_2$, NH-($C_1$-$C_8$ alkyl), N-($C_1$-$C_8$ alkyl)$_2$, NH(C=O)-($C_1$-$C_8$ alkyl), $CH_2NH_2$, $CH_2$NH-($C_1$-$C_8$ alkyl), $CH_2$N-($C_1$-$C_8$ alkyl)$_2$, $CH_2$NH(C=O)-($C_1$-$C_8$ alkyl), CHO, C(=O)-($C_1$-$C_8$ alkyl), $CO_2$-($C_1$-$C_8$ alkyl), $CONH_2$, $NO_2$, CN, $CF_3$ and -$S(O)_mR^{19}$ where $R^{19}$ represents $C_1$-$C_8$ alkyl, $C_6$-$C_{15}$ aryl or $C_7$-$C_{23}$ aralkyl;

W is N or N→O;

the broken line in the seven-membered ring represents an optional double bond;

n is 0,1,2 or 3;

X represents

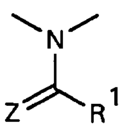 , 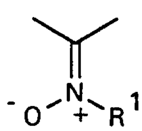 or 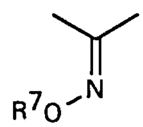

in which:

$R^7$ represents H, $C_1$-$C_8$ alkyl, $C_1$-$C_8$ acyl or $C_7$-$C_{16}$ aroyl, Z is O or S, and $R^1$ is H, $C_1$-$C_8$ alkyl, $C_5$-$C_8$ cycloalkyl, $CF_3$, $C_6$-$C_{15}$ aryl, $C_2$-$C_{14}$ heteroaryl, $NR^5R^6$ wherein $R^5$ and $R^6$ are as defined above, $C_1$-$C_8$ alkylthio or $C_1$-$C_8$ alkoxy, or $R^1$ and $R^4$ together represent-$(CH_2)_k$- where k is 1,2, or 3 so as to form a fused ring,

excluding those compounds where Q is CH, ring $A^1$ is a fused, 6 membered heterocyclic ring having one ring nitrogen and X represents

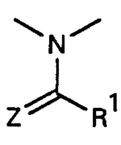

wherein

(a) Z = S or Z = O and $R^1$ is part of a fused ring or is selected from H, alkyl, cycloalkyl, aryl, alkylthio, -$NH_2$, N(alkyl)$_2$, and heteroaryl in which the heteroatom is O, S or N; or

(b) Z = O and $R^1$ is $CF_3$; or

(c) Z = S and $R^1$ is alkoxy,

the method comprising:

(a) when X represents

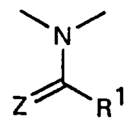

reacting a compound of formula 9.1 with compound 8.11 in the presence of base

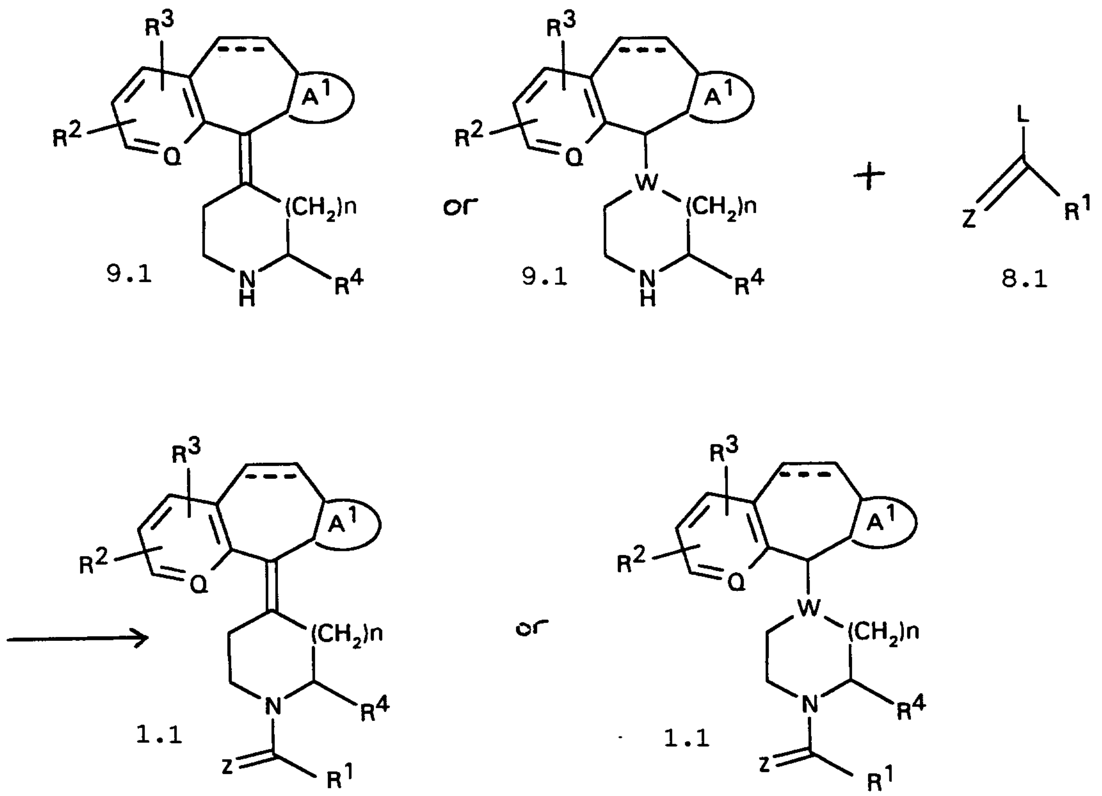

9.1 or 9.1 + 8.1

1.1 or 1.1

b) where X represents

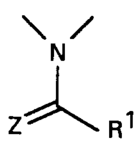

where Z is =O or =S, reacting a compound of formula 11.1 with a compound of formula 8.1

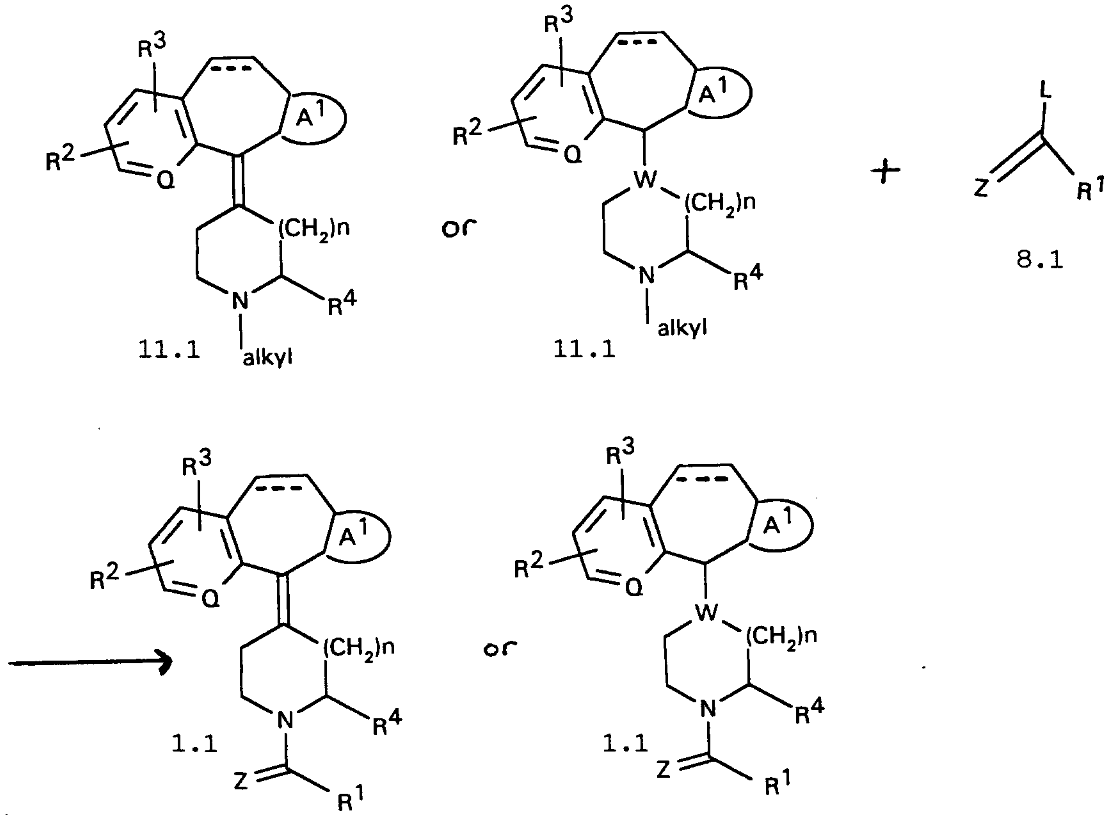

11.1 or 11.1 + 8.1

1.1 or 1.1

c) where X represents

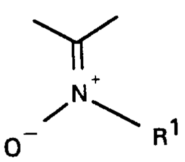

or

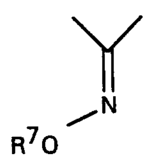

reacting a compound of formula 13 in the presence of hydroxylamine or O-substituted hydroxylamine or N-substituted hydroxylamine;

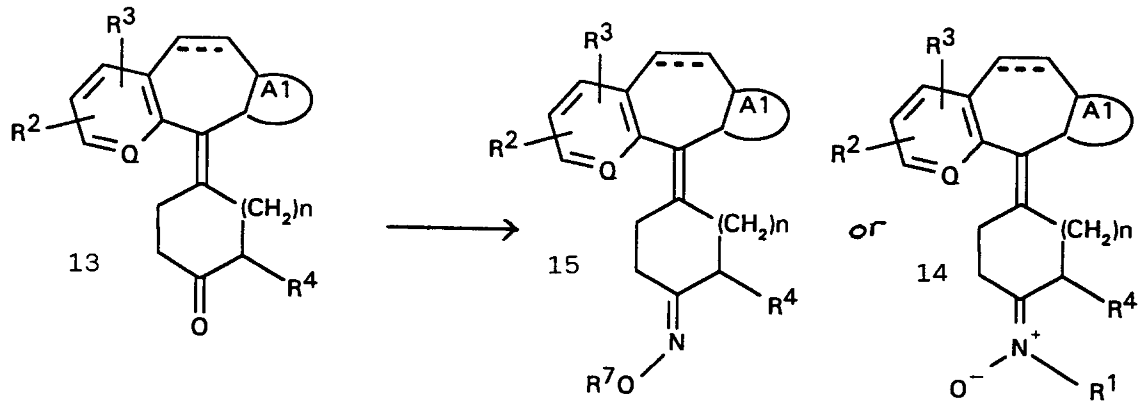

13 → 15 or 14

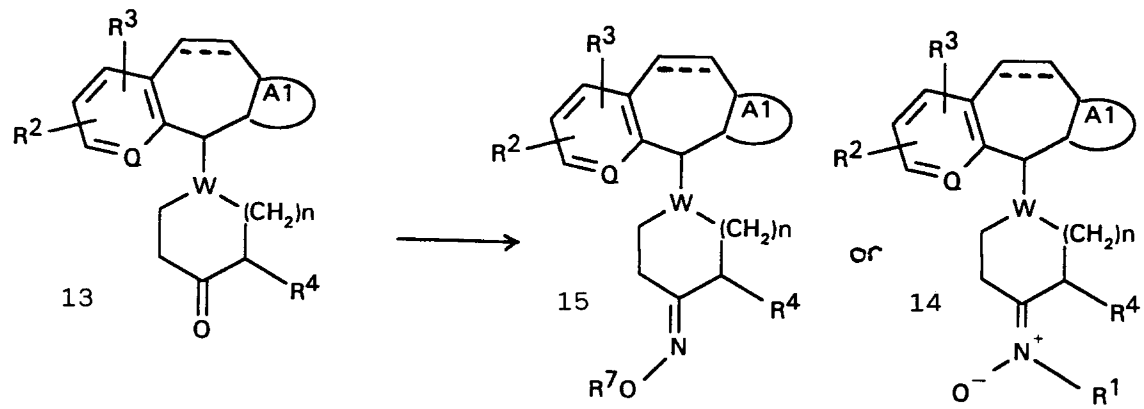

13 → 15 or 14

d) reacting a compound of formula 9.2 with an acid

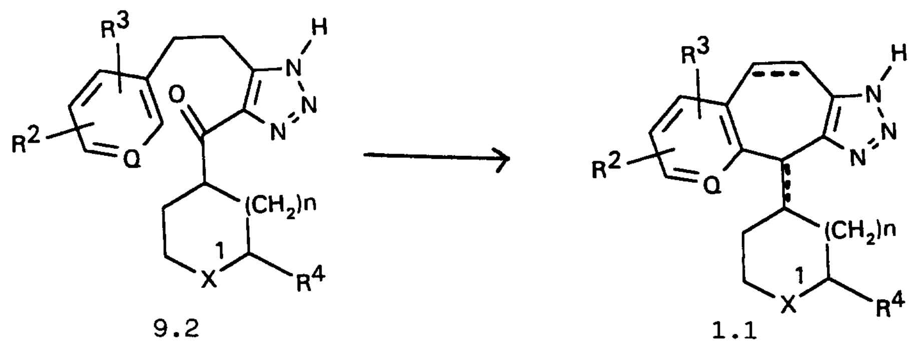

9.2 → 1.1

e) reacting a compound of formula 200 with an alkali or alkaline metal hydroxide

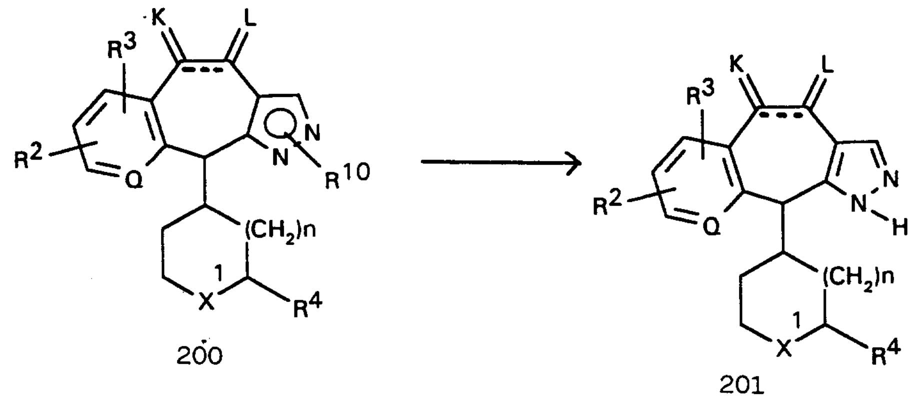

200

201

f) Reacting a compound of formula 17 with a suitable acid

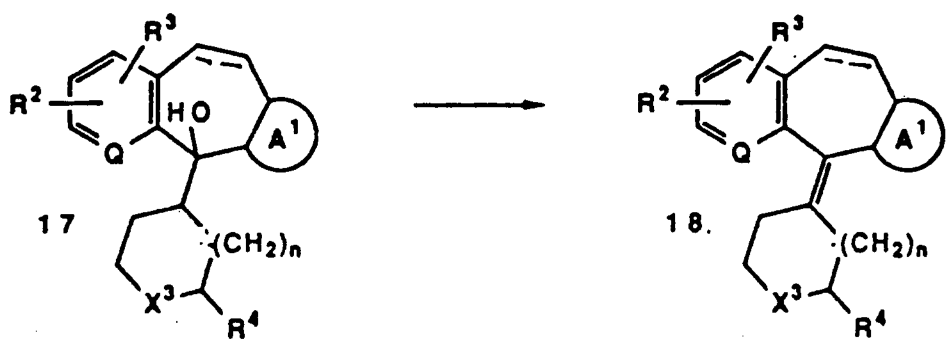

17

18.

wherein $X^3$ represents

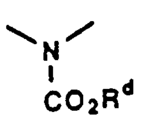

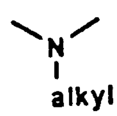

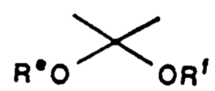

or

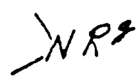

$R^d$ represents an optionally substituted alkyl, alkenyl, aryl or aralkyl group;

$R^e$ and $R^f$ each independently represent lower alkyl or together represent an alkanediyl chain of 2 or 3 methylene groups;

$R^g$ is H or $C(Z)R^1$;

g) Reacting a compound of formula 19 with a compound of formula 20 where L is a leaving group and $X^3$ represents $NR^g$ or

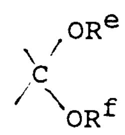

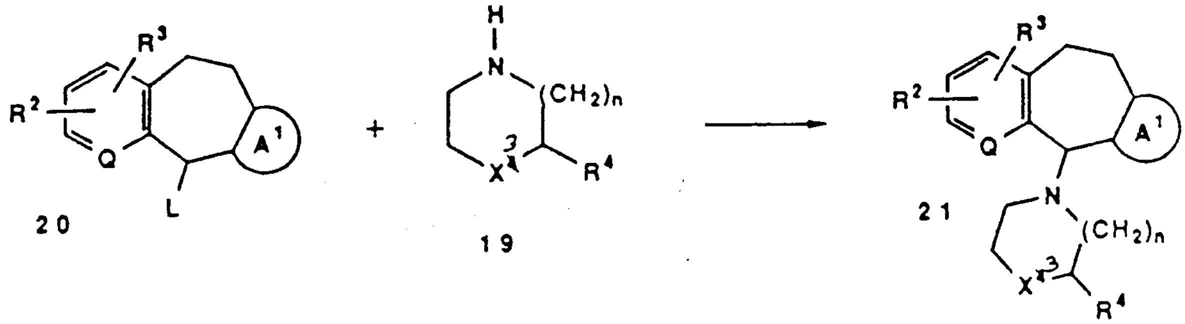

20 19 21

h) Reacting a compound of formula 4.0 with a compound of formula 19

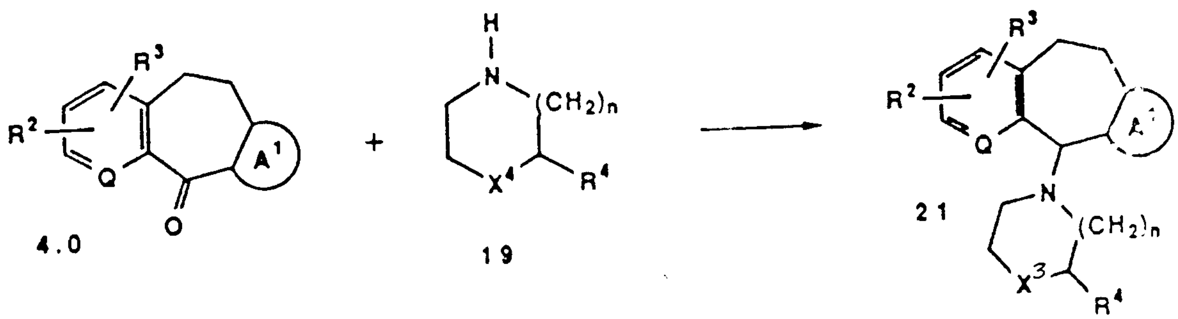

4.0 19 21

i) Dehydrating a compound of formula 26 to produce compound 27

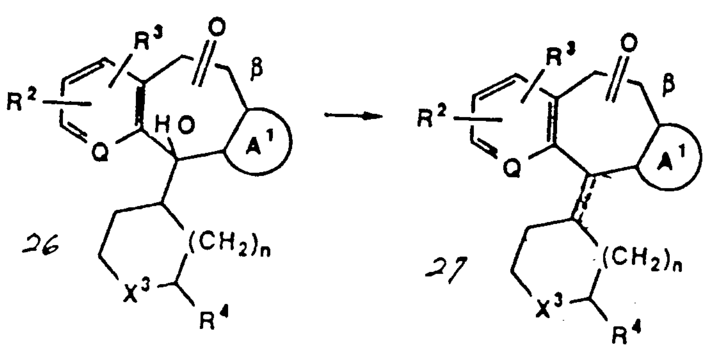

26 27

j) Hydrolyzing compound 30 with a strong aqueous acid to produce compound 31

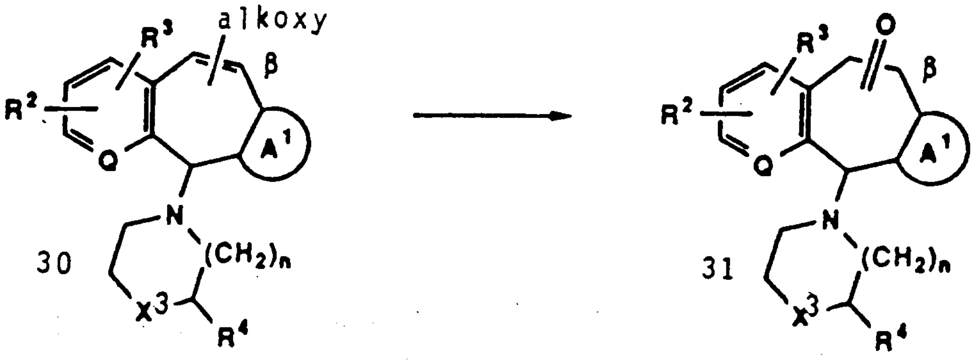

30 31

k) Reacting a compound of formula 5.1 with a compound jof formula $R^{11}CO_2H$, and $(R^{11}CO)_2O$ or $(R^{11}CO_2)_2O$ alone in the presence of HCl

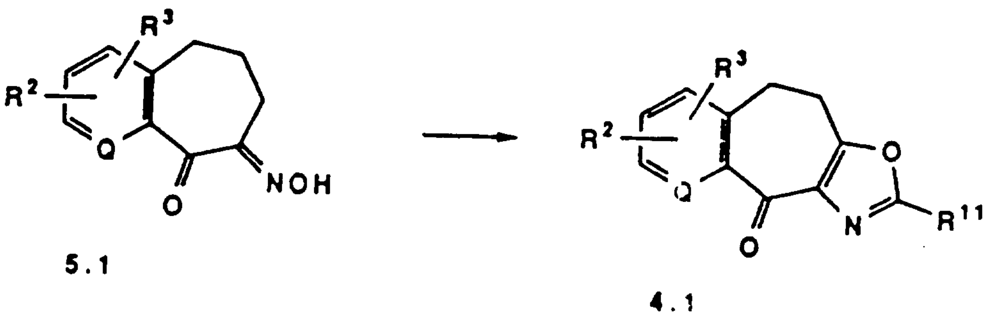

5.1 4.1

l) Reacting a compound of formula 5.2 with an acidic reagent and wherein Ar represents an appropriate aryl group

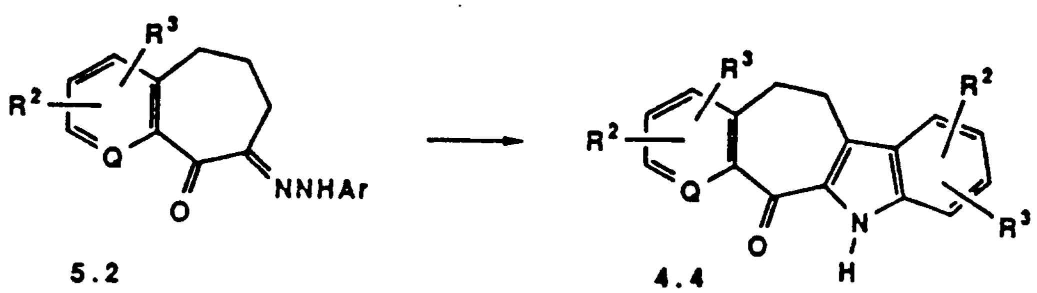

5.2 4.4

m) Reacting a compound of formula 5.3 with a hydrazine reagent $R^{10}NHNH_2$ and an acid catalyst

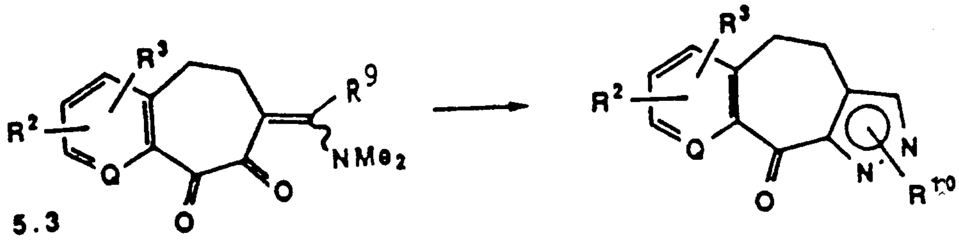

5.3

4.5

n) Reacting a compound of formula 5.4 with an acid or peracid, to produce a compound of formula 4.6

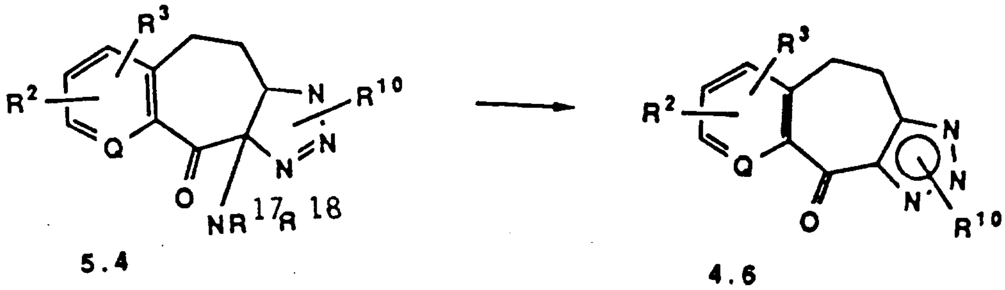

5.4

4.6

o) Reacting a compound of formula 5.5 with an appropriate alkali metal dithionite in a hot solvent

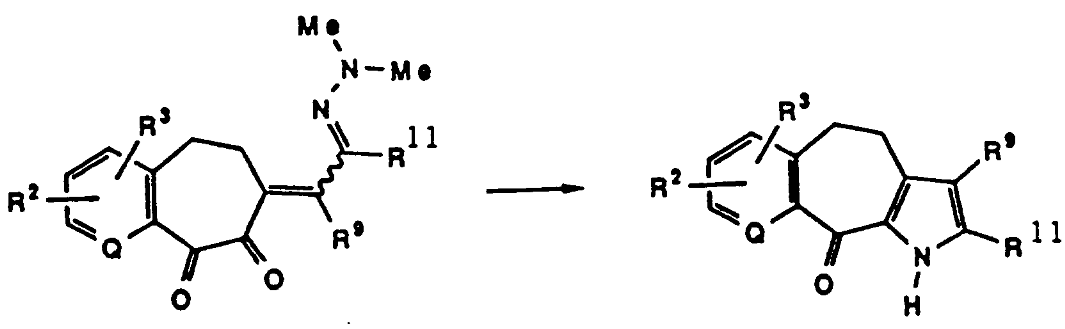

5.5

4.7

p) Reacting a compound of formula 5.3 with a hydroxylamine hydrochloride

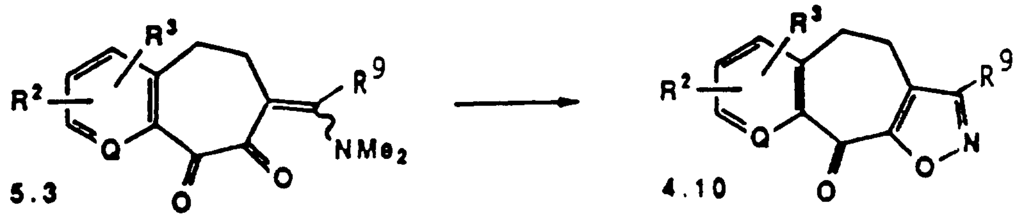

5.3 4.10

q) Reacting a compound of formula 5.3 with a compound of formula $H_2N-C(=NH)R^y$ where $R^y$ represents one of H, lower alkyl, OH, O-(lower alkyl), SH, S-(lower alkyl), $NH_2$, NH-(lower alkyl), N-(lower alkyl)$_2$, and NH(C=O)-(lower alkyl)

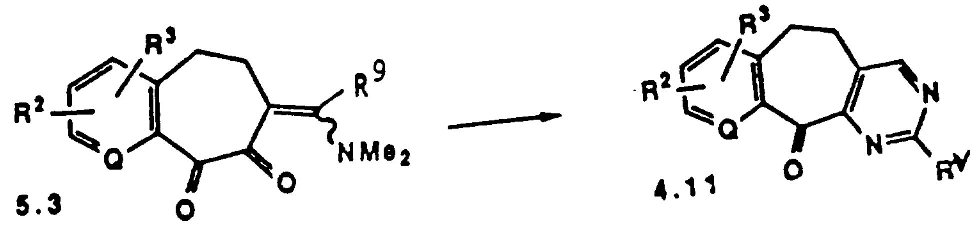

5.3 4.11

r) Reacting a compound of formula 5.3 with a compound of formula $H_2NC(=O)CH_2E$, in which E is taken from among CHO, C(=O)-(lower alkyl), $CO_2$-(lower alkyl), $CONH_2$, $NO_2$, CN, $SO_2CH_3$, and $CF_3$:

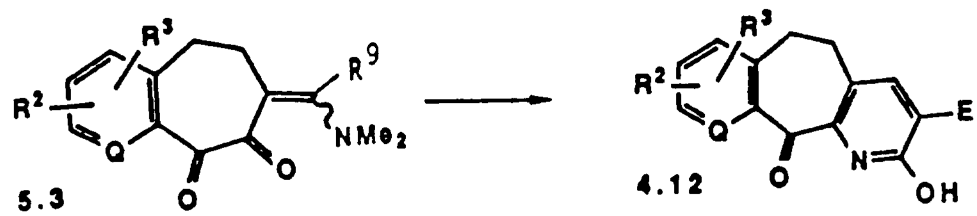

5.3 4.12

s) Reacting a compound of formula 6.0 with formamide, p-toluenesulfonic acid, and N,N',N"-methylidynetrisformamide $(HC(NHCHO)_3)$

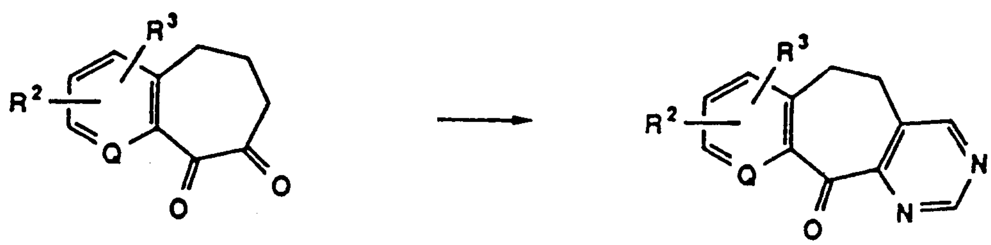

6.0 4.8

t) Reacting a compound of 5.6 with hydrazine followed by oxidation

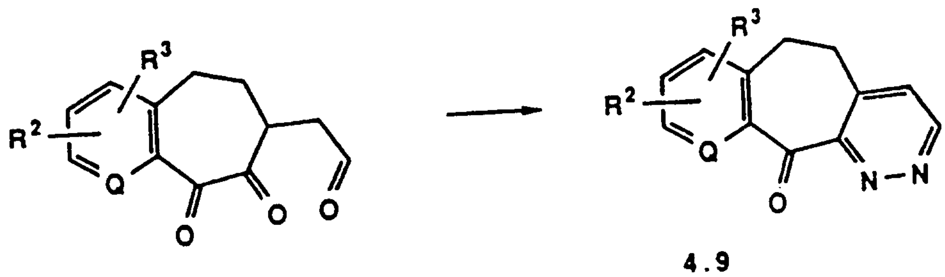

4.9

5.6

2. Use of a compound of formula 1.0 in the manufacture of a medicament for the treatment of PAF disorders, allergy and/or inflammation.

## Patentansprüche

### Patentansprüche für folgende Vertragsstaaten : AT, BE, CH, DE, FR, GB, IT, LI, LU, NL, SE

1. Verbindung der Formel 1.0

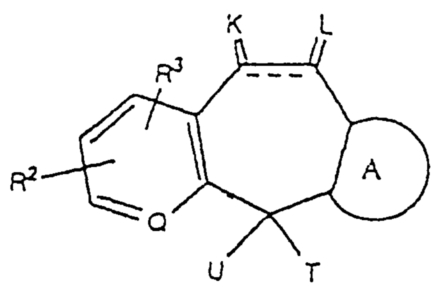

oder ein pharmazeutisch annehmbares Salz oder Solvat derselben, worin
U und T zusammen =O darstellen oder U H darstellt und T

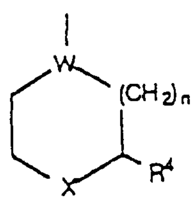

darstellt, oder
U und T zusammen

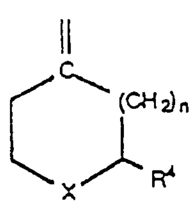

darstellen;

Q CH, N oder N→O ist;
$R^2$ und $R^3$ gleich oder verschieden sein können und jeweils unabhängig H, $C_1$-$C_8$-Alkyl, $CF_3$, $NO_2$, Halogen, $OR^5$, $NR^5R^6$ oder $C_1$-$C_8$-$S(O)_m$-Alkyl bedeuten, worin
$R^5$ und $R^6$ gleich oder verschieden sein können und jeweils unabhängig aus H, $C_1$-$C_8$-Alkyl oder $C_1$-$C_8$-Acyl, $C_7$-$C_{16}$-Aroyl ausgewählt sind und m 0, 1 oder 2 ist;
$R^4$ H, $C_1$-$C_8$-Alkyl oder $C_6$-$C_{15}$-Aryl darstellt;
K -H, -H oder -H, $C_1$-$C_8$-Alkyl oder $C_1$-$C_8$-Alkyl, $C_1$-$C_8$-Alkyl oder -H, -OH oder =O darstellt;
L -H, -H oder -H, $C_1$-$C_8$-Alkyl oder $C_1$-$C_8$-Alkyl, $C_1$-$C_8$-Alkyl oder -H, -OH oder =O darstellt, mit der Maßgabe, daß wenn L oder K -H, -OH oder =O ist, das andere K beziehungsweise L -H, -H oder -H, $C_1$-$C_8$-Alkyl oder $C_1$-$C_8$-Alkyl, $C_1$-$C_8$-Alkyl ist;
Ring

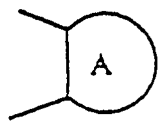 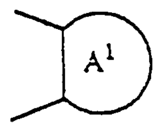

darstellt, wenn U H darstellt und
T

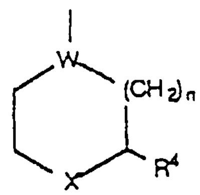

darstellt, oder wenn T und U zusammen

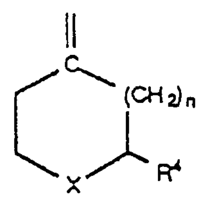

darstellen;
und Ring

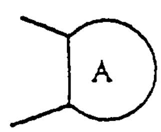 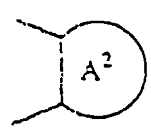

darstellt, wenn T und U zusammen =O darstellen;
Ring

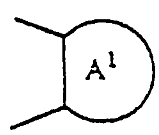

einen kondensierten 5-gliedrigen heterocyclischen aromatischen Ring mit wenigstens einem Heteroatom darstellt, das aus O, S oder N in dem Ring ausgewählt ist, wobei der Ring gegebenenfalls durch 1 bis 3 Gruppen R, die aus

$C_1$-$C_8$-Alkyl, $C_6$-$C_{15}$-Aryl und $C_7$-$C_{16}$-Arylmethyl ausgewählt sind, oder durch zwei Gruppen R an benachbarten Ringatomen substituiert ist, wobei die Gruppen R zusammen mit derartigen benachbarten Ringatomen einen kondensierten Benzol- oder kondensierten Pyridinring darstellen, oder Ring

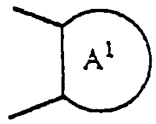

einen kondensierten,6-gliedrigen, heterocyclischen, aromatischen Ring mit 1, 2 oder 3 Ringstickstoffatomen darstellt;
Ring

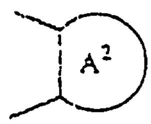

(i) wenn Q N oder N→O ist

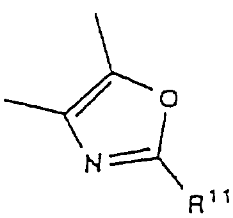 , 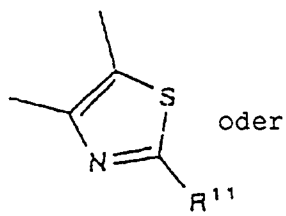 oder 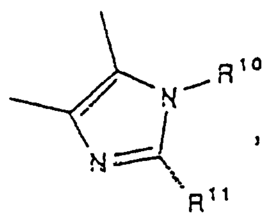 ,

oder (ii) wenn Q CH, N oder N→O ist

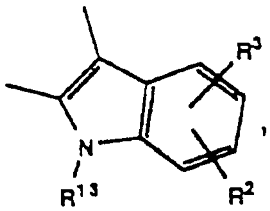, 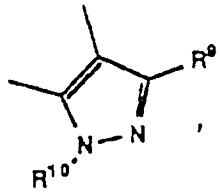, 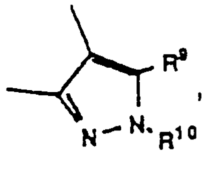, 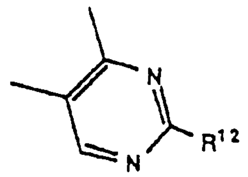,

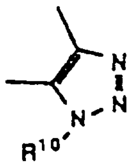, 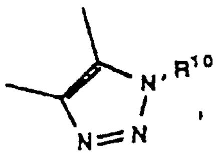, 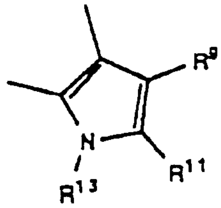, 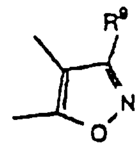,

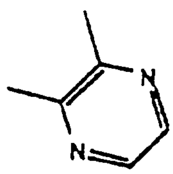, 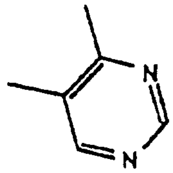, 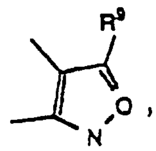, 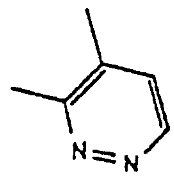,

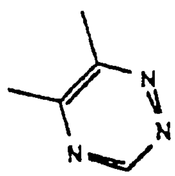 oder 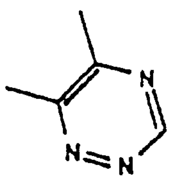;

darstellt, worin

$R^9$ H, $C_1$-$C_8$-Alkyl oder $C_6$-$C_{15}$-Aryl ist;

$R^{10}$ H, $C_1$-$C_8$-Alkyl, $C_6$-$C_{15}$-Aryl, $C_7$-$C_{23}$-Arylalkyl, $C_1$-$C_8$-Acyl, $C_7$-$C_{16}$-Aroyl und $C_2$-$C_{14}$-Heteroaryl ist, worin die Aryl-Struktureinheit gegebenenfalls durch einen oder mehr Substituenten substituiert ist, die aus H, Halogen, $NO_2$, $CF_3$, -SH, $C_1$-$C_8$-S-Alkyl, $C_1$-$C_8$-$S(O)_m$-Alkyl, $C_1$-$C_8$-Alkyl, $CO_2H$, $CH_2OH$, C(OH)-($C_1$-$C_8$-Niederalkyl), $NH_2$, NH-($C_1$-$C_8$-Niederalkyl), N-($C_1$-$C_8$-Niederalkyl)$_2$, OH, O-($C_1$-$C_8$-Niederalkyl), O-($C_6$-$C_{15}$-Aryl), O-($C_7$-$C_{16}$-Aroyl), O-($C_2$-$C_{14}$-Heteroaroyl), NH($C_1$-$C_8$-Acyl), N($C_1$-$C_8$-Acyl)$_2$, NH($C_7$-$C_{16}$-Aroyl), N($C_7$-$C_{16}$-Aroyl)$_2$, NH($C_2$-$C_{14}$-Heteroaryl), N($C_2$-$C_{14}$-Heteroaryl)$_2$,

$$-\underset{\underset{O}{\|}}{C}-NH_2,\quad -\underset{\underset{O}{\|}}{C}-O(C_{1-8}\text{-Alkyl}),$$

-CHO,

$$-\underset{\underset{O}{\|}}{C}-(C_{1-8}\text{-Alkyl}),\quad -\underset{\underset{O}{\|}}{C}-(C_{7-16}\text{-Aryl})$$

und -C≡N ausgewählt sind;

$R^{14}$ H, $C_1$-$C_8$-Alkyl, $C_6$-$C_{15}$-Aryl oder $C_7$-$C_{23}$-Aralkyl darstellt;

$R^{12}$ H, $C_1$-$C_8$-Alkyl, OH, O-($C_1$-$C_8$-Alkyl), SH, S($C_1$-$C_8$-Alkyl), $NH_2$, NH-($C_1$-$C_8$-Alkyl), N-($C_1$-$C_8$-Alkyl)$_2$ und NH(C=O)-($C_1$-$C_8$-Alkyl) darstellt;

$R^{13}$ H, $C_1$-$C_8$-Alkyl, $C_6$-$C_{15}$-Aryl oder $C_7$-$C_{16}$-Arylmethyl ist, und

$R^{14}$ H, $CO_2H$, $CH_2OH$, C(OH)-($C_1$-$C_8$-Alkyl)$NH_2$, NH-($C_1$-$C_8$-Alkyl), N-($C_1$-$C_8$-Alkyl)$_2$, NH(C=O)-($C_1$-$C_8$-Alkyl), $CH_2NH_2$, $CH_2NH$-($C_1$-$C_8$-Alkyl), $CH_2N$-($C_1$-$C_8$-Alkyl)$_2$, $CH_2NH$(C=O)-($C_1$-$C_8$-Alkyl), CHO, C(=O)-($C_1$-$C_8$-Alkyl), $CO_2$-($C_1$-$C_8$-Alkyl), $CONH_2$, $NO_2$, CN, $CF_3$ und -S(O)$_m$$R^{19}$ darstellt , worin $R^{19}$ $C_1$-$C_8$-Alkyl, $C_6$-$C_{15}$-Aryl oder $C_7$-$C_{23}$-Aralkyl darstellt;
W N oder N→O ist;
die durchbrochene Linie in dem siebengliedrigen Ring eine wahlfreie Doppelbindung darstellt;
n 0, 1, 2 oder 3 ist;
X

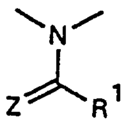 , 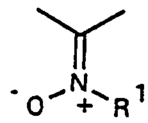 oder 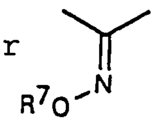

darstellt, worin
$R^7$ H, $C_1$-$C_8$-Alkyl, $C_1$-$C_8$-Acyl oder $C_7$-$C_{16}$-Aroyl darstellt,
Z O oder S ist und $R^1$ H, $C_1$-$C_8$-Alkyl, $C_5$-$C_8$-Cycloalkyl, $CF_3$, $C_6$-$C_{15}$-Aryl, $C_2$-$C_{14}$-Heteroaryl, $NR^5R^6$, worin $R^5$ und $R^6$ wie vorstehend definiert sind, $C_1$-$C_8$-Alkylthio oder $C_1$-$C_8$-Alkoxy darstellt oder $R^1$ und $R^4$ zusammen unter Bilden eines kondensierten Ringes -($CH_2$)$_k$- darstellen, worin k 1, 2 oder 3 ist,
wobei diejenigen Verbindungen ausgeschlossen sind, worin Q CH ist, Ring $A^1$ ein kondensierter, 6-gliedriger, heterocyclischer Ring mit einem Ringstickstoff ist und X

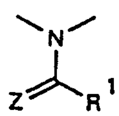

darstellt, worin

(a) Z = S oder Z = O und $R^1$ Teil eines kondensierten Rings ist oder aus H, Alkyl, Cycloalkyl, Aryl, Alkylthio, -$NH_2$, N(Alkyl)$_2$ und Heteroaryl, worin das Heteroatom O, S oder N ist, ausgewählt ist, oder
(b) Z = O und $R^1$ $CF_3$ ist, oder
(c) Z = S und $R^1$ Alkoxy ist.

**2.** Verbindung des Anspruchs 1, die weiter dadurch gekennzeichnet ist, daß sie die Struktur

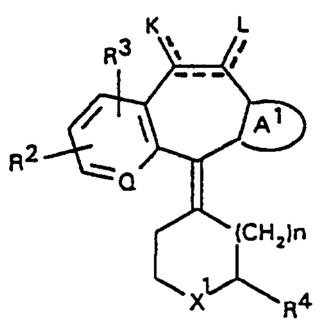

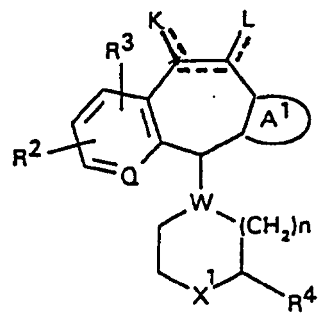

besitzt, worin $X^1$

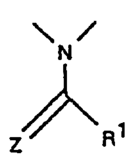

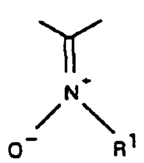 oder 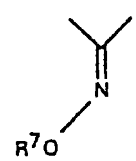

darstellt und worin Ring

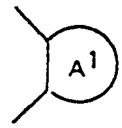

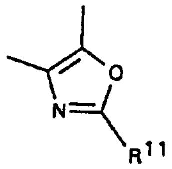
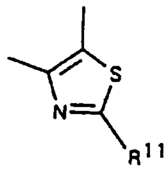
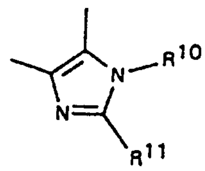
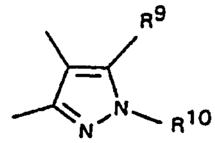

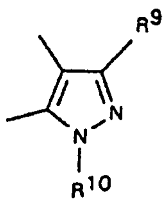
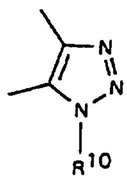
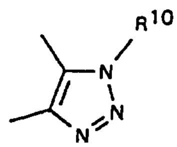
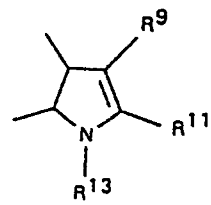

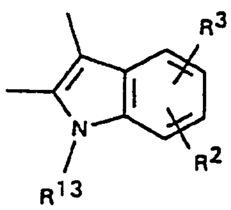
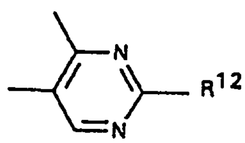
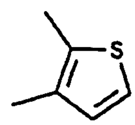

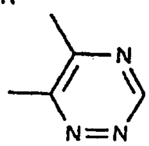
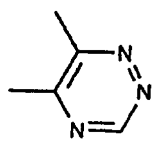
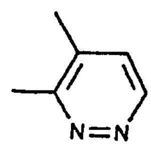

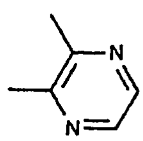 oder 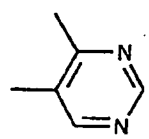

darstellt, worin
$R^9$ aus H, $C_1$-$C_8$-Alkyl oder $C_6$-$C_{15}$-Aryl ausgewählt ist,
$R^{10}$ aus H, $C_1$-$C_8$-Alkyl, $C_6$-$C_{15}$-Aryl oder $C_6$-$C_{15}$-Arylmethyl ausgewählt ist,
$R^{11}$ aus H, $C_1$-$C_8$-Alkyl, $C_6$-$C_{15}$-Aryl oder $C_6$-$C_{15}$-Arylmethyl ausgewählt ist und
$R^{13}$ aus H, $C_1$-$C_8$-Alkyl, $C_6$-$C_{15}$-Aryl oder $C_6$-$C_{15}$-Arylmethyl ausgewählt ist.

3. Verbindung des Anspruchs 1, die weiter dadurch gekennzeichnet ist, daß sie die Struktur

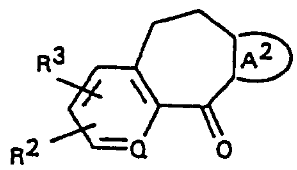

besitzt.

4. Verbindung eines der Ansprüche 1 oder 2, die weiter dadurch gekennzeichnet ist, daß K und L beide H, H darstellen und worin $R^2$, $R^3$ und $R^4$ alle H darstellen.

5. Verbindung eines der Ansprüche 1-4 mit der Strukturformel:

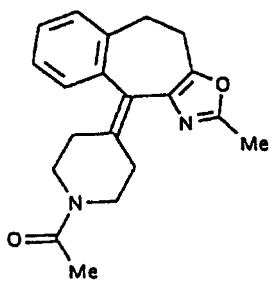

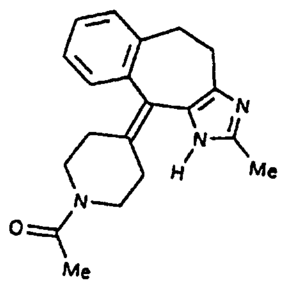

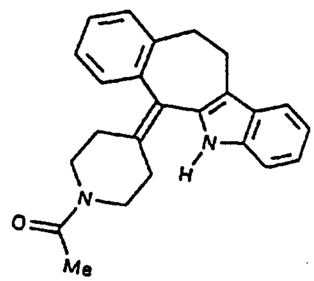

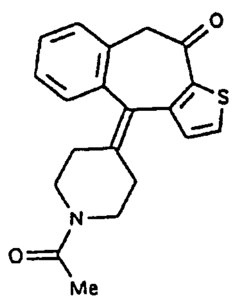

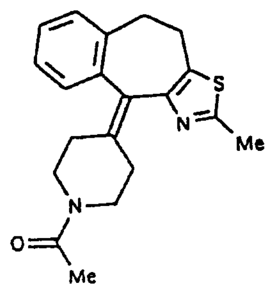

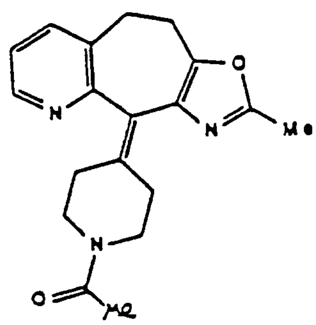

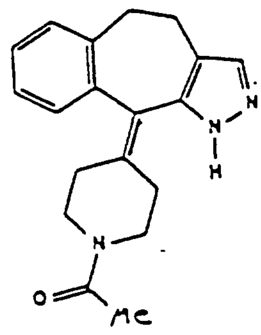

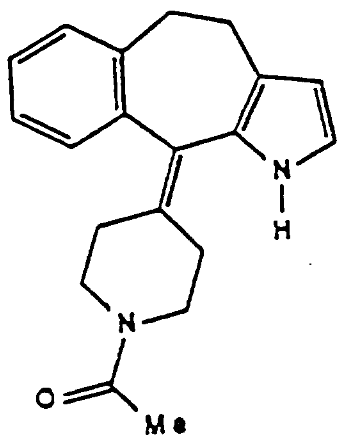

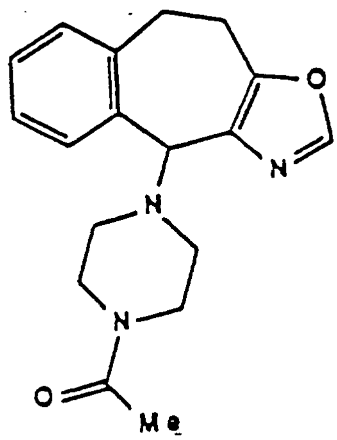

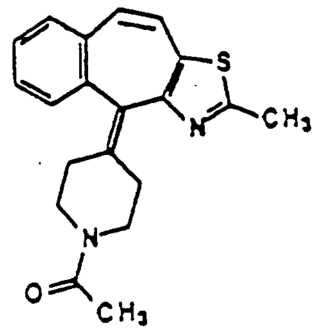

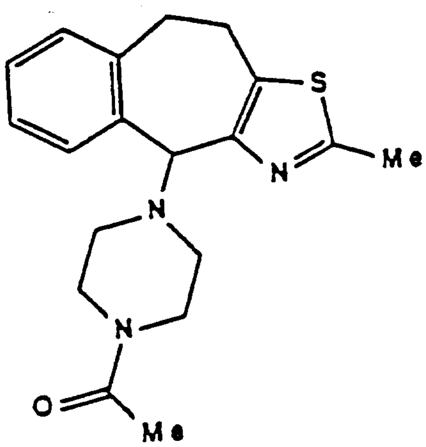

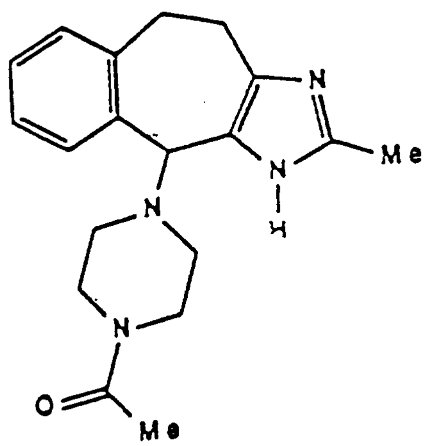

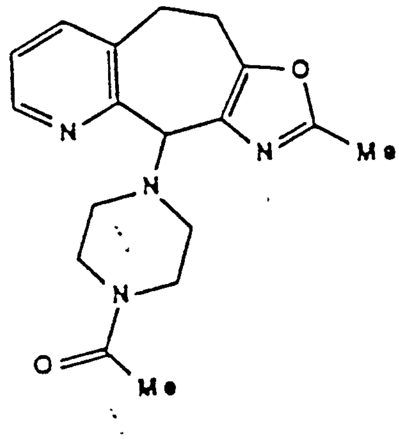

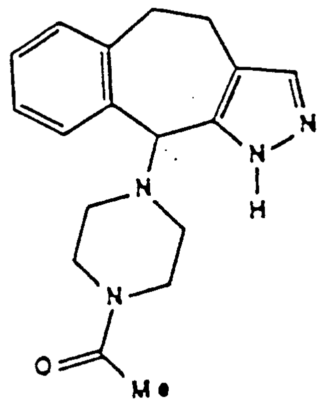

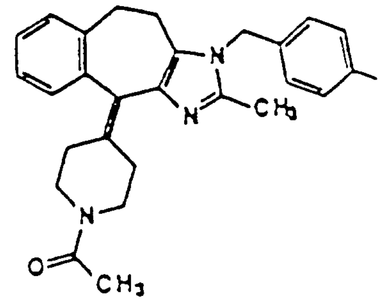

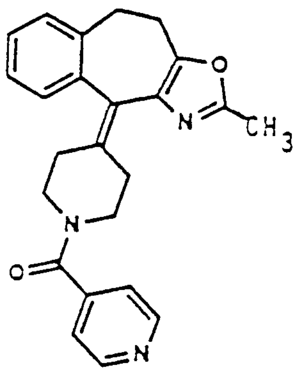

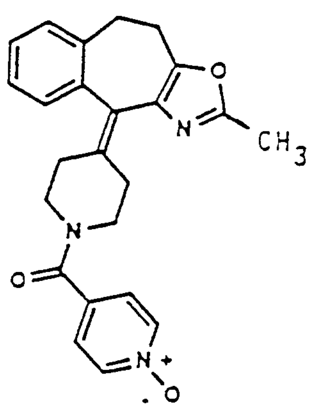

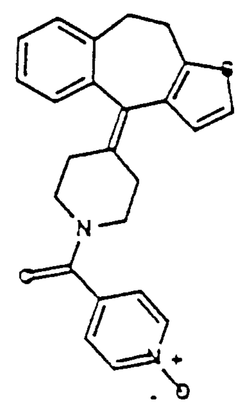

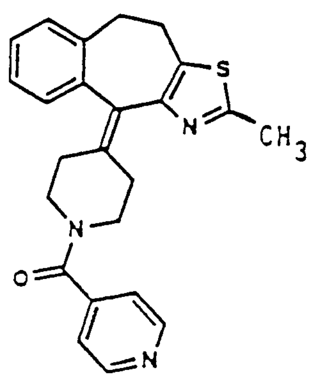

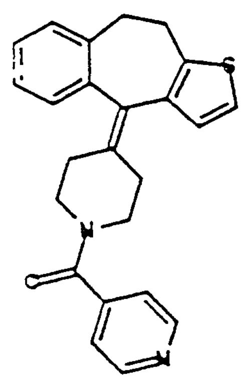

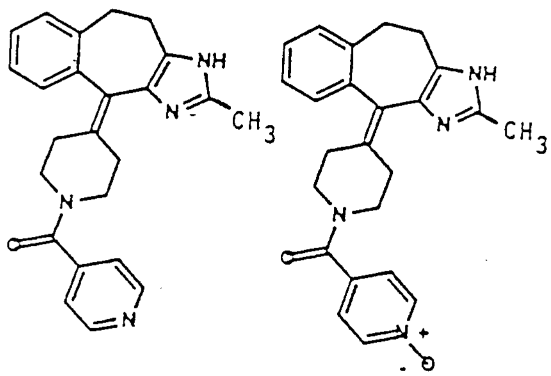

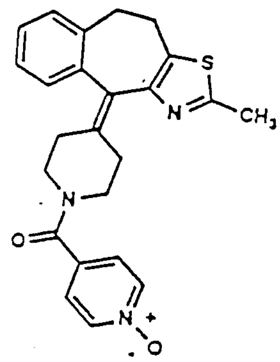

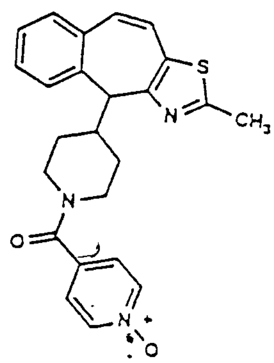

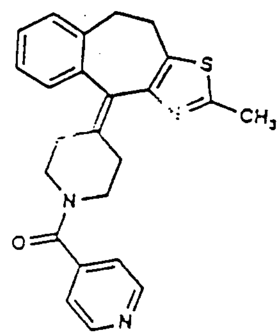

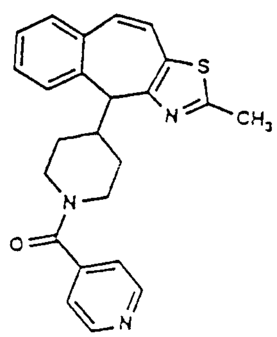

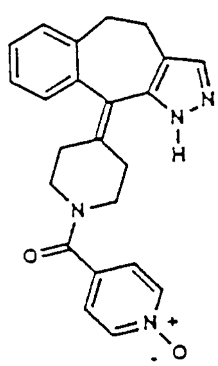

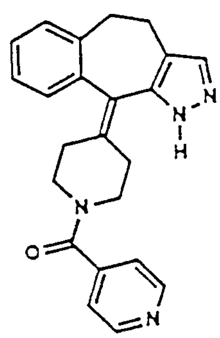

**6.** Pharmazeutische Zusammensetzung, die eine Verbindung der Formel 1.0 des Anspruchs 1 in Kombination mit einem pharmazeutisch annehmbaren Träger umfaßt.

**7.** Verfahren zum Herstellen einer pharmazeutischen Zusammensetzung, welches das Vermischen einer Zusammensetzung der Formel 1.0 des Anspruchs 1 mit einem pharmazeutisch annehmbaren Träger umfaßt.

**8.** Verwendung einer Verbindung der Formel 1.0 des Anspruchs 1 und der pharmazeutisch annehmbaren Salze derselben zur Herstellung eines Arzneimittels für die Behandlung von PAF-Störungen, Allergie und/oder Entzündungen.

**9.** Verfahren zum Herstellen einer Verbindung der Formel 1.0 des Anspruchs 1, welches

a) wenn X

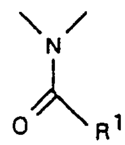

darstellt, das Umsetzen einer Verbindung der Formel 9.1 mit Verbindung 8.11 in Gegenwart einer Base

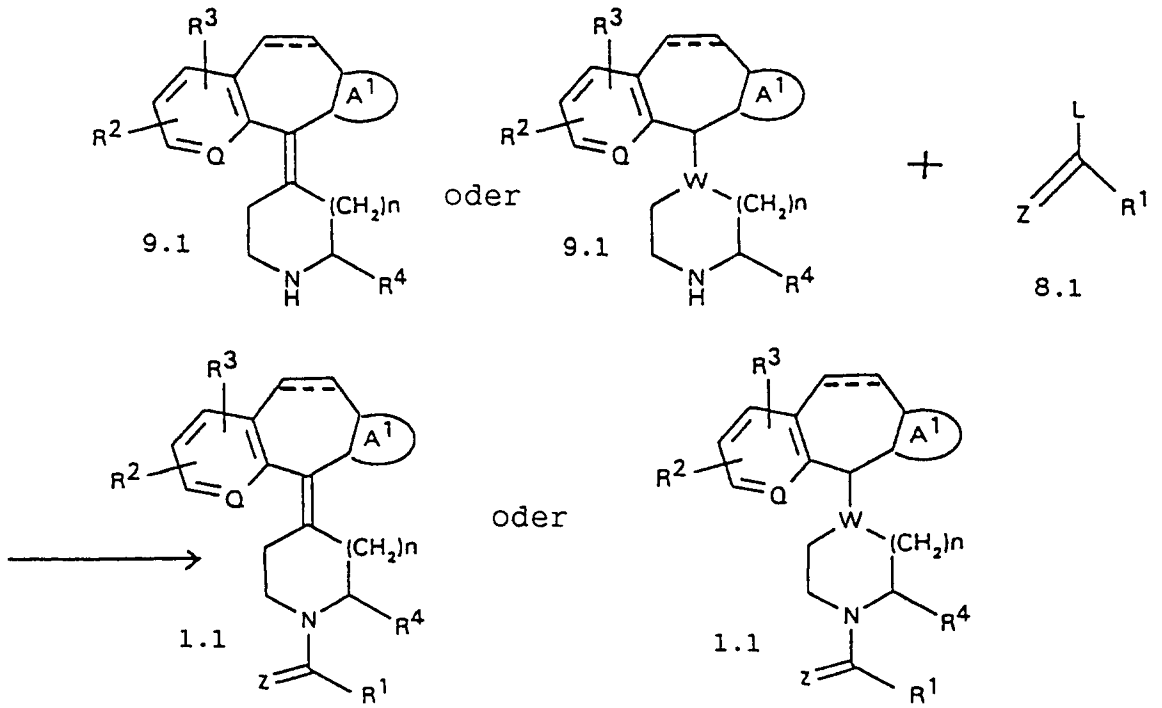

9.1 oder 9.1

8.1

1.1 oder 1.1

b) wenn X

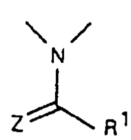

darstellt, worin Z =O oder =S ist, das Umsetzen einer Verbindung der Formel 11.1 mit einer Verbindung der Formel 8.1

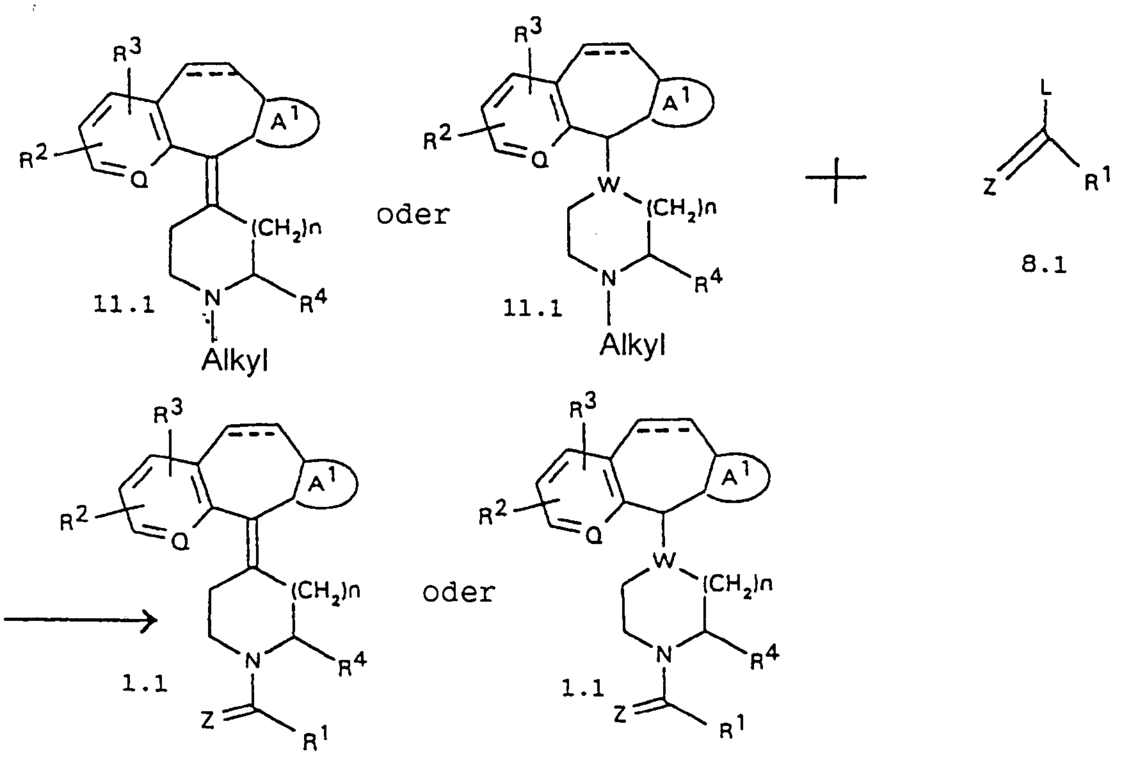

11.1 oder 11.1 + 8.1

1.1 oder 1.1

c) wenn X

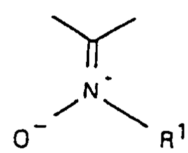

oder

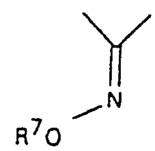

darstellt, das Umsetzen einer Verbindung der Formel 13 in Gegenwart von Hydroxylamin oder eines O-substituierten Hydroxylamins oder N-substituierten Hydroxylamins

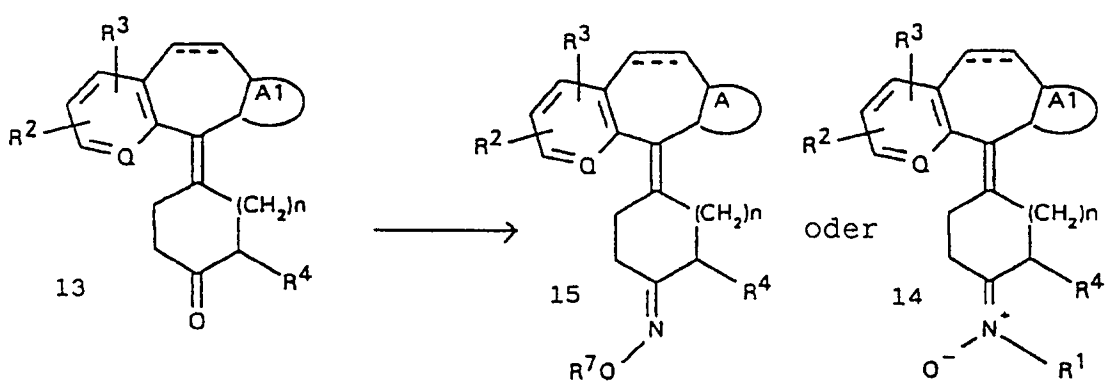

13

15 oder 14

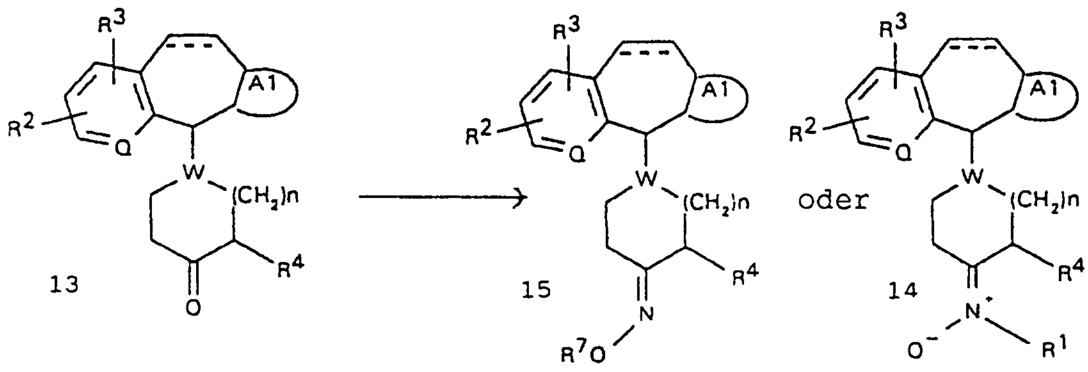

13

15

oder

14

d) Umsetzen einer Verbindung der Formel 9.2 mit einer Säure

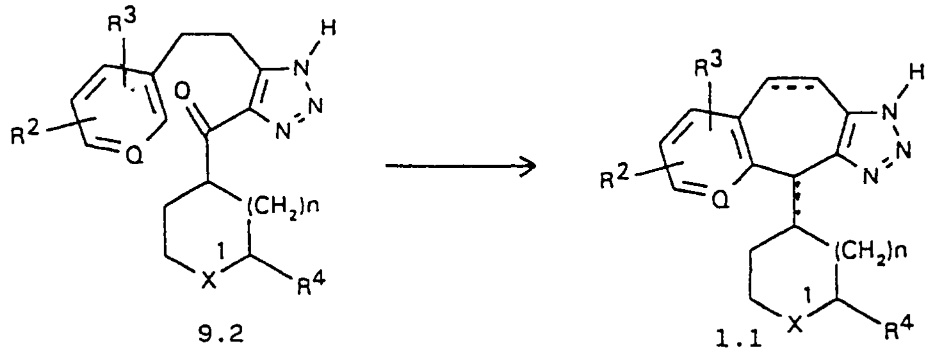

9.2

1.1

e) Umsetzen einer Verbindung der Formel 200 mit einer Alkalie oder einem Alkalimetallhydroxid

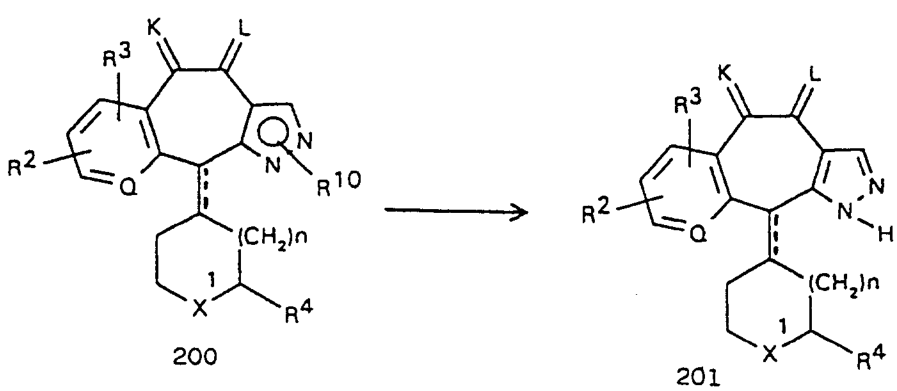

200

201

f) Umsetzen einer Verbindung der Formel 17 mit einer geeigneten Säure

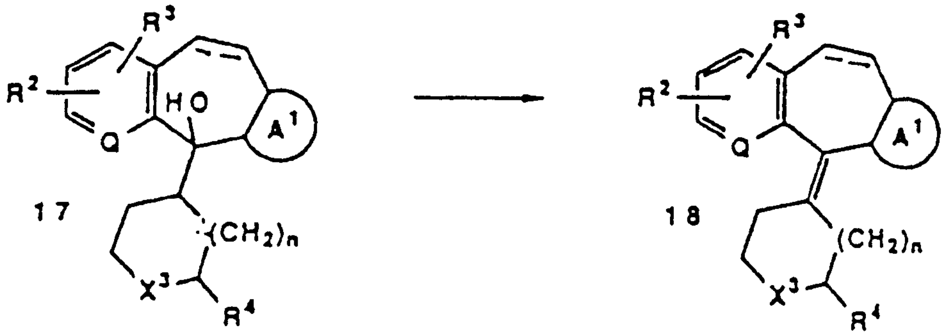

17 18

worin $X^3$

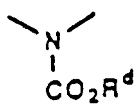

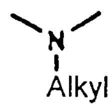

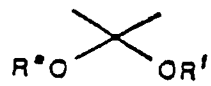

oder

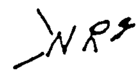

darstellt,
$R^d$ eine gegebenenfalls substituierte Alkyl-, Alkenyl-, Aryl- oder Aralkylgruppe darstellt,
$R^e$ und $R^f$ jeweils unabhängig Niederalkyl darstellen oder zusammen eine Alkandiylkette aus 2 oder 3 Methylengruppen darstellen,
$R^g$ H oder $C(Z)R^1$ ist,

g) Umsetzen einer Verbindung der Formel 19 mit einer Verbindung der Formel 20, worin L eine Abgangsgruppe ist und $X^3$
$NR^g$ oder

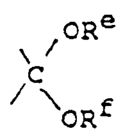

darstellt,

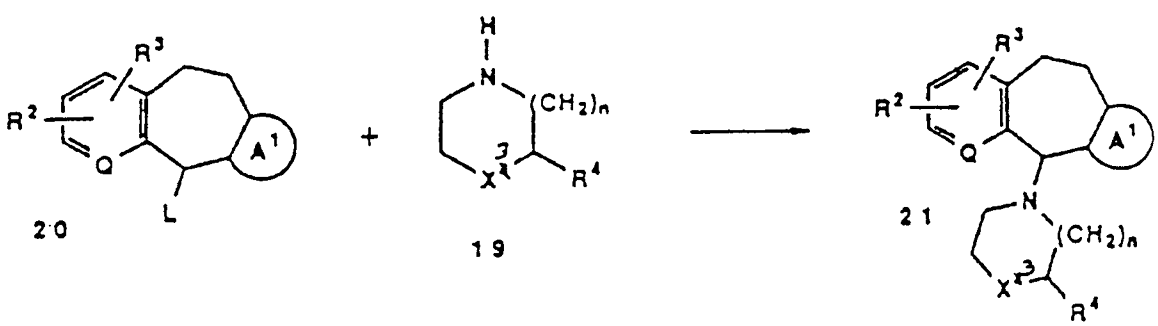

20 19 21

h) Umsetzen einer Verbindung der Formel 4.0 mit einer Verbindung der Formel 19

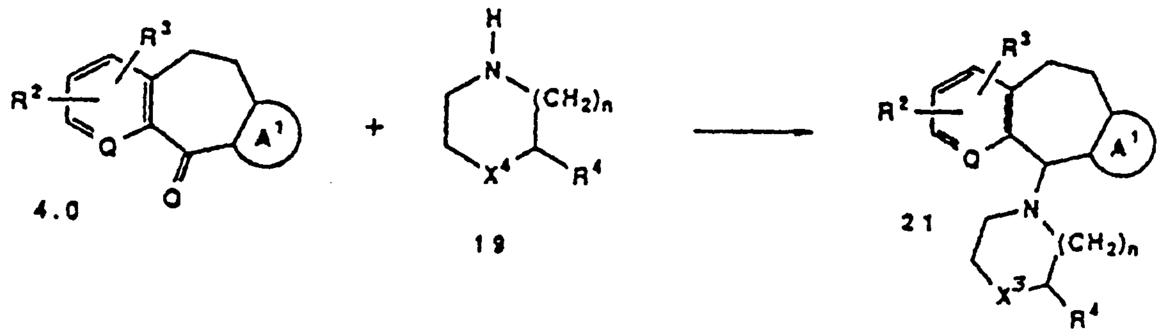

4.0 19 21

i) Dehydratisieren einer Verbindung der Formel 26 unter Herstellen einer Verbindung 27

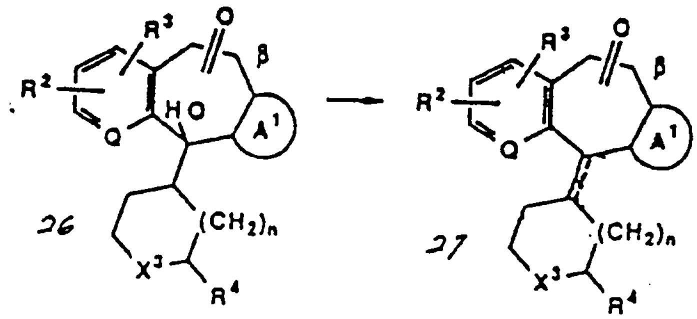

26 27

j) Hydrolysieren von Verbindung 30 mit einer starken wäßrigen Säure unter Herstellen von Verbindung 31

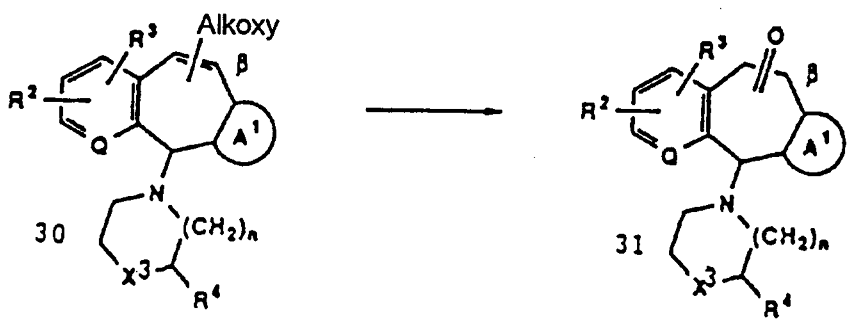

30 31

k) Umsetzen einer Verbindung der Formel 5.1 mit einer Verbindung der Formel $R^{11}CO_2H$ und $(R^{11}CO)_2O$ oder $(R^{11}CO_2)_2O$ allein in Gegenwart von HCl

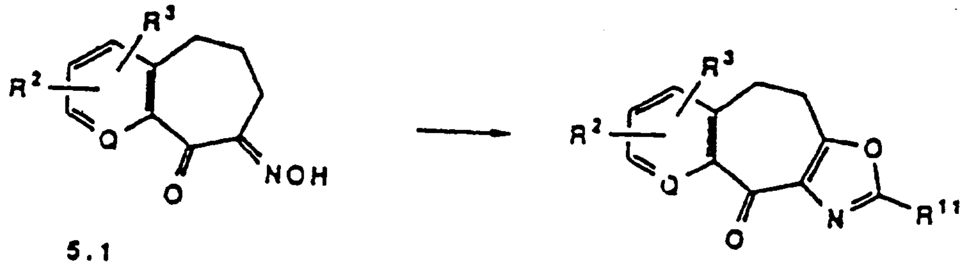

5.1 4.1

l) Umsetzen einer Verbindung der Formel 5.2 mit einem sauren Reagenz und wobei Ar eine geeignete Arylgruppe darstellt

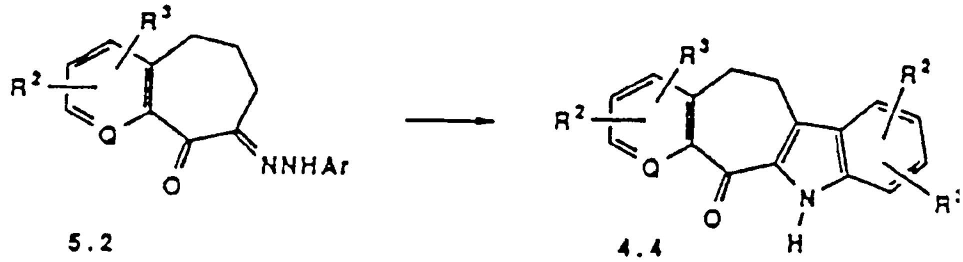

5.2 4.4

m) Umsetzen einer Verbindung der Formel 5.3 mit einem Hydrazinreagenz $R^{10}NHNH_2$ und einem sauren Katalysator

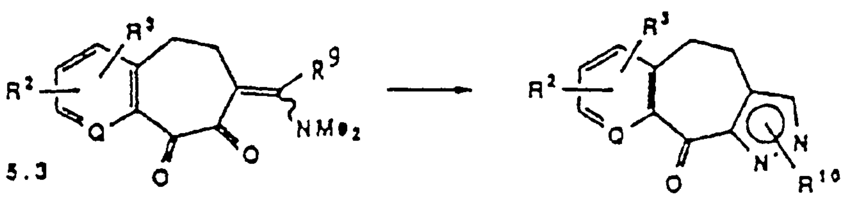

5.3 4.5

n) Umsetzen einer Verbindung der Formel 5.4 mit einer Säure oder Persäure unter Herstellen einer Verbindung der Formel 4.6

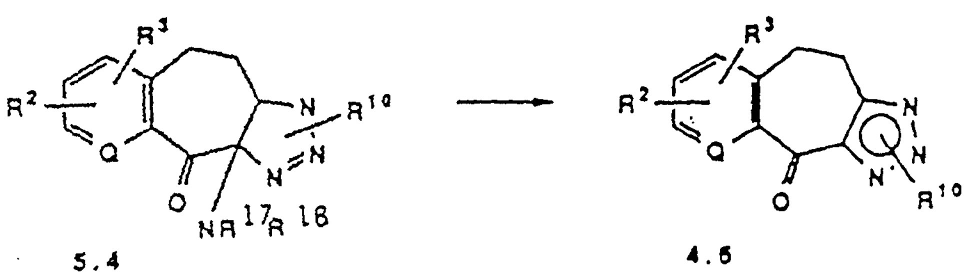

5.4 4.6

o) Umsetzen einer Verbindung der Formel 5.5 mit einem geeigneten Alkalimetalldithionit in einem heißen Lösungsmittel

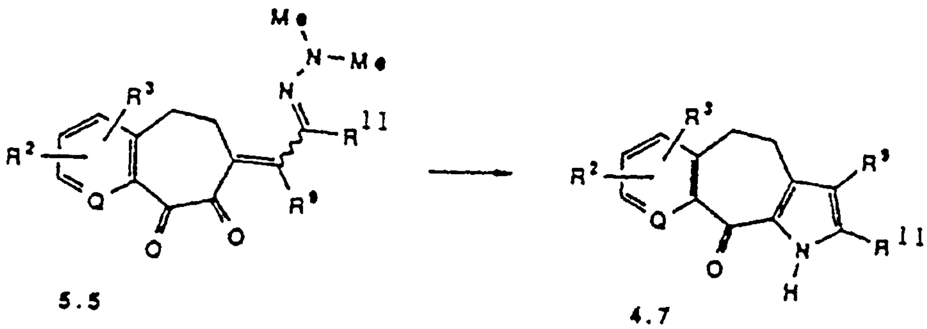

5.5 4.7

p) Umsetzen einer Verbindung der Formel 5.3 mit einem Hydroxylaminhydrochlorid

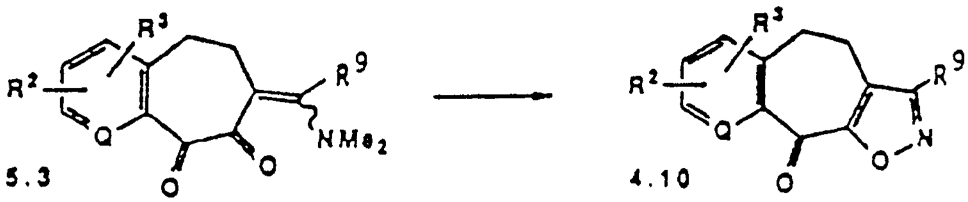

5.3 4.10

q) Umsetzen einer Verbindung der Formel 5.3 mit einer Verbindung der Formel $H_2N\text{-}C(=NH)R^Y$, worin $R^Y$ eines von H, Niederalkyl, OH, O-(Niederalkyl), SH, S-(Niederalkyl), $NH_2$, NH-(Niederalkyl), N-(Niederalkyl)$_2$ und NH(C=O)-(Niederalkyl) darstellt

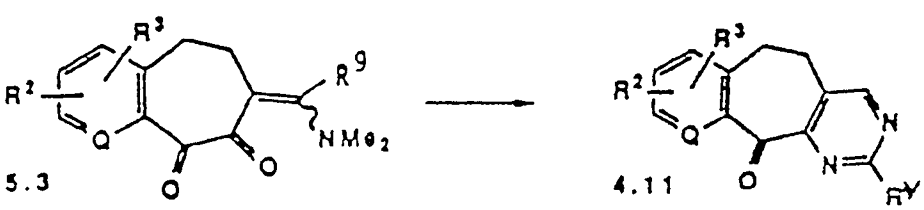

5.3 4.11

r) Umsetzen einer Verbindung der Formel 5.3 mit einer Verbindung der Formel $H_2NC(=O)CH_2E$, worin E aus CHO, C(=O)-(Niederalkyl), $CO_2$-(Niederalkyl), $CONH_2$, $NO_2$, CN, $SO_2CH_3$ und $CF_3$ ausgewählt ist:

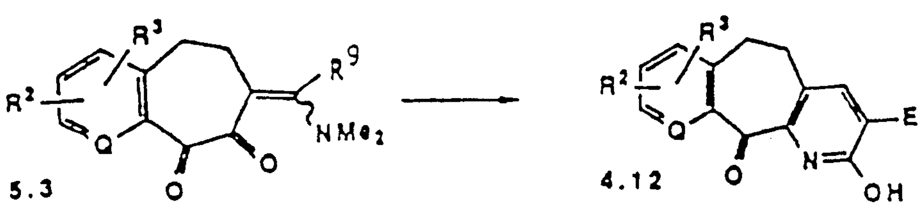

5.3 4.12

s) Umsetzen einer Verbindung der Formel 6.0 mit Formamid, p-Toluolsulfonsäure und N,N',N''-Methylidintris-formamid ($HC(NHCHO)_3$)

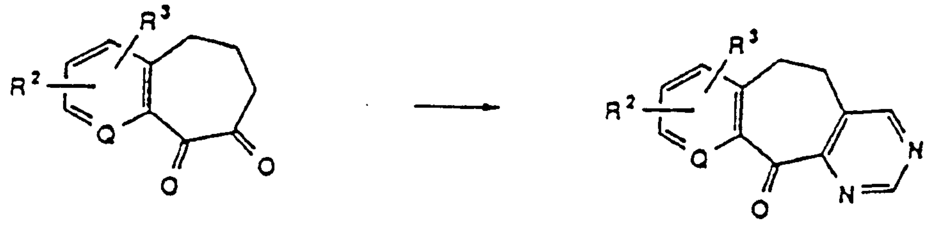

6.0 4.8

t) Umsetzen einer Verbindung 5.6 mit Hydrazin gefolgt von der Oxidation umfaßt.

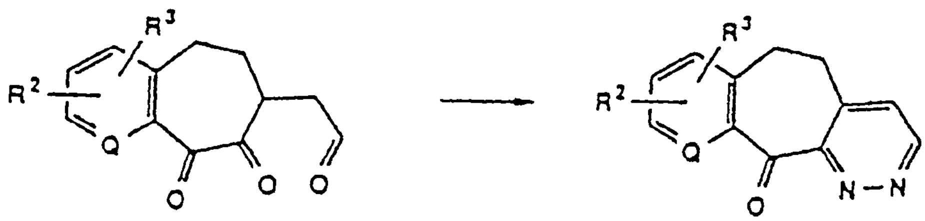

5.6 4.9

**Patentansprüche für folgende Vertragsstaaten : ES, GR**

1. Verfahren zum Herstellen einer Verbindung der Formel 1.0

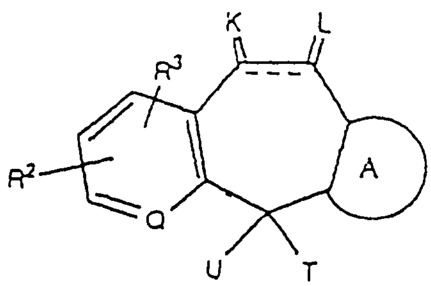

oder eines pharmazeutisch annehmbaren Salzes oder Solvats derselben, worin U und T zusammen =O darstellen oder U H darstellt und T

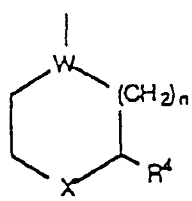

darstellt, oder U und T zusammen

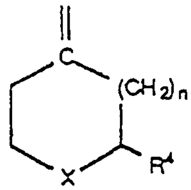

darstellen;
Q CH, N oder N→O ist;
$R^2$ und $R^3$ gleich oder verschieden sein können und jeweils unabhängig H, $C_1$-$C_8$-Alkyl, $CF_3$, $NO_2$, Halogen, $OR^5$, $NR^5R^6$ oder $C_1$-$C_8$-$S(O)_m$-Alkyl bedeuten, worin
$R^5$ und $R^6$ gleich oder verschieden sein können und jeweils unabhängig aus H, $C_1$-$C_8$-Alkyl oder $C_1$-$C_8$-Acyl, $C_7$-$C_{16}$-Aroyl ausgewählt sind und m 0, 1 oder 2 ist;
$R^4$ H, $C_1$-$C_8$-Alkyl oder $C_6$-$C_{15}$-Aryl darstellt;
K -H, -H oder -H, $C_1$-$C_8$-Alkyl oder $C_1$-$C_8$-Alkyl, $C_1$-$C_8$-Alkyl oder -H, -OH oder =O darstellt;
L -H, -H oder -H, $C_1$-$C_8$-Alkyl oder $C_1$-$C_8$-Alkyl, $C_1$-$C_8$-Alkyl oder -H, -OH oder =O darstellt, mit der Maßgabe, daß wenn L oder K -H, -OH oder =O ist, das andere K beziehungsweise L -H, -H oder -H, $C_1$-$C_8$-Alkyl oder $C_1$-$C_8$-Alkyl, $C_1$-$C_8$-Alkyl ist;
Ring

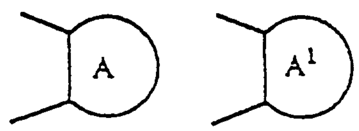

darstellt, wenn U H darstellt und
T

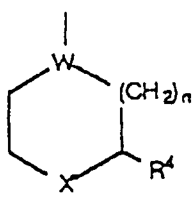

darstellt, oder wenn T und U zusammen

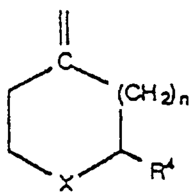

darstellen;
und Ring

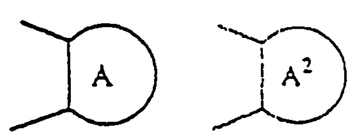

darstellt, wenn T und U zusammen =O darstellen;

Ring

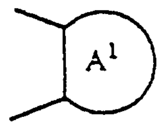

einen kondensierten 5-gliedrigen heterocyclischen aromatischen Ring mit wenigstens einem Heteroatom darstellt, das aus O, S oder N in dem Ring ausgewählt ist, wobei der Ring gegebenenfalls durch 1 bis 3 Gruppen R, die aus $C_1$-$C_8$-Alkyl, $C_6$-$C_{15}$-Aryl und $C_7$-$C_{16}$-Arylmethyl ausgewählt sind, oder durch zwei Gruppen R an benachbarten Ringatomen substituiert ist, wobei die Gruppen R zusammen mit derartigen benachbarten Ringatomen einen kondensierten Benzol- oder kondensierten Pyridinring darstellen, oder Ring

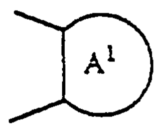

einen kondensierten, 6-gliedrigen, heterocyclischen, aromatischen Ring mit 2 oder 3 Ringstickstoffatomen darstellt;

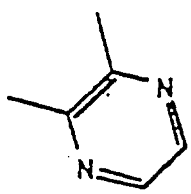 , 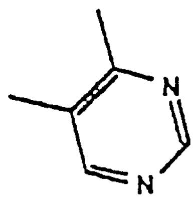 , 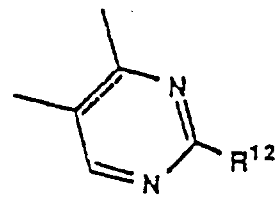 ,

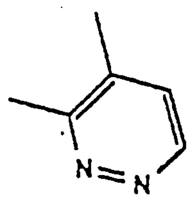 , 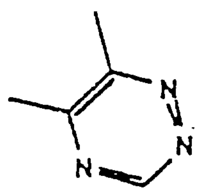 or 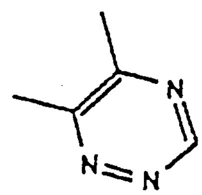 ;

oder Ring

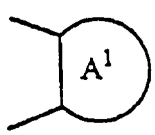

einen kondensierten 6-gliedrigen heterocyclischen aromatischen Ring mit 1 Ring Stickstoffatom darstellt, worin Q CH ist und X

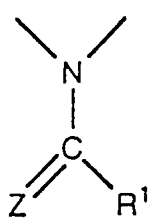

darstellt, in der

$R^1$ $C_{2-14}$ Heteroaryl darstellt, wobei das Heteroatom N→O ist;
Ring

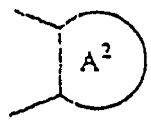

(i) wenn Q N oder N→O ist

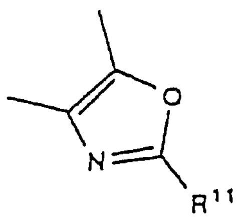

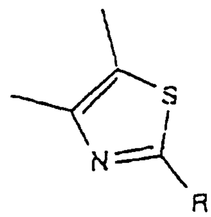

oder

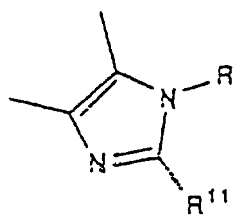

,

oder (ii) wenn Q CH, N oder N→O ist

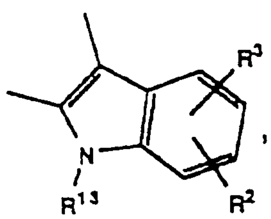

,

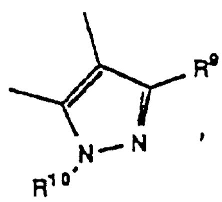

,

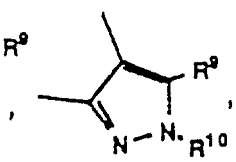

,

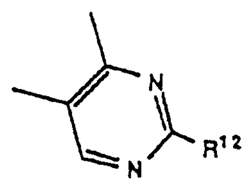

,

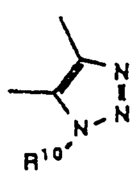

,

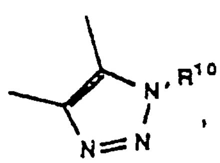

,

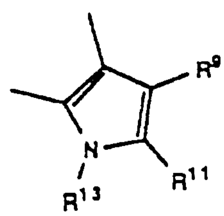

,

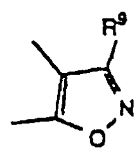

,

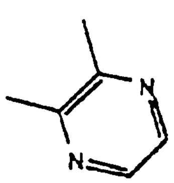

,

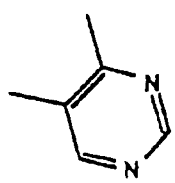

,

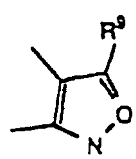

,

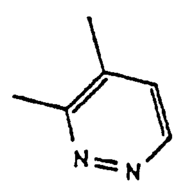

,

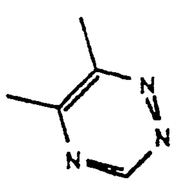

oder

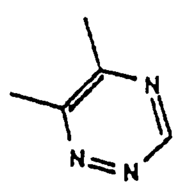

;

darstellt, worin
$R^9$ H, $C_1$-$C_8$-Alkyl oder $C_6$-$C_{15}$-Aryl ist;
$R^{10}$ H, $C_1$-$C_8$-Alkyl, $C_6$-$C_{15}$-Aryl, $C_7$-$C_{23}$-Arylalkyl, $C_1$-$C_8$-Acyl, $C_7$-$C_{16}$-Aroyl und $C_2$-$C_{14}$-Heteroaryl ist, worin die

Aryl-Struktureinheit gegebenenfalls durch einen oder mehr Substituenten substituiert ist, die aus H, Halogen, $NO_2$, $CF_3$, -SH, $C_1$-$C_8$-S-Alkyl, $C_1$-$C_8$-$S(O)_m$-Alkyl, $C_1$-$C_8$-Alkyl, $CO_2H$, $CH_2OH$, C(OH)-($C_1$-$C_8$-Niederalkyl), $NH_2$, NH-($C_1$-$C_8$-Niederalkyl), N-($C_1$-$C_8$-Niederalkyl)$_2$, OH, O-($C_1$-$C_8$-Niederalkyl), O-($C_6$-$C_{15}$-Aryl), O-($C_7$-$C_{16}$-Aroyl), O-($C_2$-$C_{14}$-Heteroaroyl), NH($C_1$-$C_8$-Acyl), N($C_1$-$C_8$-Acyl)$_2$, NH($C_7$-$C_{16}$-Aroyl), N($C_7$-$C_{16}$-Aroyl)$_2$, NH($C_2$-$C_{14}$-Heteroaryl), N($C_2$-$C_{14}$-Heteroaryl)$_2$,

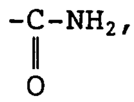 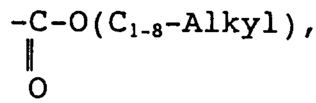

-CHO,

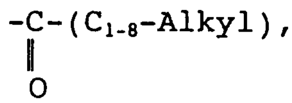 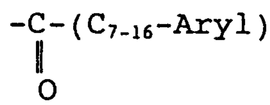

und -C≡N ausgewählt sind;
$R^{11}$ H, $C_1$-$C_8$-Alkyl, $C_6$-$C_{15}$-Aryl oder $C_7$-$C_{23}$-Aralkyl darstellt;
$R^{12}$ H, $C_1$-$C_8$-Alkyl, OH, O-($C_1$-$C_8$-Alkyl), SH, S($C_1$-$C_8$-Alkyl), $NH_2$, NH-($C_1$-$C_8$-Alkyl), N-($C_1$-$C_8$-Alkyl)$_2$ und NH(C=O)-($C_1$-$C_8$-Alkyl) darstellt;
$R^{13}$ H, $C_1$-$C_8$-Alkyl, $C_6$-$C_{15}$-Aryl oder $C_7$-$C_{16}$-Arylmethyl ist, und
$R^{14}$ H, $CO_2H$, $CH_2OH$, C(OH)-($C_1$-$C_8$-Alkyl)$NH_2$, NH-($C_1$-$C_8$-Alkyl), N-($C_1$-$C_8$-Alkyl)$_2$, NH(C=O)-($C_1$-$C_8$-Alkyl), $CH_2NH_2$, $CH_2NH$-($C_1$-$C_8$-Alkyl), $CH_2N$-($C_1$-$C_8$-Alkyl)$_2$, $CH_2NH$(C=O)-($C_1$-$C_8$-Alkyl), CHO, C(=O)-($C_1$-$C_8$-Alkyl), $CO_2$-($C_1$-$C_8$-Alkyl), $CONH_2$, $NO_2$, CN, $CF_3$ und -$S(O)_mR^{19}$ darstellt, worin $R^{19}$ $C_1$-$C_8$-Alkyl, $C_6$-$C_{15}$-Aryl oder $C_7$-$C_{23}$-Aralkyl darstellt;
W N oder N→O ist;
die durchbrochene Linie in dem siebengliedrigen Ring eine wahlfreie Doppelbindung darstellt;
n 0, 1, 2 oder 3 ist;
X

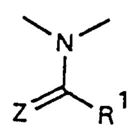 , 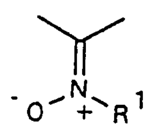 oder 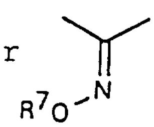

darstellt, worin
$R^7$ H, $C_1$-$C_8$-Alkyl, $C_1$-$C_8$-Acyl oder $C_7$-$C_{16}$-Aroyl darstellt,
Z O oder S ist und $R^1$ H, $C_1$-$C_8$-Alkyl, $C_5$-$C_8$-Cycloalkyl, $CF_3$, $C_6$-$C_{15}$-Aryl, $C_2$-$C_{14}$-Heteroaryl, $NR^5R^6$, worin $R^5$ und $R^6$ wie vorstehend definiert sind, $C_1$-$C_8$-Alkylthio oder $C_1$-$C_8$-Alkoxy darstellt oder $R^1$ und $R^4$ zusammen unter Bilden eines kondensierten Ringes -$(CH_2)_k$- darstellen, worin k 1, 2 oder 3 ist,
wobei diejenigen Verbindungen ausgeschlossen sind, worin Q CH ist, Ring $A^1$ ein kondensierter, 6-gliedriger, heterocyclischer Ring mit einem Ringstickstoff ist und X

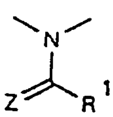

darstellt, worin

(a) Z = S oder Z = O und $R^1$ Teil eines kondensierten Rings ist oder aus H, Alkyl, Cycloalkyl, Aryl, Alkylthio, -$NH_2$, N(Alkyl)$_2$ und Heteroaryl, worin das Heteroatom O, S oder N ist, ausgewählt ist, oder
(b) Z = O und $R^1$ $CF_3$ ist, oder
(c) Z = S und $R^1$ Alkoxy ist,

wobei das Verfahren,

a) wenn X

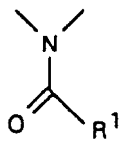

darstellt, das Umsetzen einer Verbindung der Formel 9.1 mit Verbindung 8.11 in Gegenwart einer Base

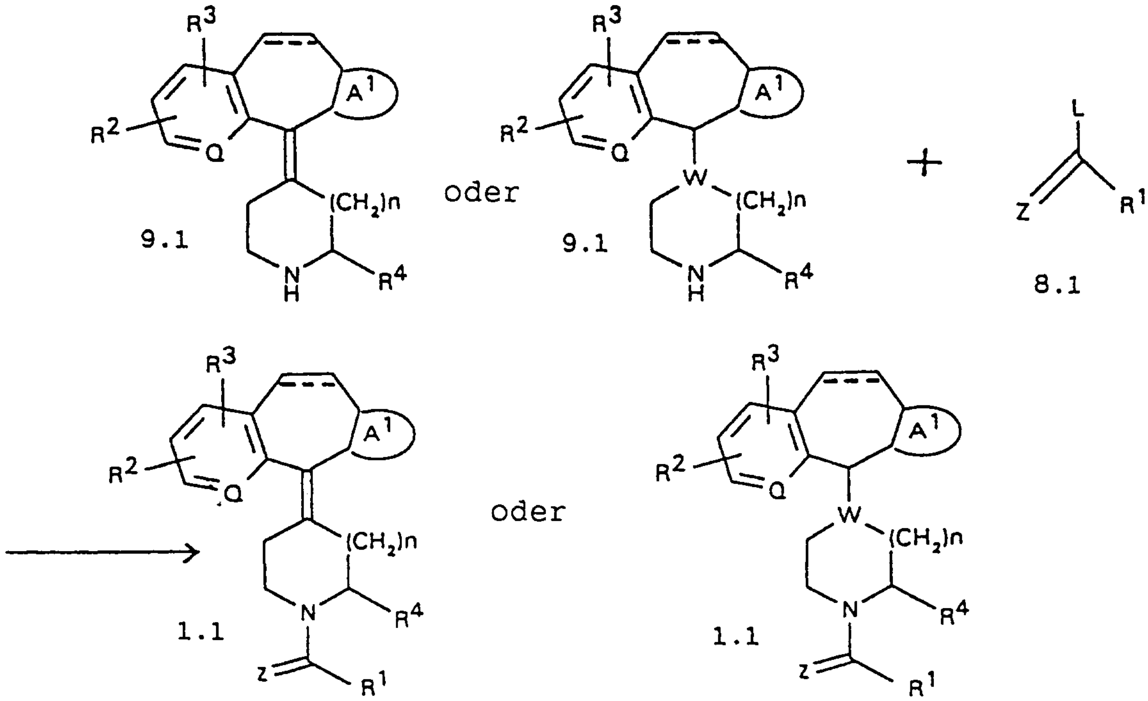

9.1 oder 9.1 + 8.1

1.1 oder 1.1

b) wenn X

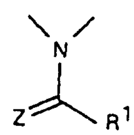

darstellt, worin Z =O oder =S ist, das Umsetzen einer Verbindung der Formel 11.1 mit einer Verbindung der Formel 8.1

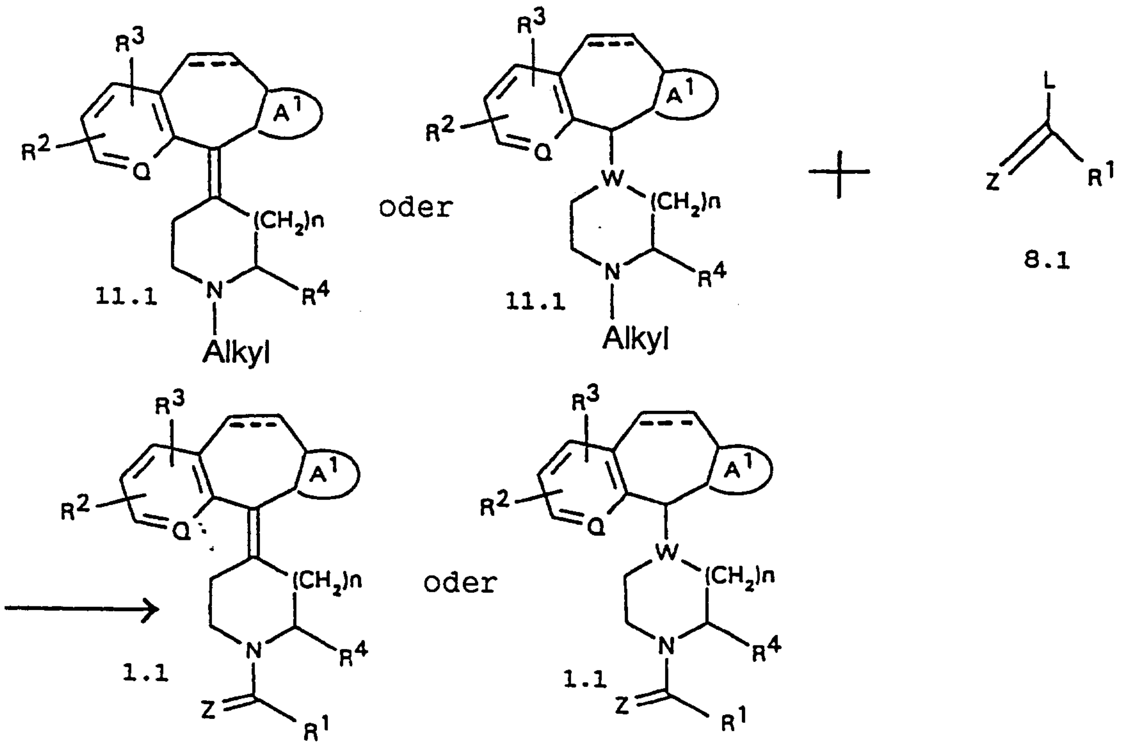

11.1 oder 11.1 8.1

1.1 oder 1.1

c) wenn X

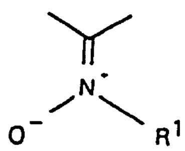

oder

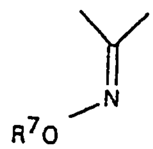

darstellt, das Umsetzen einer Verbindung der Formel 13 in Gegenwart von Hydroxylamin oder eines O-substituierten Hydroxylamins oder N-substituierten Hydroxylamins

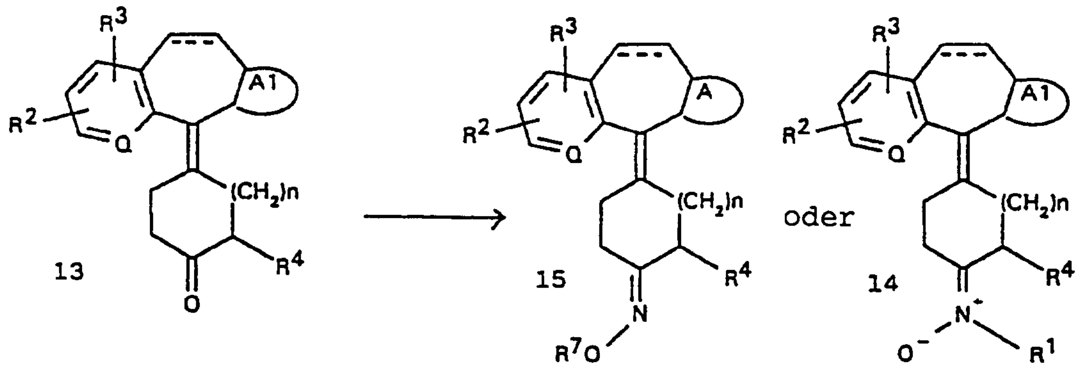

13 15 oder 14

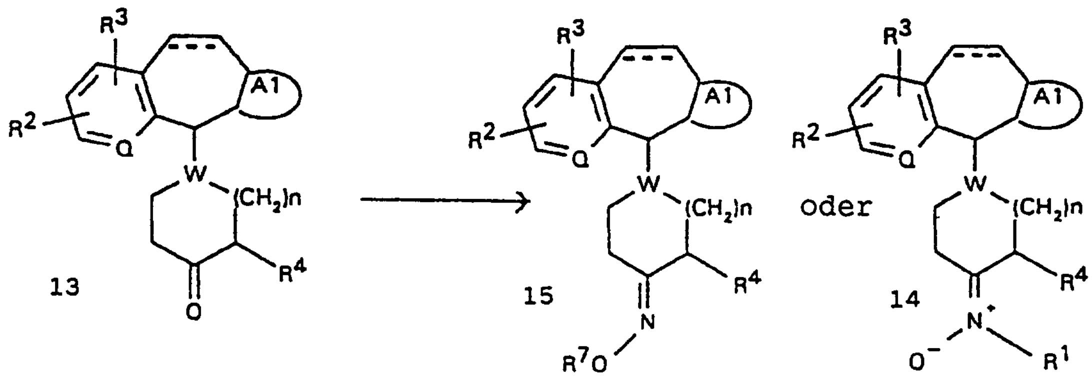

13 15 oder 14

d) Umsetzen einer Verbindung der Formel 9.2 mit einer Säure

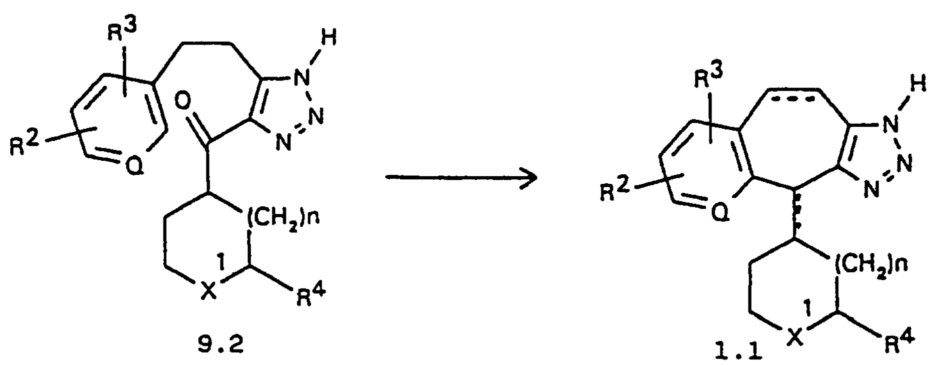

9.2 1.1

e) Umsetzen einer Verbindung der Formel 200 mit einer Alkalie oder einem Alkalimetallhydroxid

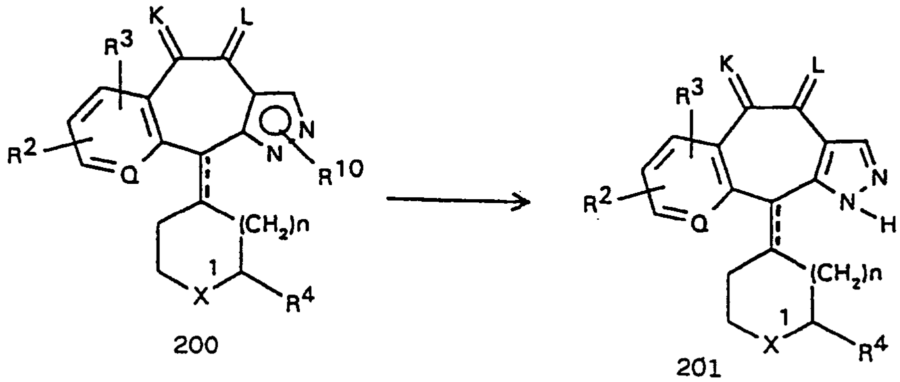

200 201

f) Umsetzen einer Verbindung der Formel 17 mit einer geeigneten Säure

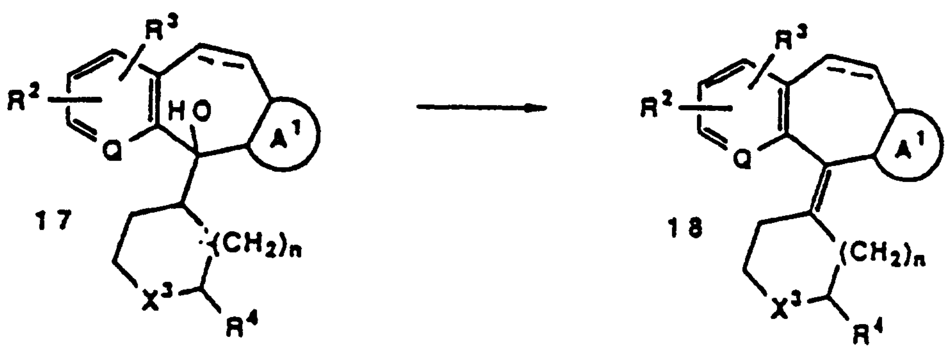

17 18

worin $X^3$

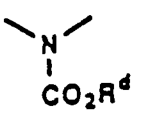 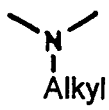 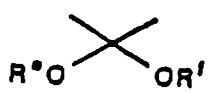 oder 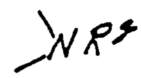

darstellt,
$R^d$ eine gegebenenfalls substituierte Alkyl-, Alkenyl-, Aryl- oder Aralkylgruppe darstellt,
$R^e$ und $R^f$ jeweils unabhängig Niederalkyl darstellen oder zusammen eine Alkandiylkette aus 2 oder 3 Methylengruppen darstellen,
$R^g$ H oder $C(Z)R^1$ ist,

g) Umsetzen einer Verbindung der Formel 19 mit einer Verbindung der Formel 20, worin L eine Abgangsgruppe ist und $X^3$ $NR^g$ oder

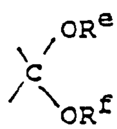

darstellt,

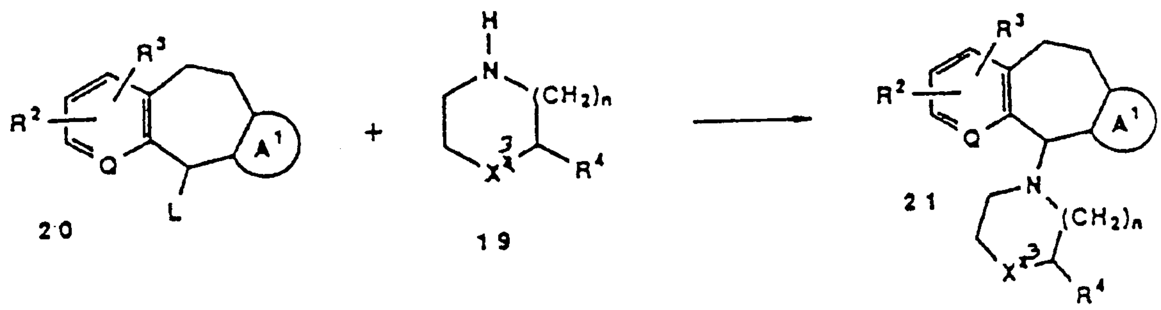

20 19 21

h) Umsetzen einer Verbindung der Formel 4.0 mit einer Verbindung der Formel 19

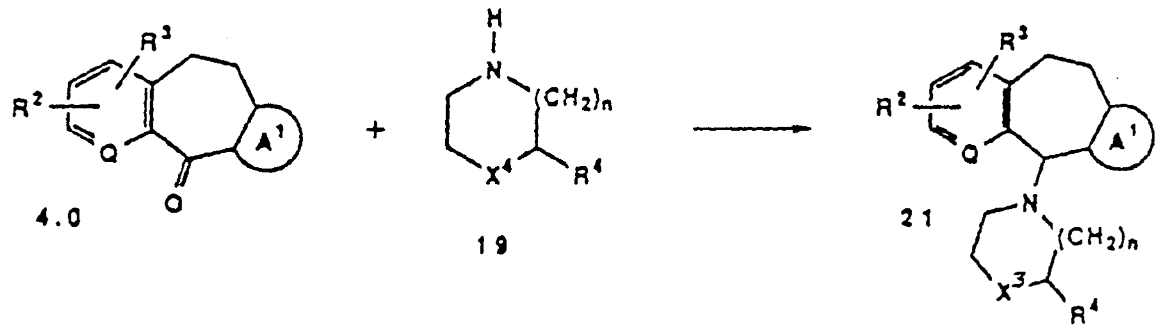

4.0 19 21

i) Dehydratisieren einer Verbindung der Formel 26 unter Herstellen einer Verbindung 27

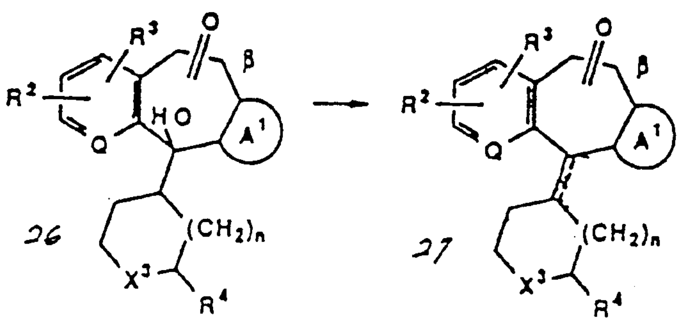

26 27

j) Hydrolysieren von Verbindung 30 mit einer starken wäßrigen Säure unter Herstellen von Verbindung 31

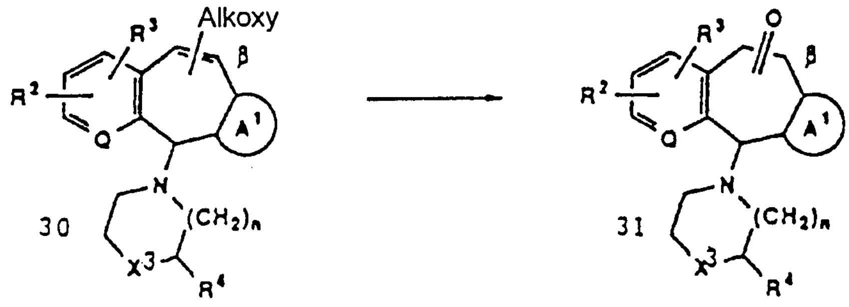

30

31

k) Umsetzen einer Verbindung der Formel 5.1 mit einer Verbindung der Formel $R^{11}CO_2H$ und $(R^{11}CO)_2O$ oder $(R^{11}CO_2)_2O$ allein in Gegenwart von HCl

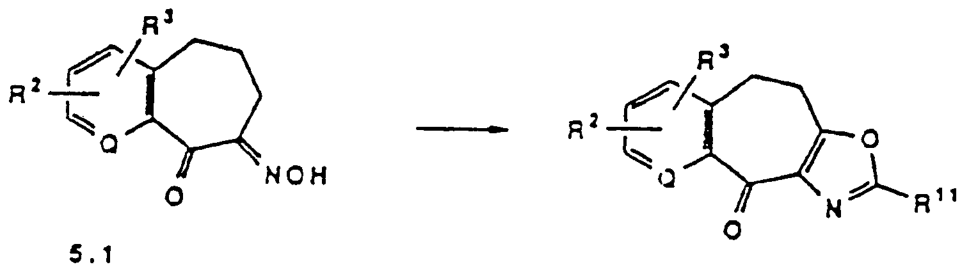

5.1

4.1

l) Umsetzen einer Verbindung der Formel 5.2 mit einem sauren Reagenz und wobei Ar eine geeignete Arylgruppe darstellt

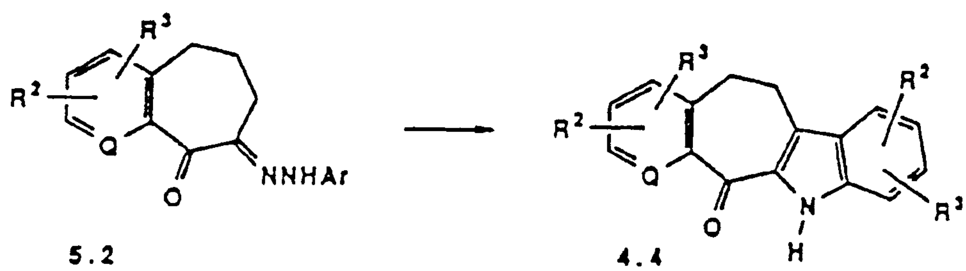

5.2

4.4

m) Umsetzen einer Verbindung der Formel 5.3 mit einem Hydrazinreagenz $R^{10}NHNH_2$ und einem sauren Katalysator

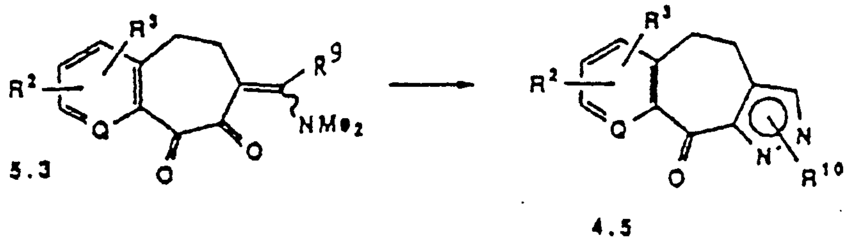

5.3 4.5

n) Umsetzen einer Verbindung der Formel 5.4 mit einer Säure oder Persäure unter Herstellen einer Verbindung der Formel 4.6

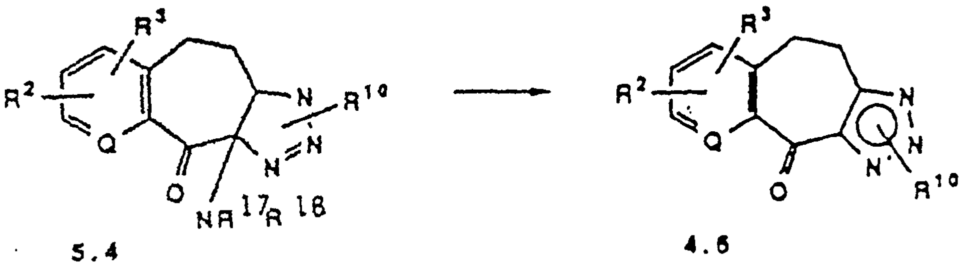

5.4 4.6

o) Umsetzen einer Verbindung der Formel 5.5 mit einem geeigneten Alkalimetalldithionit in einem heißen Lösungsmittel

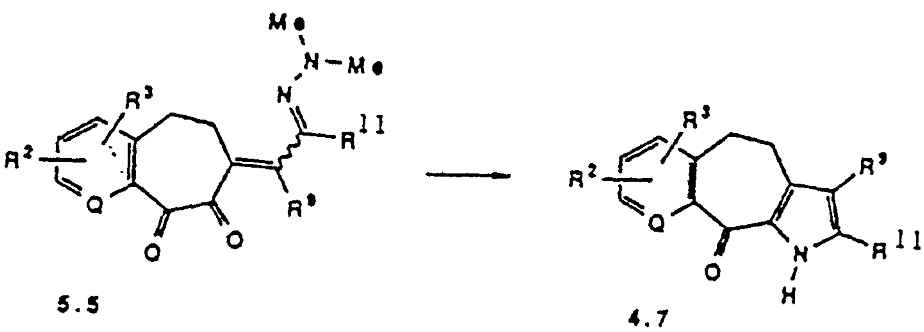

5.5 4.7

p) Umsetzen einer Verbindung der Formel 5.3 mit einem Hydroxylaminhydrochlorid

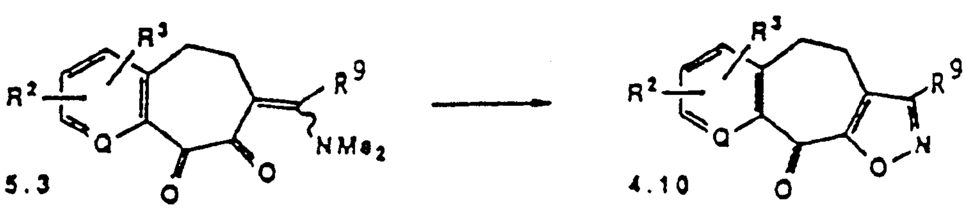

5.3 4.10

q) Umsetzen einer Verbindung der Formel 5.3 mit einer Verbindung der Formel $H_2N-C(=NH)R^Y$, worin $R^Y$ eines von H, Niederalkyl, OH, O-(Niederalkyl), SH, S-(Niederalkyl), $NH_2$, NH-(Niederalkyl), N-(Niederalkyl)$_2$ und NH(C=O)-(Niederalkyl) darstellt

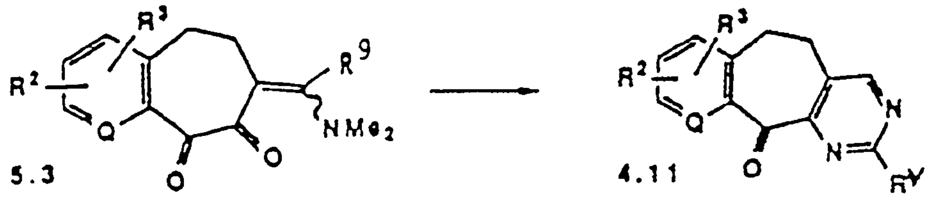

5.3 4.11

r) Umsetzen einer Verbindung der Formel 5.3 mit einer Verbindung der Formel $H_2NC(=O)CH_2E$, worin E aus CHO, C(=O)-(Niederalkyl), $CO_2$-(Niederalkyl), $CONH_2$, $NO_2$, CN, $SO_2CH_3$ und $CF_3$ ausgewählt ist:

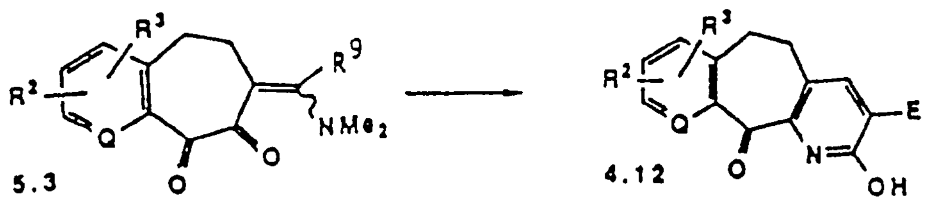

5.3 4.12

s) Umsetzen einer Verbindung der Formel 6.0 mit Formamid, p-Toluolsulfonsäure und N,N',N''-Methylidintrisformamid ($HC(NHCHO)_3$)

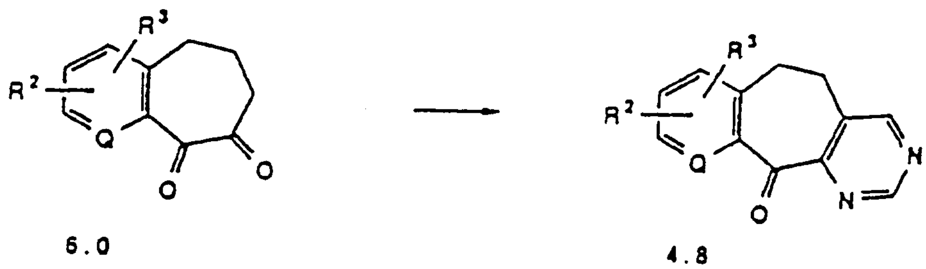

6.0 4.8

t) Umsetzen einer Verbindung 5.6 mit Hydrazin gefolgt von der Oxidation umfaßt.

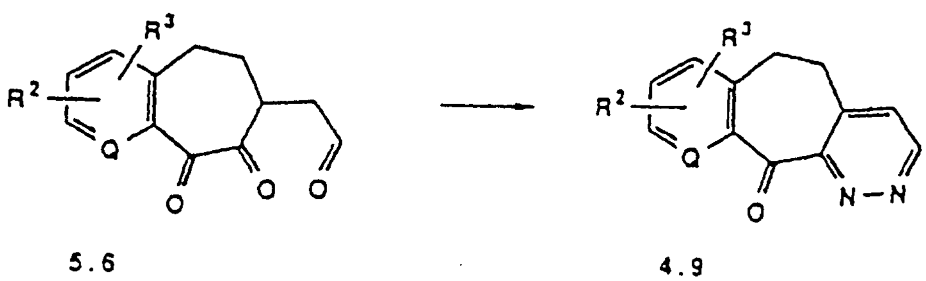

5.6 4.9

2. Verwendung einer Verbindung der Formel 1.0 bei der Herstellung eines Arzneimittels für die Behandlung von PAF-Störungen, Allergie und/oder Entzündungen.

## Revendications

**Revendications pour les Etats contractants suivants : AT, BE, CH, DE, FR, GB, IT, LI, LU, NL, SE**

**1.** Un composé de la formule 1.0

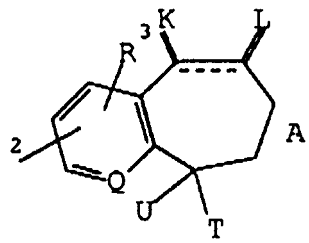

ou son sel ou solvate pharmaceutiquement acceptables, où :

U et T représentent ensemble =O, ou U représente H et T représente

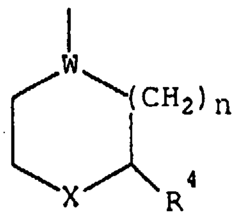

,

ou

U et T représentent ensemble

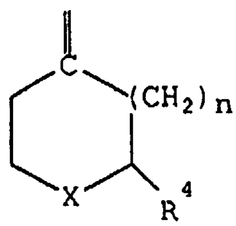

;

Q est CH, N ou N→O;

$R^2$ et $R^3$ peuvent être identiques ou différents et chacun représente indépendamment H, alkyle $C_1$-$C_8$, $CF_3$, $NO_2$, halo, $OR^5$, $NR^5R^6$, ou S(O)m-alkyle $C_1$-$C_8$, dans lesquels :

$R^5$ et $R^6$ peuvent être identiques ou différents et chacun est indépendamment sélectionné parmi H, alkyle $C_1$-$C_8$ ou bien acyle $C_1$-$C_8$, aroyle $C_7$-$C_{16}$, et m est 0, 1 ou 2,

$R^4$ représente H, alkyle $C_1$-$C_8$ ou aryle $C_6$-$C_{15}$

K représente représente-H, -H ou -H, alkyle $C_1$-$C_8$ ou alkyle $C_1$-$C_8$, alkyle $C_1$-$C_8$, ou -H, -OH ou =O;

L représente -H, -H ou -H, alkyle $C_1$-$C_8$ ou alkyle $C_1$-$C_8$, alkyle $C_1$-$C_8$ ou -H, -OH ou =O, à condition que quand L ou K est -H, -OH ou =O, alors l'autre de K ou L soit, respectivement, -H, -H ou -H, alkyle $C_1$-$C_8$ ou alkyle $C_1$-$C_8$, alkyle C1-$C_8$;

le cycle

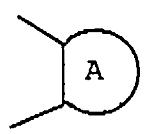

représente

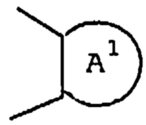

quand U représente
H et T représente

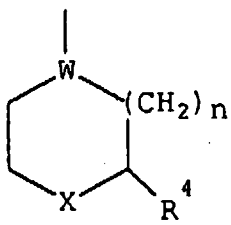

ou bien quand T et U représentent ensemble

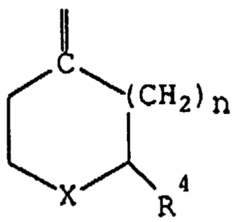

;

et le cycle

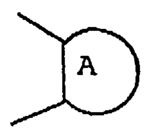

représente

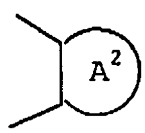

quand T et U représentent ensemble =O;
le cycle

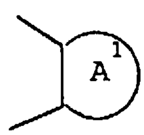

représente un cycle aromatique hétérocyclique fusionné à 5 membres ayant au moins un hétéroatome sélectionné parmi O, S ou N dans le cycle, ledit cycle étant facultativement substitué par 1 à 3 groupes R sélectionnés parmi alkyle $C_1$-$C_8$, aryle $C_6$-$C_{15}$ et arylméthyle $C_7$-$C_{16}$ ou par deux groupes R sur des atomes adjacents du cycle, lesquels groupes R avec ces atomes adjacents du cycle représentent un cycle de benzène fusionné ou de pyridine

fusionnée, ou bien le cycle

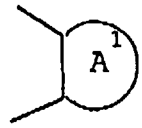

représente un cycle aromatique hétérocyclique fusionné à 6 membres ayant 1, 2 ou 3 atomes d'azote dans le cycle;

le cycle

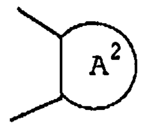

représente

(i) quand Q est N ou N→O :

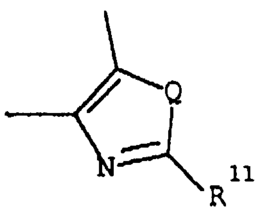

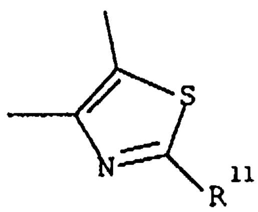

ou

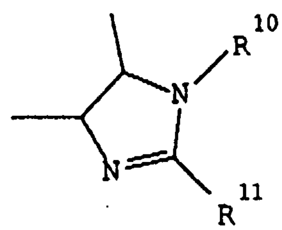

,

ou bien (ii) quand Q est CH, N ou N→O :

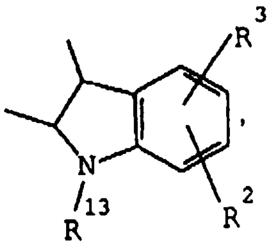 , 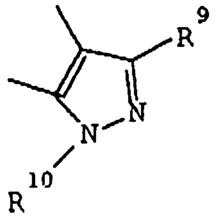 , 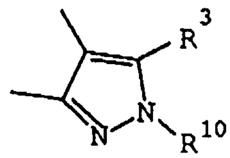 , 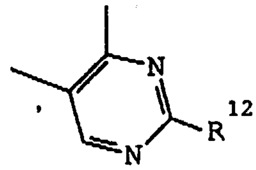 ,

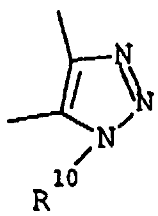 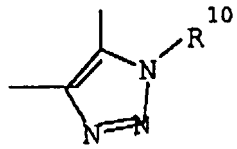 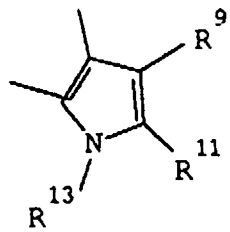 , 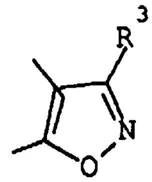 ,

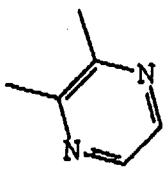 , 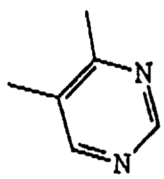 , 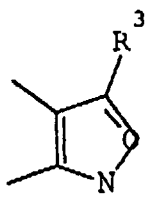 , 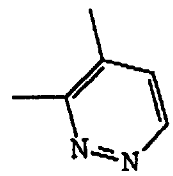 ,

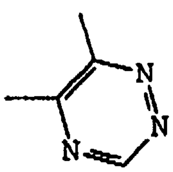 ou 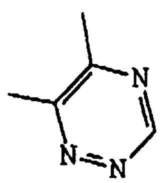 ;

dans lesquels :

$R^9$ est H, alkyle $C_1$-$C_8$ ou aryle $C_6$-$C_{15}$;

$R^{10}$ est H, alkyle $C_1$-$C_8$, aryle $C_6$-$C_{15}$, arylalkyle $C_7$-$C_{23}$, acyle $C_1$-$C_8$, aroyle $C_7$-$C_{16}$ et hétéroaroyle $C_2$-$C_{14}$ où l'entité aryle est facultativement substituée par un ou plusieurs substituants sélectionnés parmi H, halogène, $NO_2$, $CF_3$, -SH, S-alkyle $C_1$-$C_8$, $S(O)_m$-alkyle $C_1$-$C_8$, alkyle $C_1$-$C_8$, $CO_2H$, $CH_2OH$, C(OH)-(alkyle inférieur $C_1$-$C_8$), $NH_2$, NH-(alkyle inférieur $C_1$-$C_8$), N-(alkyle inférieur $C_1$-$C_8$)$_2$, OH, O-(alkyle inférieur $C_1$-$C_8$), O-(aryle $C_6$-$C_{15}$), O-(aroyle $C_7$-$C_{16}$), O-(hétéroaroyle $C_2$-$C_{14}$), NH(acyle $C_1$-$C_8$), N(acyle $C_1$-$C_8$)$_2$, NH(aroyle $C_7$-$C_{16}$), N(aroyle $C_7$-$C_{16}$)$_2$, NH(hétéroaroyle $C_2$-$C_{14}$), N(hétéroaryle $C_2$-$C_{14}$)$_2$,

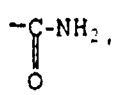

-C-O(alkyle $C_{1-8}$), -CHO,

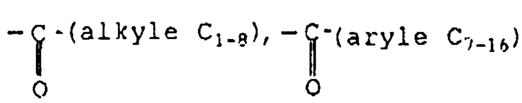

et-C≡N;

$R^{11}$ représente H, alkyle $C_1$-$C_8$, aryle $C_6$-$C_{15}$ ou aralkyle $C_7$-$C_{23}$;

$R^{12}$ représente H, alkyle $C_1$-$C_8$, OH, O-(alkyle $C_1$-$C_8$), SH, S(alkyle $C_1$-$C_8$), $NH_2$, NH-(alkyle $C_1$-$C_8$), N-(alkyle $C_1$-$C_8$)$_2$, et NH(C=O)-(alkyle $C_1$-$C_8$);

$R^{13}$ est H, alkyle $C_1$-$C_8$, aryle $C_6$-$C_{15}$ ou arylméthyle $C_7$-$C_{16}$; et

$R^{14}$ représente H, $CO_2H$, $CH_2OH$, C(OH)-(alkyle $C_1$-$C_8$)$NH_2$, NH-(alkyle $C_1$-$C_8$), N-(alkyle $C_1$-$C_8$)$_2$, NH(C=O)-(alkyle $C_1$-$C_8$), $CH_2NH_2$, $CH_2NH$-(alkyle $C_1$-$C_8$), $CH_2N$-(alkyle $C_1$-$C_8$)$_2$, $CH_2NH$(C=O)-(alkyle $C_1$-$C_8$), CHO, C(=O)-(alkyle $C_1$-$C_8$), $CO_2$-(alkyle $C_1$-$C_8$), $CONH_2$, $NO_2$, CN, $CF_3$ et -S(O)$_m$$R^{19}$ où $R^{19}$ représente alkyle $C_1$-$C_8$, aryle $C_6$-$C_{15}$ ou aralkyle $C_7$-$C_{23}$;

W est N ou N→O;

la ligne en pointillé dans le cycle à sept membres représente une double liaison facultative;

n est 0, 1, 2 ou 3;

X représente

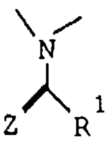 , 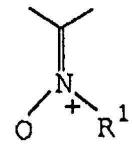 ou 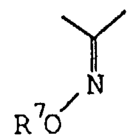

dans lesquels :

$R^7$ représente H, alkyle $C_1$-$C_8$, acyle $C_1$-$C_8$ ou aroyle $C_7$-$C_{16}$, Z est O ou S, et $R^1$ est H, alkyle $C_1$-$C_8$, cycloalkyle $C_5$-$C_8$, $CF_3$, aryle $C_6$-$C_{15}$, hétéroaryle $C_2$-$C_{14}$, $NR^5R^6$ où $R^5$ et $R^6$ sont tels que définis ci-dessus, alkylthio $C_1$-$C_8$ ou alcoxy $C_1$-$C_8$, ou bien $R^1$ et $R^4$ représentent ensemble -$(CH_2)_k$- où k est 1, 2, ou 3, afin de former un cycle fusionné,

à l'exclusion des composés où Q est CH, le cycle $A^1$ est un cycle hétérocyclique fusionné à 6 membres ayant un azote dons le cycle et X représente

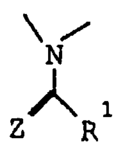

où (a)Z=S ou Z=O et $R^1$ fait partie d'un cycle fusionné ou bien est sélectionné parmi H, alkyle, cycloalkyle, aryle, alkylthio, -$NH_2$, N(alkyle)$_2$, et hétéroaryle où l'hétéroatome est O, S ou N; ou bien

(b)Z = O et $R^1$ est $CF_3$; ou

(c)Z = S et $R^1$ est alcoxy.

2. Composé de la revendication 1 caractérisé de plus en ce qu'il a la structure :

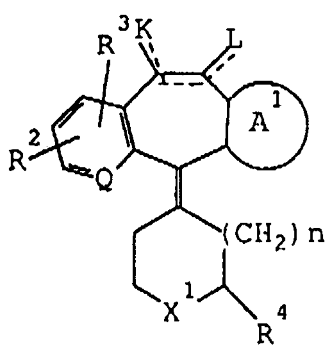 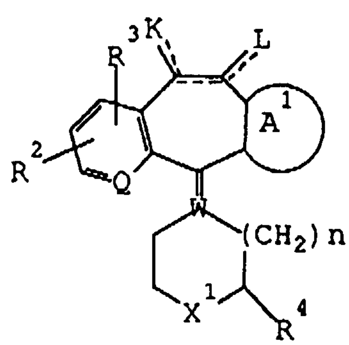

où $X^1$ représente

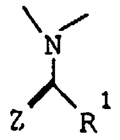 , 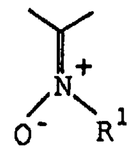 ou 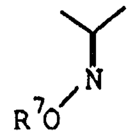

et où le cycle

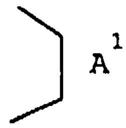

représente

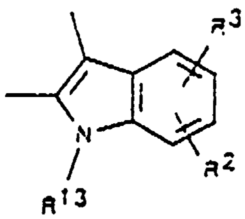

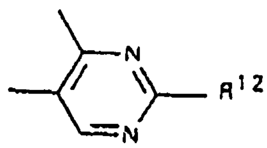

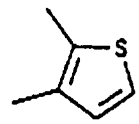

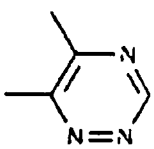

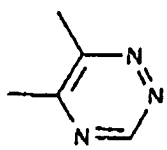

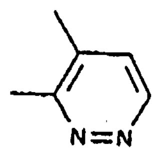

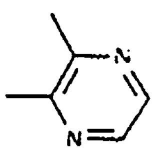 ou 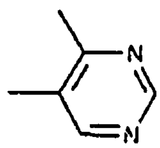

où
$R^9$ est sélectionné parmi H, alkyle $C_1$-$C_8$ ou aryle $C_6$-$C_{15}$;
$R^{10}$ est sélectionné parmi H, alkyle $C_1$-$C_8$, aryle $C_6$-$C_{15}$ ou arylméthyle $C_6$-$C_{15}$;;
$R^{11}$ est sélectionné parmi H, alkyle $C_1$-$C_8$, aryle $C_6$-$C_{15}$ ou arylméthyle $C_6$-$C_{15}$; et
$R^{13}$ est sélectionné parmi H, alkyle $C_1$-$C_8$, aryle $C_6$-$C_{15}$ ou arylméthyle $C_6$-$C_{15}$.

**3.** Composé de la revendication 1 caractérisé de plus en ce qu'il a la formule de structure :

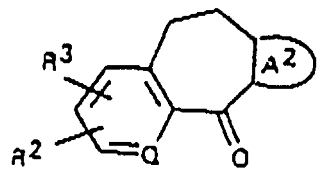

**4.** Composé selon l'une des revendications 1 ou 2, caractérisé de plus en ce que K et L représentent tous deux H, H et où $R^2$, $R^3$ et $R^4$ représentent tous H.

**5.** Composé selon l'une des revendications 1-4 ayant la formule de structure :

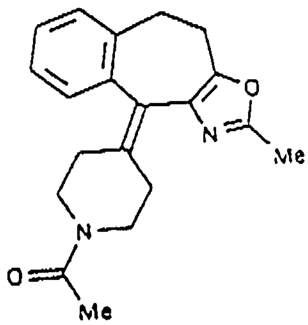

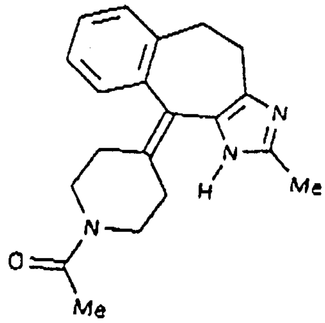

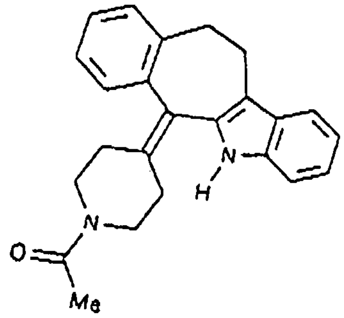

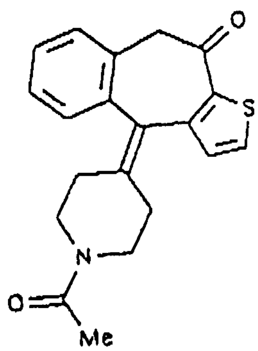

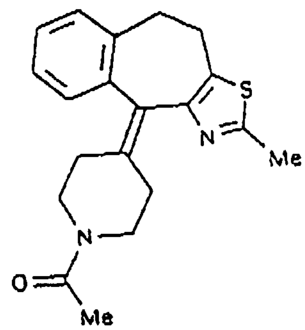

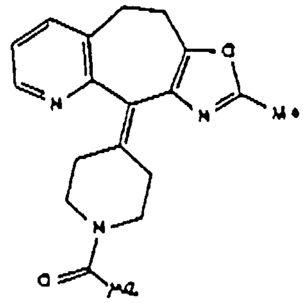

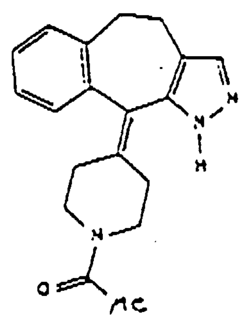

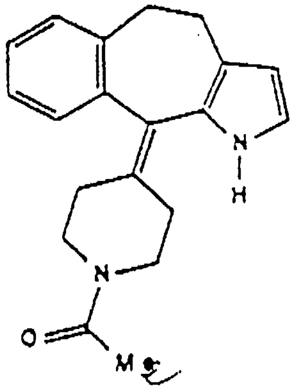

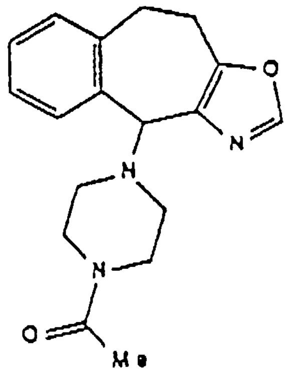

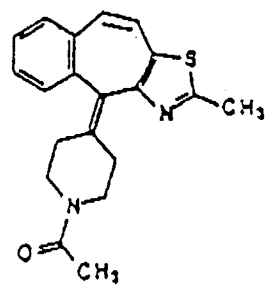

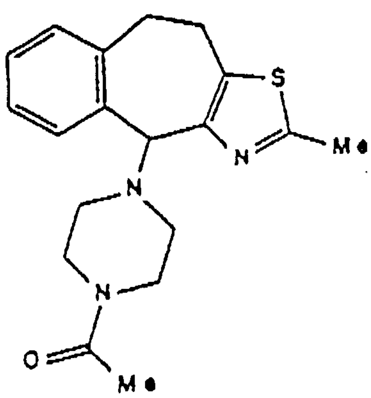

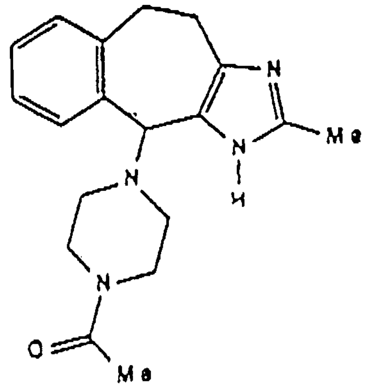

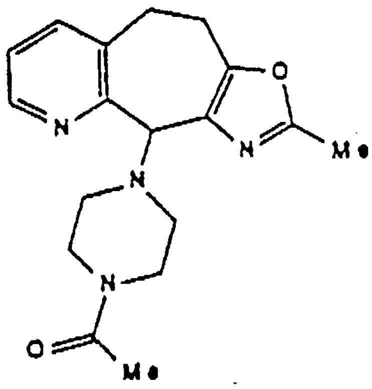

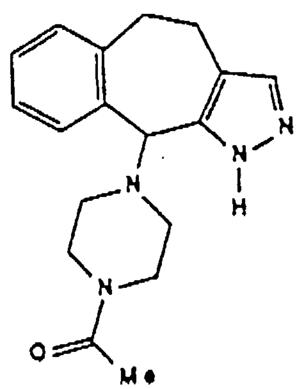

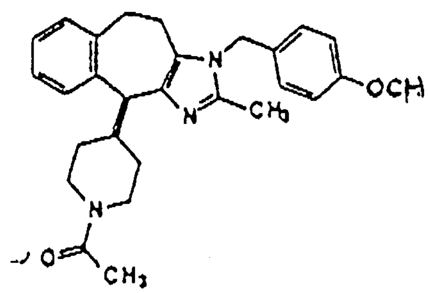

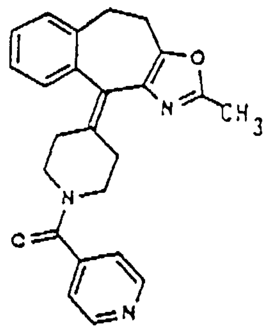

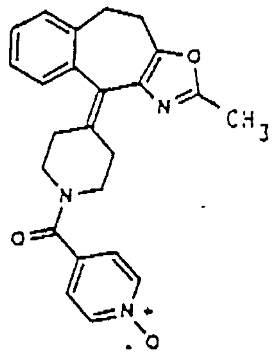

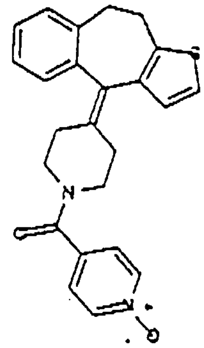

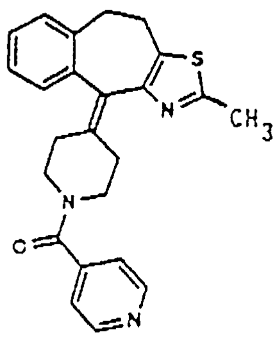

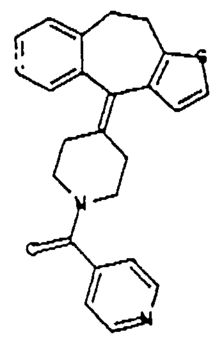

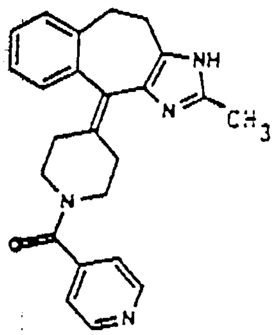

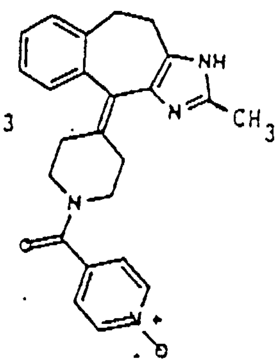

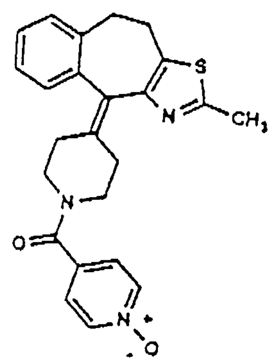

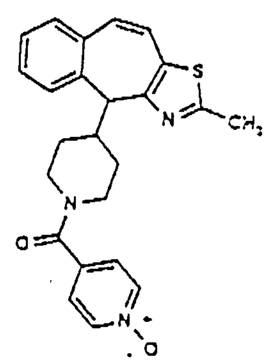

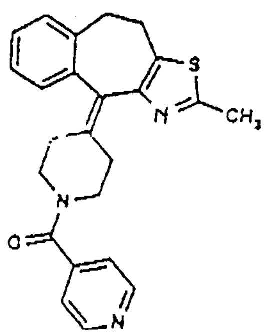

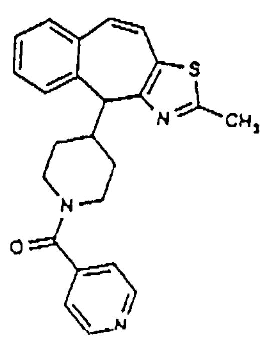

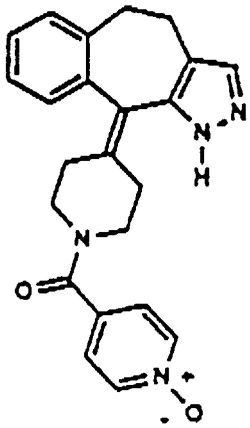 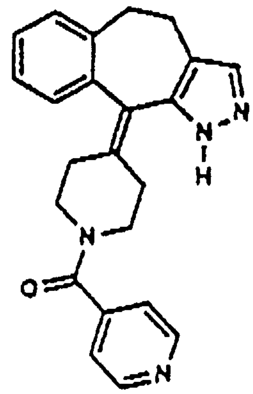

6. Composition pharmaceutique comprenant un composé de la formule 1.0 de la revendication 1 en association avec un support pharmaceutiquement acceptable.

7. Méthode de préparation d'une composition pharmaceutique consistant à mélanger une composition de la formule 1.0 de la revendication 1 avec un support pharmaceutiquement acceptable.

8. Utilisation d'un composé de formule 1.0 de la revendication 1 et ses sels pharmaceutiquement acceptables pour la préparation d'un médicament pour le traitement des troubles de PAF.

9. Méthode de production d'un composé de formule 1.0 de la revendication 1 comprenant :

a) quand X représente

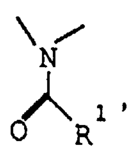

la réaction d'un composé de formule 9.1 avec un composé 8.11 en présence d'une base

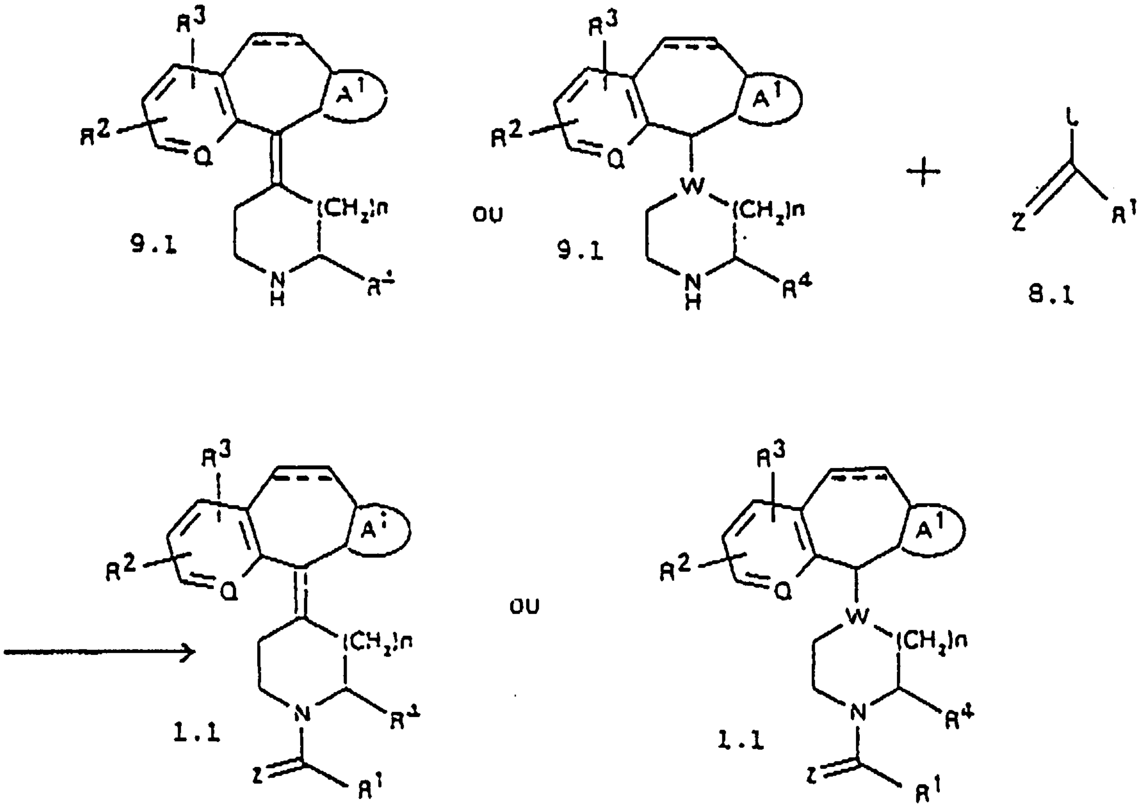

9.1 ou 9.1 + 8.1

1.1 ou 1.1

b) quand X représente

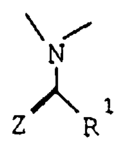

où Z est =O ou =S, la réaction d'un composé de formule 11.1 avec un composé de formule 8.1

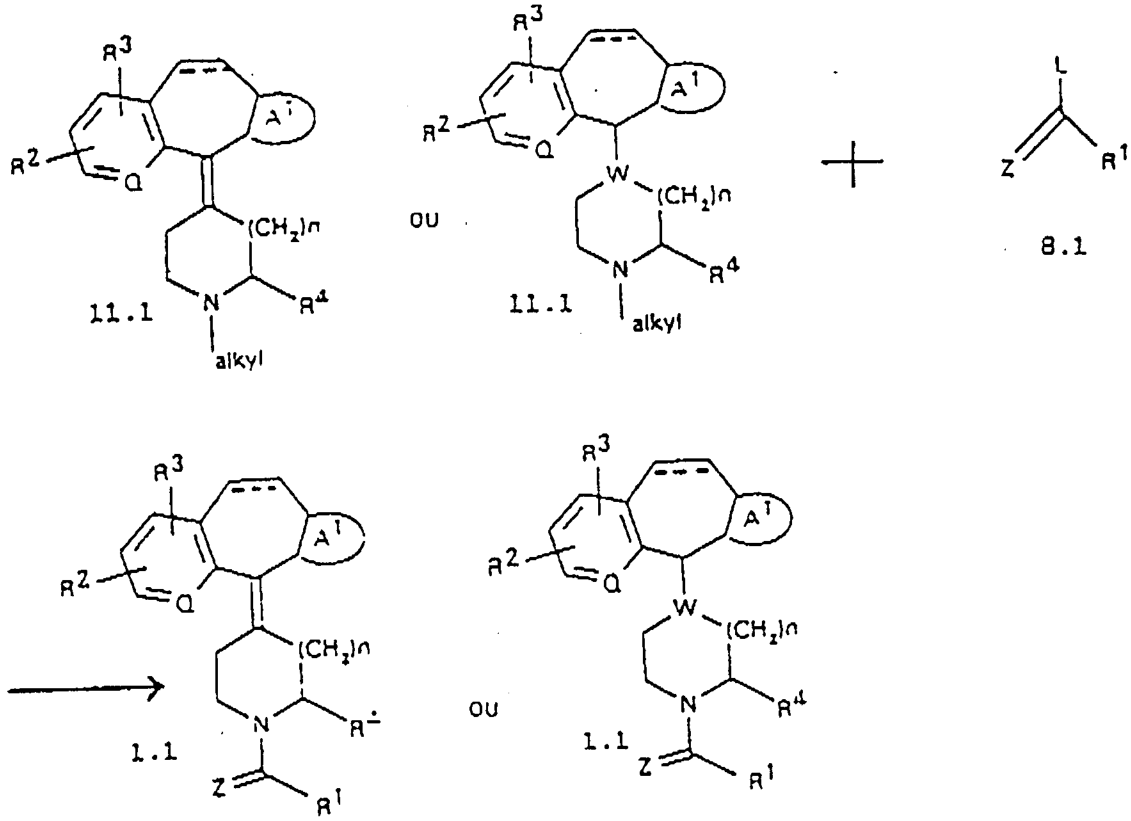

11.1 ou 11.1 8.1

1.1 ou 1.1

c) quand X représente

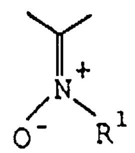
ou
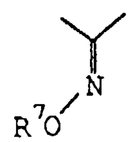
,

la réaction d'un composé de formule 13 en présence d'une hydroxyalmine ou d'une hydroxylamine O-substituée ou d'une hydroxylamine N-substituée;

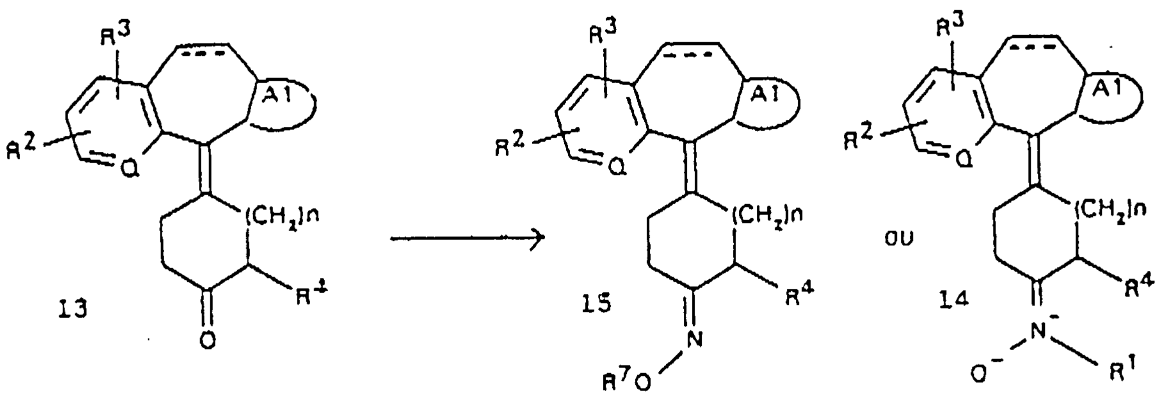

13 15 ou 14

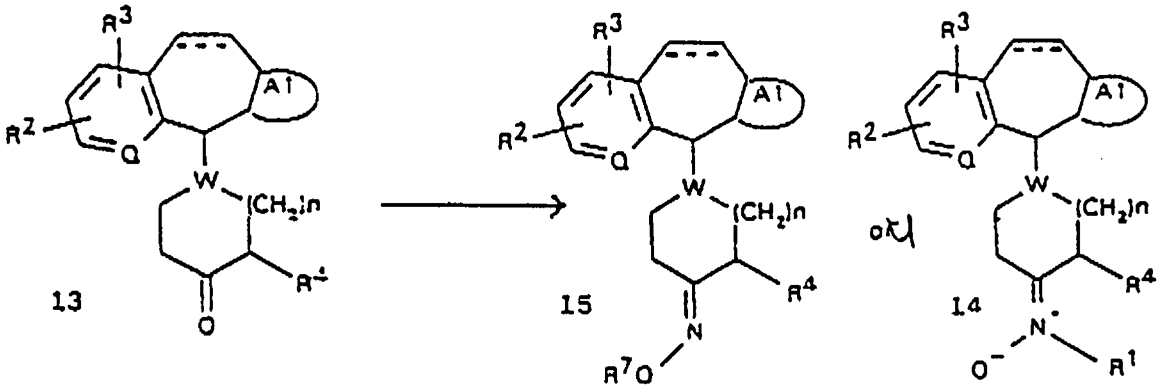

13 15 ou 14

d) la réaction d'un composé de formule 9.2 avec un acide

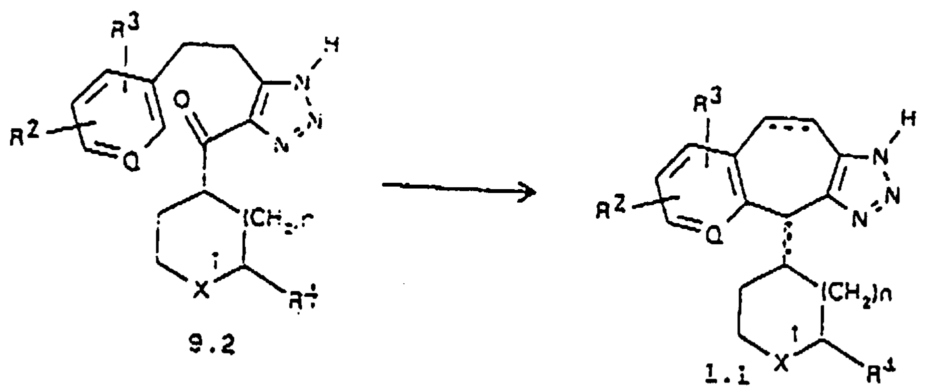

9.2 1.1

e) la réaction d'un composé de formule 200 avec un alcali ou hydroxyde d'un métal alcalin

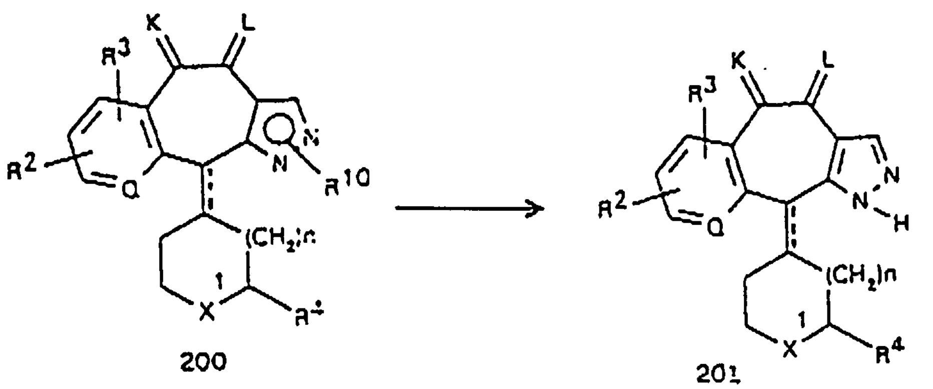

200 201

f) la réaction d'un composé de formule 17 avec un acide approprié

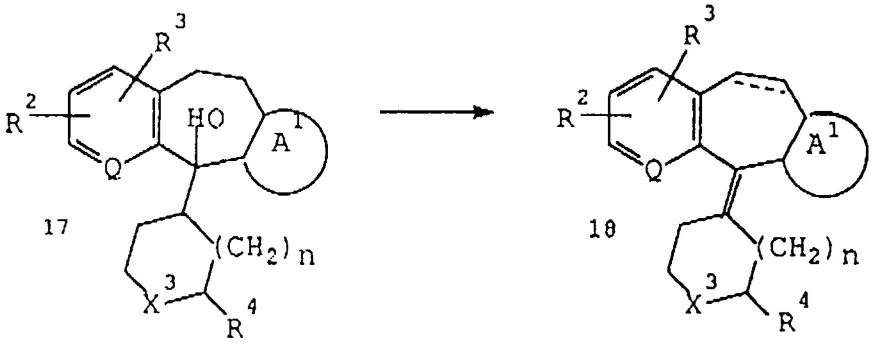

17 18

où $X^3$ représente

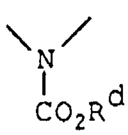

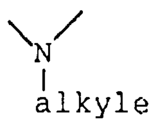

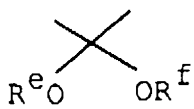

ou

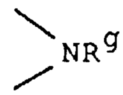

$R^d$ représente un groupe alkyle, alkényle, aryle ou aralkyle facultativement substitué;

$R^e$ et $R^f$ représentent chacun indépendamment alkyle inférieur ou ensemble représentent une chaîne alcanediyle de 2 ou 3 groupes méthylène;

$R^g$ est H ou $C(Z)R^1$;

g) la réaction d'un composé de formule 19 avec un composé de formule 20 où L est un groupe partant et $X^3$ représente $NR^g$ ou

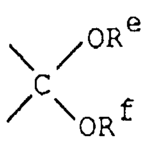

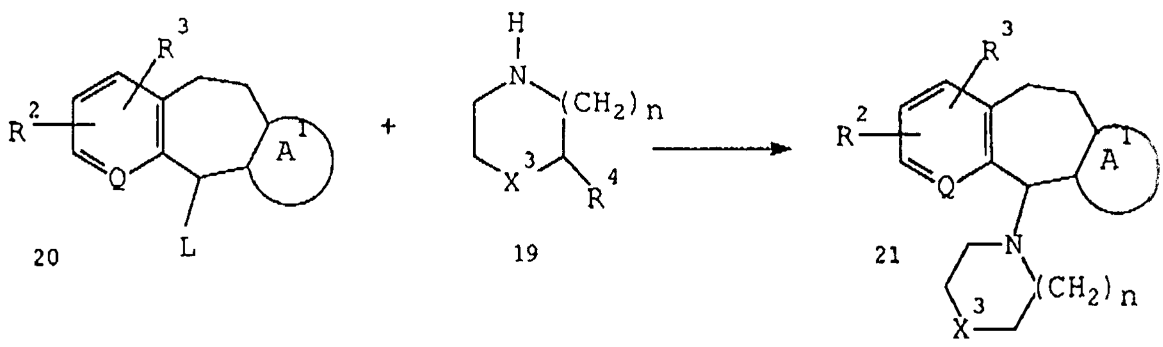

20 19 21

h) la réaction d'un composé de formule 4.0 avec un composé de formule 19

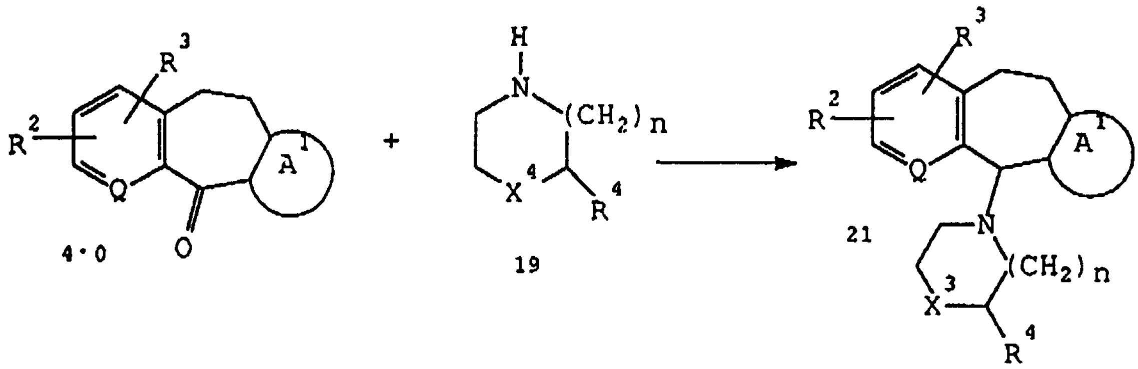

4·0 19 21

i) la déshydratation d'un composé de formule 26 pour produire un composé 27

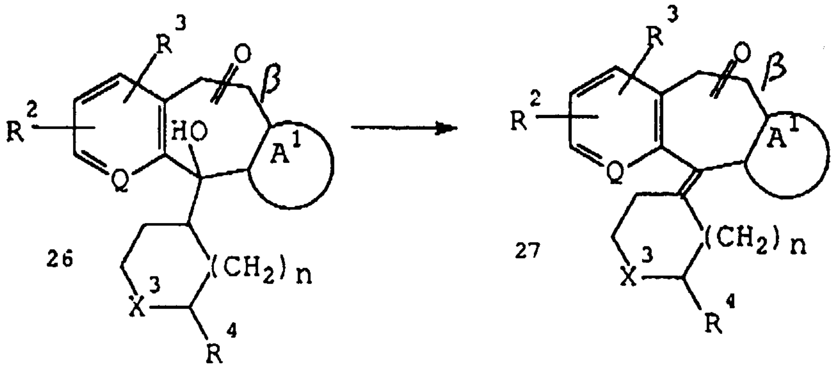

26 27

j) l'hydrolyse d'un composé 30 avec un acide aqueux fort pour produire le composé 31

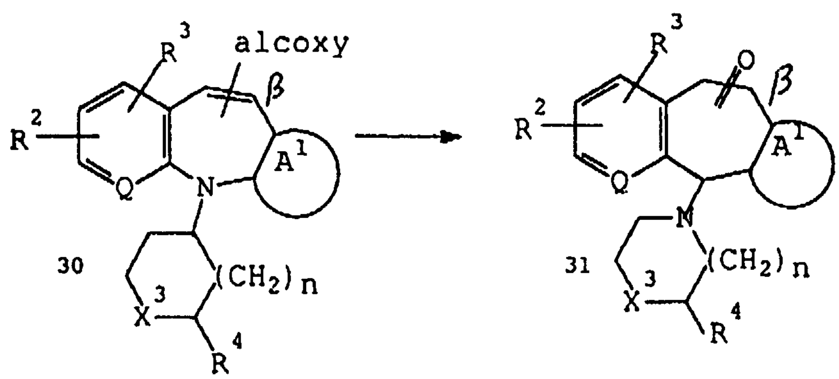


30 31

k) la réaction d'un composé de formule 5.1 avec un composé de la formule $R^{11}CO_2H$, et $(R^{11}CO)_2O$ ou $(R^{11}CO_2)_2O$ seul en présence de HCl

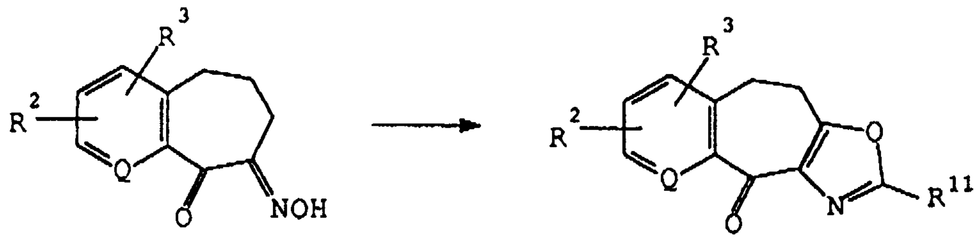

5.1 4.1

l) la réaction d'un composé de formule 5.2 avec un réactif acide et où Ar représente un groupe aryle approprié

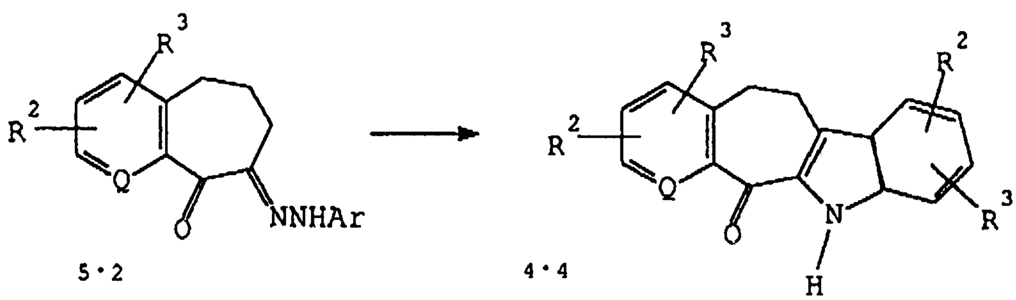

5.2 4.4

m) la réaction d'un composé de formule 5.3 avec une hydrazine réactive $R^{10}NHNH_2$ et un catalyseur acide

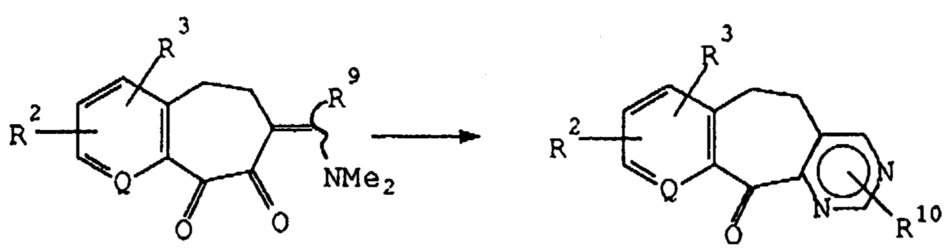

5.3 4.5

n) la réaction d'un composé de formule 5.4 avec un acide ou péracide, pour produire un composé de formule 4.6

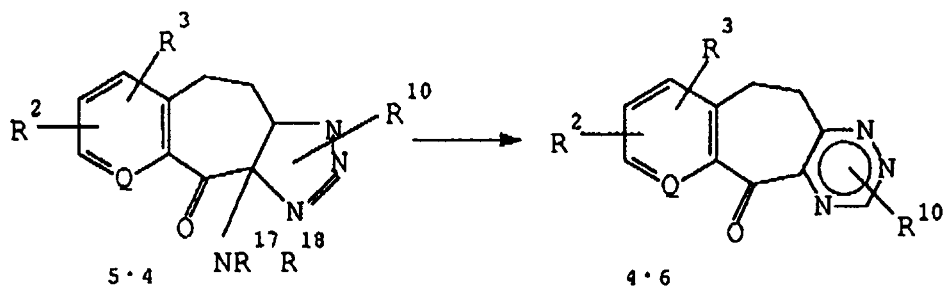

5.4 4.6

o) la réaction d'un composé de formule 5.5 avec un dithionite d'un métal alcalin approprié dans un solvant chaud

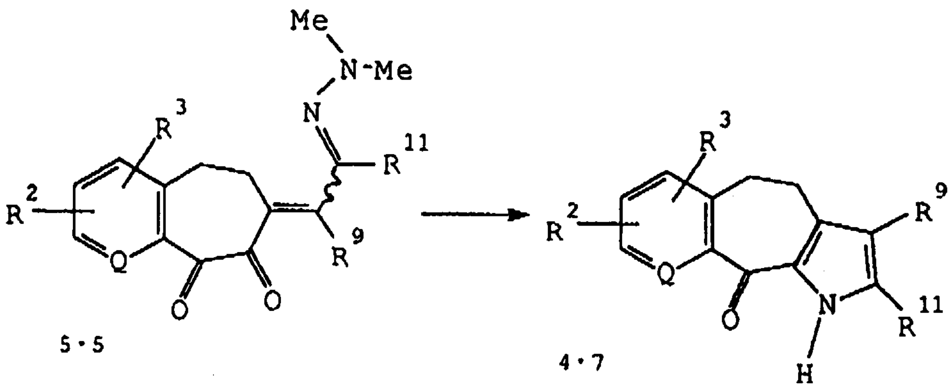

5·5

4·7

p) la réaction d'un composé de formule 5.3 avec un chlorhydrate d'hydroxylamine

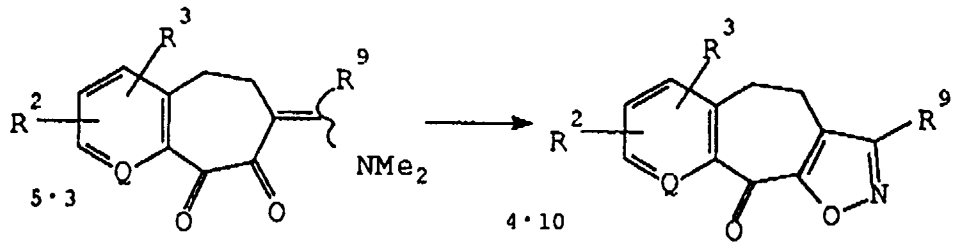

5·3

4·10

q) la réaction d'un composé de formule 5.3 avec le composé de formule $H_2N-C(=NH)R^y$ où $R^y$ représente l'un parmi H, alkyle inférieur, OH, O-(alkyle inférieur), SH, S-(alkyle inférieur), $NH_2$, NH-(alkyle inférieur), N-(alkyle inférieur)$_2$, et NH(C=O)-(alkyle inférieur)

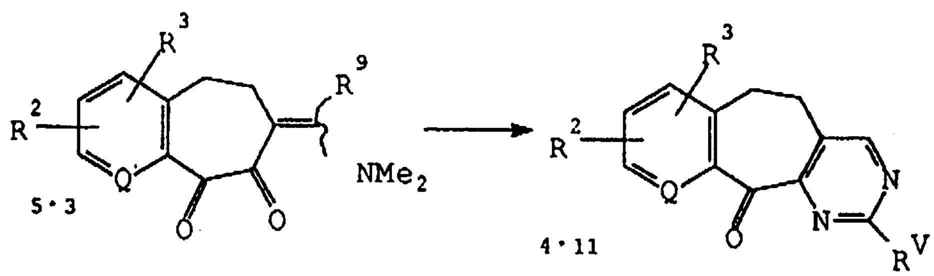

5·3

4·11

r) la réaction d'un composé de formule 5.3 avec un composé de formule $H_2NC(=O)CH_2E$, dans laquelle E est pris parmi CHO, C(=O)-(alkyle inférieur), $CO_2$-(alkyle inférieur), $CONH_2$, $NO_2$, CN, $SO_2CH_3$, et $CF_3$ :

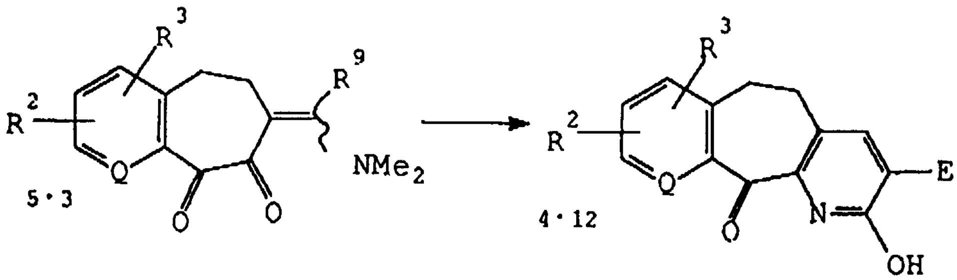

5 • 3

4 • 12

s) la réaction d'un composé de formule 6.0 avec du formamide, de l'acide P-toluènesulfonique, et du N,N', N''-méthylidynetrisformamide (HC(NHCHO)$_3$)

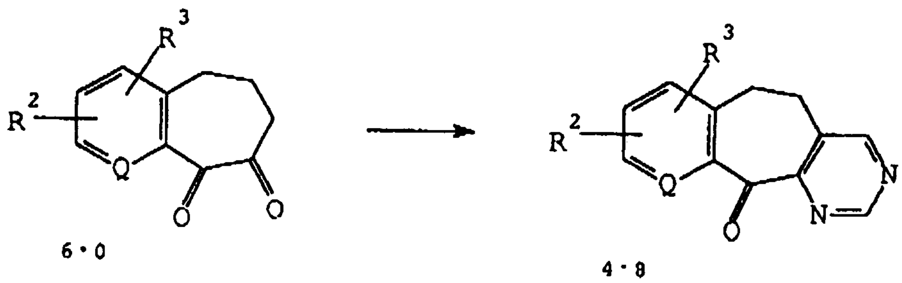

6 • 0

4 • 8

t) la réaction d'un composé de 5.6 avec de l'hydrazine avec ensuite une oxydation

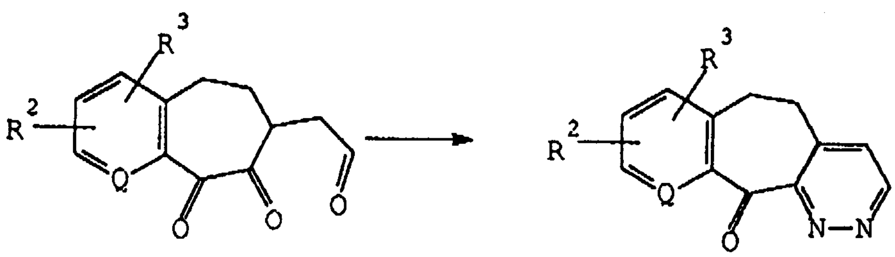

5 • 6

4 • 9

**Revendications pour les Etats contractants suivants : ES, GR**

**1.** Méthode de fabrication d'un composé de formule 1.0

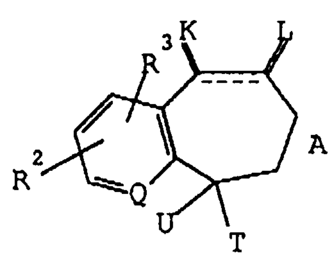

ou son sel ou solvate pharmaceutiquement acceptables, où :
U et T représentent ensemble =O, ou U représente H et T représente

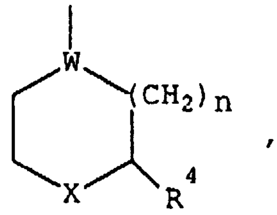

ou
U et T représentent ensemble

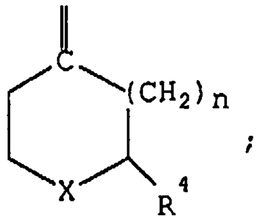

Q est CH, N ou N→O;
$R^2$ et $R^3$ peuvent être identiques ou différents et chacun représente indépendamment H, alkyle $C_1$-$C_8$, $CF_3$, $NO_2$, halo, $OR^5$, $NR^5R^6$, ou S(O)m-alkyle $C_1$-$C_8$, dans lesquels :
$R^5$ et $R^6$ peuvent être identique ou différent et chacun est indépendamment sélectionné parmi H, alkyle $C_1$-$C_8$, ou bien acyle $C_1$-$C_8$, aroyle $C_7$-$C_{16}$; et m est 0, 1 ou 2,
$R^4$ représente H, alkyle $C_1$-$C_8$ ou aryle $C_6$-$C_{15}$
K représente représente-H, -H ou -H, alkyle $C_1$-$C_8$ ou alkyle $C_1$-$C_8$, alkyle $C_1$-$C_8$, ou -H, -OH ou =O;
L représente -H, -H ou -H, alkyle $C_1$-$C_8$ ou alkyle $C_1$-$C_8$, alkyle $C_1$-$C_8$ ou -H, -OH ou =O, à condition que quand L ou K est -H, -OH ou =O, alors l'autre de K ou L soit, respectivement, -H, -H ou -H, alkyle $C_1$-$C_8$ ou alkyle $C_1$-$C_8$, alkyle C1-$C_8$;
le cycle

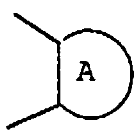

représente

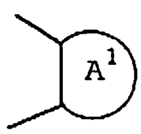

quand U représente
H et T représentent

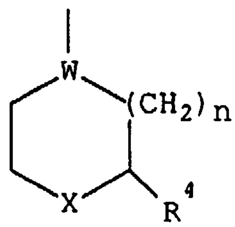

ou bien quand T et U représentent ensemble

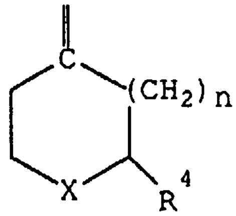

;

et le cycle

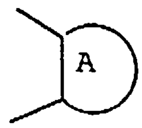

représente

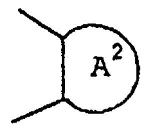

quand T et U représentent ensemble =O;
le cycle

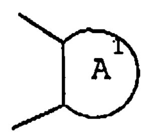

représente un cycle aromatique hétérocyclique fusionné à 5 membres ayant au moins un hétéroatome sélectionné parmi O, S ou N dans le cycle, ledit cycle étant facultativement substitué par 1 à 3 groupes R sélectionnés parmi alkyle $C_1$-$C_8$, aryle $C_6$-$C_{15}$ et arylméthyle $C_7$-$C_{16}$ ou par deux groupes R sur des atomes adjacents du cycle, lesquels groupes R avec ces atomes adjacents du cycle représentent un cycle de benzène fusionné ou de pyridine fusionnée, ou bien le cycle

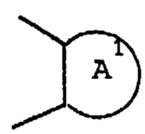

représente un cycle aromatique hétérocyclique fusionné à 6 membres ayant 1, 2 ou 3 atomes d'azote dans le cycle comme

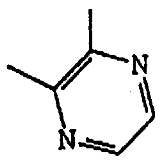 , 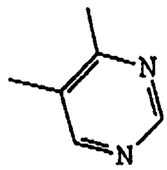 , 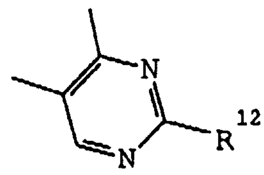 ,

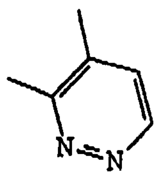 , 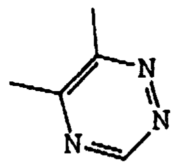 ou 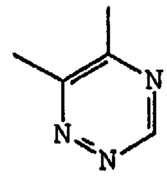 ;

ou bien le cycle

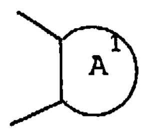

représente un cycle aromatique hétérocyclique fusionné à 6 membres ayant 1 atome d'azote dans le cycle et où Q est CH et X représente

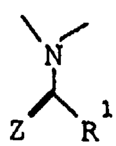

et $R^1$ représente un hétéroaryle $C_{2-14}$ où l'hétéro atome est N→O;

le cycle

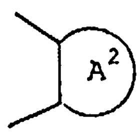

représente

(i) quand Q est N ou N→O :

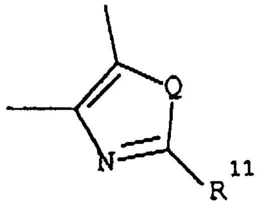 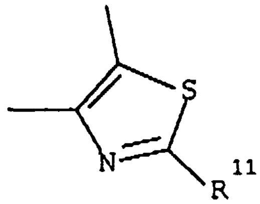 ou 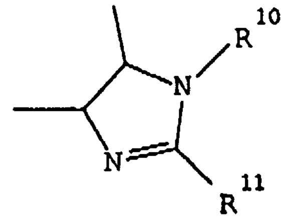 ,

ou bien (ii) quand Q est CH, N ou N→O :

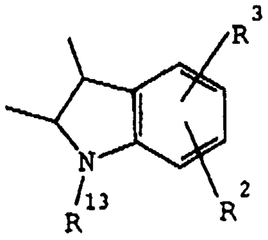 , 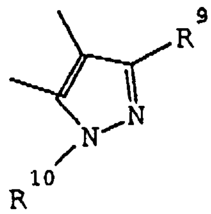 , 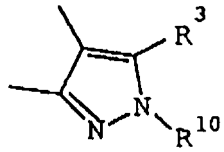 , 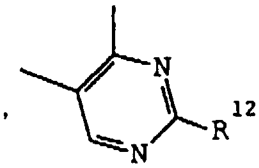 ,

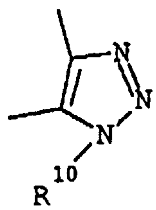 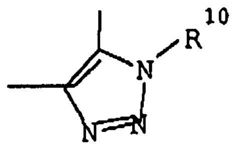 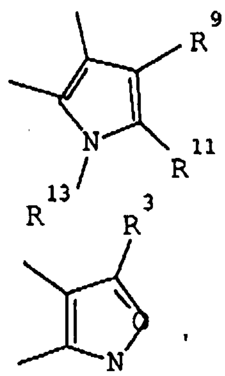 , 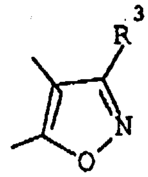 ,

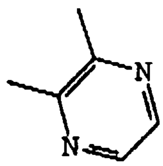 , 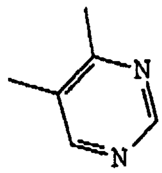 , , 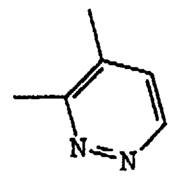 ,

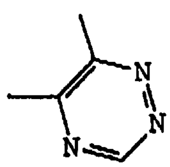 ou 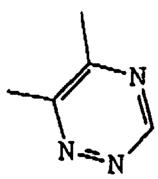 ;

dans lesquels :

$R^9$ est H, alkyle $C_1$-$C_8$ ou aryle $C_6$-$C_{15}$;

$R^{10}$ est H, alkyle $C_1$-$C_8$, aryle $C_6$-$C_{15}$, arylalkyle $C_7$-$C_{23}$, acyle $C_1$-$C_8$, aroyle $C_7$-$C_{16}$ et hétéroaroyle $C_2$-$C_{14}$ où l'entité aryle est facultativement substituée par un ou plusieurs substituants sélectionnés parmi H, halogène, $NO_2$, $CF_3$, -SH, S-alkyle $C_1$-$C_8$, $S(O)_m$-alkyle $C_1$-$C_8$, alkyle $C_1$-$C_8$, $CO_2H$, $CH_2OH$, C(OH)-(alkyle inférieur $C_1$-$C_8$), $NH_2$, NH-(alkyle inférieur $C_1$-$C_8$), N-(alkyle inférieur $C_1$-$C_8)_2$, OH, O-(alkyle inférieur $C_1$-$C_8$), O-(aryle $C_6$-$C_{15}$), O-(aroyle $C_7$-$C_{16}$), O-(hétéroaroyle $C_2$-$C_{14}$), NH(acyle $C_1$-$C_8$), N(acyle $C_1$-$C_8)_2$, NH(aroyle $C_7$-$C_{16}$), N(aroyle $C_7$-$C_{16})_2$, NH(hétéroaroyle $C_2$-$C_{14}$), N(hétéroaryle $C_2$-$C_{14})_2$,

$$-\underset{\underset{O}{\|}}{C}-NH_2,$$

-C-O(alkyle $C_{1-8}$),

$$-CHO, -\underset{\underset{O}{\|}}{C}-(\text{alkyle } C_{1-8}), -\underset{\underset{O}{\|}}{C}-(\text{aryle } C_{7-16})$$

et-C≡N;

$R^{11}$ représente H, alkyle $C_1$-$C_8$, aryle $C_6$-$C_{15}$ ou aralkyle $C_7$-$C_{23}$;

$R^{12}$ représente H, alkyle $C_1$-$C_8$, OH, O-(alkyle $C_1$-$C_8$), SH, S(alkyle $C_1$-$C_8$), $NH_2$, NH-(alkyle $C_1$-$C_8$), N-(alkyle $C_1$-$C_8)_2$, et NH(C=O)-(alkyle $C_1$-$C_8$);

$R^{13}$ est H, alkyle $C_1$-$C_8$, aryle $C_6$-$C_{15}$ ou arylméthyle $C_7$-$C_{16}$; et

$R^{14}$ représente H, $CO_2H$, $CH_2OH$, C(OH)-(alkyle $C_1$-$C_8$)$NH_2$, NH-(alkyle $C_1$-$C_8$), N-(alkyle $C_1$-$C_8)_2$, NH(C=O)-(alk-

yle $C_1$-$C_8$), $CH_2NH_2$, $CH_2NH$-(alkyle $C_1$-$C_8$), $CH_2N$-(alkyle $C_1$-$C_8$)$_2$, $CH_2NH(C{=}O)$-(alkyle $C_1$-$C_8$), CHO, C(=O)-alkyle $C_1$-$C_8$, $CO_2$-alkyle $C_1$-$C_8$), $CONH_2$, $NO_2$, CN, $CF_3$ et $-S(O)_mR^{19}$ où $R^{19}$ représente alkyle $C_1$-$C_8$, aryle $C_6$-$C_{15}$ ou aralkyle $C_7$-$C_{23}$;
W est N ou N→O;
la ligne en pointillé dans le cycle à sept membres représente une double liaison facultative;
n est 0, 1, 2 ou 3;
X représente

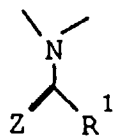 , 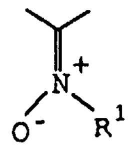 ou 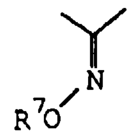

dans lesquels :
$R^7$ représente H, alkyle $C_1$-$C_8$, acyle $C_1$-$C_8$ ou aroyle $C_7$-$C_{16}$, Z est O ou S, et $R^1$ est H, alkyle $C_1$-$C_8$, cycloalkyle $C_5$-$C_8$, $CF_3$, aryle $C_6$-$C_{15}$, hétéroaryle $C_2$-$C_{14}$, $NR^5R^6$ où $R^5$ et $R^6$ sont tels que définis ci-dessus, alkylthio $C_1$-$C_8$ ou alcoxy $C_1$-$C_8$, ou bien $R^1$ et $R^4$ représentent ensemble - $(CH_2)_k$- où k est 1, 2, ou 3, afin de former un cycle fusionné,
à l'exclusion des composés où Q est CH, le cycle $A^1$ est un cycle hétérocyclique fusionné à 6 membres ayant un azote dans le cycle et X représente

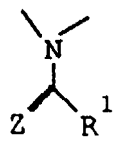

où (a)Z=S ou Z=O et $R^1$ fait partie d'un cycle fusionné ou bien est sélectionné parmi H, alkyle, cycloalkyle, aryle, alkylthio, $-NH_2$, N(alkyle)$_2$, et hétéroaryle où l'hétéroatome est O, S ou N; ou bien

(b)Z = O et $R^1$ est $CF_3$; ou

(c)Z = S et $R^1$ est alcoxy.

la méthode comprenant :

(a) quand X représente

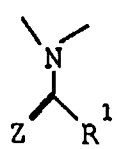 ,

la réaction d'un composé de formule 9.1 avec un composé 8.11 en présence d'une base

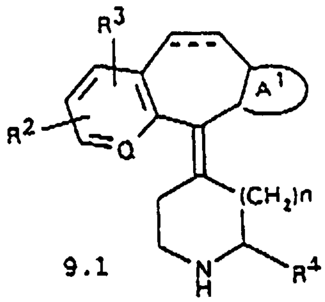

9.1

ou

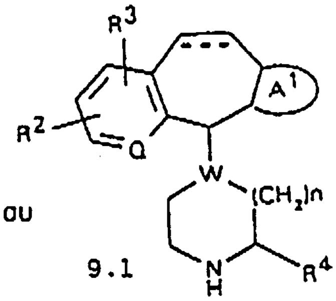

9.1

+

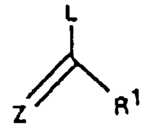

8.1

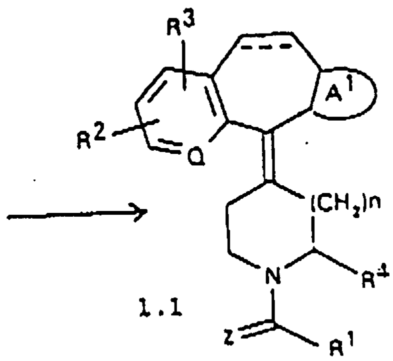

1.1

ou

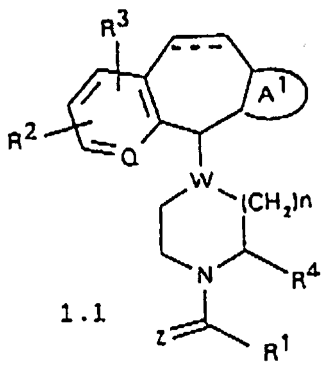

1.1

b) quand X représente

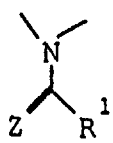

où Z est =O ou =S, la réaction d'un composé de formule 11.1 avec un composé de formule 8.1

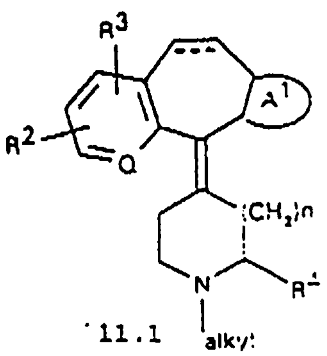

11.1

ou

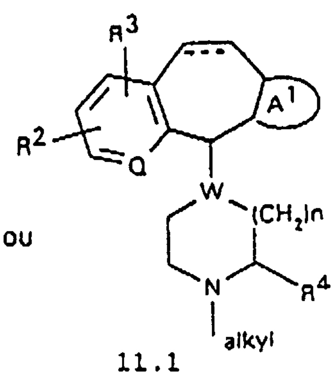

11.1

+

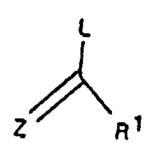

8.1

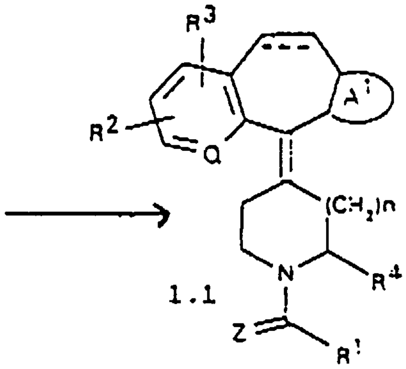

1.1

ou

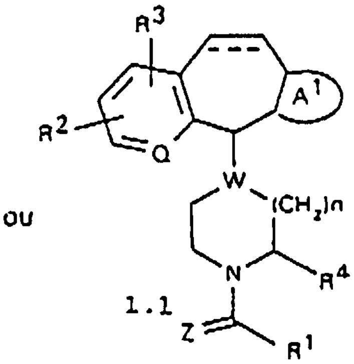

1.1

c) quand X représente

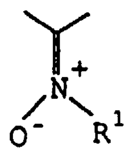

ou

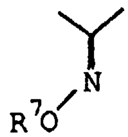

,

la réaction d'un composé de formule 13 en présence d'une hydroxyalmine ou d'une hydroxylamine O-substituée ou d'une hydroxylamine N-substituée;

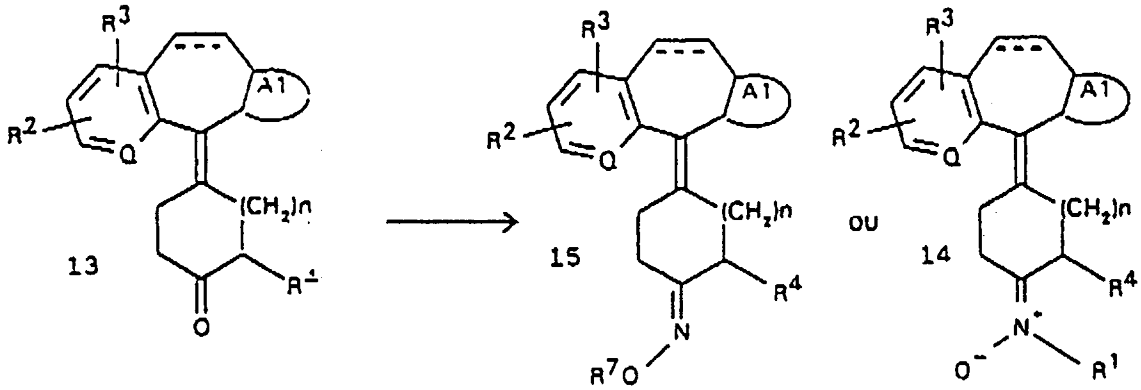

13 15 ou 14

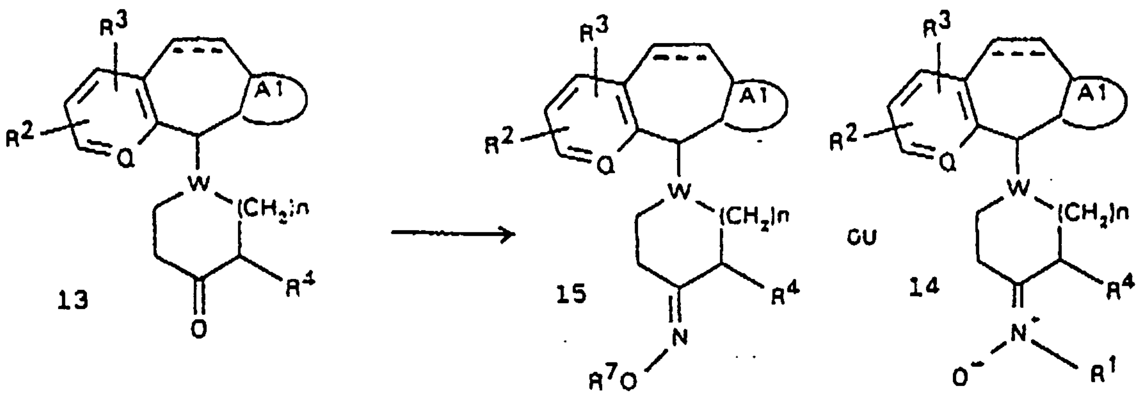

13 15 ou 14

d) la réaction d'un composé de formule 9.2 avec un acide

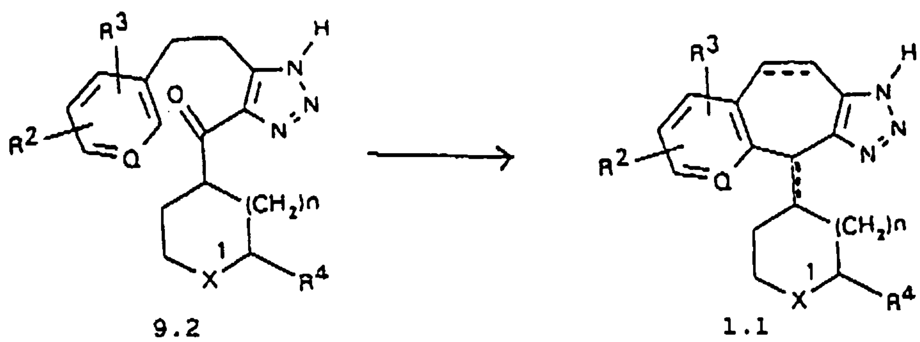

9.2 1.1

e) la réaction d'un composé de formule 200 avec un alcali ou hydroxyde d'un métal alcalin

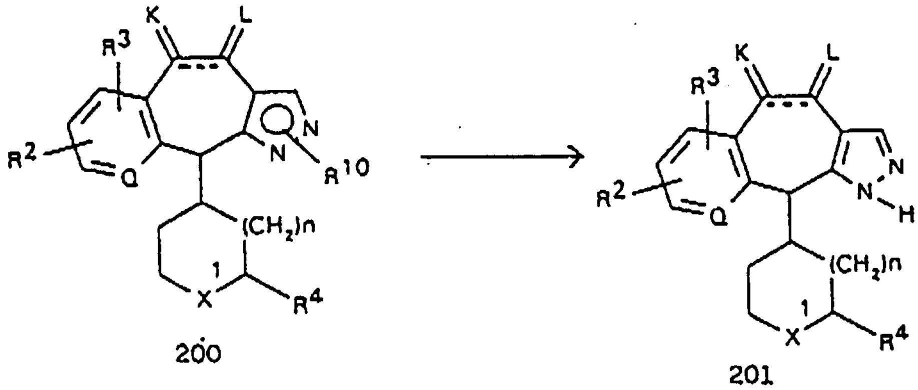

200

201

f) la réaction d'un composé de formule 17 avec un acide approprié

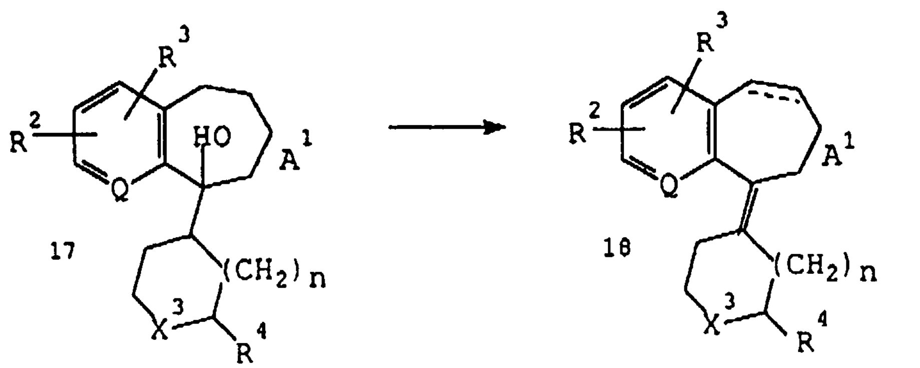

17

18

où $X^3$ représente

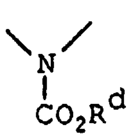

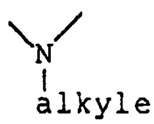

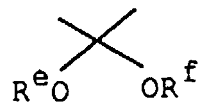

ou

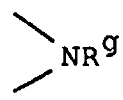

$R^d$ représente un groupe alkyle, alkényle, aryle ou aralkyle facultativement substitué;
$R^e$ et $R^f$ représentent chacun indépendamment alkyle inférieur ou ensemble représentent une chaîne alcanediyle de 2 ou 3 groupes méthylène;
$R^g$ est H ou $C(Z)R^1$;

g) la réaction d'un composé de formule 19 avec un composé de formule 20 où L est un groupe partant et $X^3$ représente $NR^g$ ou

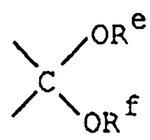

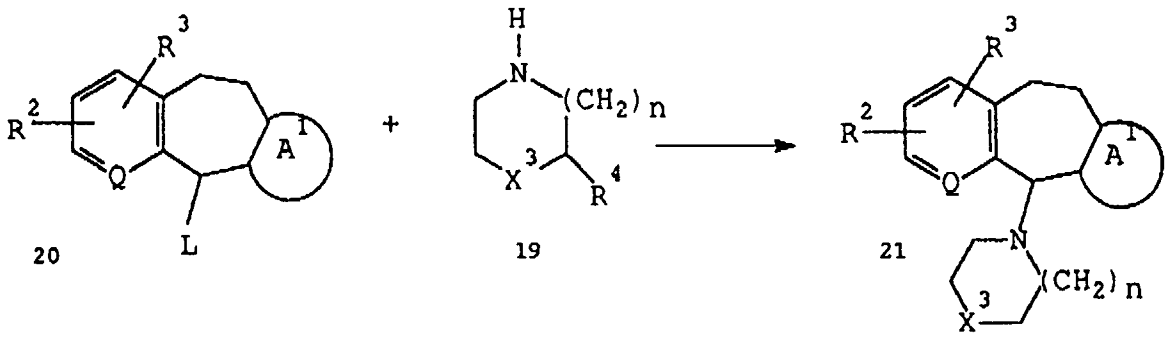

20 19 21

h) la réaction d'un composé de formule 4.0 avec un composé de formule 19

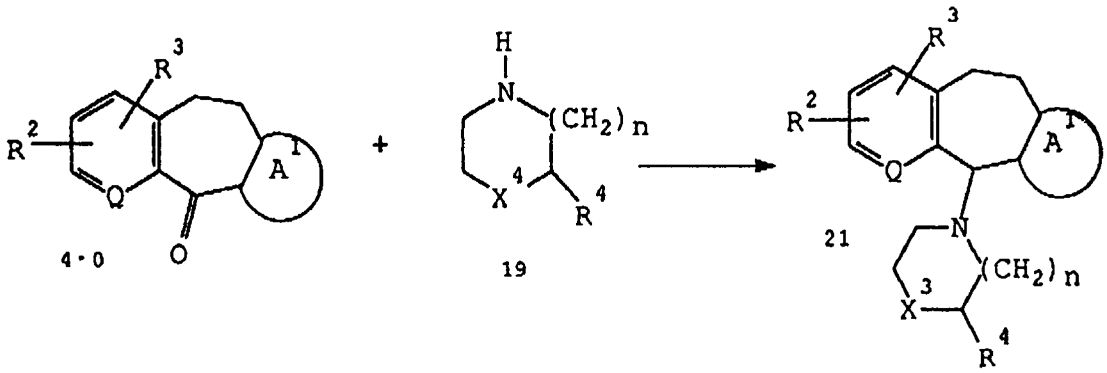

4·0 19 21

i) la déshydratation d'un composé de formule 26 pour produire un composé 27

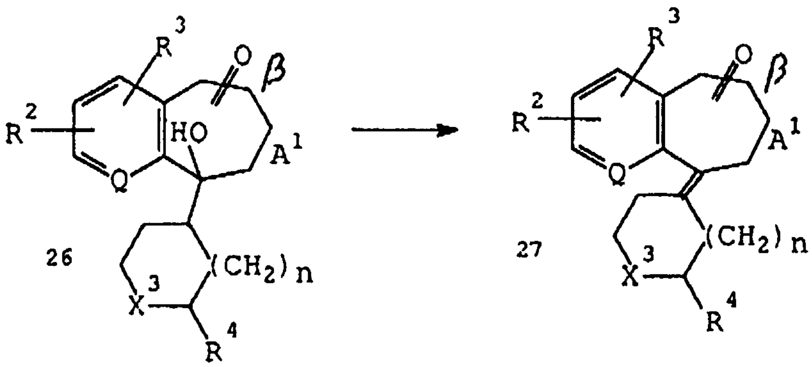

26 27

j) l'hydrolyse d'un composé 30 avec un acide aqueux fort pour produire le composé 31

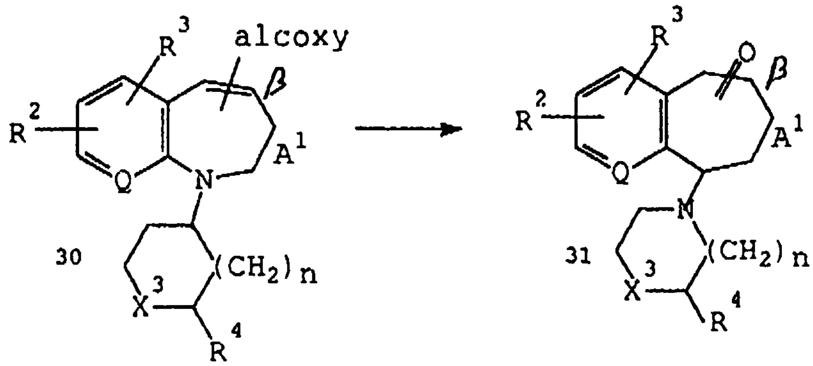

30

31

k) la réaction d'un composé de formule 5.1 avec un composé de la formule $R^{11}CO_2H$, et $(R^{11}CO)_2O$ ou $(R^{11}CO_2)_2O$ seul en présence de HCl

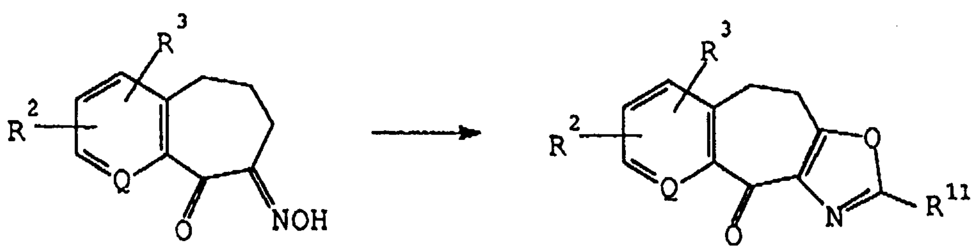

5·1

4·1

l) la réaction d'un composé de formule 5.2 avec un réactif acide et où Ar représente un groupe aryle approprié

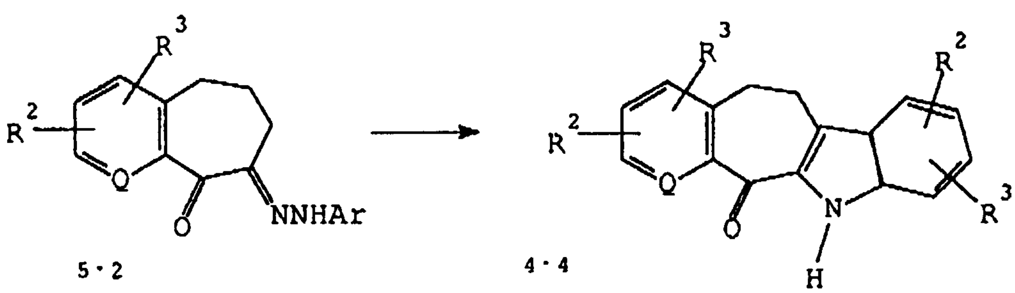

5·2

4·4

m) la réaction d'un composé de formule 5.3 avec une hydrazine réactive $R^{10}NHNH_2$ et un catalyseur acide

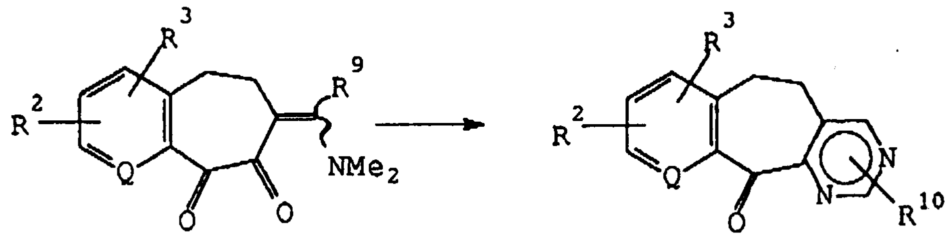

5·3

4·5

n) la réaction d'un composé de formule 5.4 avec un acide ou péracide, pour produire un composé de formule 4.6

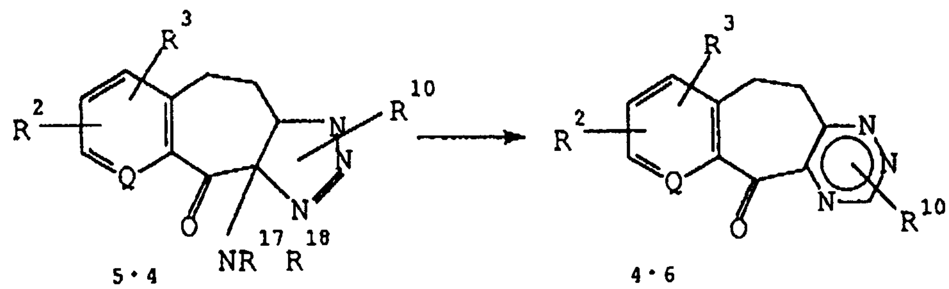

5·4

4·6

o) la réaction d'un composé de formule 5.5 avec un dithionite d'un métal alcalin approprié dans un solvant chaud

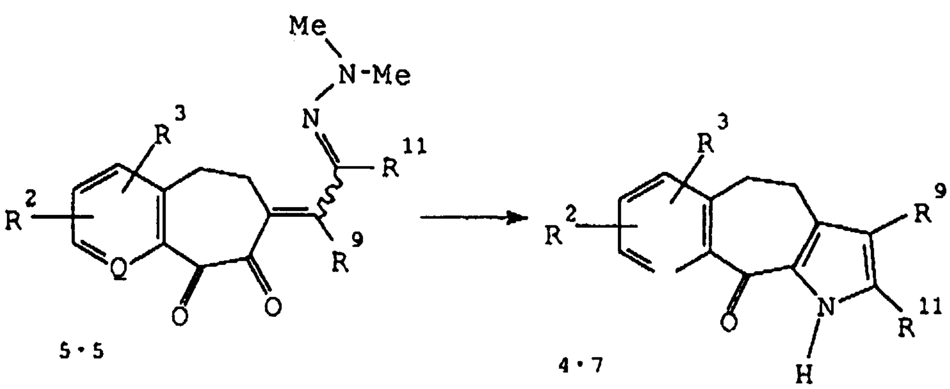

5·5

4·7

p) la réaction d'un composé de formule 5.3 avec un chlorhydrate d'hydroxylamine

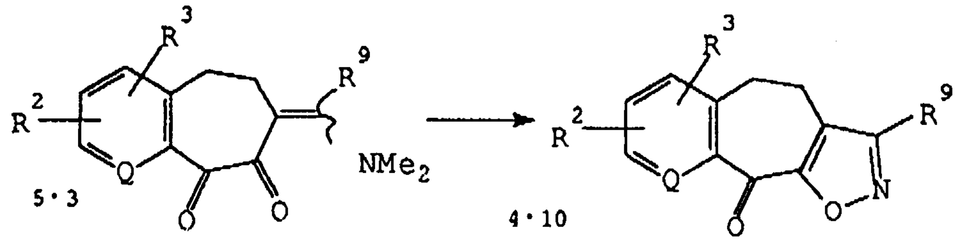

5·3

4·10

q) la réaction d'un composé de formule 5.3 avec le composé de formule $H_2N\text{-}C(=NH)R^y$ où $R^y$ représente l'un parmi H, alkyle inférieur, OH, O-(alkyle inférieur), SH, S-(alkyle inférieur), $NH_2$, NH-(alkyle inférieur), N-(alkyle inférieur)$_2$, et NH(C=O)-(alkyle inférieur)

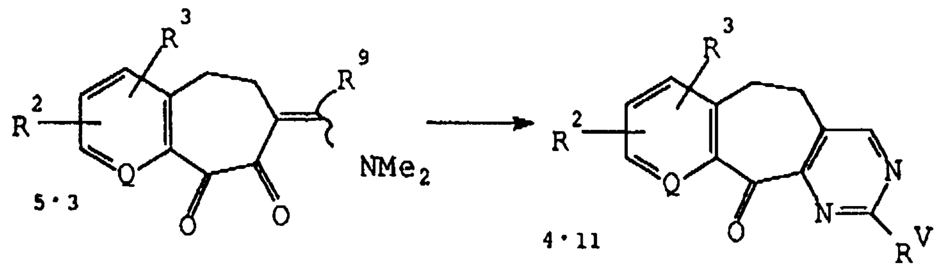

5·3

4·11

r) la réaction d'un composé de formule 5.3 avec un composé de formule $H_2NC(=O)CH_2E$, dans laquelle E est pris parmi CHO, C(=O)-(alkyle inférieur), $CO_2$-(alkyle inférieur), $CONH_2$, $NO_2$, CN, $SO_2CH_3$, et $CF_3$ :

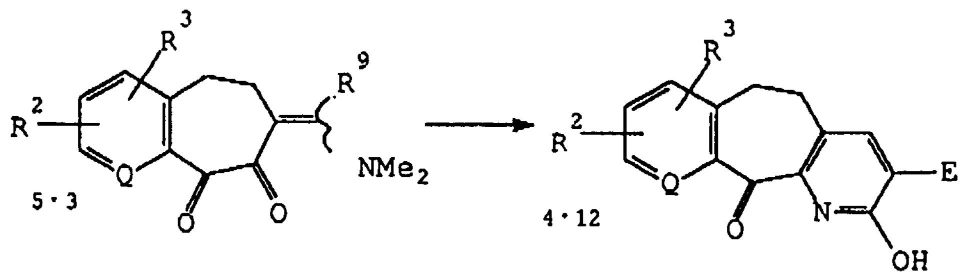

5·3

4·12

s) la réaction d'un composé de formule 6.0 avec du formamide, de l'acide p-toluènesulfonique, et du N,N', N"-méthylidynetrisformamide ($HC(NHCHO)_3$)

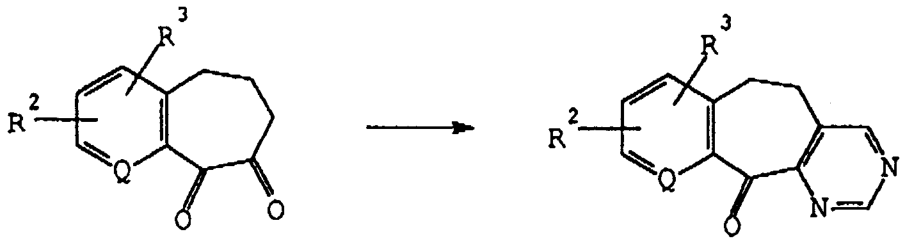

6 • 0

4 • 8

t) la réaction d'un composé de 5.6 avec de l'hydrazine avec ensuite une oxydation

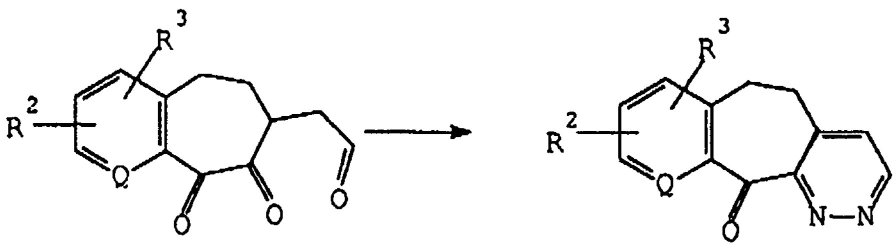

5 • 6

4 • 9

**2.** Utilisation d'un composé de formule 1.0 dans la fabrication d'un médicament pour le traitement de troubles de PAF , d'un allergie et/ou d'une inflammation.